# EUROPEAN PATENT APPLICATION

(11) **EP 3 020 813 A1**
(43) Date of publication of application: **18.05.2016**
(21) Application number: 14193368.9
(22) Date of filing: 16.11.2014
(51) Int. Cl.: C12N 15/113, A61P 25/00, A61P 35/00, A61P 37/02, A61P 43/00, A61K 31/7088

(54) **Antisense-oligonucleotides as inhibitors of TGF-R signaling**

(71) Applicant: Neurovision Pharma GmbH, 82031 Grünwald (DE)
(72) Inventor: The designation of the inventor has not yet been filed
(74) Representative: Arth, Hans-Lothar

(57) **Abstract**

The present invention relates to antisense-oligonucleotides having a length of at least 8 nucleotides, wherein at least two of the nucleotides are LNAs, their use as inhibitors of TGF-R signaling, pharmaceutical compositions containing such antisense-oligonucleotides and the use for prophylaxis and treatment of neurological, neurodegenerative and hyperproliferative diseases.

## Description

The present invention relates to antisense-oligonucleotides, their use as inhibitors of TGF-R signaling, pharmaceutical compositions containing such antisense-oligonucleotides and the use for prophylaxis and treatment of neurological, neurodegenerative and hyperproliferative diseases.

The TGF Receptor II (TGF-R_{II}) was validated as target for the treatment of neurodegenerative diseases such as ALS and hyperproliferative diseases such as cancer.

Many studies tried to increase TGF-beta1 levels for neuroprotective or immunoregulatory purposes. Agonist studies have demonstrated that TGF-beta1 reduces neuronal cell death and infarct size following middle cerebral artery occlusion (MCAO), while conversely, antagonist studies have shown increased neuronal cell death and infarct size after MCAO, suggesting that TGF-beta1 has a neuroprotective role in cerebral ischemia. Recent work with adenoviral-mediated overexpression of TGF-beta1 in vivo in mice has further implicated a neuroprotective role for TGF-beta1 in cerebral ischemia, as evidenced by a reduction in neuronal cell death, infarct size, and neurological outcome. Additionally, numerous in vitro studies have documented the neuroprotective ability of TGF-beta1 in neurons from a variety of species, including rats, mice, chicks, and humans. Of significant interest, TGFbeta1 was shown to be protective against a wide variety of death-inducing agents/insults, including hypoxialischemia, glutamate excitotoxicity, beta-amyloid, oxidative damage, and human immunodeficiency virus. The neuroprotective effect of TGF-beta1 has been related to its ability to maintain the mitochondrial membrane potential, to stabilize Ca²⁺ homeostasis, to increase the expression of the anti-apoptotic proteins Bcl-2 and Bcl-xl, to inhibit caspase-3 activation and to induce plasminogen activator inhibitor-1.

The use of TGF-beta for immunomodulation in humans is severely limited by its toxicity, including excessive stimulation of matrix production, nephrotoxicity and other detrimental effects. TGF-beta has oncogenic potential and has been implicated inglomerulopathies, pulmonary fibrosis, scleroderma and chronic graft versus host disease. In addition, while TGF beta is an extremely potent immunosuppressive cytokine, several lines of evidence indicate that chronic stimulation of TGF-beta expression - both disease-related or in transgenic animal models - can paradoxically lead to or enhance autoimmune inflammation.

Thus objective of the present application is to provide pharmaceutically active compounds able to decrease the activity of the TGF Receptor II (TGF-R_{II}) or inhibit expression of the TGF Receptor II (TGF-R_{II}).

The objective of the present invention is solved by the teaching of the independent claims. Further advantageous features, aspects and details of the invention are evident from the dependent claims, the description, the figures, and the examples of the present application.

Surprisingly under thousands of candidate substances such as protein-nucleotide complexes, RNAi, siRNA, mikro miRNA, ribozymes, aptamers, CpG-oligos, DNA-zymes, riboswitches, lipids, peptides, small molecules, modifyers of rafts or caveoli, modifyers of golgi apparatus, antibodies and their derivatives, especially chimeras, Fab-fragments, Fc-fragments the antisense-oligonucleotides were found the most promising candidates for the uses disclosed herein.

Thus the present invention is directed to antisense-oligonucleotides having a length of at least 8 nucleotides, wherein at least two of the nucleotides are LNAs and the sequence of the antisense-oligonucleotide of the at least 8 nucleotides is selected from the group of sequences of at least 8 nucleotides contained in a sequence selected from the following group:
GAATACTCTTGAATCTTGAATATCTCATGAATGGACCAGTATTCTAGAAACTCACCACTAG
   (Seq. ID No. 4: 373-433 of Seq. ID No. 1),
GCATGCCCTACGGTGCAAGTGGAATTTCTAGGCGCCTCTATGCTACTGCAGCCACACTGTCTT
   (Seq. ID No. 5: 468-530 of Seq. ID No. 1),
AACATAGCTATTCATATTTATATACAGGCATTAATAAAGTGCAAATGTTATTGGCTATTGT
   (Seq. ID No. 6: 2235-2295 of Seq. ID No. 1),
TCTCATTTCCTGAGGAAGTGCTAACACAGCTTATCCTATGACAATGTCAAAGGCATAGAATG
   (Seq. ID No. 7: 2305-2366 of Seq. ID No. 1),
ACATGGCCCAGCCTGCCCCAGAAGAGCTATTTGGTAGTGTTTAGGGAGCCGTCTTCAGGAATCTTCTCC
   (Seq. ID No. 8: 2518-2586 of Seq. ID No. 1), 
AGAAGCTGGGAATTTCTGGTCGCCCTCGATCTCTCAACACGTTGTCCTTCATGCTTTCGACACAGGGGTGCTCCC
GCACCTTGGAACCAAATGGAGGCTCATAATCTT
(Seq. ID No. 9: 2738-2845 of Seq. ID No. 1),
GCCCAGCTTGCGCAGGTCCTCCCAGCTGATGACATGCCGCGTCAGGTACTCCTGTAGGTTGCCCTTGGC
   (Seq. ID No. 10: 3195-3263 of Seq. ID No. 1),
GTTTCCCAGGTTGAACTCAGCTTCTGCTGCCGGTTAACGCGGTAGCAGTAGAAGATGATGATGACAGATA
   (Seq. ID No. 11: 3640-3709 of Seq. ID No. 1), 
AGTGACTATCATGTCGTTATTAACCGACTTCTGAACGTGCGGTGGGATCGTGCTGGCGATACGCGTCCACAGGAC
GATGTGCAGCGGCCACAGGCCCCTGAGCAGCCCCCGACCCATGGCAGACCCCGCTGCTCGTCATAGACCGAGCCC
CCAGCGCAGCGGACGGCGC
(Seq. ID No. 12: 4131-4299 of Seq. ID No. 1), 
TGTCCCGAGCGGGTGCACGCGCGGGGGTGTCGTCGCTCCGTGCGCGCGAGTGACTCACTCAACTTCAACTCAGCG
CTGCGGG
(Seq. ID No. 15: 4480-4561 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Alternatively the present invention is directed to antisense-oligonucleotides having a length of at least 8 nucleotides, wherein at least two of the nucleotides are LNAs and the sequence of the antisense-oligonucleotide of the at least 8 nucleotides is selected from the group of sequences of at least 8 nucleotides contained in a sequence selected from the following group:
GAATACTCTTGAATCTTGAATATCTCATGAATGGACCAGTATTCTAGAAACTCACCACTAG
   (Seq. ID No. 4: 373-433 of Seq. ID No. 1),
GCATGCCCTACGGTGCAAGTGGAATTTCTAGGCGCCTCTATGCTACTGCAGCCACACTGTCTT
   (Seq. ID No. 5: 468-530 of Seq. ID No. 1),
AACATAGCTATTCATATTTATATACAGGCATTAATAAAGTGCAAATGTTATTGGCTATTGT
   (Seq. ID No. 6: 2235-2295 of Seq. ID No. 1),
TCTCATTTCCTGAGGAAGTGCTAACACAGCTTATCCTATGACAATGTCAAAGGCATAGAATG
   (Seq. ID No. 7: 2305-2366 of Seq. ID No. 1),
ACATGGCCCAGCCTGCCCCAGAAGAGCTATTTGGTAGTGTTTAGGGAGCCGTCTTCAGGAATCTTCTCC
   (Seq. ID No. 8: 2518-2586 of Seq. ID No. 1), 
AGAAGCTGGGAATTTCTGGTCGCCCTCGATCTCTCAACACGTTGTCCTTCATGCTTTCGACACAGGGGTGCTCCC
GCACCTTGGAACCAAATGGAGGCTCATAATCTT
(Seq. ID No. 9: 2738-2845 of Seq. ID No. 1),
GCCCAGCTTGCGCAGGTCCTCCCAGCTGATGACATGCCGCGTCAGGTACTCCTGTAGGTTGCCCTTGGC
   (Seq. ID No. 10: 3195-3263 of Seq. ID No. 1),
GTTTCCCAGGTTGAACTCAGCTTCTGCTGCCGGTTAACGCGGTAGCAGTAGAAGATGATGATGACAGATA
   (Seq. ID No. 11: 3640-3709 of Seq. ID No. 1), 
AGTGACTATCATGTCGTTATTAACCGACTTCTGAACGTGCGGTGGGATCGTGCTGGCGATACGCGTCCACAGGAC
GATGTGCAGCGGCCACAGGCCCC
(Seq. ID No. 13: 4131-4228 of Seq. ID No. 1), 
GATGTGCAGCGGCCACAGGCCCCTGAGCAGCCCCCGACCCATGGCAGACCCCGCTGCTCGTCATAGACCGAGCCC
CCAGCGCAGCGGACGGCGC
(Seq. ID No. 14: 4206-4299 of Seq. ID No. 1), 
TGTCCCGAGCGGGTGCACGCGCGGGGGTGTCGTCGCTCCGTGCGCGCGAGTGACTCACTCAACTTCAACTCAGCG
CTGCGGG
(Seq. ID No. 15: 4480-4561 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

More preferably the present invention is directed to antisense-oligonucleotides having a length of at least 10 nucleotides, wherein at least two of the nucleotides are LNAs and the sequence of the antisense-oligonucleotide of the at least 10 nucleotides is selected from the group of sequences of at least 10 nucleotides contained in a sequence selected from the following group:
GAATACTCTTGAATCTTGAATATCTCATGAATGGACCAGTATTCTAGAAACTCACCACTAG
   (Seq. ID No. 4: 373-433 of Seq. ID No. 1),
GCATGCCCTACGGTGCAAGTGGAATTTCTAGGCGCCTCTATGCTACTGCAGCCACACTGTCTT
   (Seq. ID No. 5: 468-530 of Seq. ID No. 1),
AACATAGCTATTCATATTTATATACAGGCATTAATAAAGTGCAAATGTTATTGGCTATTGT
   (Seq. ID No. 6: 2235-2295 of Seq. ID No. 1),
TCTCATTTCCTGAGGAAGTGCTAACACAGCTTATCCTATGACAATGTCAAAGGCATAGAATG
   (Seq. ID No. 7: 2305-2366 of Seq. ID No. 1),
ACATGGCCCAGCCTGCCCCAGAAGAGCTATTTGGTAGTGTTTAGGGAGCCGTCTTCAGGAATCTTCTCC
   (Seq. ID No. 8: 2518-2586 of Seq. ID No. 1), 
AGAAGCTGGGAATTTCTGGTCGCCCTCGATCTCTCAACACGTTGTCCTTCATGCTTTCGACACAGGGGTGCTCCC
GCACCTTGGAACCAAATGGAGGCTCATAATCTT
(Seq. ID No. 9: 2738-2845 of Seq. ID No. 1),
GCCCAGCTTGCGCAGGTCCTCCCAGCTGATGACATGCCGCGTCAGGTACTCCTGTAGGTTGCCCTTGGC
   (Seq. ID No. 10: 3195-3263 of Seq. ID No. 1),
GTTTCCCAGGTTGAACTCAGCTTCTGCTGCCGGTTAACGCGGTAGCAGTAGAAGATGATGATGACAGATA
   (Seq. ID No. 11: 3640-3709 of Seq. ID No. 1), 
AGTGACTATCATGTCGTTATTAACCGACTTCTGAACGTGCGGTGGGATCGTGCTGGCGATACGCGTCCACAGGAC
GATGTGCAGCGGCCACAGGCCCCTGAGCAGCCCCCGACCCATGGCAGACCCCGCTGCTCGTCATAGACCGAGCCC
CCAGCGCAGCGGACGGCGC
(Seq. ID No. 12: 4131-4299 of Seq. ID No. 1), 
TGTCCCGAGCGGGTGCACGCGCGGGGGTGTCGTCGCTCCGTGCGCGCGAGTGACTCACTCAACTTCAACTCAGCG
CTGCGGG
(Seq. ID No. 15: 4480-4561 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Also more preferably the present invention is directed to antisense-oligonucleotides having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of the 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides contained in a sequence selected from the following group:
GAATACTCTTGAATCTTGAATATCTCATGAATGGACCAGTATTCTAGAAACTCACCACTAG
   (Seq. ID No. 4: 373-433 of Seq. ID No. 1),
GCATGCCCTACGGTGCAAGTGGAATTTCTAGGCGCCTCTATGCTACTGCAGCCACACTGTCTT
   (Seq. ID No. 5: 468-530 of Seq. ID No. 1),
AACATAGCTATTCATATTTATATACAGGCATTAATAAAGTGCAAATGTTATTGGCTATTGT
   (Seq. ID No. 6: 2235-2295 of Seq. ID No. 1),
TCTCATTTCCTGAGGAAGTGCTAACACAGCTTATCCTATGACAATGTCAAAGGCATAGAATG
   (Seq. ID No. 7: 2305-2366 of Seq. ID No. 1),
ACATGGCCCAGCCTGCCCCAGAAGAGCTATTTGGTAGTGTTTAGGGAGCCGTCTTCAGGAATCTTCTCC
   (Seq. ID No. 8: 2518-2586 of Seq. ID No. 1), 
AGAAGCTGGGAATTTCTGGTCGCCCTCGATCTCTCAACACGTTGTCCTTCATGCTTTCGACACAGGGGTGCTCCC
GCACCTTGGAACCAAATGGAGGCTCATAATCTT
(Seq. ID No. 9: 2738-2845 of Seq. ID No. 1),
GCCCAGCTTGCGCAGGTCCTCCCAGCTGATGACATGCCGCGTCAGGTACTCCTGTAGGTTGCCCTTGGC
   (Seq. ID No. 10: 3195-3263 of Seq. ID No. 1),
GTTTCCCAGGTTGAACTCAGCTTCTGCTGCCGGTTAACGCGGTAGCAGTAGAAGATGATGATGACAGATA
   (Seq. ID No. 11: 3640-3709 of Seq. ID No. 1), 
AGTGACTATCATGTCGTTATTAACCGACTTCTGAACGTGCGGTGGGATCGTGCTGGCGATACGCGTCCACAGGAC
GATGTGCAGCGGCCACAGGCCCCTGAGCAGCCCCCGACCCATGGCAGACCCCGCTGCTCGTCATAGACCGAGCCC
CCAGCGCAGCGGACGGCGC
(Seq. ID No. 12: 4131-4299 of Seq. ID No. 1), 
TGTCCCGAGCGGGTGCACGCGCGGGGGTGTCGTCGCTCCGTGCGCGCGAGTGACTCACTCAACTTCAACTCAGCG
CTGCGGG
(Seq. ID No. 15: 4480-4561 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

The above disclosure has to be understood in the following way. Disclosed are antisense-oligonucleotides of certain length such as 15 nucleotides which are capable of hybridizing with the open reading frame of the gene encoding TGF-R_{II} or with a region of the mRNA encoding TGF-R_{II} and salts and optical isomers of said antisense-oligonucleotide are also disclosed. The antisense-oligonucleotides of certain length such as 15 nucleotides contain at least two LNA nucleotides, preferably the terminal 3' nucleotide is an LNA nucleotide and also the terminal 5' nucleotide is an LNA nucleotide. The sequence of the antisense-oligonucleotide consisting, for instance, of 15 nucleotides is contained in one of the sequences 4 to 15 (seq. ID No. 4 to Seq. ID No. 15). If, for instance Seq. ID No. 6 which reads as follows
AACATAGCTATTCATATTTATATACAGGCATTAATAAAGTGCAAATGTTATTGGCTATTGT

Is taken, the exemplary antisense-oligonucleotide consisting of 15 nucleotides refers to all deletion sequences of seq. ID No. 6 having 15 nucleotides, which are the following deletion sequences (Seq. ID No. 16 - 62):

AACATAGCTATTCAT, ACATAGCTATTCATA, CATAGCTATTCATAT, ATAGCTATTCATATT,
TAGCTATTCATATTT, AGCTATTCATATTTA, GCTATTCATATTTAT, CTATTCATATTTATA,
TATTCATATTTATAT, ATTCATATTTATATA, TTCATATTTATATAC, TCATATTTATATACA,
CATATTTATATACAG, ATATTTATATACAGG, TATTTATATACAGGC, ATTTATATACAGGCA,
TTTATATACAGGCAT, TTATATACAGGCATT, TATATACAGGCATTA, ATATACAGGCATTAA,
TATACAGGCATTAAT, ATACAGGCATTAATA, TACAGGCATTAATAA, ACAGGCATTAATAAA,
CAGGCATTAATAAAG, AGGCATTAATAAAGT, GGCATTAATAAAGTG, GCATTAATAAAGTGC,
CATTAATAAAGTGCA, ATTAATAAAGTGCAA, TTAATAAAGTGCAAA, TAATAAAGTGCAAAT,
AATAAAGTGCAAATG, ATAAAGTGCAAATGT, TAAAGTGCAAATGTT, AAAGTGCAAATGTTA,
AAGTGCAAATGTTAT, AGTGCAAATGTTATT, GTGCAAATGTTATTG, TGCAAATGTTATTGG,
GCAAATGTTATTGGC, CAAATGTTATTGGCT, AAATGTTATTGGCTA, AATGTTATTGGCTAT,
ATGTTATTGGCTATT, TGTTATTGGCTATTG, and GTTATTGGCTATTGT.

Alternatively the present invention relates to antisense-oligonucleotide(s) having a length of at least 8 nucleotides, wherein at least two of the nucleotides are LNAs and the sequence of the antisense-oligonucleotide of the at least 8 nucleotides is selected from the group of sequences of at least 8 nucleotides which hybridize within the following regions of Seq. ID No. 2 starting with the nucleotide 69 and ending with the nucleotide 150, starting with the nucleotide 331 and ending with the nucleotide 424, starting with the nucleotide 402 and ending with the nucleotide 499, starting with the nucleotide 921 and ending with the nucleotide 990, starting with the nucleotide 1367 and ending with the nucleotide 1435, starting with the nucleotide 1785 and ending with the nucleotide 1892, starting with the nucleotide 2044 and ending with the nucleotide 2112, starting with the nucleotide 2264 and ending with the nucleotide 2325, starting with the nucleotide 2335 and ending with the nucleotide 2395, starting with the nucleotide 4100 and ending with the nucleotide 4162 or starting with the nucleotide 4197 and ending with the nucleotide 4257, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 10 nucleotides, wherein at least two of the nucleotides are LNAs and the sequence of the antisense-oligonucleotide of the at least 10 nucleotides is selected from the group of sequences of at least 10 nucleotides which hybridize within the following regions of Seq. ID No. 2 starting with the nucleotide 69 and ending with the nucleotide 150, starting with the nucleotide 331 and ending with the nucleotide 424, starting with the nucleotide 402 and ending with the nucleotide 499, starting with the nucleotide 921 and ending with the nucleotide 990, starting with the nucleotide 1367 and ending with the nucleotide 1435, starting with the nucleotide 1785 and ending with the nucleotide 1892, starting with the nucleotide 2044 and ending with the nucleotide 2112, starting with the nucleotide 2264 and ending with the nucleotide 2325, starting with the nucleotide 2335 and ending with the nucleotide 2395, starting with the nucleotide 4100 and ending with the nucleotide 4162 or starting with the nucleotide 4197 and ending with the nucleotide 4257, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides which hybridize within the following regions of Seq. ID No. 2 starting with the nucleotide 69 and ending with the nucleotide 150, starting with the nucleotide 331 and ending with the nucleotide 424, starting with the nucleotide 402 and ending with the nucleotide 499, starting with the nucleotide 921 and ending with the nucleotide 990, starting with the nucleotide 1367 and ending with the nucleotide 1435, starting with the nucleotide 1785 and ending with the nucleotide 1892, starting with the nucleotide 2044 and ending with the nucleotide 2112, starting with the nucleotide 2264 and ending with the nucleotide 2325, starting with the nucleotide 2335 and ending with the nucleotide 2395, starting with the nucleotide 4100 and ending with the nucleotide 4162 or starting with the nucleotide 4197 and ending with the nucleotide 4257, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 8 nucleotides, wherein at least two of the nucleotides are LNAs and the sequence of the antisense-oligonucleotide of the at least 8 nucleotides is selected from the group of sequences of at least 8 nucleotides contained in the region of Seq. ID No. 1 starting with the nucleotide 373 and ending with the nucleotide 433, starting with the nucleotide 468 and ending with the nucleotide 530, starting with the nucleotide 2235 and ending with the nucleotide 2295, starting with the nucleotide 2305 and ending with the nucleotide 2366, starting with the nucleotide 2518 and ending with the nucleotide 2586, starting with the nucleotide 2738 and ending with the nucleotide 2845, starting with the nucleotide 3195 and ending with the nucleotide 3263, starting with the nucleotide 3640 and ending with the nucleotide 3709, starting with the nucleotide 4131 and ending with the nucleotide 4228, starting with the nucleotide 4206 and ending with the nucleotide 4299 or starting with the nucleotide 4480 and ending with the nucleotide 4561, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 10 nucleotides, wherein at least two of the nucleotides are LNAs and the sequence of the antisense-oligonucleotide of the at least 10 nucleotides is selected from the group of sequences of at least 10 nucleotides contained in the region of Seq. ID No. 1 starting with the nucleotide 373 and ending with the nucleotide 433, starting with the nucleotide 468 and ending with the nucleotide 530, starting with the nucleotide 2235 and ending with the nucleotide 2295, starting with the nucleotide 2305 and ending with the nucleotide 2366, starting with the nucleotide 2518 and ending with the nucleotide 2586, starting with the nucleotide 2738 and ending with the nucleotide 2845, starting with the nucleotide 3195 and ending with the nucleotide 3263, starting with the nucleotide 3640 and ending with the nucleotide 3709, starting with the nucleotide 4131 and ending with the nucleotide 4228, starting with the nucleotide 4206 and ending with the nucleotide 4299 or starting with the nucleotide 4480 and ending with the nucleotide 4561, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides, wherein at least two of the nucleotides are LNAs and the sequence of the antisense-oligonucleotide of the 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides is selected from the group of sequences of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides contained in the region of Seq. ID No. 1 starting with the nucleotide 373 and ending with the nucleotide 433, starting with the nucleotide 468 and ending with the nucleotide 530, starting with the nucleotide 2235 and ending with the nucleotide 2295, starting with the nucleotide 2305 and ending with the nucleotide 2366, starting with the nucleotide 2518 and ending with the nucleotide 2586, starting with the nucleotide 2738 and ending with the nucleotide 2845, starting with the nucleotide 3195 and ending with the nucleotide 3263, starting with the nucleotide 3640 and ending with the nucleotide 3709, starting with the nucleotide 4131 and ending with the nucleotide 4228, starting with the nucleotide 4206 and ending with the nucleotide 4299 or starting with the nucleotide 4480 and ending with the nucleotide 4561, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

In case the antisense-oligonucleotide has between 10 and 24 nucleotides, the above disclosure reads as follows. Preferred are antisense-oligonucleotide(s) having a length of 10 to 24 nucleotides, wherein at least two of the nucleotides are LNAs and the sequence of the antisense-oligonucleotide of the 10 to 24 nucleotides is selected from the group of sequences of 10 to 24 nucleotides contained in the region of Seq. ID No. 1 starting with the nucleotide 373 and ending with the nucleotide 433, starting with the nucleotide 468 and ending with the nucleotide 530, starting with the nucleotide 2235 and ending with the nucleotide 2295, starting with the nucleotide 2305 and ending with the nucleotide 2366, starting with the nucleotide 2518 and ending with the nucleotide 2586, starting with the nucleotide 2738 and ending with the nucleotide 2845, starting with the nucleotide 3195 and ending with the nucleotide 3263, starting with the nucleotide 3640 and ending with the nucleotide 3709, starting with the nucleotide 4131 and ending with the nucleotide 4228, starting with the nucleotide 4206 and ending with the nucleotide 4299 or starting with the nucleotide 4480 and ending with the nucleotide 4561, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

However, it shall be clarified here that wherever it is stated that an antisense-oligonucleotide hybridizes within certain regions of Seq. ID No. 2 (defined by a starting nucleotide and an ending nucleotide), Seq. ID No. 2 can also be replaced by Seq ID No. 3, the only difference between Seq. ID No. 2 and Seq. ID No. 3 being that Seq. ID No. 2 is written in the DNA code (A, T, C, G as nucleotides) and Seq. ID No. 3 is written in the RNA code (A, U, C, G as nucleotides). Since U can also hybridize with A (as does T) an antisense-oligonucleotide that hybridizies with a certain region of Seq. ID No. 2 can also hybridize with the corresponding region of Seq. ID No. 3 (the nucleotide positions between Seq. ID No. 2 and Seq. ID No. 3 also remain the same).

More preferably the present invention relates to the following embodiments.

The present invention is directed to antisense-oligonucleotides having a length of at least 8 nucleotides and preferably 10 nucleotides, wherein at least two of the at least 8 or 10 nucleotides are LNAs and the sequence of the antisense-oligonucleotide of the at least 8 or 10 nucleotides is selected from the group of sequences of at least 8 or 10 nucleotides contained in a sequence selected from the following group:
GAATACTCTTGAATCTTGAATATCTCATGAATGGACCAGTATTCTAGAAACTCACCACTAG
   (Seq. ID No. 4: 373-433 of Seq. ID No. 1),
AACATAGCTATTCATATTTATATACAGGCATTAATAAAGTGCAAATGTTATTGGCTATTGT
   (Seq. ID No. 6: 2235-2295 of Seq. ID No. 1),
TCTCATTTCCTGAGGAAGTGCTAACACAGCTTATCCTATGACAATGTCAAAGGCATAGAATG
   (Seq. ID No. 7: 2305-2366 of Seq. ID No. 1),
ACATGGCCCAGCCTGCCCCAGAAGAGCTATTTGGTAGTGTTTAGGGAGCCGTCTTCAGGAATCTTCTCC
   (Seq. ID No. 8: 2518-2586 of Seq. ID No. 1),
GCCCAGCTTGCGCAGGTCCTCCCAGCTGATGACATGCCGCGTCAGGTACTCCTGTAGGTTGCCCTTGGC
   (Seq. ID No. 10: 3195-3263 of Seq. ID No. 1), 
AGTGACTATCATGTCGTTATTAACCGACTTCTGAACGTGCGGTGGGATCGTGCTGGCGATACGCGTCCACAGGAC
GATGTGCAGCGGCCACAGGCCCCTGAGCAGCCCCCGACCCATGGCAGACCCCGCTGCTCGTCATAGACCGAGCCC
CCAGCGCAGCGGACGGCGC
(Seq. ID No. 12: 4131-4299 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

The present invention is preferably directed to antisense-oligonucleotides having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of the 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides contained in a sequence selected from the following group:
GAATACTCTTGAATCTTGAATATCTCATGAATGGACCAGTATTCTAGAAACTCACCACTAG
   (Seq. ID No. 4: 373-433 of Seq. ID No. 1),
AACATAGCTATTCATATTTATATACAGGCATTAATAAAGTGCAAATGTTATTGGCTATTGT
   (Seq. ID No. 6: 2235-2295 of Seq. ID No. 1),
TCTCATTTCCTGAGGAAGTGCTAACACAGCTTATCCTATGACAATGTCAAAGGCATAGAATG
   (Seq. ID No. 7: 2305-2366 of Seq. ID No. 1),
ACATGGCCCAGCCTGCCCCAGAAGAGCTATTTGGTAGTGTTTAGGGAGCCGTCTTCAGGAATCTTCTCC
   (Seq. ID No. 8: 2518-2586 of Seq. ID No. 1),
GCCCAGCTTGCGCAGGTCCTCCCAGCTGATGACATGCCGCGTCAGGTACTCCTGTAGGTTGCCCTTGGC
   (Seq. ID No. 10: 3195-3263 of Seq. ID No. 1), 
AGTGACTATCATGTCGTTATTAACCGACTTCTGAACGTGCGGTGGGATCGTGCTGGCGATACGCGTCCACAGGAC
GATGTGCAGCGGCCACAGGCCCCTGAGCAGCCCCCGACCCATGGCAGACCCCGCTGCTCGTCATAGACCGAGCCC
CCAGCGCAGCGGACGGCGC
(Seq. ID No. 12: 4131-4299 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Alternatively the present invention relates to antisense-oligonucleotide(s) having a length of at least 8 or 10 nucleotides, wherein at least two of the nucleotides are LNAs and the sequence of the antisense-oligonucleotide of the at least 8 or 10 nucleotides is selected from the group of sequences of at least 8 or 10 nucleotides which hybridize within the following regions of Seq. ID No. 2 starting with the nucleotide 331 and ending with the nucleotide 424, starting with the nucleotide 402 and ending with the nucleotide 499, starting with the nucleotide 1367 and ending with the nucleotide 1435, starting with the nucleotide 2044 and ending with the nucleotide 2112, starting with the nucleotide 2264 and ending with the nucleotide 2325, starting with the nucleotide 2335 and ending with the nucleotide 2395, or starting with the nucleotide 4197 and ending with the nucleotide 4257, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides which hybridize within the following regions of Seq. ID No. 2 starting with the nucleotide 331 and ending with the nucleotide 424, starting with the nucleotide 402 and ending with the nucleotide 499, starting with the nucleotide 1367 and ending with the nucleotide 1435, starting with the nucleotide 2044 and ending with the nucleotide 2112, starting with the nucleotide 2264 and ending with the nucleotide 2325, starting with the nucleotide 2335 and ending with the nucleotide 2395, or starting with the nucleotide 4197 and ending with the nucleotide 4257, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 8 or 10 nucleotides, wherein at least two of the nucleotides are LNAs and the sequence of the antisense-oligonucleotide of the at least 8 or 10 nucleotides is selected from the group of sequences of at least 8 or 10 nucleotides contained in the region of Seq. ID No. 1 starting with the nucleotide 373 and ending with the nucleotide 433, starting with the nucleotide 2235 and ending with the nucleotide 2295, starting with the nucleotide 2305 and ending with the nucleotide 2366, starting with the nucleotide 2518 and ending with the nucleotide 2586, starting with the nucleotide 3195 and ending with the nucleotide 3263, starting with the nucleotide 4131 and ending with the nucleotide 4228, or starting with the nucleotide 4206 and ending with the nucleotide 4299, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides, wherein at least two of the nucleotides are LNAs and the sequence of the antisense-oligonucleotide of the 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides is selected from the group of sequences of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides contained in the region of Seq. ID No. 1 starting with the nucleotide 373 and ending with the nucleotide 433, starting with the nucleotide 2235 and ending with the nucleotide 2295, starting with the nucleotide 2305 and ending with the nucleotide 2366, starting with the nucleotide 2518 and ending with the nucleotide 2586, starting with the nucleotide 3195 and ending with the nucleotide 3263, starting with the nucleotide 4131 and ending with the nucleotide 4228, or starting with the nucleotide 4206 and ending with the nucleotide 4299, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

In case the antisense-oligonucleotide has between 10 and 24 nucleotides, the above disclosure reads as follows. Preferred are antisense-oligonucleotide(s) having a length of 10 to 24 nucleotides, wherein at least two of the nucleotides are LNAs and the sequence of the antisense-oligonucleotide of the 10 to 24 nucleotides is selected from the group of sequences of 10 to 24 nucleotides contained in the region of Seq. ID No. 1 starting with the nucleotide 373 and ending with the nucleotide 433, starting with the nucleotide 2235 and ending with the nucleotide 2295, starting with the nucleotide 2305 and ending with the nucleotide 2366, starting with the nucleotide 2518 and ending with the nucleotide 2586, starting with the nucleotide 3195 and ending with the nucleotide 3263, starting with the nucleotide 4131 and ending with the nucleotide 4228, or starting with the nucleotide 4206 and ending with the nucleotide 4299, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Still more preferably the present invention relates to the following embodiments.

The present invention is preferably directed to antisense-oligonucleotides having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in a sequence selected from the following group:
CTCTTGAATCTTGAATATCTCATGAATGGACCAGTATTCTAGAAACTCACC
   (Seq. ID No. 63: 378-428 of Seq. ID No. 1),
CCCTACGGTGCAAGTGGAATTTCTAGGCGCCTCTATGCTACTGCAGCCACACT
   (Seq. ID No. 64: 473-525 of Seq. ID No. 1),
AGCTATTCATATTTATATACAGGCATTAATAAAGTGCAAATGTTATTGGCT
   (Seq. ID No. 65: 2240-2290 of Seq. ID No. 1),
TTTCCTGAGGAAGTGCTAACACAGCTTATCCTATGACAATGTCAAAGGCATA
   (Seq. ID No. 66: 2310-2361 of Seq. ID No. 1),
GCCCAGCCTGCCCCAGAAGAGCTATTTGGTAGTGTTTAGGGAGCCGTCTTCAGGAATCT
   (Seq. ID No. 67: 2523-2581 of Seq. ID No. 1), 
CTGGGAATTTCTGGTCGCCCTCGATCTCTCAACACGTTGTCCTTCATGCTTTCGACACAGGGGTGCTCCCGCACC
TTGGAACCAAATGGAGGCTCATA
(Seq. ID No. 68: 2743-2840 of Seq. ID No. 1),
GCTTGCGCAGGTCCTCCCAGCTGATGACATGCCGCGTCAGGTACTCCTGTAGGTTGCCC
   (Seq. ID No. 69: 3200-3258 of Seq. ID No. 1),
CCAGGTTGAACTCAGCTTCTGCTGCCGGTTAACGCGGTAGCAGTAGAAGATGATGATGAC
   (Seq. ID No. 70: 3645-3704 of Seq. ID No. 1), 
CTATCATGTCGTTATTAACCGACTTCTGAACGTGCGGTGGGATCGTGCTGGCGATACGCGTCCACAGGACGATGT
GCAGCGGCCACAG
(Seq. ID No. 71: 4136-4223 of Seq. ID No. 1), 
GCAGCGGCCACAGGCCCCTGAGCAGCCCCCGACCCATGGCAGACCCCGCTGCTCGTCATAGACCGAGCCCCCAGC
GCAGCGGAC
(Seq. ID No. 72: 4211-4294 of Seq. ID No. 1),
CGAGCGGGTGCACGCGCGGGGGTGTCGTCGCTCCGTGCGCGCGAGTGACTCACTCAACTTCAACTCAGCGCT
   (Seq. ID No. 73: 4485-4556 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Instead of Seq. ID No. 71 and Seq. ID No. 72 a combined sequence (Seq. ID No. 74) could be used: 
CTATCATGTCGTTATTAACCGACTTCTGAACGTGCGGTGGGATCGTGCTGGCGATACGCGTCCACAGGACGATGT
GCAGCGGCCACAGGCCCCTGAGCAGCCCCCGACCCATGGCAGACCCCGCTGCTCGTCATAGACCGAGCCCCCAGC
GCAGCGGAC
(Seq. ID No. 74: 4136-4294 of Seq. ID No. 1),

Also more preferably the present invention is directed to antisense-oligonucleotides having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of the 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides contained in a sequence selected from the following group:
CTCTTGAATCTTGAATATCTCATGAATGGACCAGTATTCTAGAAACTCACC
   (Seq. ID No. 63: 378-428 of Seq. ID No. 1),
CCCTACGGTGCAAGTGGAATTTCTAGGCGCCTCTATGCTACTGCAGCCACACT
   (Seq. ID No. 64: 473-525 of Seq. ID No. 1),
AGCTATTCATATTTATATACAGGCATTAATAAAGTGCAAATGTTATTGGCT
   (Seq. ID No. 65: 2240-2290 of Seq. ID No. 1),
TTTCCTGAGGAAGTGCTAACACAGCTTATCCTATGACAATGTCAAAGGCATA
   (Seq. ID No. 66: 2310-2361 of Seq. ID No. 1),
GCCCAGCCTGCCCCAGAAGAGCTATTTGGTAGTGTTTAGGGAGCCGTCTTCAGGAATCT
   (Seq. ID No. 67: 2523-2581 of Seq. ID No. 1), 
CTGGGAATTTCTGGTCGCCCTCGATCTCTCAACACGTTGTCCTTCATGCTTTCGACACAGGGGTGCTCCCGCACC
TTGGAACCAAATGGAGGCTCATA
(Seq. ID No. 68: 2743-2840 of Seq. ID No. 1),
GCTTGCGCAGGTCCTCCCAGCTGATGACATGCCGCGTCAGGTACTCCTGTAGGTTGCCC
   (Seq. ID No. 69: 3200-3258 of Seq. ID No. 1),
CCAGGTTGAACTCAGCTTCTGCTGCCGGTTAACGCGGTAGCAGTAGAAGATGATGATGAC
   (Seq. ID No. 70: 3645-3704 of Seq. ID No. 1),
(Seq. ID No. 71: 4136-4223 of Seq. ID No. 1), 
GCAGCGGCCACAGGCCCCTGAGCAGCCCCCGACCCATGGCAGACCCCGCTGCTCGTCATAGACCGAGCCCCCAGC
GCAGCGGAC
(Seq. ID No. 72: 4211-4294 of Seq. ID No. 1),
CGAGCGGGTGCACGCGCGGGGGTGTCGTCGCTCCGTGCGCGCGAGTGACTCACTCAACTTCAACTCAGCGCT
   (Seq. ID No. 73: 4485-4556 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Instead of Seq. ID No. 71 and Seq. ID No. 72 a combined sequence (Seq. ID No. 74) could be used: 
CTATCATGTCGTTATTAACCGACTTCTGAACGTGCGGTGGGATCGTGCTGGCGATACGCGTCCACAGGACGATGT
GCAGCGGCCACAGGCCCCTGAGCAGCCCCCGACCCATGGCAGACCCCGCTGCTCGTCATAGACCGAGCCCCCAGC
GCAGCGGAC
(Seq. ID No. 74: 4136-4294 of Seq. ID No. 1),

Alternatively the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides which hybridize within the following regions of Seq. ID No. 2 starting with the nucleotide 74 and ending with the nucleotide 145, starting with the nucleotide 336 and ending with the nucleotide 419, starting with the nucleotide 407 and ending with the nucleotide 494, starting with the nucleotide 926 and ending with the nucleotide 985, starting with the nucleotide 1372 and ending with the nucleotide 1430, starting with the nucleotide 1790 and ending with the nucleotide 1887, starting with the nucleotide 2049 and ending with the nucleotide 2107, starting with the nucleotide 2269 and ending with the nucleotide 2320, starting with the nucleotide 2340 and ending with the nucleotide 2390, starting with the nucleotide 4105 and ending with the nucleotide 4157 or starting with the nucleotide 4202 and ending with the nucleotide 4252, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides which hybridize within the following regions of Seq. ID No. 2 starting with the nucleotide 74 and ending with the nucleotide 145, starting with the nucleotide 336 and ending with the nucleotide 419, starting with the nucleotide 407 and ending with the nucleotide 494, starting with the nucleotide 926 and ending with the nucleotide 985, starting with the nucleotide 1372 and ending with the nucleotide 1430, starting with the nucleotide 1790 and ending with the nucleotide 1887, starting with the nucleotide 2049 and ending with the nucleotide 2107, starting with the nucleotide 2269 and ending with the nucleotide 2320, starting with the nucleotide 2340 and ending with the nucleotide 2390, starting with the nucleotide 4105 and ending with the nucleotide 4157 or starting with the nucleotide 4202 and ending with the nucleotide 4252, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in the region of Seq. ID No. 1 starting with the nucleotide 378 and ending with the nucleotide 428, starting with the nucleotide 473 and ending with the nucleotide 525, starting with the nucleotide 2240 and ending with the nucleotide 2290, starting with the nucleotide 2310 and ending with the nucleotide 2361, starting with the nucleotide 2523 and ending with the nucleotide 2581, starting with the nucleotide 2743 and ending with the nucleotide 2840, starting with the nucleotide 3200 and ending with the nucleotide 3258, starting with the nucleotide 3645 and ending with the nucleotide 3704, starting with the nucleotide 4136 and ending with the nucleotide 4223, starting with the nucleotide 4211 and ending with the nucleotide 4294 or starting with the nucleotide 4485 and ending with the nucleotide 4556, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides, wherein at least two of the nucleotides are LNAs and the sequence of the antisense-oligonucleotide of the 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides is selected from the group of sequences of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides contained in the region of Seq. ID No. 1 starting with the nucleotide 378 and ending with the nucleotide 428, starting with the nucleotide 473 and ending with the nucleotide 525, starting with the nucleotide 2240 and ending with the nucleotide 2290, starting with the nucleotide 2310 and ending with the nucleotide 2361, starting with the nucleotide 2523 and ending with the nucleotide 2581, starting with the nucleotide 2743 and ending with the nucleotide 2840, starting with the nucleotide 3200 and ending with the nucleotide 3258, starting with the nucleotide 3645 and ending with the nucleotide 3704, starting with the nucleotide 4136 and ending with the nucleotide 4223, starting with the nucleotide 4211 and ending with the nucleotide 4294 or starting with the nucleotide 4485 and ending with the nucleotide 4556, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

In case the antisense-oligonucleotide has between 10 and 24 nucleotides, the above disclosure reads as follows. Preferred are antisense-oligonucleotide(s) having a length of 10 to 24 nucleotides, wherein at least two of the nucleotides are LNAs and the sequence of the antisense-oligonucleotide of the 10 to 24 nucleotides is selected from the group of sequences of 10 to 24 nucleotides contained in the region of Seq. ID No. 1 starting with the nucleotide 378 and ending with the nucleotide 428, starting with the nucleotide 473 and ending with the nucleotide 525, starting with the nucleotide 2240 and ending with the nucleotide 2290, starting with the nucleotide 2310 and ending with the nucleotide 2361, starting with the nucleotide 2523 and ending with the nucleotide 2581, starting with the nucleotide 2743 and ending with the nucleotide 2840, starting with the nucleotide 3200 and ending with the nucleotide 3258, starting with the nucleotide 3645 and ending with the nucleotide 3704, starting with the nucleotide 4136 and ending with the nucleotide 4223, starting with the nucleotide 4211 and ending with the nucleotide 4294 or starting with the nucleotide 4485 and ending with the nucleotide 4556, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Still more preferably the present invention relates to the following embodiments.

The present invention is preferably directed to antisense-oligonucleotides having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in a sequence selected from the following group:
CTCTTGAATCTTGAATATCTCATGAATGGACCAGTATTCTAGAAACTCACC
   (Seq. ID No. 63: 378-428 of Seq. ID No. 1),
AGCTATTCATATTTATATACAGGCATTAATAAAGTGCAAATGTTATTGGCT
   (Seq. ID No. 65: 2240-2290 of Seq. ID No. 1),
TTTCCTGAGGAAGTGCTAACACAGCTTATCCTATGACAATGTCAAAGGCATA
   (Seq. ID No. 66: 2310-2361 of Seq. ID No. 1),
GCCCAGCCTGCCCCAGAAGAGCTATTTGGTAGTGTTTAGGGAGCCGTCTTCAGGAATCT
   (Seq. ID No. 67: 2523-2581 of Seq. ID No. 1),
GCTTGCGCAGGTCCTCCCAGCTGATGACATGCCGCGTCAGGTACTCCTGTAGGTTGCCC
   (Seq. ID No. 69: 3200-3258 of Seq. ID No. 1), 
CTATCATGTCGTTATTAACCGACTTCTGAACGTGCGGTGGGATCGTGCTGGCGATACGCGTCCACAGGACGATGT
GCAGCGGCCACAG
(Seq. ID No. 71: 4136-4223 of Seq. ID No. 1), 
GCAGCGGCCACAGGCCCCTGAGCAGCCCCCGACCCATGGCAGACCCCGCTGCTCGTCATAGACCGAGCCCCCAGC
GCAGCGGAC
(Seq. ID No. 72: 4211-4294 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Instead of seq. ID No. 71 and Seq. ID No. 72 a combined sequence (Seq. ID No. 74) could be used: 
CTATCATGTCGTTATTAACCGACTTCTGAACGTGCGGTGGGATCGTGCTGGCGATACGCGTCCACAGGACGATGT
GCAGCGGCCACAGGCCCCTGAGCAGCCCCCGACCCATGGCAGACCCCGCTGCTCGTCATAGACCGAGCCCCCAGC
GCAGCGGAC
(Seq. ID No. 74: 4136-4294 of Seq. ID No. 1),

Also more preferably the present invention is directed to antisense-oligonucleotides having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of the 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides contained in a sequence selected from the following group:
CTCTTGAATCTTGAATATCTCATGAATGGACCAGTATTCTAGAAACTCACC
   (Seq. ID No. 63: 378-428 of Seq. ID No. 1),
AGCTATTCATATTTATATACAGGCATTAATAAAGTGCAAATGTTATTGGCT
   (Seq. ID No. 65: 2240-2290 of Seq. ID No. 1),
TTTCCTGAGGAAGTGCTAACACAGCTTATCCTATGACAATGTCAAAGGCATA
   (Seq. ID No. 66: 2310-2361 of Seq. ID No. 1),
GCCCAGCCTGCCCCAGAAGAGCTATTTGGTAGTGTTTAGGGAGCCGTCTTCAGGAATCT
   (Seq. ID No. 67: 2523-2581 of Seq. ID No. 1),
GCTTGCGCAGGTCCTCCCAGCTGATGACATGCCGCGTCAGGTACTCCTGTAGGTTGCCC
   (Seq. ID No. 69: 3200-3258 of Seq. ID No. 1), 
CTATCATGTCGTTATTAACCGACTTCTGAACGTGCGGTGGGATCGTGCTGGCGATACGCGTCCACAGGACGATGT
GCAGCGGCCACAG
(Seq. ID No. 71: 4136-4223 of Seq. ID No. 1), 
GCAGCGGCCACAGGCCCCTGAGCAGCCCCCGACCCATGGCAGACCCCGCTGCTCGTCATAGACCGAGCCCCCAGC
GCAGCGGAC
(Seq. ID No. 72: 4211-4294 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Instead of seq. ID No. 71 and Seq. ID No. 72 a combined sequence (Seq. ID No. 74) could be used: 
CTATCATGTCGTTATTAACCGACTTCTGAACGTGCGGTGGGATCGTGCTGGCGATACGCGTCCACAGGACGATGT
GCAGCGGCCACAGGCCCCTGAGCAGCCCCCGACCCATGGCAGACCCCGCTGCTCGTCATAGACCGAGCCCCCAGC
GCAGCGGAC
(Seq. ID No. 74: 4136-4294 of Seq. ID No. 1),

Alternatively the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides which hybridize within the following regions of Seq. ID No. 2 starting with the nucleotide 336 and ending with the nucleotide 419, starting with the nucleotide 407 and ending with the nucleotide 494, starting with the nucleotide 1372 and ending with the nucleotide 1430, starting with the nucleotide 2049 and ending with the nucleotide 2107, starting with the nucleotide 2269 and ending with the nucleotide 2320, starting with the nucleotide 2340 and ending with the nucleotide 2390, or starting with the nucleotide 4202 and ending with the nucleotide 4252, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides which hybridize within the following regions of Seq. ID No. 2 starting with the nucleotide 336 and ending with the nucleotide 419, starting with the nucleotide 407 and ending with the nucleotide 494, starting with the nucleotide 1372 and ending with the nucleotide 1430, starting with the nucleotide 2049 and ending with the nucleotide 2107, starting with the nucleotide 2269 and ending with the nucleotide 2320, starting with the nucleotide 2340 and ending with the nucleotide 2390, or starting with the nucleotide 4202 and ending with the nucleotide 4252, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in the region of Seq. ID No. 1 starting with the nucleotide 378 and ending with the nucleotide 428, starting with the nucleotide 2240 and ending with the nucleotide 2290, starting with the nucleotide 2310 and ending with the nucleotide 2361, starting with the nucleotide 2523 and ending with the nucleotide 2581, starting with the nucleotide 3200 and ending with the nucleotide 3258, starting with the nucleotide 4136 and ending with the nucleotide 4223, or starting with the nucleotide 4211 and ending with the nucleotide 4294, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides, wherein at least two of the nucleotides are LNAs and the sequence of the antisense-oligonucleotide of the 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides is selected from the group of sequences of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides contained in the region of Seq. ID No. 1 starting with the nucleotide 378 and ending with the nucleotide 428, starting with the nucleotide 2240 and ending with the nucleotide 2290, starting with the nucleotide 2310 and ending with the nucleotide 2361, starting with the nucleotide 2523 and ending with the nucleotide 2581, starting with the nucleotide 3200 and ending with the nucleotide 3258, starting with the nucleotide 4136 and ending with the nucleotide 4223, or starting with the nucleotide 4211 and ending with the nucleotide 4294, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

In case the antisense-oligonucleotide has between 10 and 24 nucleotides, the above disclosure reads as follows. Preferred are antisense-oligonucleotide(s) having a length of 10 to 24 nucleotides, wherein at least two of the nucleotides are LNAs and the sequence of the antisense-oligonucleotide of the 10 to 24 nucleotides is selected from the group of sequences of 10 to 24 nucleotides contained in the region of Seq. ID No. 1 starting with the nucleotide 378 and ending with the nucleotide 428, starting with the nucleotide 2240 and ending with the nucleotide 2290, starting with the nucleotide 2310 and ending with the nucleotide 2361, starting with the nucleotide 2523 and ending with the nucleotide 2581, starting with the nucleotide 3200 and ending with the nucleotide 3258, starting with the nucleotide 4136 and ending with the nucleotide 4223, or starting with the nucleotide 4211 and ending with the nucleotide 4294, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Still more preferably the present invention relates to the following embodiments.

The present invention is preferably directed to antisense-oligonucleotides having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in a sequence selected from the following group:
GAATCTTGAATATCTCATGAATGGACCAGTATTCTAGAAAC
   (Seq. ID No. 75: 383-423 of Seq. ID No. 1),
CGGTGCAAGTGGAATTTCTAGGCGCCTCTATGCTACTGCAGCC
   (Seq. ID No. 76: 478-520 of Seq. ID No. 1),
TTCATATTTATATACAGGCATTAATAAAGTGCAAATGTTAT
   (Seq. ID No. 77: 2245-2285 of Seq. ID No. 1),
TGAGGAAGTGCTAACACAGCTTATCCTATGACAATGTCAAAG
   (Seq. ID No. 78: 2315-2356 of Seq. ID No. 1),
GCCTGCCCCAGAAGAGCTATTTGGTAGTGTTTAGGGAGCCGTCTTCAGG
   (Seq. ID No. 79: 2528-2576 of Seq. ID No. 1), 
AATTTCTGGTCGCCCTCGATCTCTCAACACGTTGTCCTTCATGCTTTCGACACAGGGGTGCTCCCGCACCTTGGA
ACCAAATGGAGGC
(Seq. ID No. 80: 2748-2835 of Seq. ID No. 1),
CGCAGGTCCTCCCAGCTGATGACATGCCGCGTCAGGTACTCCTGTAGGT
   (Seq. ID No. 81: 3205-3253 of Seq. ID No. 1),
TTGAACTCAGCTTCTGCTGCCGGTTAACGCGGTAGCAGTAGAAGATGATG
   (Seq. ID No. 82: 3650-3699 of Seq. ID No. 1), 
ATGTCGTTATTAACCGACTTCTGAACGTGCGGTGGGATCGTGCTGGCGATACGCGTCCACAGGACGATGTGCAGC
GGC
(Seq. ID No. 83: 4141-4218 of Seq. ID No. 1),
GGCCACAGGCCCCTGAGCAGCCCCCGACCCATGGCAGACCCCGCTGCTCGTCATAGACCGAGCCCCCAGCGCAG
   (Seq. ID No. 84: 4216-4289 of Seq. ID No. 1),
GGGTGCACGCGCGGGGGTGTCGTCGCTCCGTGCGCGCGAGTGACTCACTCAACTTCAACTCA
   (Seq. ID No. 85: 4490-4551 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Instead of Seq. ID No. 83 and Seq. ID No. 84 a combined sequence (Seq. ID No. 86) could be used: 
ATGTCGTTATTAACCGACTTCTGAACGTGCGGTGGGATCGTGCTGGCGATACGCGTCCACAGGACGATGTGCAGC
GGCCACAGGCCCCTGAGCAGCCCCCGACCCATGGCAGACCCCGCTGCTCGTCATAGACCGAGCCCCCAGCGCAG
(Seq. ID No. 86: 4141-4289 of Seq. ID No. 1),

Also more preferably the present invention is directed to antisense-oligonucleotides having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of the 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides contained in a sequence selected from the following group:
GAATCTTGAATATCTCATGAATGGACCAGTATTCTAGAAAC
   (Seq. ID No. 75: 383-423 of Seq. ID No. 1),
CGGTGCAAGTGGAATTTCTAGGCGCCTCTATGCTACTGCAGCC
   (Seq. ID No. 76: 478-520 of Seq. ID No. 1),
TTCATATTTATATACAGGCATTAATAAAGTGCAAATGTTAT
   (Seq. ID No. 77: 2245-2285 of Seq. ID No. 1),
TGAGGAAGTGCTAACACAGCTTATCCTATGACAATGTCAAAG
   (Seq. ID No. 78: 2315-2356 of Seq. ID No. 1),
GCCTGCCCCAGAAGAGCTATTTGGTAGTGTTTAGGGAGCCGTCTTCAGG
   (Seq. ID No. 79: 2528-2576 of Seq. ID No. 1), 
AATTTCTGGTCGCCCTCGATCTCTCAACACGTTGTCCTTCATGCTTTCGACACAGGGGTGCTCCCGCACCTTGGA
ACCAAATGGAGGC
(Seq. ID No. 80: 2748-2835 of Seq. ID No. 1),
CGCAGGTCCTCCCAGCTGATGACATGCCGCGTCAGGTACTCCTGTAGGT
   (Seq. ID No. 81: 3205-3253 of Seq. ID No. 1),
TTGAACTCAGCTTCTGCTGCCGGTTAACGCGGTAGCAGTAGAAGATGATG
   (Seq. ID No. 82: 3650-3699 of Seq. ID No. 1), 
ATGTCGTTATTAACCGACTTCTGAACGTGCGGTGGGATCGTGCTGGCGATACGCGTCCACAGGACGATGTGCAGC
GGC
(Seq. ID No. 83: 4141-4218 of Seq. ID No. 1),
GGCCACAGGCCCCTGAGCAGCCCCCGACCCATGGCAGACCCCGCTGCTCGTCATAGACCGAGCCCCCAGCGCAG
   (Seq. ID No. 84: 4216-4289 of Seq. ID No. 1),
GGGTGCACGCGCGGGGGTGTCGTCGCTCCGTGCGCGCGAGTGACTCACTCAACTTCAACTCA
   (Seq. ID No. 85: 4490-4551 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Instead of Seq. ID No. 83 and Seq. ID No. 84 a combined sequence (Seq. ID No. 86) could be used: 
ATGTCGTTATTAACCGACTTCTGAACGTGCGGTGGGATCGTGCTGGCGATACGCGTCCACAGGACGATGTGCAGC
GGCCACAGGCCCCTGAGCAGCCCCCGACCCATGGCAGACCCCGCTGCTCGTCATAGACCGAGCCCCCAGCGCAG
(Seq. ID No. 86: 4141-4289 of Seq. ID No. 1),

Alternatively the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides which hybridize within the following regions of Seq. ID No. 2 starting with the nucleotide 79 and ending with the nucleotide 140, starting with the nucleotide 341 and ending with the nucleotide 414, starting with the nucleotide 412 and ending with the nucleotide 489, starting with the nucleotide 931 and ending with the nucleotide 980, starting with the nucleotide 1377 and ending with the nucleotide 1425, starting with the nucleotide 1795 and ending with the nucleotide 1882, starting with the nucleotide 2054 and ending with the nucleotide 2102, starting with the nucleotide 2274 and ending with the nucleotide 2315, starting with the nucleotide 2345 and ending with the nucleotide 2385, starting with the nucleotide 4110 and ending with the nucleotide 4152 or starting with the nucleotide 4207 and ending with the nucleotide 4247, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides which hybridize within the following regions of Seq. ID No. 2 starting with the nucleotide 79 and ending with the nucleotide 140, starting with the nucleotide 341 and ending with the nucleotide 414, starting with the nucleotide 412 and ending with the nucleotide 489, starting with the nucleotide 931 and ending with the nucleotide 980, starting with the nucleotide 1377 and ending with the nucleotide 1425, starting with the nucleotide 1795 and ending with the nucleotide 1882, starting with the nucleotide 2054 and ending with the nucleotide 2102, starting with the nucleotide 2274 and ending with the nucleotide 2315, starting with the nucleotide 2345 and ending with the nucleotide 2385, starting with the nucleotide 4110 and ending with the nucleotide 4152 or starting with the nucleotide 4207 and ending with the nucleotide 4247, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in the region of Seq. ID No. 1 starting with the nucleotide 383 and ending with the nucleotide 423, starting with the nucleotide 478 and ending with the nucleotide 520, starting with the nucleotide 2245 and ending with the nucleotide 2285, starting with the nucleotide 2315 and ending with the nucleotide 2356, starting with the nucleotide 2528 and ending with the nucleotide 2576, starting with the nucleotide 2748 and ending with the nucleotide 2835, starting with the nucleotide 3205 and ending with the nucleotide 3253, starting with the nucleotide 3650 and ending with the nucleotide 3699, starting with the nucleotide 4141 and ending with the nucleotide 4218, starting with the nucleotide 4216 and ending with the nucleotide 4289 or starting with the nucleotide 4490 and ending with the nucleotide 4551, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides, wherein at least two of the nucleotides are LNAs and the sequence of the antisense-oligonucleotide of the 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides is selected from the group of sequences of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides contained in the region of Seq. ID No. 1 starting with the nucleotide 383 and ending with the nucleotide 423, starting with the nucleotide 478 and ending with the nucleotide 520, starting with the nucleotide 2245 and ending with the nucleotide 2285, starting with the nucleotide 2315 and ending with the nucleotide 2356, starting with the nucleotide 2528 and ending with the nucleotide 2576, starting with the nucleotide 2748 and ending with the nucleotide 2835, starting with the nucleotide 3205 and ending with the nucleotide 3253, starting with the nucleotide 3650 and ending with the nucleotide 3699, starting with the nucleotide 4141 and ending with the nucleotide 4218, starting with the nucleotide 4216 and ending with the nucleotide 4289 or starting with the nucleotide 4490 and ending with the nucleotide 4551, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

In case the antisense-oligonucleotide has between 10 and 24 nucleotides, the above disclosure reads as follows. Preferred are antisense-oligonucleotide(s) having a length of 10 to 24 nucleotides, wherein at least two of the nucleotides are LNAs and the sequence of the antisense-oligonucleotide of the 10 to 24 nucleotides is selected from the group of sequences of 10 to 24 nucleotides contained in the region of Seq. ID No. 1 starting with the nucleotide 383 and ending with the nucleotide 423, starting with the nucleotide 478 and ending with the nucleotide 520, starting with the nucleotide 2245 and ending with the nucleotide 2285, starting with the nucleotide 2315 and ending with the nucleotide 2356, starting with the nucleotide 2528 and ending with the nucleotide 2576, starting with the nucleotide 2748 and ending with the nucleotide 2835, starting with the nucleotide 3205 and ending with the nucleotide 3253, starting with the nucleotide 3650 and ending with the nucleotide 3699, starting with the nucleotide 4141 and ending with the nucleotide 4218, starting with the nucleotide 4216 and ending with the nucleotide 4289 or starting with the nucleotide 4490 and ending with the nucleotide 4551, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Still more preferably the present invention relates to the following embodiments.

The present invention is preferably directed to antisense-oligonucleotides having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in a sequence selected from the following group:
GAATCTTGAATATCTCATGAATGGACCAGTATTCTAGAAAC
   (Seq. ID No. 75: 383-423 of Seq. ID No. 1),
TTCATATTTATATACAGGCATTAATAAAGTGCAAATGTTAT
   (Seq. ID No. 77: 2245-2285 of Seq. ID No. 1),
TGAGGAAGTGCTAACACAGCTTATCCTATGACAATGTCAAAG
   (Seq. ID No. 78: 2315-2356 of Seq. ID No. 1),
GCCTGCCCCAGAAGAGCTATTTGGTAGTGTTTAGGGAGCCGTCTTCAGG
   (Seq. ID No. 79: 2528-2576 of Seq. ID No. 1),
CGCAGGTCCTCCCAGCTGATGACATGCCGCGTCAGGTACTCCTGTAGGT
   (Seq. ID No. 81: 3205-3253 of Seq. ID No. 1), 
ATGTCGTTATTAACCGACTTCTGAACGTGCGGTGGGATCGTGCTGGCGATACGCGTCCACAGGACGATGTGCAGC
GGC
(Seq. ID No. 83: 4141-4218 of Seq. ID No. 1),
GGCCACAGGCCCCTGAGCAGCCCCCGACCCATGGCAGACCCCGCTGCTCGTCATAGACCGAGCCCCCAGCGCAG
   (Seq. ID No. 84: 4216-4289 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Instead of Seq. ID No. 83 and Seq. ID No. 84 a combined sequence (Seq. ID No. 86) could be used: 
ATGTCGTTATTAACCGACTTCTGAACGTGCGGTGGGATCGTGCTGGCGATACGCGTCCACAGGACGATGTGCAGC
GGCCACAGGCCCCTGAGCAGCCCCCGACCCATGGCAGACCCCGCTGCTCGTCATAGACCGAGCCCCCAGCGCAG
(Seq. ID No. 86: 4141-4289 of Seq. ID No. 1),

Also more preferably the present invention is directed to antisense-oligonucleotides having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of the 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides contained in a sequence selected from the following group:
GAATCTTGAATATCTCATGAATGGACCAGTATTCTAGAAAC
   (Seq. ID No. 75: 383-423 of Seq. ID No. 1),
TTCATATTTATATACAGGCATTAATAAAGTGCAAATGTTAT
   (Seq. ID No. 77: 2245-2285 of Seq. ID No. 1),
TGAGGAAGTGCTAACACAGCTTATCCTATGACAATGTCAAAG
   (Seq. ID No. 78: 2315-2356 of Seq. ID No. 1),
GCCTGCCCCAGAAGAGCTATTTGGTAGTGTTTAGGGAGCCGTCTTCAGG
   (Seq. ID No. 79: 2528-2576 of Seq. ID No. 1),
CGCAGGTCCTCCCAGCTGATGACATGCCGCGTCAGGTACTCCTGTAGGT
   (Seq. ID No. 81: 3205-3253 of Seq. ID No. 1), 
ATGTCGTTATTAACCGACTTCTGAACGTGCGGTGGGATCGTGCTGGCGATACGCGTCCACAGGACGATGTGCAGC
GGC
(Seq. ID No. 83: 4141-4218 of Seq. ID No. 1),
GGCCACAGGCCCCTGAGCAGCCCCCGACCCATGGCAGACCCCGCTGCTCGTCATAGACCGAGCCCCCAGCGCAG
   (Seq. ID No. 84: 4216-4289 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Instead of Seq. ID No. 83 and Seq. ID No. 84 a combined sequence (Seq. ID No. 86) could be used: 
ATGTCGTTATTAACCGACTTCTGAACGTGCGGTGGGATCGTGCTGGCGATACGCGTCCACAGGACGATGTGCAGC
GGCCACAGGCCCCTGAGCAGCCCCCGACCCATGGCAGACCCCGCTGCTCGTCATAGACCGAGCCCCCAGCGCAG
(Seq. ID No. 86: 4141-4289 of Seq. ID No. 1),

Alternatively the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides which hybridize within the following regions of Seq. ID No. 2 starting with the nucleotide 341 and ending with the nucleotide 414, starting with the nucleotide 412 and ending with the nucleotide 489, starting with the nucleotide 1377 and ending with the nucleotide 1425, starting with the nucleotide 2054 and ending with the nucleotide 2102, starting with the nucleotide 2274 and ending with the nucleotide 2315, starting with the nucleotide 2345 and ending with the nucleotide 2385, or starting with the nucleotide 4207 and ending with the nucleotide 4247, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides which hybridize within the following regions of Seq. ID No. 2 starting with the nucleotide 341 and ending with the nucleotide 414, starting with the nucleotide 412 and ending with the nucleotide 489, starting with the nucleotide 1377 and ending with the nucleotide 1425, starting with the nucleotide 2054 and ending with the nucleotide 2102, starting with the nucleotide 2274 and ending with the nucleotide 2315, starting with the nucleotide 2345 and ending with the nucleotide 2385, or starting with the nucleotide 4207 and ending with the nucleotide 4247, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in the region of Seq. ID No. 1 starting with the nucleotide 383 and ending with the nucleotide 423, starting with the nucleotide 2245 and ending with the nucleotide 2285, starting with the nucleotide 2315 and ending with the nucleotide 2356, starting with the nucleotide 2528 and ending with the nucleotide 2576, starting with the nucleotide 3205 and ending with the nucleotide 3253, starting with the nucleotide 4141 and ending with the nucleotide 4218, or starting with the nucleotide 4216 and ending with the nucleotide 4289, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides, wherein at least two of the nucleotides are LNAs and the sequence of the antisense-oligonucleotide of the 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides is selected from the group of sequences of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides contained in the region of Seq. ID No. 1 starting with the nucleotide 383 and ending with the nucleotide 423, starting with the nucleotide 2245 and ending with the nucleotide 2285, starting with the nucleotide 2315 and ending with the nucleotide 2356, starting with the nucleotide 2528 and ending with the nucleotide 2576, starting with the nucleotide 3205 and ending with the nucleotide 3253, starting with the nucleotide 4141 and ending with the nucleotide 4218, or starting with the nucleotide 4216 and ending with the nucleotide 4289, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

In case the antisense-oligonucleotide has between 10 and 24 nucleotides, the above disclosure reads as follows. Preferred are antisense-oligonucleotide(s) having a length of 10 to 24 nucleotides, wherein at least two of the nucleotides are LNAs and the sequence of the antisense-oligonucleotide of the 10 to 24 nucleotides is selected from the group of sequences of 10 to 24 nucleotides contained in the region of Seq. ID No. 1 starting with the nucleotide 383 and ending with the nucleotide 423, starting with the nucleotide 2245 and ending with the nucleotide 2285, starting with the nucleotide 2315 and ending with the nucleotide 2356, starting with the nucleotide 2528 and ending with the nucleotide 2576, starting with the nucleotide 3205 and ending with the nucleotide 3253, starting with the nucleotide 4141 and ending with the nucleotide 4218, or starting with the nucleotide 4216 and ending with the nucleotide 4289, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Still more preferably the present invention relates to the following embodiments.

The present invention is preferably directed to antisense-oligonucleotides having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in a sequence selected from the following group:
TTGAATATCTCATGAATGGACCAGTATTCTA
   (Seq. ID No. 87: 388-418 of Seq. ID No. 1),
CAAGTGGAATTTCTAGGCGCCTCTATGCTACTG
   (Seq. ID No. 88: 483-515 of Seq. ID No. 1),
ATTTATATACAGGCATTAATAAAGTGCAAAT
   (Seq. ID No. 89: 2250-2280 of Seq. ID No. 1),
AAGTGCTAACACAGCTTATCCTATGACAATGT
   (Seq. ID No. 90: 2320-2351 of Seq. ID No. 1),
CCCCAGAAGAGCTATTTGGTAGTGTTTAGGGAGCCGTCT
   (Seq. ID No. 91: 2533-2571 of Seq. ID No. 1), 
CTGGTCGCCCTCGATCTCTCAACACGTTGTCCTTCATGCTTTCGACACAGGGGTGCTCCCGCACCTTGGAACCAA

ATG
(Seq. ID No. 92: 2753-2830 of Seq. ID No. 1),
GTCCTCCCAGCTGATGACATGCCGCGTCAGGTACTCCTG
   (Seq. ID No. 93: 3210-3248 of Seq. ID No. 1),
CTCAGCTTCTGCTGCCGGTTAACGCGGTAGCAGTAGAAGA
   (Seq. ID No. 94: 3655-3694 of Seq. ID No. 1),
GTTATTAACCGACTTCTGAACGTGCGGTGGGATCGTGCTGGCGATACGCGTCCACAGGACGATGTGCA
   (Seq. ID No. 95: 4146-4213 of Seq. ID No. 1),
CAGGCCCCTGAGCAGCCCCCGACCCATGGCAGACCCCGCTGCTCGTCATAGACCGAGCCCCCAG
   (Seq. ID No. 96: 4221-4284 of Seq. ID No. 1),
CACGCGCGGGGGTGTCGTCGCTCCGTGCGCGCGAGTGACTCACTCAACTTCA
   (Seq. ID No. 97: 4495-4546 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Also more preferably the present invention is directed to antisense-oligonucleotides having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of the 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides contained in a sequence selected from the following group:
TTGAATATCTCATGAATGGACCAGTATTCTA
   (Seq. ID No. 87: 388-418 of Seq. ID No. 1),
CAAGTGGAATTTCTAGGCGCCTCTATGCTACTG
   (Seq. ID No. 88: 483-515 of Seq. ID No. 1),
ATTTATATACAGGCATTAATAAAGTGCAAAT
   (Seq. ID No. 89: 2250-2280 of Seq. ID No. 1),
AAGTGCTAACACAGCTTATCCTATGACAATGT
   (Seq. ID No. 90: 2320-2351 of Seq. ID No. 1),
CCCCAGAAGAGCTATTTGGTAGTGTTTAGGGAGCCGTCT
   (Seq. ID No. 91: 2533-2571 of Seq. ID No. 1), 
CTGGTCGCCCTCGATCTCTCAACACGTTGTCCTTCATGCTTTCGACACAGGGGTGCTCCCGCACCTTGGAACCAA

ATG
(Seq. ID No. 92: 2753-2830 of Seq. ID No. 1),
GTCCTCCCAGCTGATGACATGCCGCGTCAGGTACTCCTG
   (Seq. ID No. 93: 3210-3248 of Seq. ID No. 1),
CTCAGCTTCTGCTGCCGGTTAACGCGGTAGCAGTAGAAGA
   (Seq. ID No. 94: 3655-3694 of Seq. ID No. 1),
GTTATTAACCGACTTCTGAACGTGCGGTGGGATCGTGCTGGCGATACGCGTCCACAGGACGATGTGCA
   (Seq. ID No. 95: 4146-4213 of Seq. ID No. 1),
CAGGCCCCTGAGCAGCCCCCGACCCATGGCAGACCCCGCTGCTCGTCATAGACCGAGCCCCCAG
   (Seq. ID No. 96: 4221-4284 of Seq. ID No. 1),
CACGCGCGGGGGTGTCGTCGCTCCGTGCGCGCGAGTGACTCACTCAACTTCA
   (Seq. ID No. 97: 4495-4546 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Alternatively the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides which hybridize within the following regions of Seq. ID No. 2 starting with the nucleotide 84 and ending with the nucleotide 135, starting with the nucleotide 346 and ending with the nucleotide 409, starting with the nucleotide 417 and ending with the nucleotide 484, starting with the nucleotide 936 and ending with the nucleotide 975, starting with the nucleotide 1382 and ending with the nucleotide 1420, starting with the nucleotide 1800 and ending with the nucleotide 1877, starting with the nucleotide 2059 and ending with the nucleotide 2097, starting with the nucleotide 2279 and ending with the nucleotide 2310, starting with the nucleotide 2350 and ending with the nucleotide 2380, starting with the nucleotide 4115 and ending with the nucleotide 4147 or starting with the nucleotide 4212 and ending with the nucleotide 4242, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides which hybridize within the following regions of Seq. ID No. 2 starting with the nucleotide 84 and ending with the nucleotide 135, starting with the nucleotide 346 and ending with the nucleotide 409, starting with the nucleotide 417 and ending with the nucleotide 484, starting with the nucleotide 936 and ending with the nucleotide 975, starting with the nucleotide 1382 and ending with the nucleotide 1420, starting with the nucleotide 1800 and ending with the nucleotide 1877, starting with the nucleotide 2059 and ending with the nucleotide 2097, starting with the nucleotide 2279 and ending with the nucleotide 2310, starting with the nucleotide 2350 and ending with the nucleotide 2380, starting with the nucleotide 4115 and ending with the nucleotide 4147 or starting with the nucleotide 4212 and ending with the nucleotide 4242, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in the region of Seq. ID No. 1 starting with the nucleotide 388 and ending with the nucleotide 418, starting with the nucleotide 483 and ending with the nucleotide 515, starting with the nucleotide 2250 and ending with the nucleotide 2280, starting with the nucleotide 2320 and ending with the nucleotide 2351, starting with the nucleotide 2533 and ending with the nucleotide 2571, starting with the nucleotide 2753 and ending with the nucleotide 2830, starting with the nucleotide 3210 and ending with the nucleotide 3248, starting with the nucleotide 3655 and ending with the nucleotide 3694, starting with the nucleotide 4146 and ending with the nucleotide 4213, starting with the nucleotide 4221 and ending with the nucleotide 4284 or starting with the nucleotide 4495 and ending with the nucleotide 4546, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides, wherein at least two of the nucleotides are LNAs and the sequence of the antisense-oligonucleotide of the 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides is selected from the group of sequences of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides contained in the region of Seq. ID No. 1 starting with the nucleotide 388 and ending with the nucleotide 418, starting with the nucleotide 483 and ending with the nucleotide 515, starting with the nucleotide 2250 and ending with the nucleotide 2280, starting with the nucleotide 2320 and ending with the nucleotide 2351, starting with the nucleotide 2533 and ending with the nucleotide 2571, starting with the nucleotide 2753 and ending with the nucleotide 2830, starting with the nucleotide 3210 and ending with the nucleotide 3248, starting with the nucleotide 3655 and ending with the nucleotide 3694, starting with the nucleotide 4146 and ending with the nucleotide 4213, starting with the nucleotide 4221 and ending with the nucleotide 4284 or starting with the nucleotide 4495 and ending with the nucleotide 4546, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

In case the antisense-oligonucleotide has between 10 and 24 nucleotides, the above disclosure reads as follows. Preferred are antisense-oligonucleotide(s) having a length of 10 to 24 nucleotides, wherein at least two of the nucleotides are LNAs and the sequence of the antisense-oligonucleotide of the 10 to 24 nucleotides is selected from the group of sequences of 10 to 24 nucleotides contained in the region of Seq. ID No. 1 starting with the nucleotide 388 and ending with the nucleotide 418, starting with the nucleotide 483 and ending with the nucleotide 515, starting with the nucleotide 2250 and ending with the nucleotide 2280, starting with the nucleotide 2320 and ending with the nucleotide 2351, starting with the nucleotide 2533 and ending with the nucleotide 2571, starting with the nucleotide 2753 and ending with the nucleotide 2830, starting with the nucleotide 3210 and ending with the nucleotide 3248, starting with the nucleotide 3655 and ending with the nucleotide 3694, starting with the nucleotide 4146 and ending with the nucleotide 4213, starting with the nucleotide 4221 and ending with the nucleotide 4284 or starting with the nucleotide 4495 and ending with the nucleotide 4546, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Still more preferably the present invention relates to the following embodiments.

The present invention is preferably directed to antisense-oligonucleotides having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in a sequence selected from the following group:
TTGAATATCTCATGAATGGACCAGTATTCTA
   (Seq. ID No. 87: 388-418 of Seq. ID No. 1),
ATTTATATACAGGCATTAATAAAGTGCAAAT
   (Seq. ID No. 89: 2250-2280 of Seq. ID No. 1),
AAGTGCTAACACAGCTTATCCTATGACAATGT
   (Seq. ID No. 90: 2320-2351 of Seq. ID No. 1),
CCCCAGAAGAGCTATTTGGTAGTGTTTAGGGAGCCGTCT
   (Seq. ID No. 91: 2533-2571 of Seq. ID No. 1),
GTCCTCCCAGCTGATGACATGCCGCGTCAGGTACTCCTG
   (Seq. ID No. 93: 3210-3248 of Seq. ID No. 1),
GTTATTAACCGACTTCTGAACGTGCGGTGGGATCGTGCTGGCGATACGCGTCCACAGGACGATGTGCA
   (Seq. ID No. 95: 4146-4213 of Seq. ID No. 1),
CAGGCCCCTGAGCAGCCCCCGACCCATGGCAGACCCCGCTGCTCGTCATAGACCGAGCCCCCAG
   (Seq. ID No. 96: 4221-4284 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Also more preferably the present invention is directed to antisense-oligonucleotides having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of the 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides contained in a sequence selected from the following group:
TTGAATATCTCATGAATGGACCAGTATTCTA
   (Seq. ID No. 87: 388-418 of Seq. ID No. 1),
ATTTATATACAGGCATTAATAAAGTGCAAAT
   (Seq. ID No. 89: 2250-2280 of Seq. ID No. 1),
AAGTGCTAACACAGCTTATCCTATGACAATGT
   (Seq. ID No. 90: 2320-2351 of Seq. ID No. 1),
CCCCAGAAGAGCTATTTGGTAGTGTTTAGGGAGCCGTCT
   (Seq. ID No. 91: 2533-2571 of Seq. ID No. 1),
GTCCTCCCAGCTGATGACATGCCGCGTCAGGTACTCCTG
   (Seq. ID No. 93: 3210-3248 of Seq. ID No. 1),
GTTATTAACCGACTTCTGAACGTGCGGTGGGATCGTGCTGGCGATACGCGTCCACAGGACGATGTGCA
   (Seq. ID No. 95: 4146-4213 of Seq. ID No. 1),
CAGGCCCCTGAGCAGCCCCCGACCCATGGCAGACCCCGCTGCTCGTCATAGACCGAGCCCCCAG
   (Seq. ID No. 96: 4221-4284 of Seq. ID No. 1),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Alternatively the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides which hybridize within the following regions of Seq. ID No. 2 starting with the nucleotide 346 and ending with the nucleotide 409, starting with the nucleotide 417 and ending with the nucleotide 484, starting with the nucleotide 1382 and ending with the nucleotide 1420, starting with the nucleotide 2059 and ending with the nucleotide 2097, starting with the nucleotide 2279 and ending with the nucleotide 2310, starting with the nucleotide 2350 and ending with the nucleotide 2380, or starting with the nucleotide 4212 and ending with the nucleotide 4242, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 10 to 28 nucleotides, preferably 10 to 26 nucleotides, more preferably 10 to 24 nucleotides or 10 to 22 nucleotides or 10 to 20 nucleotides, wherein at least two of the nucleotides are LNAs and the sequence of the antisense-oligonucleotide of 10 to 28 nucleotides, preferably 10 to 26 nucleotides, more preferably 10 to 24 nucleotides or 10 to 22 nucleotides or 10 to 20 nucleotides is selected from the group of sequences of 10 to 28 nucleotides, preferably 10 to 26 nucleotides, more preferably 10 to 24 nucleotides or 10 to 22 nucleotides or 10 to 20 nucleotides which hybridize within the following regions of Seq. ID No. 2 starting with the nucleotide 346 and ending with the nucleotide 409, starting with the nucleotide 417 and ending with the nucleotide 484, starting with the nucleotide 1382 and ending with the nucleotide 1420, starting with the nucleotide 2059 and ending with the nucleotide 2097, starting with the nucleotide 2279 and ending with the nucleotide 2310, starting with the nucleotide 2350 and ending with the nucleotide 2380, or starting with the nucleotide 4212 and ending with the nucleotide 4242, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of at least 10 or 8 nucleotides, wherein at least two of the nucleotides are LNAs and the sequence of the antisense-oligonucleotide of the at least 10 or 8 nucleotides is selected from the group of sequences of at least 10 or 8 nucleotides contained in the region of Seq. ID No. 1 starting with the nucleotide 388 and ending with the nucleotide 418, starting with the nucleotide 2250 and ending with the nucleotide 2280, starting with the nucleotide 2320 and ending with the nucleotide 2351, starting with the nucleotide 2533 and ending with the nucleotide 2571, starting with the nucleotide 3210 and ending with the nucleotide 3248, starting with the nucleotide 4146 and ending with the nucleotide 4213, or starting with the nucleotide 4221 and ending with the nucleotide 4284, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

Moreover the present invention relates to antisense-oligonucleotide(s) having a length of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides, wherein at least two of the nucleotides are LNAs and the sequence of the antisense-oligonucleotide of the 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides is selected from the group of sequences of 8 to 28 nucleotides, preferably of 10 to 28 nucleotides, more preferably 10 to 26 nucleotides, still more preferably 10 to 24 nucleotides or 10 to 22 nucleotides or 10 to 20 nucleotides contained in the region of Seq. ID No. 1 starting with the nucleotide 388 and ending with the nucleotide 418, starting with the nucleotide 2250 and ending with the nucleotide 2280, starting with the nucleotide 2320 and ending with the nucleotide 2351, starting with the nucleotide 2533 and ending with the nucleotide 2571, starting with the nucleotide 3210 and ending with the nucleotide 3248, starting with the nucleotide 4146 and ending with the nucleotide 4213, or starting with the nucleotide 4221 and ending with the nucleotide 4284, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

In case the antisense-oligonucleotide has between 10 and 24 nucleotides, the above disclosure reads as follows. Preferred are antisense-oligonucleotide(s) having a length of 10 to 24 nucleotides, wherein at least two of the nucleotides are LNAs and the sequence of the antisense-oligonucleotide of the 10 to 24 nucleotides is selected from the group of sequences of 10 to 24 nucleotides contained in the region of Seq. ID No. 1 starting with the nucleotide 388 and ending with the nucleotide 418, starting with the nucleotide 2250 and ending with the nucleotide 2280, starting with the nucleotide 2320 and ending with the nucleotide 2351, starting with the nucleotide 2533 and ending with the nucleotide 2571, starting with the nucleotide 3210 and ending with the nucleotide 3248, starting with the nucleotide 4146 and ending with the nucleotide 4213, or starting with the nucleotide 4221 and ending with the nucleotide 4284, wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

The **Seq. ID No. 1** represents a non-coding DNA strand (5'-3' antisense-sequence) of the Homo sapiens transforming growth factor, beta receptor II (70/80kDa) (TGF-R_{II}), transcript variant 2.

The **Seq. ID No. 2** represents a coding DNA strand (5'-3' sense-sequence) of the Homo sapiens transforming growth factor, beta receptor II (70/80kDa) (TGF-R_{II}), transcript variant 2. Thus, Seq. ID No. 2 is reverse complementary to Seq. ID No. 1. Alternatively, one can also regard the sequence of Seq. ID No. 2 to be similar to the mRNA (5'-3' sense-sequence) of the Homo sapiens transforming growth factor, beta receptor II (70/80kDa) (TGF-R_{II}), transcript variant 2 (see also Seq. ID No. 3), except that it is written in the DNA code, i.e. represented in G, C, A, T code, and not in the RNA code.

The **Seq. ID No. 3** represents the mRNA (5'-3' sense-sequence) of the Homo sapiens transforming growth factor, beta receptor II (70/80kDa) (TGF-R_{II}), transcript variant 2. It is evident that the mRNA displayed in Seq. ID No. 3 is written in the RNA code, i.e. represented in G, C, A, U code.

It shall be understood, that "coding DNA strand", as used herein, refers to the DNA strand that is identical to the mRNA (except that is written in the DNA code) and that encompasses the codons that used for protein translation. It is not used as template for the transcription into mRNA. Thus, the terms "coding DNA strand", "sense DNA strand" and "non-template DNA strand" can be used interchangeably. Furthermore, "non-coding DNA strand", as used herein, refers to the DNA strand that is complementary to the "coding DNA strand" and serves as a template for the transcription of mRNA. Thus, the terms "non-coding DNA strand", "antisense DNA strand" and "template DNA strand" can be used interchangeably

The term **"antisense-oligonucleotide"** refers to an oligomer or polymer of ribonucleic acid (RNA) or deoxyribonucleic acid (DNA) or mimetics or variants thereof such as antisense-oligonucleotides having a modified internucleotide linkage like a phosphorothioate linkage and/or one or more modified nucleobases such as 5-methylcytosine and/or one or more modified nucleotide units such as LNAs like β-D-oxy-LNA. The term "antisense-oligonucleotide" includes oligonucleotides composed of naturally-occurring nucleobases, sugars and covalent internucleotide (backbone) linkages as well as oligonucleotides having non-naturally-occurring portions which function similarly. Such modified or substituted oligonucleotides are often preferred over native forms, because of desirable properties such as, for example, enhanced cellular uptake, enhanced affinity for nucleic acid target and increased stability in the presence of nucleases. The antisense-oligonucleotides are short catalytic RNAs or catalytic oligonucleotides which hybridize to the target nucleic acid and inhibit its expression.

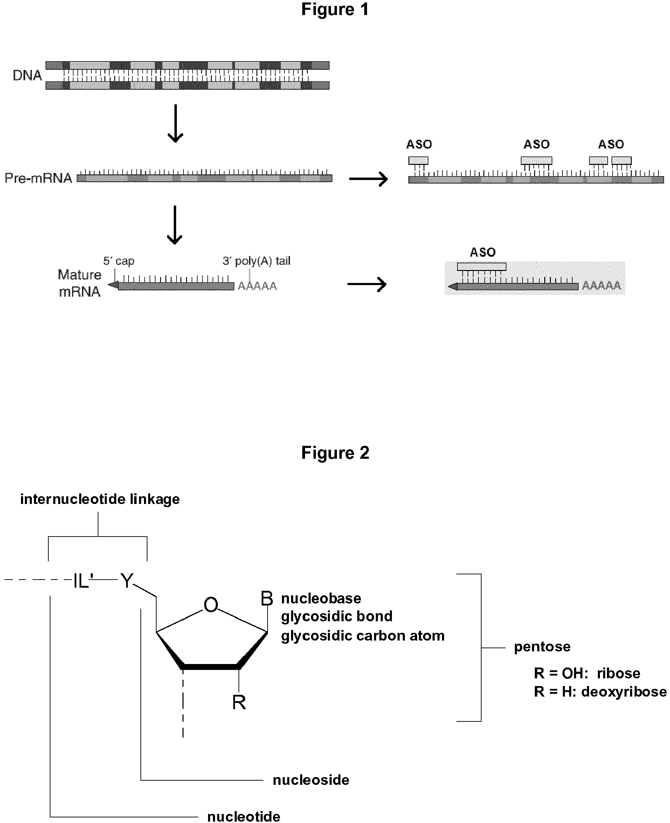

The term **"nucleoside"** is well known to a skilled person and refers to a pentose sugar moiety like ribose, desoxyribose or a modified or locked ribose or a modified or locked desoxyribose like the LNAs which are below disclosed in detail. A nucleobase is linked to the glycosidic carbon atom (position 1' of the pentose) and an internucleotide linkage is formed between the 3' oxygen or sulfur atom and preferably the 3' oxygen atom of a nucleoside and the 5' oxygen or sulfur atom and preferably the 5' oxygen atom of the adjacent nucleoside, while the internucleotide linkage does not belong to the nucleoside (see Fig. 2).

The term **"nucleotide"** is well known to a skilled person and refers to a pentose sugar moiety like ribose, desoxyribose or a modified or locked ribose or a modified or locked desoxyribose like the LNAs which are below disclosed in detail. A nucleobase is linked to the glycosidic carbon atom (position 1' of the pentose) and an internucleotide linkage is formed between the 3' oxygen or sulfur atom and preferably the 3' oxygen atom of a nucleotide and the 5' oxygen or sulfur atom and preferably the 5' oxygen atom of the adjacent nucleotide, while the internucleotide linkage is a part of the nucleotide (see Fig. 2).

The term **"nucleobase"** is herein abbreviated with "B" refers to the five standard nucleotide bases adenine (A), thymine (T), guanine (G), cytosine (C), and uracile (U) as well as to modifications or analogues thereof. Modified nucleobases include other synthetic and natural nucleobases such as 5-methylcytosine (5-me-C), 5-methyldeoxycytosine (5-me-dC), 5-hydroxymethyl cytosine, xanthine, hypoxanthine, 2-aminoadenine, 6-methyladenine, 6-methylguanine, 6-ethyladenine, 6-ethylguanine, 2-propyladenine, 2-propylguanine, 6-carboxyuracile, N⁶-methyl-adenine, 5-halouracil, 5-halocytosine, 5-propynyl uracil, 5-propynyl cytosine, 6-azo uracil, 6-azo cytosine, 6-azo thymine, 5-uracil (pseudouracil), 4-thiouracil, 8-halo-, 8-amino-, 8-thiol-, 8-thioalkyl-, 8-hydroxyl- and other 8-substituted adenines and -guanine, 5-halo-particularly 5-bromo-, 5-trifluoromethyl- and other 5-substituted uracils and -cytosines, 7-methylguanine, 7-methyladenine, 8-azaguanine, 8-azaadenine, 7-deazaguanine, 7-deazaadenine, 3-deazaguanine, 3-deazaadenine, 2-thiouracil, 2-thiothymine and 2-thiocytosine etc., with 5-methylcytosine and/or 5-methyldeoxycytosine substitutions being preferred since these modifications have been shown to increase nucleic acid duplex stability.

Preferred antisense-oligonucleotides of the present invention can comprise analogues of nucleobases. The nucleobase of only one nucleotide unit of the antisense-oligonucleotide could be replaced by an analogue of a nucleobase or two, three, four, five or even all nucleobases in an antisense-oligonucleotide could be replaced by analogues of nucleobases, such as uracile (U), 5-halouracil, 5-methylcytosine, or N⁶-methyl-adenine. Preferably the LNA units might be connected to analogues of nucleobases. Especially preferred are antisense-oligonucleotides of the Seq. ID No.s 260 - 310, wherein one, two, three or all nucleobases are substituted by the above-mentioned analogues of nucleobases.

The antisense-oligonucleotides of the present invention are complementary sequences to the mRNA, i.e. antisense-sequences of sequences of Seq. ID No. 2, for example, within the following areas of Seq. ID No. 2
starting with the nucleotide 69 and ending with the nucleotide 150,
starting with the nucleotide 331 and ending with the nucleotide 424,
starting with the nucleotide 402 and ending with the nucleotide 499,
starting with the nucleotide 921 and ending with the nucleotide 990,
starting with the nucleotide 1367 and ending with the nucleotide 1435,
starting with the nucleotide 1785 and ending with the nucleotide 1892,
starting with the nucleotide 2044 and ending with the nucleotide 2112,
starting with the nucleotide 2264 and ending with the nucleotide 2325,
starting with the nucleotide 2335 and ending with the nucleotide 2395,
starting with the nucleotide 4100 and ending with the nucleotide 4162 or
starting with the nucleotide 4197 and ending with the nucleotide 4257.

Within these ranges the first nucleotide such as nucleotide 331 as well as the last nucleotide such as nucleotide 424 are included so that this range or subsequence starting with the nucleotide 331 and ending with the nucleotide 424 consists of the sequence from nucleotide 331 to nucleotide 424.

As an example, the subsequence of Seq. ID No. 2 from nucleotide 331 to nucleotide 424 consists of 94 nucleotides and reads as follows:

GCGCCGTCCGCTGCGCTGGGGGCTCGGTCTATGACGAGCAGCGGGGTCTG
CCATGGGTCGGGGGCTGCTCAGGGGCCTGTGGCCGCTGCACATC
331
424

The antisense-oligonucleotides of the present invention bind or hybridize to the complementary sequence within these areas. If, for instance, the area of Seq. ID No. 2 where the antisense-oligonucleotide shall bind or hybridize is defined as the area from nucleotide 331 to 424, the antisense-oligonucleotides consisting of, e.g. 10 to 20 nucleotides can bind or hybridize anywhere within this area. If, for instance, the antisense-oligonucleotide has a length of 18 nucleotides, this antisense-oligonucleotide binds or hybridizes to the complementary sequence within the area from nucleotide 331 to 424 which are the following subsequences:
starting with nucleotide 331 and extending over 18 nucleotides thus ending at nucleotide 348 (GCGCCGTCCGCTGCGCTG) or
starting with nucleotide 332 and extending over 18 nucleotides thus ending at nucleotide 349 (CGCCGTCCGCTGCGCTGG) or
starting with nucleotide 333 and extending over 18 nucleotides thus ending at nucleotide 350 (GCCGTCCGCTGCGCTGGG) or
   and so on until
starting with nucleotide 407 and extending over 18 nucleotides thus ending at nucleotide 424 (CTGTGGCCGCTGCACATC).

It will be recognized that when referring to a sequence of nucleotides or monomers, what is referred to, is the sequence of bases, such as A, T, G, C or U.

The antisense-oligonucleotides as well as the salts of the antisense-oligonucleotides as disclosed herein have been proven to be complementary to the target which is the gene encoding for the TGF-R_{II} or the mRNA encoding the TGF-R_{II}, i.e., hybridize sufficiently well and with sufficient specificity to give the desired inhibitory effect.

The term **"salt"** refers to physiologically and/or pharmaceutically acceptable salts of the antisense-oligonucleotides of the present invention. The antisense-oligonucleotides contain nucleobases like adenine, guanine, thymine, cytosine or derivatives thereof which are basic and which form a salt like a chloride or mesylate salt. The internucleotide linkage preferably contains a negatively charged oxygen or sulfur atom which form salts like the sodium, lithium or potassium salt. Thus, pharmaceutically acceptable base addition salts are formed with inorganic bases or organic bases. Examples for suitable organic and inorganic bases are bases derived from metal ions, e.g., aluminum, alkali metal ions, such as sodium or potassium, alkaline earth metal ions such as calcium or magnesium, or an amine salt ion or alkali- or alkaline-earth hydroxides, -carbonates or -bicarbonates. Examples include aqueous LiOH, NaOH, KOH, NH₄OH, potassium carbonate, ammonia and sodium bicarbonate, ammonium salts, primary, secondary and tertiary amines, such as, e.g., tetraalkylammonium hydroxide, lower alkylamines such as methylamine, t-butylamine, procaine, ethanolamine, arylalkylamines such as dibenzylamine and N,N-dibenzylethylenediamine, lower alkylpiperidines such as N-ethylpiperidine, cycloalkylamines such as cyclohexylamine or dicyclohexylamine, morpholine, glucamine, N-methyl- and N,N-dimethylglucamine, 1-adamantylamine, benzathine, or salts derived from amino acids like arginine, lysine, ornithine or amides of originally neutral or acidic amino acids, chloroprocaine, choline, procaine or the like.

Since the antisense-oligonucleotides are basic, they form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphersulfonic acid, china acid, mandelic acid, o-methylmandelic acid, hydrogen-benzenesulfonic acid, picric acid, adipic acid, D-o-tolyltartaric acid, tartronic acid, α-toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

In the context of this invention, **"hybridization"** means hydrogen bonding, which may be Watson-Crick, Hoogsteen or reversed Hoogsteen hydrogen bonding, between complementary nucleoside or nucleotide bases. As used herein, the term "complementary" refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense compound does not need to be 100% complementary to that of its target nucleic acid to be specifically hybridizable although a 100% complementarity is preferred. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired, i.e., in the case of therapeutic treatment.

### Specificity

The antisense-oligonucleotides of the present invention have in common that they are specific in regard to the region where they bind to the gene or to the mRNA encoding TGF-R_{II}. There is no other region in the complete human transcriptome where these antisense-oligonucleotides could bind with 100% complementarity. Thus the antisense-oligonucleotides of the present invention are highly specific concerning hybridization with the gene or with the mRNA encoding TGF-R_{II}. The antisense-oligonucleotides bind with 100% complementarity specific to the gene encoding TGF-R_{II} or to the mRNA encoding TGF-R_{II} and do not bind to any other region in the complete human transcriptome.

The term **"mRNA",** as used herein, may encompass both mRNA containing introns (also refered to as pre-mRNA) as well as mRNA which does not contain any introns.

The antisense-oligonucleotides of the present invention are able to bind or hybridize with the Pre-mRNA and/or with the mRNA. That means the antisense-oligonucleotides can bind to or hybridize at an intron region or within an intron region of the Pre-mRNA or can bind to or hybridize at an overlapping intron - exon region of the Pre-mRNA or can bind to or hybridize at an exon region or within an exon region of the Pre-mRNA and the exon region of the mRNA (see Fig. 1). Preferred are antisense-oligonucleotides which are able to bind to or hybridize with Pre-mRNA and mRNA. Binding or hybridization of the antisense-oligonucleotides (ASO) to the Pre-mRNA inhibits the 5' cap formation, inhibits splicing of the Pre-mRNA in order to obtain the mRNA and activates RNase H which cleaves the Pre-mRNA. Binding or hybridization of the antisense-oligonucleotides (ASO) to the mRNA activates RNase H which cleaves the mRNA and inhibits binding of the ribosomal subunits.

The antisense-oligonucleotides of the present invention consist of at least 10 and no more than 20 nucleotides and consequently consist of 10, 11, 12, 13, 14, 15, 16, 17, 18, 19, or 20 nucleotides, preferably of 11 to 19 nucleotides and more preferably of 12 to 18 nucleotides, wherein at least two of these nucleotides are locked nucleic acis (LNA). Shorter antisense-oligonucleotides, i.e. antisense-oligonucleotides having less than 10 nucleotides are also possible but the shorter the antisense-oligonucleotides the higher the risk that the hybridization is not sufficiently strong anymore and that selectivity will decrease or will get lost. Non-selective antisense-oligonucleotides bear the risk to bind to undesired regions in the human transcriptome and to undesired mRNAs coding for other proteins than TFG-R_{II} thereby causing undesired side effects. Longer antisense-oligonucleotides having more than 20 nucleotides are also possible but further increasing the length make the synthesis of such antisense-oligonucleotides even more complicated and expensive without any further benefit in increasing selectivity or strength of hybridization or better stability in regard to degradation.

Thus the present invention is directed to antisense-oligonucleotides consisting of 10 to 20 nucleotides, wherein at least two nucleotides and preferably the 3' and 5' terminal nucleotides are LNAs. Thus it is preferred that at least the terminal 3' nucleotide is an LNA and also at least the 5' terminal nucleotide is an LNA. In case more than 2 LNAs are present, it is preferred that the further LNAs are linked to the 3' or 5' terminal LNA like it is the case in gapmers as disclosed herein.

One nucleotide building block present in an antisense-oligonucleotide of the present invention can be represented by the following general formula (B1) and (B2): 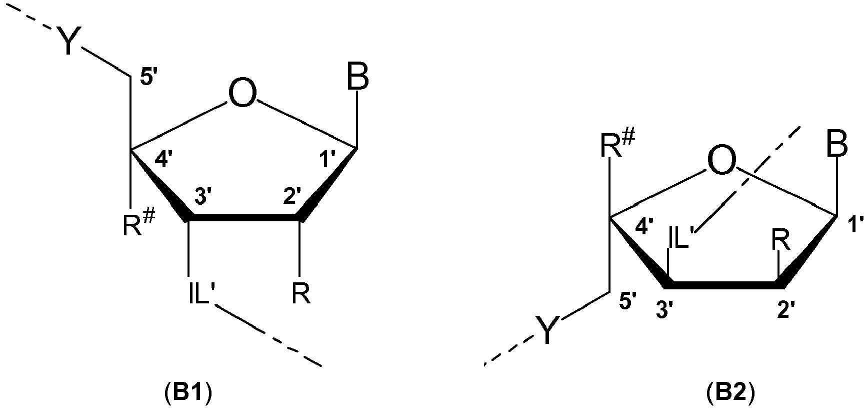 wherein
B represents a nucleobase;
IL' represents -X"-P(=X')(X)⁻;
R represents -H, -F, -OH, -NH₂, -OCH₃, -OCH₂CH₂OCH₃ and R⁴ represents -H; or R and R⁴ form together the bridge -R^{#}-R- which is selected from -CH₂-O-, -CH₂-S-, -CH₂-NH-, -CH₂-N(CH₃)-, -CH₂-N(C₂H₅)-, -CH₂-CH₂-O-, -CH₂-CH₂-S-, -CH₂-CH₂-NH-, -CH₂-CH₂-N(CH₃)-, or -CH₂-CH₂-N(C₂H₅)-;
X' represents =O or =S;
X⁻ represents -O⁻, -OH, -OR^{H}, -NHR^{H}, -N(R^{H})₂, -OCH₂CH₂OR^{H}, -OCH₂CH₂SR^{H}, -BH₃⁻, -R^{H}, -SH, -SR^{H}, or -S⁻;
X" represents -O-, -NH-, -NR^{H}-, -CH₂-, or -S-;
Y is -O-, -NH-, -NR^{H}-, -CH₂- or -S-;
R^{H} is selected from hydrogen and C₁₋₄-alkyl and preferably -CH₃ or -C₂H₅ and most preferably -CH₃.

Preferably X⁻ represents -O⁻, -OH, -OCH₃, -NH(CH₃), -N(CH₃)₂, -OCH₂CH₂OCH₃, -OCH₂CH₂SCH₃, -BH₃⁻, -CH₃, -SH, -SCH₃, or -S⁻; and more preferably -O⁻, -OH, -OCH₃, -N(CH₃)₂, -OCH₂CH₂OCH₃, -BH₃⁻, -SH, -SCH₃, or -S⁻.

IL' represents preferably -O-P(O)(O⁻), -O-P(O)(S⁻)-, -O-P(S)(S⁻)-, -S-P(O)(O⁻)-, -S-P(O)(S⁻)-, -S-P(S)(S⁻)-, -O-P(O)(O⁻)-, -O-P(O)(S⁻)-, -S-P(O)(O⁻)-, -O-P(O)(R^{H})-, -O-P(O)(OR^{H})-, -O-P(O)(NHR^{H})-, -O-P(O)[N(R^{H})₂]-, -O-P(O)(BH₃⁻)-, -O-P(O)(OCH₂CH₂OR^{H})-, -O-P(O)(OCH₂CH₂SR^{H})-, -O-P(O)(O⁻)-, -NR^{H}-P(O)(O⁻)-, wherein R^{H} is selected from hydrogen and C₁₋₄-alkyl.

The group -O-P(O)(R^{H})-O- is preferably -O-P(O)(CH₃)-O- or -O-P(O)(C₂H₅)-O-and most preferably -O-P(O)(CH₃)-O-.

The group -O-P(O)(OR^{H})-O- is preferably -O-P(O)(OCH₃)-O- or -O-P(O)(OC₂H₅)-O- and most preferably -O-P(O)(OCH₃)-O-.

The group -O-P(O)(NHR^{H})-O- is preferably -O-P(O)(NHCH₃)-O- or -OP(O)(NHC₂H₅)-O- and most preferably -O-P(O)(NHCH₃)-O-.

The group -O-P(O)[N(R^{H})₂]-O- is preferably -O-P(O)[N(CH₃)₂]-O- or -O-P(O)[N(C₂H₅)₂]-O- and most preferably -O-P(O)[N(CH₃)₂]-O-.

The group -O-P(O)(OCH₂CH₂OR^{H})-O- is preferably -O-P(O)(OCH₂CH₂OCH₃)-O- or -O-P(O)(OCH₂CH₂OC₂H₅)-O- and most preferably -O-P(O)(OCH₂CH₂OCH₃)-O-. The group -O-P(O)(OCH₂CH₂SR^{H})-O- is preferably -O-P(O)(OCH₂CH₂SCH₃)-O- or -O-P(O)(OCH₂CH₂SC₂H₅)-O- and most preferably -O-P(O)(OCH₂CH₂SCH₃)-O-. The group -O-P(O)(O⁻)-NR^{H}- is preferably -O-P(O)(O⁻)-NH- or -O-P(O)(O⁻)-N(CH₃)- and most preferably -O-P(O)(O⁻)-NH-.

The group -NR^{H}-P(O)(O⁻)-O- is preferably -NH-P(O)(O⁻)-O- or -N(CH₃)-P(O)(O⁻)-O- and most preferably -NH-P(O)(O⁻)-O-.

Even more preferably IL' represents -O-P(O)(O⁻)-, -O-P(O)(S⁻)-, -O-P(S)(S⁻)-, -O-P(O)(NHR^{H})-, or -O-P(O)[N(R^{H})₂]-, and still more preferably IL' represents -O-P(O)(O⁻)-, -O-P(O)(S⁻)-, or -O-P(S)(S⁻)-, and most preferably IL' represents -O-P(O)(S⁻)-, or -O-P(S)(S⁻)-.

Preferably Y represents -O-.

Preferably B represents a standard nucleobase selected from A, T, G, C.

Preferably IL represents -O-P(=O)(S⁻)- or -O-P(=S)(S^{H})-.

The above definitions of B, Y and IL' apply also to the formula b¹ to b⁶.

Thus the following general formula (B3) to (B6) are preferred: 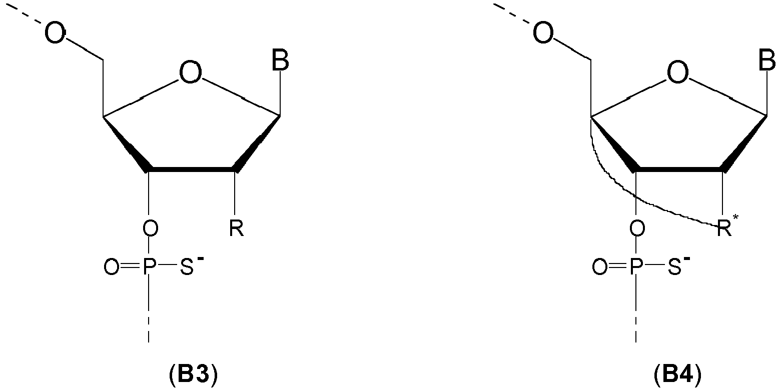 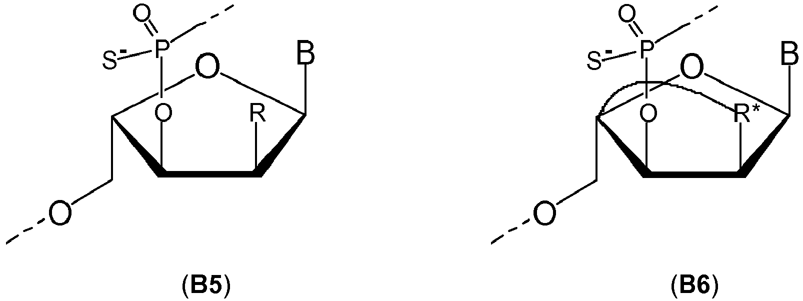 wherein
B represents a nucleobase and preferably A, T, G, C;
R represents -H, -F, -OH, -NH₂, -N(CH₃)₂, -OCH₃, -OCH₂CH₂OCH₃, -OCH₂CH₂CH₂OH, -OCH₂CH₂CH₂NH₂ and preferably -H;
R^{*} represents the moiety -R^{#}-R- as defined below and is, for instance, preferably selected from -C(R^{a}R^{b})-O-, -C(R^{a}R^{b})-NR^{c}-, -C(R^{a}R^{b})-S-, and -C(R^{a}R^{b})-C(R^{a}R^{b})-O-, wherein the substituents R^{a}, R^{b} and R^{c} have the meanings as defined herein. More preferably R^{*} is selected from -CH₂-O-, -CH₂-S-, -CH₂-NH-, -CH₂-N(CH₃)-, -CH₂-CH₂-O-, or -CH₂-CH₂-S-, and more preferably -CH₂-O-, -CH₂-S-, -CH₂-CH₂-O-, or -CH₂-CH₂-S-, and still more preferably -CH₂-O-, -CH₂-S-, or -CH₂-CH₂-O-, and still more preferably -CH₂-O- or -CH₂-S-, and most preferably -CH₂-O-.

Examples of preferred nucleotides which are non-LNA units are the following:

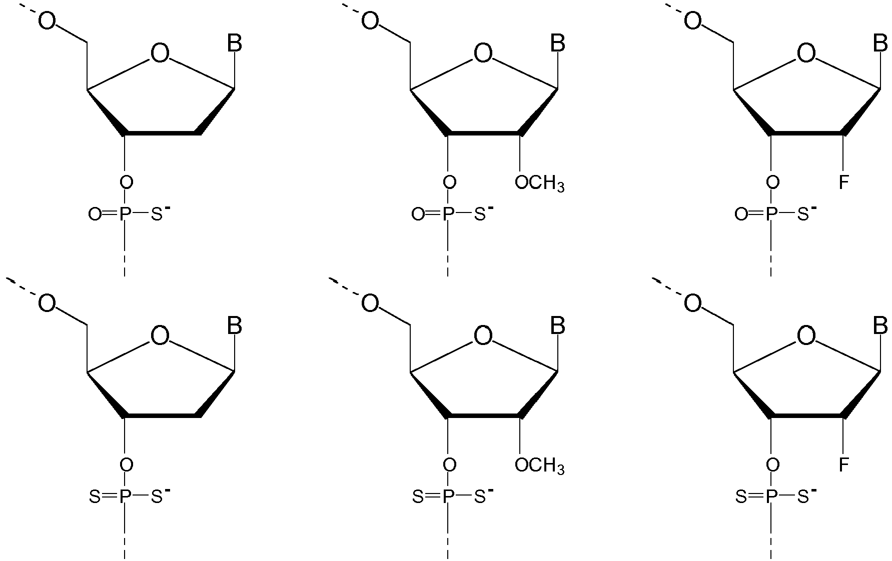

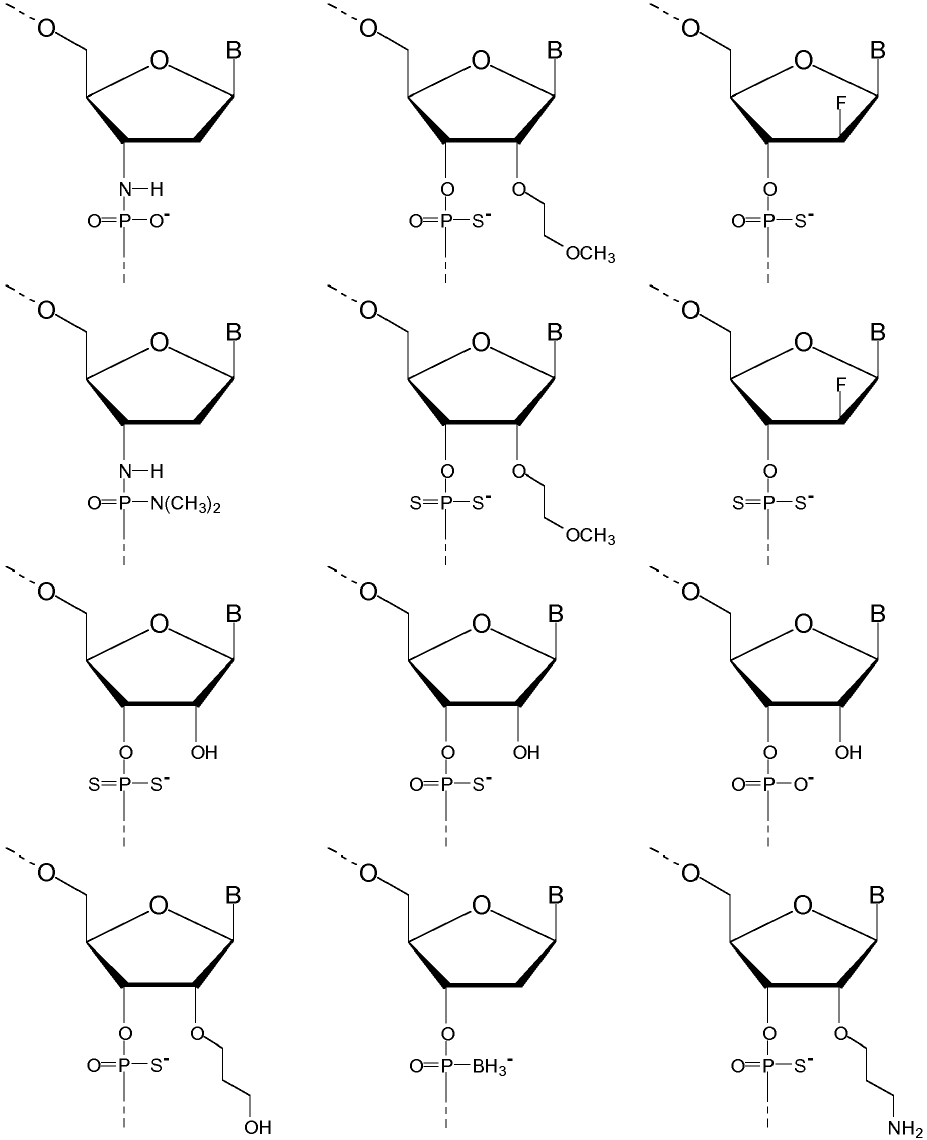

### Internucleotide Linkages (IL)

The monomers of the antisense-oligonucleotides described herein are coupled together via an internucleotide linkage. Suitably, each monomer is linked to the 3' adjacent monomer via an internucleotide linkage. The person having ordinary skill in the art would understand that, in the context of the present invention, the 5' monomer at the end of an oligomer does not comprise a 5' internucleotide linkage, although it may or may not comprise a 5' terminal group. The term "internucleotide linkage" is intended to mean a group capable of covalently coupling together two nucleotides, two nucleotide analogues like two LNAs, and a nucleotide and a nucleotide analogue like an LNA. Specific and preferred examples include phosphate groups and phosphorothioate groups.

The nucleotides of the antisense-oligonucleotides of the present invention or contiguous nucleotide sequences thereof are coupled together via internucleotide linkages. Suitably each nucleotide is linked through the 5' position to the 3' adjacent nucleotide via an internucleotide linkage.

The antisense-oligonucleotides can be modified by several different ways. Modifications within the backbone are possible and refer to antisense-oligonucleotides wherein the phosphate groups (also named phosphodiester groups) in their internucleotide backbone are partially or completely replaced by other groups. Preferred modified antisense-oligonucleotide backbones include, for instance, phosphorothioates, chiral phorphorothioates, phosphorodithioates, phosphotriester, aminoalkylphosphotriesters, methyl, ethyl and C₃-C₁₀-alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters and boranophosphates having normal 3'-5' linkages, 2'-5' linked analogues of these, and those having inverted polarity wherein the adjacent pairs of nucleotide units are linked 3'-5' to 5'-3' or 2'-5' to 5'-2'. Various salts, mixed salts and free acids forms thereof are also included and disclosed herein in further detail.

Suitable internucleotide linkages include those listed within WO2007/031091, for example the internucleotide linkages listed on the first paragraph of page 34 of WO2007/031091 (hereby incorporated by reference). It is, in some embodiments, preferred to modify the internucleotide linkage from its normal phosphodiester to one that is more resistant to nuclease attack, such as phosphorothioate or boranophosphate - these two, being cleavable by RNase H, also allow that route of antisense inhibition in reducing the expression of the target gene.

The internucleotide linkage consists of the group IL' which is the group bound to the 3' carbon atom of the ribose moiety and the group Y which is the group bound to the 5' carbon atom of the contiguous ribose moiety as shown in the formula (IL'Y) below

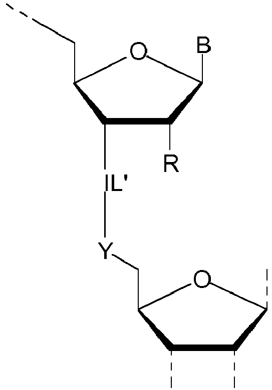

The internucleotide linkage IL is represented by -IL'-Y-. IL' represents -X"-P(=X')(X⁻)- so that IL is represented by -X"-P(=X')(X⁻)-Y- wherein the substituents X⁻, X', X" and Y have the meanings as disclosed herein.

The internucleotide linkage IL = -X"-P(=X')(X⁻)-Y- is preferably selected form the group consisting of:
- O-P(O)(O⁻)-O-, -O-P(O)(S⁻)-O-, -O-P(S)(S⁻)-O-, -S-P(O)(O⁻)-O-, -S-P(O)(S⁻)-O-, -S-P(S)(S⁻)-O-, -O-P(O)(O⁻)-S-, -O-P(O)(S⁻)-S-, -S-P(O)(O⁻)-S-, -O-P(O)(R^{H})-O-, -O-P(O)(OR^{H})-O-, -O-P(O)(NHR^{H})-O-, -O-P(O)[N(R^{H})₂]-O-, -O-P(O)(BH₃⁻)-O-, -O-P(O)(OCH₂CH₂OR^{H})-O-, -O-P(O)(OCH₂CH₂SR^{H})-O-, -O-P(O)(O⁻)-NR^{H}-, -NR^{H}-P(O)(O⁻)-O-, where R^{H} is selected from hydrogen and C1-4-alkyl.

The group -O-P(O)(R^{H})-O- is preferably -O-P(O)(CH₃)-O- or -O-P(O)(C₂H₅)-O-and most preferably -O-P(O)(CH₃)-O-.

The group -O-P(O)(OR^{H})-O- is preferably -O-P(O)(OCH₃)-O- or -O-P(O)(OC₂H₅)-O-and most preferably -O-P(O)(OCH₃)-O-.

The group -O-P(O)(NHR^{H})-O- is preferably -O-P(O)(NHCH₃)-O- or -O-P(O)(NHC₂H₅)-O- and most preferably -O-P(O)(NHCH₃)-O-.

The group -O-P(O)[N(R^{H})₂]-O- is preferably -O-P(O)[N(CH₃)₂]-O- or -O-P(O)[N(C₂H₅)₂]-O- and most preferably -O-P(O)[N(CH₃)₂]-O-.

The group -O-P(O)(OCH₂CH₂OR^{H})-O- is preferably -O-P(O)(OCH₂CH₂OCH₃)-O- or -O-P(O)(OCH₂CH₂OC₂H₅)-O- and most preferably -O-P(O)(OCH₂CH₂OCH₃)-O-.

The group -O-P(O)(OCH₂CH₂SR^{H})-O- is preferably -O-P(O)(OCH₂CH₂SCH₃)-O- or -O-P(O)(OCH₂CH₂SC₂H₅)-O- and most preferably -O-P(O)(OCH₂CH₂SCH₃)-O-.

The group -O-P(O)(O⁻)-NR^{H}- is preferably -O-P(O)(O⁻)-NH- or -O-P(O)(O⁻)-N(CH₃)- and most preferably -O-P(O)(O⁻)-NH-.

The group -NR^{H}-P(O)(O⁻)-O- is preferably -NH-P(O)(O⁻)-O- or -N(CH₃)-P(O)(O⁻)-O-and most preferably -NH-P(O)(O⁻)-O-.

Even more preferably IL represents -O-P(O)(O⁻)-O-, -O-P(O)(S⁻)-O-, -O-P(S)(S⁻)-O-, -O-P(O)(NHR^{H})-O-, or -O-P(O)[N(R^{H})₂]-O-, and still more preferably IL represents -O-P(O)(O⁻)-O-, -O-P(O)(S⁻)-O-, or -O-P(S)(S⁻)-O-, and most preferably IL represents -O-P(O)(S⁻)-O-, or -O-P(S)(S⁻)-O-.

Thus IL is preferably a phosphate group (-O-P(O)(O⁻)-O-), a phosphorothioate group (-O-P(O)(S⁻)-O-) or a phosphorodithioate group (-O-P(S)(S⁻)-O-).

The nucleotide units or the nucleosides of the antisense-oligonucleotides are connected to each other by internucleotide linkages so that within one antisense-oligonucleotide different internucleotide linkages can be present. The LNA units are preferably linked by internucleotide linkages which are not phosphate groups. The LNA units are linked to each other by a group IL which is preferably selected from -O-P(O)(S⁻)-O-, -O-P(S)(S⁻)-O-, -O-P(O)(NHR^{H})-O-, and -O-P(O)[N(R^{H})₂]-O- and more preferably from -O-P(O)(S⁻)-O- and -O-P(S)(S⁻)-O-.

The non-LNA units are linked to each other by a group IL which is preferably selected from -O-P(O)(O⁻)-O-, -O-P(O)(S⁻)-O-, -O-P(S)(S⁻)-O-, -O-P(O)(NHR^{H})-O-, and -O-P(O)[N(R^{H})₂]-O- and more preferably from -O-P(O)(O⁻)-O-, -O-P(O)(S⁻)-O- and -O-P(S)(S⁻)-O-.

A non-LNA unit is linked to an LNA unit by a group IL which is preferably selected from -O-P(O)(S⁻)-O-, -O-P(S)(S⁻)-O-, -O-P(O)(NHR^{H})-O-, and -O-P(O)[N(R^{H})₂]-O-and more preferably from -O-P(O)(S⁻)-O- and -O-P(S)(S⁻)-O-.

The term **"LNA unit"** as used herein refers to a nucleotide which is locked, i.e. to a nucleotide which has a bicyclic structure and especially a bicyclic ribose structure and more especially a bicyclic ribose structure as shown in general formula (II).

The term **"non-LNA unit"** as used herein refers to a nucleotide which is not locked, i.e. to a nucleotide which has no bicyclic structure and especially no bicyclic ribose structure and more especially no bicyclic ribose structure as shown in general formula (II).

The term **"unit"** as used herein refers to a part or a fragment or a moiety of an antisense-oligonucleotide of the present invention. Thus a "unit" is not a complete molecule, it is a part or a fragment or a moiety of an antisense-oligonucleotide which has at least one position for a covalent linkage to another part or fragment or moiety of the antisense-oligonucleotide. For example, the general structures **(B1)** to **(B6)** are units, because they can be covalently linked through the group Y and IL' or O and O-P(O)(S⁻) respectively. Preferably a unit is a moiety consisting of a pentose structure, a nucleobase connected to the pentose structure a 5' radical group and an IL' radical group.

The term **"building block"** or **"monomer"** as used herein refers to a molecule and especially to a nucleoside which is used in the synthesis of an antisense-oligonucleotide of the present invention. Examples are the LNA molecules of general formula (I), wherein Y represents a 5'-terminal group and IL' represents a 3'-terminal group.

Suitable sulphur (S) containing internucleotide linkages as provided herein are preferred.

Furthermore, pure diastereomeric antisense-oligonucleotides are preferred. Preferred are Sp- and Rp-diastereomers as shown below:

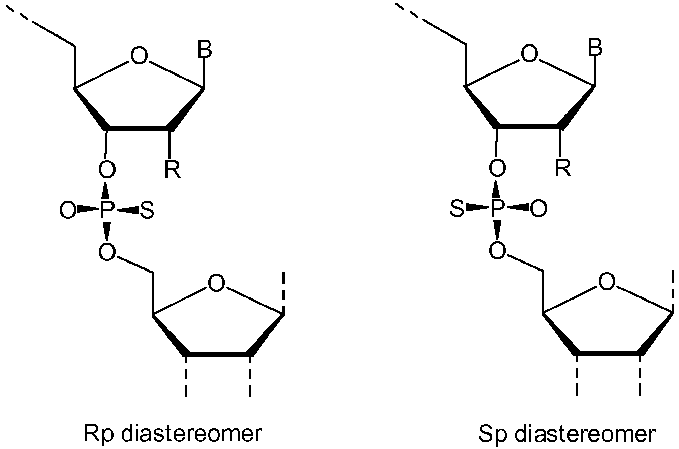

Preferred are phosphorothioate moieties in the backbone where at least 50% of the internucleotide linkages are phosphorothioate groups. Also preferred is that the LNA units, if present, are linked through phosphorothioates as internucleotide linkages. Most preferred is a complete phosphorothioate backbone, i.e. most preferred is when all nucleotide units and also the LNA units (if present) are linked to each other through phosphorothioate groups which are defined as follows: -O-P(O)(S⁻)-O-which is synonymous to -O-P(O,S)-O- or to -O-P(O⁻)(S)-O-.

In case the antisense-oligonucleotide is a **gapmer,** it is preferred that the LNA regions have internucleotide linkages selected from -O-P(O)(S⁻)-O- and -O-P(S) (S⁻)-O- and that the non-LNA region, the middle part, has internucleotide linkages selected from -O-P(O)(O⁻)-O-, -O-P(O)(S⁻)-O- and -O-P(S)(S⁻)-O- and that the LNA regions are connected to the non-LNA region through internucleotide linkages selected from -O-P(O)(O⁻)-O-, -O-P(O)(S⁻)-O- and -O-P(S)(S⁻)-O-.

It is even more preferred if all internucleotide linkages which are 9 in a 10-mer and 19 in a 20-mer are selected from -O-P(O)(S⁻)-O- and -O-P(S)(S⁻)-O-. Still more preferred is that all internucleotide linkages are phosphorothioate groups (-O-P(O)(S⁻)-O-) or are phosphorodithioate groups (-O-P(S)(S⁻)-O-).

### Locked Nucleic Acids (LNA^{®})

It is especially preferred that some of the nucleotides of the general formula **(B1)** or **(B2)** in the antisense-oligonucleotides are replaced by so-called LNAs (Locked Nucleic Acids). The abbreviation LNA is a registered trademark, but herein the term "LNA" is solely used in a descriptive manner.

Preferably the terminal nucleotides are replaced by LNAs and more preferred the last 1 to 4 nucleotides at the 3' end and/or the last 1 to 4 nucleotides at the 5' end are replaced by LNAs. It is also preferred to have at least the terminal nucleotide at the 3' end and at the 5' end replaced by an LNA each.

The term **"LNA"** as used herein, refers to a bicyclic nucleotide analogue, known as "Locked Nucleic Acid". It may refer to an LNA monomer, or, when used in the context of an "LNA antisense-oligonucleotide" or an "antisense-oligonucleotide containing LNAs", LNA refers to an oligonucleotide containing one or more such bicyclic nucleotide analogues. LNA nucleotides are characterized by the presence of a linker group (such as a bridge) between C2' and C4' of the ribose sugar ring - for example as shown as the biradical R^{#} - R as described below. The LNA used in the antisense-oligonucleotides of the present invention preferably has the structure of the general formula (I) 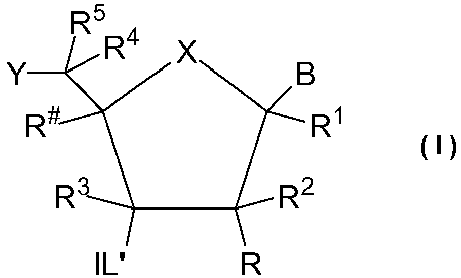 wherein for all chiral centers, asymmetric groups may be found in either R or S orientation;
wherein X is selected from -O-, -S-, -N(R^{N})-, -C(R⁶R⁷)-, and preferably X is -O-;
B is selected from hydrogen, optionally substituted C₁₋₄-alkoxy, optionally substituted C₁₋₄-alkyl, optionally substituted C₁₋₄-acyloxy, nucleobases and nucleobase analogues, and preferably B is a nucleobase or a nucleobase analogue and most preferred a standard nucleobase;
Y represents a part of an internucleotide linkage to an adjacent nucleotide in case the moiety of general formula (I) is an LNA unit of an antisense-oligonucleotide of the present invention, or a **5'-terminal group** in case the moiety of general formula (I) is a monomer or building block for synthesizing an antisense-oligonucleotide of the present invention. The 5' carbon atom optionally includes the substituent R⁴ and R⁵;
IL' represents a part of an internucleotide linkage to an adjacent nucleotide in case the moiety of general formula (I) is an LNA unit of an antisense-oligonucleotide of the present invention, or a **3'-terminal group** in case the moiety of general formula (I) is a monomer or building block for synthesizing an antisense-oligonucleotide of the present invention.
R^{#} and R together represent a bivalent linker group consisting of 1 - 4 groups or atoms selected from -C(R^{a}R^{b})-, -C(R^{a})=C(R^{b})-, -C(R^{a})=N-, -O-, -Si(R^{a})₂-, -S-, -SO₂-, -N(R^{c})- and >C=Z, wherein Z is selected from -O-, -S-, and -N(R^{a})-, and R^{a}, R^{b} and R^{c} are independently of each other selected from hydrogen, optionally substituted C₁₋₁₂-alkyl, optionally substituted C₂₋₆-alkenyl, optionally substituted C₂-₆-alkynyl, hydroxy, optionally substituted C₁₋₁₂-alkoxy, C₁₋₆-alkoxy-C₁₋₆-alkyl, C₂₋₆-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkylenyl-aminocarbonyl, mono-and di(C₁₋₆-alkyl)amino-C₁₋₆-alkylenyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, where aryl and heteroaryl may be optionally substituted and where two geminal substituents R^{a} and R^{b} together may represent optionally substituted methylene (=CH2), wherein for all chiral centers, asymmetric groups may be found in either R or S orientation, and;
each of the substituents R¹, R², R³, R⁴, R⁵, R⁶ and R⁷, which are present is independently selected from hydrogen, optionally substituted C₁₋₁₂-alkyl, optionally substituted C₂₋₆-alkenyl, optionally substituted C₂-₆-alkynyl, hydroxy, C₁₋₁₂-alkoxy, C₁₋₆-alkoxy-C₁-₆-alkyl, C₂₋₆-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, where aryl and heteroaryl may be optionally substituted, and where two geminal substituents together may designate oxo, thioxo, imino, or optionally substituted methylene;
wherein R^{N} is selected from hydrogen and C₁₋₄-alkyl, and where two adjacent (non-geminal) substituents may designate an additional bond resulting in a double bond; and R^{N}, when present and not involved in a biradical, is selected from hydrogen and C₁₋₄-alkyl; and basic salts and acid addition salts thereof. For all chiral centers, asymmetric groups may be found in either R or S orientation.

In preferred embodiments, R^{#} and R together represent a biradical consisting of a groups selected from the group consisting of -C(R^{a}R^{b})-C(R^{a}R^{b})-, -C(R^{a}R^{b})-O-, -C(R^{a}R^{b})-NR^{c}-, -C(R^{a}R^{b})-S-, and -C(R^{a}R^{b})-C(R^{a}R^{b})-O-, wherein each R^{a}, R^{b} and R^{c} may optionally be independently selected.

In some embodiments, R^{a} and R^{b} may be, optionally independently selected from the group consisting of hydrogen and C₁₋₆-alkyl, such as methyl, and preferred is hydrogen.

In preferred embodiments, R¹, R², R³, R⁴, and R⁵ are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂-₆-alkynyl, C₁₋₆-alkoxyl, substituted C₁₋₆-alkoxyl, acyl, substituted acyl, C₁₋₆-aminoalkyl or substituted C₁₋₆-aminoalkyl. For all chiral centers, asymmetric groups may be found in either R or S orientation.

In preferred embodiments R¹, R², R³, R⁴, and R⁵ are hydrogen.

In some embodiments, R¹, R², and R³, are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂-₆-alkynyl, C₁₋₆-alkoxyl, substituted C₁₋₆-alkoxyl, acyl, substituted acyl, C₁₋₆-aminoalkyl or substituted C₁₋₆-aminoalkyl. For all chiral centers, asymmetric groups may be found in either R or S orientation. In preferred embodiments R¹, R², and R³ are hydrogen.

In preferred embodiments, R⁴ and R⁵ are each independently selected from the group consisting of -H, -CH₃, -CH₂-CH₃, -CH₂-O-CH₃, and -CH=CH₂. Suitably in some embodiments, either R⁴ or R⁵ are hydrogen, whereas the other group (R⁴ or R⁵ respectively) is selected from the group consisting of C₁₋₆-alkyl, C₂₋₆-alkenyl, C₂₋₆-alkynyl, substituted C₁₋₆-alkyl, substituted C₂₋₆-alkenyl, substituted C₂₋₆-alkynyl or substituted acyl (-C(=O)-); wherein each substituted group is mono or poly substituted with substituent groups independently selected from halogen, C₁₋₆-alkyl, substituted C₁₋₆-alkyl, C₂₋₆-alkenyl, substituted C₂₋₆-alkenyl, C₂-₆-alkynyl, substituted C₂-₆-alkynyl, -OJ₁, -SJ₁, -NJ₁J₂, -N₃, -COOJ₁, -CN, -O-C(=O)NJ₁J₂, -N(H)C(=NH)NJ₁J₂ or -N(H)C(=X)N(H)J₂, wherein X is O or S; and each J₁ and J₂ is, independently -H, C₁₋₆-alkyl, substituted C₁₋₆-alkyl, C₂₋₆-alkenyl, substituted C₂₋₆-alkenyl, C₂-₆-alkynyl, substituted C₂-₆-alkynyl, C₁₋₆-aminoalkyl, substituted C₁₋₆-aminoalkyl or a protecting group. In some embodiments either R⁴ or R⁵ is substituted C₁₋₆-alkyl. In some embodiments either R⁴ or R⁵ is substituted methylene, wherein preferred substituent groups include one or more groups independently selected from -F, -NJ₁J₂, -N₃, -CN, -OJ₁, -SJ₁, -O-C(=O)NJ₁J₂, -N(H)C(=NH)NJ₁J₂ or -N(H)C(=O)N(H)J₂. In some embodiments each J₁ and J₂ is, independently -H or C₁₋₆-alkyl. In some embodiments either R⁴ or R⁵ is methyl, ethyl or methoxymethyl. In some embodiments either R⁴ or R⁵ is methyl. In a further embodiment either R⁴ or R⁵ is ethylenyl. In some embodiments either R⁴ or R⁵ is substituted acyl. In some embodiments either R⁴ or R⁵ is -O-C(=O)NJ₁J₂. For all chiral centers, asymmetric groups may be found in either R or S orientation. Such 5' modified bicyclic nucleotides are disclosed in WO 2007/134181 A, which is hereby incorporated by reference in its entirety.

In some embodiments B is a nucleobase, including nucleobase analogues and naturally occurring nucleobases, such as a purine or pyrimidine, or a substituted purine or substituted pyrimidine, such as a nucleobase referred to herein, such as a nucleobase selected from the group consisting of adenine, cytosine, thymine, adenine, uracil, and/or a modified or substituted nucleobase, such as 5-thiazolo-uracil, 2-thio-uracil, 5-propynyl-uracil, 2'thio-thymine, 5-methyl cytosine, 5-thiozolo-cytosine, 5-propynyl-cytosine, and 2,6- diaminopurine.

In preferred embodiments, R^{#} and R together represent a biradical selected from -C(R^{a}R^{b})-O-, -C(R^{a}R^{b})-C(R^{c}R^{d})-O-, -C(R^{a}R^{b})-C(R^{c}R^{d})-C(R^{e}R^{f})-O--C(R^{a}R^{b})-O-C(R^{d}R^{e})-, -C(R^{a}R^{b})-O-C(R^{d}R^{e})-O-, -C(R^{a}R^{b})-C(R^{d}R^{e})-, -C(R^{a}R^{b})-C(R^{c}R^{d})-C(R^{e}R^{f})-, -C(R^{a})=C(R^{b})-C(R^{d}R^{e})-, -C(R^{a}R^{b})-N(R^{c})-, -C(R^{a}R^{b})-C(R^{d}R^{e})-N(R^{c})-, -C(R^{a}R^{b})-N(R^{c})-O-, -C(R^{a}R^{b})-S-, and -C(R^{a}R^{b})-C(R^{d}R^{e})-S-, wherein R^{a}, R^{b}, R^{c}, R^{d}, R^{e}, and R^{f} each is independently selected from hydrogen, optionally substituted C₁₋₁₂-alkyl, optionally substituted C₂₋₆-alkenyl, optionally substituted C₂-₆-alkynyl, hydroxy, C₁₋₁₂-alkoxy, C₁₋₆-alkoxy-C₁₋₆-alkyl, C₂₋₆-alkenyloxy, carboxy, C₁₋₁₂-alkoxycarbonyl C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, where aryl and heteroaryl may be optionally substituted and where two geminal substituents R^{a} and R^{b} together may designate optionally substituted methylene (=CH₂). For all chiral centers, asymmetric groups may be found in either *R* or *S* orientation.

In a further embodiment R^{#} and R together designate a biradical (bivalent group) selected from -CH₂-O-, -CH₂-S-, -CH₂-NH-, -CH₂-N(CH₃)-, -CH₂-CH₂-O-, -CH₂-CH(CH₃)-, -CH₂-CH₂-S-, -CH₂-CH₂-NH-, -CH₂-CH₂-CH₂-, -CH₂-CH₂-CH₂-O-, -CH₂-CH₂-CH(CH₃)-, -CH=CH-CH₂-, -CH₂-O-CH₂-O-, -CH₂-NH-O-, -CH₂-N(CH₃)-O-, -CH₂-O-CH₂-, -CH(CH₃)-O-, -CH(CH₂-O-CH₃)-O-, -CH₂-CH₂-, and -CH=CH-. For all chiral centers, asymmetric groups may be found in either *R* or *S* orientation.

In some embodiments, R^{#} and R together designate the biradical -C(R^{a}R^{b})-N(R^{c})-O-, wherein R^{a} and R^{b} are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂-₆-alkynyl, C₁₋₆-alkoxyl, substituted C₁₋₆-alkoxyl, acyl, substituted acyl, C₁₋₆-aminoalkyl or substituted C₁₋₆-aminoalkyl, such as hydrogen, and; wherein R^{c} is selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂-₆-alkynyl, C₁₋₆-alkoxyl, substituted C₁₋₆-alkoxyl, acyl, substituted acyl, C₁₋₆-aminoalkyl or substituted C₁₋₆-aminoalkyl, and preferably hydrogen.

In preferred embodiments, R^{#} and R together represent the biradical -C(R^{a}R^{b})-O-C(R^{d}R^{e})-O-, wherein R^{a}, R^{b}, R^{d}, and R^{e} are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂-₆-alkynyl, C₁₋₆-alkoxyl,substituted C₁₋₆-alkoxyl, acyl, substituted acyl, C₁₋₆-aminoalkyl or substituted C₁₋₆-aminoalkyl, and preferably hydrogen.

In preferred embodiments, R^{#} and R form the biradical -CH(Z)-O-, wherein Z is selected from the group consisting of C₁₋₆-alkyl, C₂₋₆-alkenyl, C₂-₆-alkynyl, substituted C₁₋₆-alkyl, substituted C₂₋₆-alkenyl, substituted C₂-₆-alkynyl, acyl, substituted acyl, substituted amide, thiol or substituted thio; and wherein each of the substituted groups, is, independently, mono or poly substituted with optionally protected substituent groups independently selected from halogen, oxo, hydroxyl, -OJ₁, -NJ₁J₂, -SJ₁, -N₃, -OC(=X)J₁, -OC(=X)NJ₁J₂, -NJ³C(=X)NJ₁J₂ and -CN, wherein each J₁, J₂ and J₃ is, independently, -H or C₁₋₆-alkyl, and X is O, S or NJ₁. In preferred embodiments Z is C₁-₆-alkyl or substituted C₁₋₆-alkyl. In further preferred embodiments Z is methyl. In preferred embodiments Z is substituted C₁₋₆-alkyl. In preferred embodiments said substituent group is C₁-₆-alkoxy. In some embodiments Z is CH₃OCH₂-. For all chiral centers, asymmetric groups may be found in either *R* or *S* orientation. Such bicyclic nucleotides are disclosed in US 7,399,845 which is hereby incorporated by reference in its entirety. In preferred embodiments, R¹, R², R³, R⁴, and R⁵ are hydrogen. In preferred embodiments, R¹, R², and R³ are hydrogen, and one or both of R⁴, R⁵ may be other than hydrogen as referred to above and in WO 2007/134181.

In preferred embodiments, R^{#} and R together represent a biradical which comprise a substituted amino group in the bridge such as the biradical -CH₂-N(R^{c})-, wherein R^{c} is C₁₋₁₂-alkyloxy. In preferred embodiments R^{#} and R together represent a biradical -Cq₃q₄-NOR-, wherein q₃ and q₄ are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂-₆-alkynyl, C₁₋₆-alkoxyl, substituted C₁₋₆-alkoxyl,acyl, substituted acyl, C₁₋₆-aminoalkyl or substituted C₁₋₆-aminoalkyl; wherein each substituted group is, independently, mono or poly substituted with substituent groups independently selected from halogen, -OJ₁, -SJ₁, -NJ₁J₂, -COOJ₁, -CN, -OC(=O)NJ₁J₂, -NH-C(=NH)NJ₁J₂ or -NH-C(=X)NHJ₂, wherein X is O or S; and each of J₁ and J₂ is, independently, -H, C₁₋₆-alkyl, C₂₋₆-alkenyl, C₂-₆-alkynyl, C₁₋₆-aminoalkyl or a protecting group. For all chiral centers, asymmetric groups may be found in either R or S orientation. Such bicyclic nucleotides are disclosed in WO2008/150729 which is hereby incorporated by reference in its entirety. In preferred embodiments, R¹, R², R³, R⁴, and R⁵ are independently selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂-₆-alkynyl, C₁₋₆-alkoxyl,substituted C₁₋₆-alkoxyl, acyl, substituted acyl, C₁₋₆-aminoalkyl or substituted C₁₋₆-aminoalkyl. In preferred embodiments, R¹, R², R³, R⁴, and R⁵ are hydrogen. In preferred embodiments, R¹, R², and R³ are hydrogen and one or both of R⁴, R⁵ may be other than hydrogen as referred to above and in WO 2007/134181.

In preferred embodiments R^{#} and R together represent a biradical (bivalent group)-C(R^{a}R^{b})-O-, wherein R^{a} and R^{b} are each independently halogen, C₁₋₁₂-alkyl, substituted C₁₋₁₂-alkyl, C₂₋₆-alkenyl, substituted C₂₋₆-alkenyl, C₂-₆-alkynyl, substituted C₂-₆-alkynyl, C₁₋₁₂-alkoxy, substituted C₁₋₁₂-alkoxy, -OJ₁, -SJ₁, -S(O)J₁, -SO₂-J₁, -NJ₁J₂, -N₃, -CN, -C(=O)OJ₁, -C(=O)NJ₁J₂, -C(=O)J₁, -OC(=O)NJ₁J₂, -NH-C(=NH)NJ₁J₂, -NH-C(=O)NJ₁J₂, or , -NH-C(=S)NJ₁J₂; or R^{a} and R^{b} together are =C(q₃)(q₄); q₃ and q₄ are each, independently, - H, halogen, C₁₋₁₂-alkyl or substituted C₁₋₁₂-alkyl; each substituted group is, independently, mono or poly substituted with substituent groups independently selected from halogen, C₁₋₆-alkyl, substituted C₁₋₆-alkyl, C₂₋₆-alkenyl, substituted C₂₋₆-alkenyl, C₂-₆-alkynyl, substituted C₂₋₆-alkynyl, -OJ₁, -SJ₁, -NJ₁J₂, -N₃, -CN, -C(=O)OJ₁, -C(=O)NJ₁J₂, -C(=O)J₁, -OC(=O)NJ₁J₂, -NH-C(=O)NJ₁J₂, or -NH-C(=S)NJ₁J₂ and; each J₁ and J₂ is independently, -H, C₁₋₆-alkyl, substituted C₁₋₆-alkyl, C₂₋₆-alkenyl, substituted C₂₋₆-alkenyl, C₂-₆-alkynyl, substituted C₂₋₆-alkynyl, C₁₋₆-aminoalkyl, substituted C₁₋₆-aminoalkyl or a protecting group. Such compounds are disclosed in WO2009006478A, hereby incorporated in its entirety by reference.

In preferred embodiments, R^{#} and R form the biradical -Q-, wherein Q is -C(q₁)(q₂)C(q₃)(q₄)-, -C(q₁)=C(q₃)-, -C[=C(q₁)(q₂)]-C(q₃)(q₄)- or -C(q₁)(q₂)-C[=C(q₃)(q₄)]-;
q₁, q₂, q₃, q₄ are each independently of each other -H, halogen, C₁₋₁₂-alkyl, substituted C₁₋₁₂-alkyl, C₂₋₆-alkenyl, substituted C₁₋₁₂-alkoxy, -OJ₁, -SJ₁, -S(O)J₁, -SO₂-J₁, -NJ₁J₂, -N₃, -CN, -C(=O)OJ₁, -C(=O)NJ₁J₂, -C(=O)J₁, -OC(=O)NJ₁J₂, -NH-C(=NH)NJ₁J₂, -NH-C(=O)NJ₁J₂, or -NH-C(=S)NJ₁J₂; each J₁ and J₂ is independently of each other -H, C₁₋₆-alkyl, C₂₋₆-alkenyl, C₂-₆-alkynyl, C₁₋₆-aminoalkyl or a protecting group; and optionally when Q is -C(q₁)(q₂)C(q₃)(q₄)-and one of q₃ or q₄ is -CH₃, then at least one of the other of q₃ or q₄ or one of q₁ and q₂ is other than -H. In preferred embodiments R¹, R², R³, R⁴, and R⁵ are hydrogen. For all chiral centers, asymmetric groups may be found in either R or S orientation. Such bicyclic nucleotides are disclosed in WO2008/154401 which is hereby incorporated by reference in its entirety. In preferred embodiments R¹, R², R³, R⁴, and R⁵ are independently of each other selected from the group consisting of hydrogen, halogen, C₁₋₆-alkyl, substituted C₁₋₆-alkyl, C₂₋₆-alkenyl, substituted C₂₋₆-alkenyl, C₂₋₆-alkynyl or substituted C₂-₆-alkynyl, C₁₋₆-alkoxyl, substituted C₁₋₆-alkoxyl, acyl, substituted acyl, C₁₋₆-aminoalkyl or substituted C₁₋₆-aminoalkyl. In preferred embodiments R¹, R², R³, R⁴, and R⁵ are hydrogen. In preferred embodiments R¹, R², and R³ are hydrogen and one or both of R⁴, R⁵ may be other than hydrogen as referred to above and in WO 2007/134181 or WO2009/067647 (alpha-L-bicyclic nucleic acids analogues).

As used herein, the term "C₁-C₆-alkyl" refers to -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH₂-CH(C₂H₅)₂, and -CH(CH₃)-C(CH₃)₃. The term "C₁-C₆-alkyl" shall also include "C₁-C₆-cycloalkyl" like cyclo-C₃H₅, cyclo-C₄H₇, cyclo-C₅H₉, and cyclo-C₆H₁₁.

Preferred are -CH₃, -C₂H₅, -C₃H₇, -CH(CH₃)₂, -C₄H₉, -CH₂-CH(CH₃)₂, -CH(CH₃)-C₂H₅, -C(CH₃)₃, and -C₅H₁₁. Especially preferred are -CH₃, -C₂H₅, -C₃H₇, and -CH(CH₃)₂.

The term "C₁-C₆-alkyl" shall also include "C₁-C₆-cycloalkyl" like cyclo-C₃H₅, cyclo-C₄H₇, cyclo-C₅H₉, and cyclo-C₆H₁₁.

As used herein, the term "C₁-C₁₂-alkyl" refers to C₁-C₆-alkyl, -C₇H₁₅, -C₈H₁₇, -C₉H₁₉, -C₁₀H₂₁, -C₁H₂₃, -C₁₂H₂₅.

As used herein, the term "C₁-C₆-alkylenyl" refers to -CH₂-, -C₂H₄-, -CH(CH₃)-, -C₃H₆-, -CH₂-CH(CH₃)-, -CH(CH₃)-CH₂-, -C(CH₃)₂-, -C₄H₈-, -CH₂-C(CH₃)₂-, -C(CH₃)₂-CH₂-, -C₂H₄-CH(CH₃)-, -CH(CH₃)-C₂H₄-, -CH₂-CH(CH₃)-CH₂-, -CH(CH₃)-CH(CH₃)-, -C₅H₁₀-, -CH(CH₃)-C₃H₆-, -CH₂-CH(CH₃)-C₂H₄-, -C₂H₄-CH(CH₃)-CH₂-, -C₃H₆-CH(CH₃)-, -C₂H₄-C(CH₃)₂-, -C(CH₃)₂-C₂H₄-, -CH₂-C(CH₃)₂-CH₂-, -CH₂-CH(CH₃)-CH(CH₃)-, -CH(CH₃)-CH₂-CH(CH₃)-, -CH(CH₃)-CH(CH₃)-CH₂-, -CH(CH₃)-CH(CH₃)-CH(CH₃)-, -C(CH₃)₂-C₃H₆-, -CH₂-C(CH₃)₂-C₂H₄-, -C₂H₄-C(CH₃)₂-CH₂-, -C₃H₆-C(CH₃)₂-, -CH(CH₃)-C₄H₈-, -C₆H₁₂-, -CH₂-CH(CH₃)-C₃H₆-, -C₂H₄-CH(CH₃)-C₂H₄-, -C₃H₆-CH(CH₃)-CH₂-, -C₄H₈-CH(CH₃)-, -C₂H₄-CH(CH₃)-CH(CH₃)-, -CH₂-CH(CH₃)-CH(CH₃)-CH₂-, -CH₂-CH(CH₃)-CH₂-CH(CH₃)-, -CH(CH₃)-C₂H₄-CH(CH₃)-, -CH(CH₃)-CH₂-CH(CH₃)-CH₂-, and -CH(CH₃)-CH(CH₃)-C₂H₄-.

As used herein, the term "C₂-C₆-alkenyl" refers to -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -CH(CH₃)-C₂H₄-CH=CH₂, -C₂H₄-CH=C(CH₃)₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -CH(CH₃)-CH₂-C(CH₃)=CH₂, -CH(CH₃)-CH(CH₃)-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[C(CH₃)₃]=CH₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -CH=CH-C₂H₄-CH=CH₂, -CH₂-CH=CH-CH=CH-CH₃, -CH=CH-CH₂-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH=CH-CH=CH₂, -CH=CH-CH₂-C(CH₃)=CH₂, -CH=CH-CH(CH₃)-CH=CH₂, -CH=C(CH₃)-CH₂-CH=CH₂, -C(CH₃)=CH-CH₂-CH=CH₂, -CH=CH-CH=C(CH₃)₂, -CH=CH-C(CH₃)=CH-CH₃, -CH=C(CH₃)-CH=CH-CH₃, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, and -CH=CH-CH=CH-CH=CH₂.

Preferred are -CH=CH₂, -CH₂-CH=CH₂, -C(CH₃)=CH₂, -CH=CH-CH₃, -C₂H₄-CH=CH₂, -CH₂-CH=CH-CH₃. Especially preferred are -CH=CH₂, -CH₂-CH=CH₂, and -CH=CH-CH₃.

As used herein, the term "C₂-C₆-alkynyl" refers to -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C=C-CH₃, -CH₂-C≡C-C₂H₅, -C≡C-C₃H₇, -CH(CH₃)-C≡CH, -CH₂-CH(CH₃)-C≡CH, -CH(CH₃)-CH₂-C≡CH, -CH(CH₃)-C≡C-CH₃, -C₄H₈-C≡CH, -C₃H₆-C≡C-CH₃, -C₂H₄-C≡C-C₂H₅, -CH₂-C≡C-C₃H₇, -C≡C-C₄H₉, -C₂H₄-CH(CH₃)-C≡CH, -CH₂-CH(CH₃)-CH₂-C≡CH, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -C≡C-C(CH₃)₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C≡C-CH(CH₃)-C≡CH, -CH(CH₃)-C≡C-C≡CH, -CH(C≡CH)-CH₂-C≡CH, -C(C≡CH)₂-CH₃, -CH₂-CH(C≡CH)₂, -CH(C≡CH)-C≡C-CH₃. Preferred are -C≡CH and -C≡C-CH₃.

The term "C₁₋₆-alkoxyl" refers to "C₁₋C₆-alkyl-O-".

The term "C₁₋₁₂-alkoxyl" refers to "C₁-C₁₂-alkyl-O-".

The term "C₁₋₆-aminoalkyl" refers to "H₂N-C₁-C₆-alkyl-".

The term "C₂-C₆-alkenyloxy" refers to "C₂-C₆-alkenyl-O-".

The term "C₁₋₆-alkylcarbonyl" refers to "C₁-C₆-alkyl-CO-". Also referred to as "acyl".

The term "C₁₋₁₂-alkylcarbonyl" refers to "C₁-C₁₂-alkyl-CO-". Also referred to as "acyl".

The term "C₁₋₆-alkoxycarbonyl" refers to "C₁-C₆-alkyl-O-CO-".

The term "C₁₋₁₂-alkoxycarbonyl" refers to "C₁-C₁₂-alkyl-O-CO-".

The term "C₁-C₆-alkanoyloxy" refers to "C₁-C₆-alkyl-CO-O-".

The term "C₁₋₆-alkylthio" refers to "C₁-C₆-alkyl-S-".

The term "C₁₋₆-alkylsulphonyloxy" refers to "C₁-C₆-alkyl-SO₂-O-".

The term "C₁₋₆-alkylcarbonylamino" refers to "C₁-C₆-alkyl-CO-NH-".

The term "C₁₋₆-alkylamino" refers to "C₁-C₆-alkyl-NH-".

The term "(C₁₋₆-)₂alkylamino" refers to a dialkylamino group like "[C₁-C₆-alkyl][C₁-C₆-alkyl]N-".

The term "C₁₋₆-alkylaminocarbonyl" refers to "C₁-C₆-alkyl-NH-CO-".

The term "(C₁₋₆-)₂alkylaminocarbonyl" refers to a dialkylaminocarbonyl group like "[C₁-C₆-alkyl][C₁-C₆-alkyl]N-CO-".

The term "amino-C₁₋₆-alkylaminocarbonyl" refers to "H₂N-[C₁-C₆-alkylenyl]-NH-CO-".

The term "C₁₋₆-alkyl-amino-C₁₋₆-alkylaminocarbonyl" refers to "C₁₋₆-alkyl-HN-[C₁-C₆-alkylenyl]-NH-CO-".

The term "(C₁₋₆-)₂-alkyl-amino-C₁₋₆-alkylaminocarbonyl" refers to "[C₁-C₆-alkyl][C₁-C₆-alkyl]N-[C₁-C₆-alkylenyl]-NH-CO-".

The term "aryl" refers to phenyl, toluyl, substituted phenyl and substituted toluyl.

The term "aryloxy" refers to "aryl-O-".

The term "arylcarbonyl" refers to "aryl-CO-".

The term "aryloxycarbonyl" refers to "aryl-O-CO-".

The term "heteroaryl" refers to substituted or not substituted heteroaromatic groups which have from 4 to 9 ring atoms, from 1 to 4 of which are selected from O, N and/or S. Preferred "heteroaryl" groups have 1 or 2 heteroatoms in a 5- or 6-membered aromatic ring. Mono and bicyclic ring systems are included. Typical "heteroaryl" groups are pyridyl, furyl, thienyl, pyrrolyl, oxazolyl, thiazolyl, imidazolyl, isoxazolyl, isothiazolyl, oxadiazolyl, pyridazinyl, pyrimidyl, pyrazinyl, 1,3,5-triazinyl, 1,2,3-triazolyl, 1,3,4-thiadiazolyl, indolizinyl, indolyl, isoindolyl, benzo[b]furyl, benzo[b]thienyl, indazolyl, benzimidazolyl, benzthiazolyl, purinyl, quinolizinyl, quinolyl, isoquinolyl, quinazolinyl, quinoxalinyl, 1,8-naphthyridinyl, tetrahydroquinolyl, benzooxazolyl, chrom-2-onyl, indazolyl, and the like.

The term "heteroaryloxy" refers to "heteroaryl-O-".

The term "heteroarylcarbonyl" refers to "heteroaryl-CO-".

The term "heteroaryloxycarbonyl" refers to "heteroaryl-O-CO-".

The term "substituted" refers to groups wherein one or more hydrogen atoms are replaced by one or more of the following substituents: -OH, -OCH₃, -OC₂H₅, -OC₃H₇, -O-cyclo-C₃H₅, -OCH(CH₃)₂, -OCH₂Ph, -F, -Cl, -COCH₃, -COC₂H₅, -COC₃H₇, -CO-cyclo-C₃H₅, -COCH(CH₃)₂, -COOH, -CONH₂, -NH₂, -NHCH₃, -NHC₂H₅, -NHC₃H₇, -NH-cyclo-C₃H₅, -NHCH(CH₃)₂, -N(CH₃)₂, -N(C₂H₅)₂, -N(C₃H₇)₂, -N(cyclo-C₃H₅)₂, -N[CH(CH₃)₂]₂, -SO₃H, -OCF₃, -OC₂F₅, cyclo-C₃H₅, -CH₃, -C₂H₅, -C₃H₇, -CH(CH₃)₂, -CH=CH₂, -CH₂-CH=CH₂, -C≡CH and/or -C≡C-CH₃.

In case the general structure (I) represents monomers or building blocks for synthesizing the antisense-oligonucleotides of the present invention, the terminal groups Y and IL' are selected independently of each other from hydrogen, azido, halogen, cyano, nitro, hydroxy, PG-O-, AG-O-, mercapto, PG-S-, AG-S-, C₁₋₆-alkylthio, amino, PG-N(R^{H})-, AG-N(R^{H})-, mono- or di(C₁₋₆-alkyl)amino, optionally substituted C₁₋₆-alkoxy, optionally substituted C₁₋₆-alkyl, optionally substituted C₂₋₆-alkenyl, optionally substituted C₂₋₆-alkenyloxy, optionally substituted C₂₋₆-alkynyl, optionally substituted C₂₋₆-alkynyloxy, monophosphate, monothiophosphate, diphosphate, dithiophosphate triphosphate, trithiophosphate, carboxy, sulphono, hydroxymethyl, PG-O-CH₂-, AG-O-CH₂-, aminomethyl, PG-N(R^{H})-CH₂-, AG-N(R^{H})-CH₂-, carboxymethyl, sulphonomethyl, where PG is a protection group for -OH, -SH, and -NH(R^{H}), respectively, AG is an activation group for -OH, -SH, and -NH(R^{H}), respectively, and R^{H} is selected from hydrogen and C₁₋₆- alkyl.

The protection groups PG of hydroxy substituents comprise substituted trityl, such as 4,4'-dimethoxytrityl (DMT), 4-monomethoxytrityl (MMT), optionally substituted 9-(9-phenyl)xanthenyl (pixyl), optionally substituted methoxytetrahydropyranyl (mthp), silyl such as trimethylsilyl (TMS), triisopropylsilyl (TIPS), tert-butyldimethylsilyl (TBDMS), triethylsilyl, and phenyldimethylsilyl, tert-butylethers, acetals (including two hydroxy groups), acyl such as acetyl or halogen substituted acetyls, e.g. chloroacetyl or fluoroacetyl, isobutyryl, pivaloyl, benzoyl and substituted benzoyls, methoxymethyl (MOM), benzyl ethers or substituted benzyl ethers such as 2,6-dichlorobenzyl (2,6-Cl₂Bzl). Alternatively when Y or IL' is hydroxyl they may be protected by attachment to a solid support optionally through a linker.

When Y or IL' is an amino group, illustrative examples of the amino protection groups are fluorenylmethoxycarbonyl (Fmoc), tert-butyloxycarbonyl (BOC), trifluoroacetyl, allyloxycarbonyl (alloc or AOC), benzyloxycarbonyl (Z or Cbz), substituted benzyloxycarbonyls such as 2-chloro benzyloxycarbonyl (2-CIZ), monomethoxytrityl (MMT), dimethoxytrityl (DMT), phthaloyl, and 9-(9-phenyl)xanthenyl (pixyl).

Act represents an activation group for -OH, -SH, and -NH(R^{H}), respectively. Such activation groups are, for instance, selected from optionally substituted O-phosphoramidite, optionally substituted O-phosphortriester, optionally substituted O-phosphordiester, optionally substituted H-phosphonate, and optionally substituted O-phosphonate.

In the present context, the term "phosphoramidite" means a group of the formula -P(OR^{x})-N(R^{y})₂, wherein R^{x} designates an optionally substituted alkyl group, e.g. methyl, 2-cyanoethyl, or benzyl, and each of R^{y} designate optionally substituted alkyl groups, e.g. ethyl or isopropyl, or the group -N(R^{y})₂ forms a morpholino group (-N(CH₂CH₂)₂O). R^{x} preferably designates 2-cyanoethyl and the two R^{y} are preferably identical and designate isopropyl. Thus, an especially relevant phosphoramidite is N,N-diisopropyl-O-(2-cyanoethyl)-phosphoramidite.

### LNA monomers or LNA building blocks

The LNA monomers or LNA building blocks used as starting materials in the synthesis of the antisense-oligonucleotides of the present invention are preferably LNA nucleosides of the following general formulae:

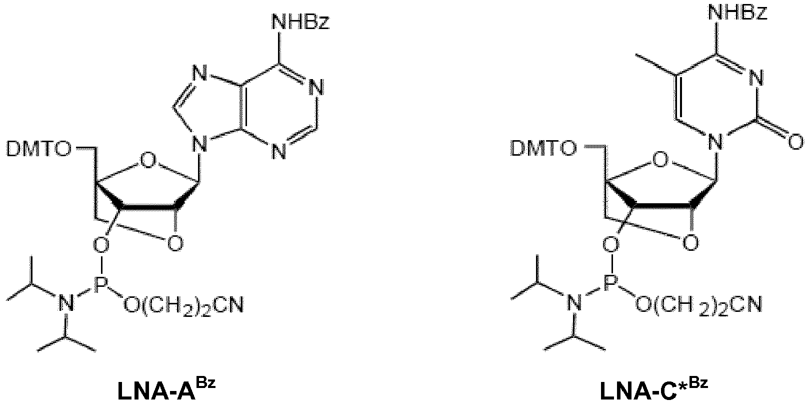

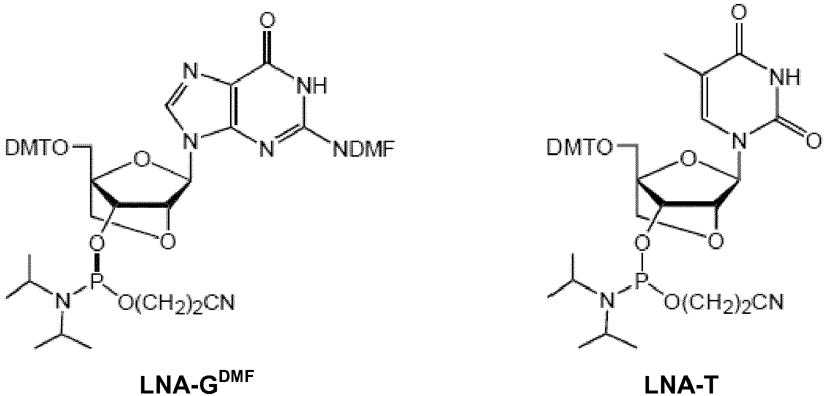

The LNA building blocks are normally provided as LNA phosphoramidites with the four different nucleobases: adenine (A), guanine (G), 5-methyl-cytosine (C*) and thymine (T). The antisense-oligonucleotides of the present invention containing LNA units are synthesized by standard phosphoramidite chemistry. In the LNA building blocks the nucleobases are protected. A preferred protecting group for the amino group of the purin base is a benzoyl group (Bz), indicated as A^{Bz}. A preferred protecting group for the amino group of the 5-methylpyrimidinone base is a benzoyl group (Bz), indicated as C*^{Bz}. A preferred protecting group for the amino group of the purinone base is a dimethylformamidine (DMF) group, a diethylformamidine (DEF), a dipropylformamidine (DPF), a dibutylformamidine (DBF), or a iso-butyryl (-CO-CH(CH₃)₂) group, indicated as G^{DMF}, G^{DEF}, G^{DPF}, G^{DBF}, or G^{iBu}. Thus the group -NDMF refers to -N=CH-N(CH₃)₂. DMT refers to 4,4'-dimethoxytrityl.

Thus, **LNA-T** refers to 5'-O-(4,4'-dimethoxytrityl)-3'-O-(2-cyanoethyl-N,N-diisopropyl)phosphoramidite-thymidine LNA. **LNA-C*^{Bz}** refers to 5'-O-(4,4'-dimethoxytrityl)-3'-O-(2-cyanoethyl-N,N-diisopropyl)phosphoramidite-4-N-benzoyl-5-methyl-2'-cytidine LNA. **LNA**-**A^{Bz}** refers to 5'-O-(4,4'-dimethoxytrityl)-3'-O-(2-cyanoethyl-N,N-diisopropyl)phosphoramidite-6-N-benzoyl-2'-adenosine LNA. **LNA-G^{DMF}** refers to 5'-O-(4,4'-dimethoxytrityl)-3'-O-(2-cyanoethyl-N,N-diisopropyl)-phosphoramidite-2-N-dimethylformamidine-2'-guanosine LNA. **LNA-G^{iBu}** refers to 5'-O-(4,4'-dimethoxytrityl)-3'-O-(2-cyanoethyl-N,N-diisopropyl)phosphoramidite-2-N-iso-butyryl-2'-guanosine LNA.

### Terminal groups

In case Y represents the 5'-terminal group of an antisense-oligonucleotide of the present invention, the residue Y is also named Y^{5'} and represents:
-OH, -O-C₁₋₆-alkyl, -S-C₁₋₆-alkyl, -O-C₆₋₉-phenyl, -O-C₇₋₁₀-benzyl, -NH-C₁₋₆-alkyl, -N(C₁₋₆-alkyl)₂, -O-C₂₋₆-alkenyl, -S-C₂₋₆-alkenyl, -NH-C₂₋₆-alkenyl, -N(C₂₋₆-alkenyl)₂, -O-C₂₋₆-alkynyl, -S-C₂₋₆-alkynyl, -NH-C₂₋₆-alkynyl, -N(C₂₋₆-alkynyl)₂, -O-C₁₋₆-alkylenyl-O-C₁₋₆-alkyl, -O-[C₁₋₆-alkylenyl-O]ₘ-C₁₋₆-alkyl, -O-CO-C₁₋₆-alkyl, -O-CO-C₁₋₆-alkenyl, -O-CO-C₁₋₆-alkynyl, -O-S(O)-C₁₋₆-alkyl, -O-SO₂-C₁₋₆-alkyl, -O-SO₂-O-C₁₋₆-alkyl, -O-P(O)(O⁻)₂, -O-P(O)(O⁻)(O-C₁₋₆-alkyl), -O-P(O)(O-C₁₋₆-alkyl)₂, -O-P(O)(S⁻)₂, -O-P(O)(S-C₁₋₆-alkyl)₂, -O-P(O)(S⁻)(O-C₁₋₆-alkyl), -O-P(O)(O⁻)(NH-C₁₋₆-alkyl), -O-P(O)(O-C₁₋₆-alkyl)(NH-C₁₋₆-alkyl), -O-P(O)(O⁻)[N(C₁₋₆-alkyl)₂], -O-P(O)(O-C₁₋₆-alkyl)[N(C₁₋₆-alkyl)₂], -O-P(O)(O⁻)(BH₃⁻), -O-P(O)(O-C₁₋₆-alkyl)(BH₃⁻), -O-P(O)(O⁻)(O-C₁₋₆-alkylenyl-O-C₁₋₆-alkyl), -O-P(O)(O-C₁₋₆-alkylenyl-O-C₁₋₆-alkyl)₂, -O-P(O)(O⁻)(O-C₁₋₆-alkylenyl-S-C₁₋₆-alkyl), -O-P(O)(O-C₁₋₆-alkylenyl-S-C₁₋₆-alkyl)₂, -O-P(O)(O⁻)(OCH₂CH₂O-C₁₋₆-alkyl), -O-P(O)(OCH₂CH₂O-C₁₋₆-alkyl)₂, -O-P(O)(O⁻)(OCH₂CH₂S-C₁₋₆-alkyl), -O-P(O)(OCH₂CH₂S-C₁₋₆-alkyl)₂, wherein the C₁₋₆-alkyl, C₂₋₆-alkenyl, C₂₋₆-alkynyl, -O-C₆₋₉-phenyl or -O-C₇₋₁₀-benzyl may be further substituted by -F, -OH, C₁₋₄-alkyl, C₂₋₄-alkenyl and/or C₂₋₄-alkynyl. where m is selected from 1, 2, 3, 4, 5, 6, 7, 8, 9 or 10.

More preferred are: -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -O-COCH₃, -O-COC₂H₅, -O-COC₃H₇, -O-CO-cyclo-C₃H₅, -O-COCH(CH₃)₂, -OCF₃, -O-S(O)CH₃, -O-S(O)C₂H₅, -O-S(O)C₃H₇, -O-S(O)-cyclo-C₃H₅, -O-SO₂CH₃, -O-SO₂C₂H₅, -O-SO₂C₃H₇, -O-SO₂-cyclo-C₃H₅, -O-SO₂-OCH₃, -O-SO₂-OC₂H₅, -O-SO₂-OC₃H₇, -O-SO₂-O-cyclo-C₃H₅, -O(CH₂)ₙN[(CH₂)ₙOH], -O(CH₂)ₙN[(CH₂)ₙ-H];
even more preferred are:
-OCH₃, -OC₂H₅, -OCH₂CH₂OCH₃, -O-(2-methoxyethoxy) (also known as MOE), -OCH₂CH₂-N(CH₃)₂ (also known as DMAOE), -O[(CH₂)ₙO]ₘCH₃, -O(CH₂)ₙOCH₃, -O(CH₂)ₙNH₂, -O(CH₂)ₙN(CH₃)₂,
where n is selected from 1, 2, 3, 4, 5, or 6; and where m is selected from 1, 2, 3, 4, 5, 6, 7, 8, 9 or 10.

In case IL' represents the 3'-terminal group of an antisense-oligonucleotide of the present invention, the residue IL' is also named IL'^{3'} and represents:
-OH, -O-C₁₋₆-alkyl, -S-C₁₋₆-alkyl, -O-C₆₋₉-phenyl, -O-C₇₋₁₀-benzyl, -NH-C₁₋₆-alkyl, -N(C₁₋₆-alkyl)₂, -O-C₂₋₆-alkenyl, -S-C₂₋₆-alkenyl, -NH-C₂₋₆-alkenyl, -N(C₂₋₆-alkenyl)₂, -O-C₂₋₆-alkynyl, -S-C₂₋₆-alkynyl, -NH-C₂₋₆-alkynyl, -N(C₂₋₆-alkynyl)₂, -O-C₁₋₆-alkylenyl-O-C₁₋₆-alkyl, -O-[C₁₋₆-alkylenyl-O]ₘ-C₁₋₆-alkyl,
-O-CO-C₁₋₆-alkyl, -O-CO-C₂₋₆-alkenyl, -O-CO-C₂₋₆-alkynyl, -O-S(O)-C₁₋₆-alkyl, -O-SO₂-C₁₋₆-alkyl, -O-SO₂-O-C₁₋₆-alkyl, -O-P(O)(O⁻)₂, -O-P(O)(O⁻)(O-C₁₋₆-alkyl), -O-P(O)(O-C₁₋₆-alkyl)₂, -O-P(O)(S⁻)₂, -O-P(O)(S-C₁₋₆-alkyl)₂, -O-P(O)(S⁻)(O-C₁₋₆-alkyl), -O-P(O)(O⁻)(NH-C₁₋₆-alkyl), -O-P(O)(O-C₁₋₆-alkyl)(NH-C₁₋₆-alkyl), -O-P(O)(O⁻)[N(C₁₋₆-alkyl)₂], -O-P(O)(O-C₁₋₆-alkyl)[N(C₁₋₆-alkyl)₂], -O-P(O)(O⁻)(BH₃⁻), -O-P(O)(O-C₁₋₆-alkyl)(BH₃⁻), -O-P(O)(O⁻)(O-C₁₋₆-alkylenyl-O-C₁₋₆-alkyl), -O-P(O)(O-C₁₋₆-alkylenyl-O-C₁₋₆-alkyl)₂, -O-P(O)(O⁻)(O-C₁₋₆-alkylenyl-S-C₁₋₆-alkyl), -O-P(O)(O-C₁₋₆-alkylenyl-S-C₁₋₆-alkyl)₂, -O-P(O)(O⁻)(OCH₂CH₂O-C₁₋₆-alkyl), -O-P(O)(OCH₂CH₂O-C₁₋₆-alkyl)₂, -O-P(O)(O⁻)(OCH₂CH₂S-C₁₋₆-alkyl), -O-P(O)(OCH₂CH₂S-C₁₋₆-alkyl)₂,
wherein the C₁₋₆-alkyl, C₂₋₆-alkenyl, C₂₋₆-alkynyl, -O-C₆₋₉-phenyl or -O-C₇₋₁₀-benzyl may be further substituted by -F, -OH, C₁₋₄-alkyl, C₂₋₄-alkenyl and/or C₂₋₄-alkynyl. where m is selected from 1, 2, 3, 4, 5, 6, 7, 8, 9 or 10.

More preferred are: -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -O-COCH₃, -O-COC₂H₅, -O-COC₃H₇, -O-CO-cyclo-C₃H₅, -O-COCH(CH₃)₂, -OCF₃, -O-S(O)CH₃, -O-S(O)C₂H₅, -O-S(O)C₃H₇, -O-S(O)-cyclo-C₃H₅, -O-SO₂CH₃, -O-SO₂C₂H₅, -O-SO₂C₃H₇, -O-SO₂-cyclo-C₃H₅, -O-SO₂-OCH₃, -O-SO₂-OC₂H₅, -O-SO₂-OC₃H₇, -O-SO₂-O-cyclo-C₃H₅, -O(CH₂)ₙN[(CH₂)ₙOH], -O(CH₂)ₙN[(CH₂)ₙ-H]; even more preferred are:
-OCH₃, -OC₂H₅, -OCH₂CH₂OCH₃, -O-(2-methoxyethoxy) (also known as MOE), -OCH₂CH₂-N(CH₃)₂ (also known as DMAOE), -O[(CH₂)ₙO]ₘCH₃, -O(CH₂)ₙOCH₃, -O(CH₂)ₙNH₂, -O(CH₂)ₙN(CH₃)₂,
where n is selected from 1, 2, 3, 4, 5, or 6; and
where m is selected from 1, 2, 3, 4, 5, 6, 7, 8, 9 or 10.

### Preferred LNAs

In preferred embodiments LNA units used in the antisense-oligonucleotides of the present invention preferably have the structure of general formula (II):

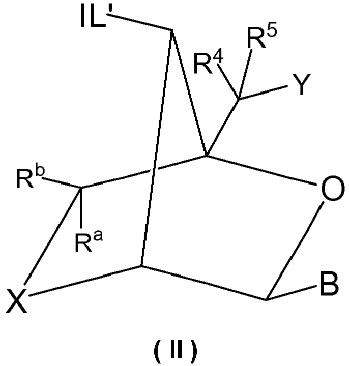

The moiety -C(R^{a}R^{b})-X- represents preferably -C(R^{a}R^{b})-O-, -C(R^{a}R^{b})-NR^{c}-, -C(R^{a}R^{b})-S-, and -C(R^{a}R^{b})-C(R^{a}R^{b})-O-, wherein the substituents R^{a}, R^{b} and R^{c} have the meanings as defined herein and are preferably C₁₋₆-alkyl and more preferably C₁₋₄-alkyl. More preferably -C(R^{a}R^{b})-X- is selected from -CH₂-O-, -CH₂-S-, -CH₂-NH-, -CH₂-N(CH₃)-, -CH₂-CH₂-O-, or -CH₂-CH₂-S-, and more preferably -CH₂-O-, -CH₂-S-, -CH₂-CH₂-O-, or -CH₂-CH₂-S-, and still more preferably -CH₂-O-, -CH₂-S-, or -CH₂-CH₂-O-, and still more preferably -CH₂-O- or -CH₂-S-, and most preferably -CH₂-O-.

All chiral centers and asymmetric substituents (if any) can be either in R or in S orientation. For example, two exemplary stereochemical isomers are the beta-D and alpha-L isoforms as shown below:

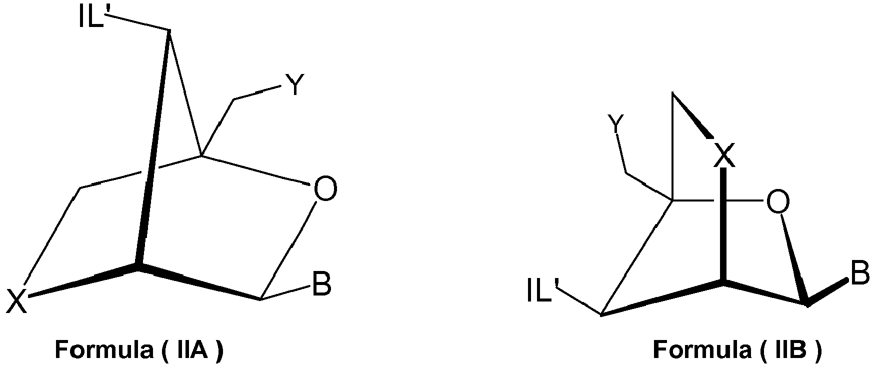

Preferred LNA units are selected from general formula (b¹) to (b⁵):

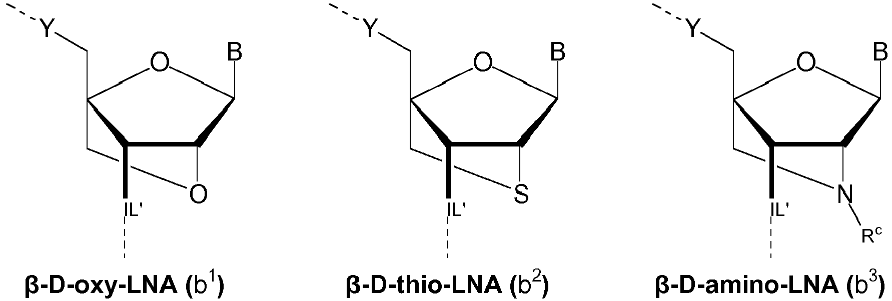

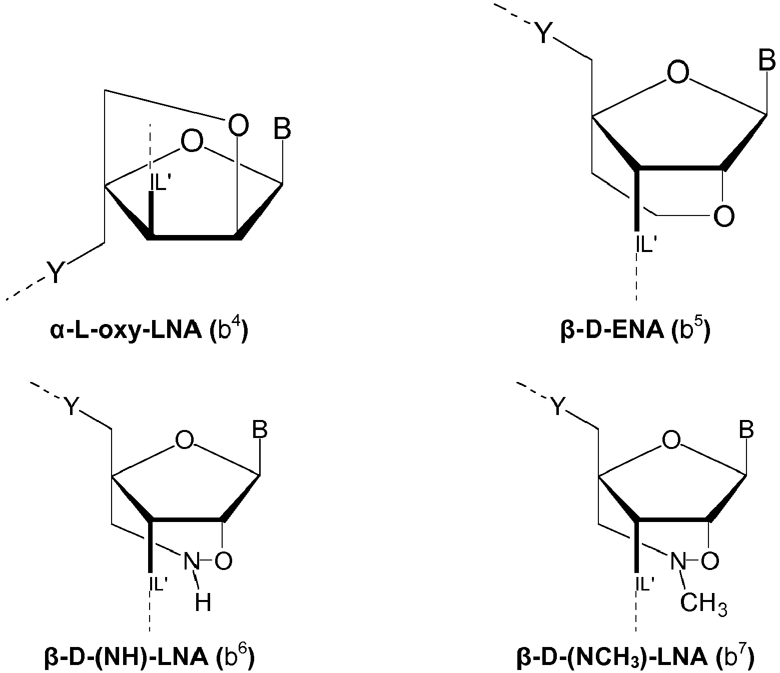

The term "thio-LNA" comprises a locked nucleotide in which Y in the general formula (II) is selected from -S- or -CH₂-S-. Thio-LNA can be in both beta-D and alpha-L-configuration.

The term "amino-LNA" comprises a locked nucleotide in which Y in the general formula (II) is selected from -NH-, -N(R)-, -CH₂-NH-, and -CH₂-N(R)-, where R is selected from hydrogen and C₁₋₄-alkyl. Amino-LNA can be in both beta-D and alpha-L-configuration.

The term "oxy-LNA" comprises a locked nucleotide in which Y in the general formula (II) is -O-. Oxy-LNA can be in both beta-D and alpha-L-configuration.

The term "ENA" comprises a locked nucleotide in which Y in the general formula (II) is -CH₂-O- (where the oxygen atom of -CH₂-O- is attached to the 2'-position relative to the base B). R^{e} is hydrogen or methyl.

In preferred exemplary embodiments LNA is selected from beta-D-oxy-LNA, alpha-L-oxy-LNA, beta-D-amino-LNA and beta-D-thio-LNA, in particular beta-D-oxy-LNA. Thus especially preferred are antisense-oligonucleotides of the present invention which contain LNA units and which especially contain 1 to 5 LNA units at the 5' terminal and 1 to 5 LNA units at the 3' terminal end of the antisense-oligonucleotide.

Preferred are also the antisense-oligonucleotides of the following general formula:
Seq. ID No. 428 represented by the following general formula (S3):
   5'-N¹-**GTCATAGA**-N²-3'
wherein
N¹ represents: CATGGCAGACCCCGCTGCTC-, ATGGCAGACCCCGCTGCTC-, TGGCAGACCCCGCTGCTC-, GGCAGACCCCGCTGCTC-, GCAGACCCCGCTGCTC-, CAGACCCCGCTGCTC-, AGACCCCGCTGCTC-, GACCCCGCTGCTC-, ACCCCGCTGCTC-, CCCCGCTGCTC-, CCCGCTGCTC-, CCGCTGCTC-, CGCTGCTC-, GCTGCTC-, CTGCTC-, TGCTC-, GCTC-, CTC-, TC-, or C-;
   and
N² is selected from: -C, -CC, -CCG, -CCGA, -CCGAG, -CCGAGC, -CCGAGCC, -CCGAGCCC, -CCGAGCCCC, -CCGAGCCCCC, -CCGAGCCCCCA, -CCGAGCCCCCAG, -CCGAGCCCCCAGC, -CCGAGCCCCCAGCG, -CCGAGCCCCCAGCGC, -CCGAGCCCCCAGCGCA, -CCGAGCCCCCAGCGCAG, -CCGAGCCCCCAGCGCAGC, -CCGAGCCCCCAGCGCAGCG, or -CCGAGCCCCCAGCGCAGCGG.

Preferred in accordance with the present invention is general formula (S3):
5'-N¹-**GTCATAGA**-N²-3'
wherein
N¹ represents: TGGCAGACCCCGCTGCTC-, GGCAGACCCCGCTGCTC-, GCAGACCCCGCTGCTC-, CAGACCCCGCTGCTC-, AGACCCCGCTGCTC-, GACCCCGCTGCTC-, ACCCCGCTGCTC-, CCCCGCTGCTC-, CCCGCTGCTC-, CCGCTGCTC-, CGCTGCTC-, GCTGCTC-, CTGCTC-, TGCTC-, GCTC-, CTC-, TC-, or C-;
   and
N² is selected from: -C, -CC, -CCG, -CCGA, -CCGAG, -CCGAGC, -CCGAGCC, -CCGAGCCC, -CCGAGCCCC, -CCGAGCCCCC, -CCGAGCCCCCA, -CCGAGCCCCCAG, -CCGAGCCCCCAGC, -CCGAGCCCCCAGCG, -CCGAGCCCCCAGCGC, -CCGAGCCCCCAGCGCA, -CCGAGCCCCCAGCGCAG, or -CCGAGCCCCCAGCGCAGC.

More preferred in accordance with the present invention is general formula (S3):
5'-N¹-**GTCATAGA**-N²-3'
wherein
N¹ represents: GACCCCGCTGCTC-, ACCCCGCTGCTC-, CCCCGCTGCTC-, CCCGCTGCTC-, CCGCTGCTC-, CGCTGCTC-, GCTGCTC-, CTGCTC-, TGCTC-, GCTC-, CTC-, TC-, or C-;
   and
N² is selected from: -C, -CC, -CCG, -CCGA, -CCGAG, -CCGAGC, -CCGAGCC, -CCGAGCCC, -CCGAGCCCC, -CCGAGCCCCC, -CCGAGCCCCCA, -CCGAGCCCCCAG, or -CCGAGCCCCCAGC.

Even more preferred in accordance with the present invention is general formula (S3):
5'-N¹-**GTCATAGA**-N²-3'
wherein
N¹ represents: CGCTGCTC-, GCTGCTC-, CTGCTC-, TGCTC-, GCTC-, CTC-, TC-, or C-;
   and
N² is selected from: -C, -CC, -CCG, -CCGA, -CCGAG, -CCGAGC, -CCGAGCC, or -CCGAGCCC.

Seq. ID No. 98 represented by the following general formula (S4):
5'-N³-**ACGCGTCC**-N⁴-3'
wherein
N³ represents: GGTGGGATCGTGCTGGCGAT-, GTGGGATCGTGCTGGCGAT-, TGGGATCGTGCTGGCGAT-, GGGATCGTGCTGGCGAT-, GGATCGTGCTGGCGAT-, GATCGTGCTGGCGAT-, ATCGTGCTGGCGAT-, TCGTGCTGGCGAT-, CGTGCTGGCGAT-, GTGCTGGCGAT-, TGCTGGCGAT-, GCTGGCGAT-, CTGGCGAT-, TGGCGAT-, GGCGAT-, GCGAT-, CGAT-, GAT-, AT-, or T-;
   and
N⁴ is selected from: -ACAGGACGATGTGCAGCGGC, -ACAGGACGATGTGCAGCGG, -ACAGGACGATGTGCAGCG, -ACAGGACGATGTGCAGC, -ACAGGACGATGTGCAG, -ACAGGACGATGTGCA, -ACAGGACGATGTGC, -ACAGGACGATGTG, -ACAGGACGATGT, -ACAGGACGATG, -ACAGGACGAT, -ACAGGACGA, -ACAGGACG, -ACAGGAC, -ACAGGA,-ACAGG,-ACAG,-ACA,-AC, or -A.

Preferred in accordance with the present invention is general formula (S4):
5'-N³-**ACGCGTCC**-N⁴-3'
wherein
N³ represents: TGGGATCGTGCTGGCGAT-, GGGATCGTGCTGGCGAT-, GGATCGTGCTGGCGAT-, GATCGTGCTGGCGAT-, ATCGTGCTGGCGAT-, TCGTGCTGGCGAT-, CGTGCTGGCGAT-, GTGCTGGCGAT-, TGCTGGCGAT-, GCTGGCGAT-, CTGGCGAT-, TGGCGAT-, GGCGAT-, GCGAT-, CGAT-, GAT-, AT-, or T-;
   and
N⁴ is selected from: -ACAGGACGATGTGCAGCG, -ACAGGACGATGTGCAGC, -ACAGGACGATGTGCAG, -ACAGGACGATGTGCA, -ACAGGACGATGTGC, -ACAGGACGATGTG, -ACAGGACGATGT, -ACAGGACGATG, -ACAGGACGAT, -ACAGGACGA, -ACAGGACG, -ACAGGAC, -ACAGGA, -ACAGG, -ACAG, -ACA, -AC, or -A.

More preferred in accordance with the present invention is general formula (S4):
5'-N³-**ACGCGTCC**-N⁴-3'
wherein
N³ represents: TCGTGCTGGCGAT-, CGTGCTGGCGAT-, GTGCTGGCGAT-, TGCTGGCGAT-, GCTGGCGAT-, CTGGCGAT-, TGGCGAT-, GGCGAT-, GCGAT-, CGAT-, GAT-,
AT-, or T-;
   and
N⁴ is selected from: -ACAGGACGATGTG, -ACAGGACGATGT, -ACAGGACGATG, -ACAGGACGAT, -ACAGGACGA, -ACAGGACG, -ACAGGAC, -ACAGGA, -ACAGG, -ACAG, -ACA, -AC, or -A.

Even more preferred in accordance with the present invention is general formula (S4):
5'-N³-**ACGCGTCC**-N⁴-3'
wherein
N³ represents: CTGGCGAT-, TGGCGAT-, GGCGAT-, GCGAT-, CGAT-, GAT-, AT-, or T-;
   and
N⁴ is selected from: -ACAGGACG, -ACAGGAC, -ACAGGA, -ACAGG, -ACAG, -ACA, -AC, or -A.

Seq. ID No. 99 represented by the following general formula (S5):
5'-N⁵-**GTAGTGTT**-N⁶-3'
wherein
N⁵ represents: CCCAGCCTGCCCCAGAAGAGCTATTTG-, CCAGCCTGCCCCAGAAGAGCTATTTG-, CAGCCTGCCCCAGAAGAGCTATTTG-, AGCCTGCCCCAGAAGAGCTATTTG-, GCCTGCCCCAGAAGAGCTATTTG-, CCTGCCCCAGAAGAGCTATTTG-, CTGCCCCAGAAGAGCTATTTG-, TGCCCCAGAAGAGCTATTTG-, GCCCCAGAAGAGCTATTTG-, CCCCAGAAGAGCTATTTG-, CCCAGAAGAGCTATTTG-, CCAGAAGAGCTATTTG-, CAGAAGAGCTATTTG-, AGAAGAGCTATTTG-, GAAGAGCTATTTG-, AAGAGCTATTTG-, AGAGCTATTTG-, GAGCTATTTG-, AGCTATTTG-, GCTATTTG-, CTATTTG-, TATTTG-, ATTTG-, TTTG-, TTG-, TG-, or G-;
   and
N⁶ is selected from: -TAGGGAGCCGTCTTCAGGAATCTTCTC, -TAGGGAGCCGTCTTCAGGAATCTTCT, -TAGGGAGCCGTCTTCAGGAATCTTC, -TAGGGAGCCGTCTTCAGGAATCTT, -TAGGGAGCCGTCTTCAGGAATCT, -TAGGGAGCCGTCTTCAGGAATC, -TAGGGAGCCGTCTTCAGGAAT, -TAGGGAGCCGTCTTCAGGAA, -TAGGGAGCCGTCTTCAGGA, -TAGGGAGCCGTCTTCAGG, -TAGGGAGCCGTCTTCAG, -TAGGGAGCCGTCTTCA, -TAGGGAGCCGTCTTC, -TAGGGAGCCGTCTT, -TAGGGAGCCGTCT, -TAGGGAGCCGTC, -TAGGGAGCCGT, -TAGGGAGCCG, -TAGGGAGCC, -TAGGGAGC, -TAGGGAG, -TAGGGA, -TAGGG, -TAGG, -TAG, -TA, or -T.

Preferred in accordance with the present invention is general formula (S5):
5'-N⁵-**GTAGTGTT**-N⁶-3'
wherein
N⁵ represents: GCCTGCCCCAGAAGAGCTATTTG-, CCTGCCCCAGAAGAGCTATTTG-, CTGCCCCAGAAGAGCTATTTG-, TGCCCCAGAAGAGCTATTTG-, GCCCCAGAAGAGCTATTTG-, CCCCAGAAGAGCTATTTG-, CCCAGAAGAGCTATTTG-, CCAGAAGAGCTATTTG-, CAGAAGAGCTATTTG-, AGAAGAGCTATTTG-, GAAGAGCTATTTG-, AAGAGCTATTTG-, AGAGCTATTTG-, GAGCTATTTG-, AGCTATTTG-, GCTATTTG-, CTATTTG-, TATTTG-, ATTTG-, TTTG-, TTG-, TG-, or G-;
   and
N⁶ is selected from: -TAGGGAGCCGTCTTC, -TAGGGAGCCGTCTT, -TAGGGAGCCGTCT, -TAGGGAGCCGTC, -TAGGGAGCCGT, -TAGGGAGCCG, -TAGGGAGCC, -TAGGGAGC, -TAGGGAG, -TAGGGA, -TAGGG, -TAGG, -TAG, -TA, or -T.

More preferred in accordance with the present invention is general formula (S5):
5'-N⁵-**GTAGTGTT**-N⁶-3'
wherein
N⁵ represents: CCCCAGAAGAGCTATTTG-, CCCAGAAGAGCTATTTG-, CCAGAAGAGCTATTTG-, CAGAAGAGCTATTTG-, AGAAGAGCTATTTG-, GAAGAGCTATTTG-, AAGAGCTATTTG-, AGAGCTATTTG-, GAGCTATTTG-, AGCTATTTG-, GCTATTTG-, CTATTTG-, TATTTG-, ATTTG-, TTTG-, TTG-, TG-, or G-;
   and
N⁶ is selected from: -TAGGGAGCCG, -TAGGGAGCC, -TAGGGAGC, -TAGGGAG, -TAGGGA, -TAGGG, -TAGG, -TAG, -TA, or -T.

Even more preferred in accordance with the present invention is general formula (S5):
5'-N⁵-**GTAGTGTT**-N⁶-3'
wherein
N⁵ represents: GAAGAGCTATTTG-, AAGAGCTATTTG-, AGAGCTATTTG-, GAGCTATTTG-, AGCTATTTG-, GCTATTTG-, CTATTTG-, TATTTG-, ATTTG-, TTTG-, TTG-, TG-, or G-;
   and
N⁶ is selected from: -TAGGG, -TAGG, -TAG, -TA, or -T.

Also preferred in accordance with the present invention is general formula (S5):
5'-N⁵-**GTAGTGTT**-N⁶-3'
wherein
N⁵ represents: CAGAAGAGCTATTTG-, AGAAGAGCTATTTG-, GAAGAGCTATTTG-, AAGAGCTATTTG-, AGAGCTATTTG-, GAGCTATTTG-, AGCTATTTG-, GCTATTTG-, CTATTTG-, TATTTG-, ATTTG-, TTTG-, TTG-, TG-, or G-;
   and
N⁶ is selected from: -TAGGGAGCCGTCTTCAGGAATCT, -TAGGGAGCCGTCTTCAGGAATC, -TAGGGAGCCGTCTTCAGGAAT, -TAGGGAGCCGTCTTCAGGAA, -TAGGGAGCCGTCTTCAGGA, -TAGGGAGCCGTCTTCAGG, -TAGGGAGCCGTCTTCAG, -TAGGGAGCCGTCTTCA, -TAGGGAGCCGTCTTC, -TAGGGAGCCGTCTT, -TAGGGAGCCGTCT, -TAGGGAGCCGTC, -TAGGGAGCCGT, -TAGGGAGCCG, -TAGGGAGCC, -TAGGGAGC, -TAGGGAG, -TAGGGA, -TAGGG, -TAGG, -TAG, -TA, or -T.

Also more preferred in accordance with the present invention is general formula (S5):
5'-N⁵-**GTAGTGTT**-N⁶-3'
wherein
N⁵ represents: GAGCTATTTG-, AGCTATTTG-, GCTATTTG-, CTATTTG-, TATTTG-, ATTTG-, TTTG-, TTG-, TG-, or G-;
   and
N⁶ is selected from: -TAGGGAGCCGTCTTCAGG, -TAGGGAGCCGTCTTCAG, -TAGGGAGCCGTCTTCA, -TAGGGAGCCGTCTTC, -TAGGGAGCCGTCTT, -TAGGGAGCCGTCT, -TAGGGAGCCGTC, -TAGGGAGCCGT, -TAGGGAGCCG, -TAGGGAGCC, -TAGGGAGC, -TAGGGAG, -TAGGGA, -TAGGG, -TAGG, -TAG, -TA, or -T.

Also even more preferred in accordance with the present invention is general formula (S5):
5'-N⁵-**GTAGTGTT**-N⁶-3'
wherein
N⁵ represents: ATTTG-, TTTG-, TTG-, TG-, or G-;
   and
N⁶ is selected from: -TAGGGAGCCGTCT, -TAGGGAGCCGTC, -TAGGGAGCCGT, -TAGGGAGCCG,
-TAGGGAGCC, -TAGGGAGC, -TAGGGAG, -TAGGGA, -TAGGG, -TAGG, -TAG, -TA, or -T.

Seq. ID No. 100 represented by the following general formula (S6):
5'-N⁷-**AATGGACC**-N⁸-3'
wherein
N⁷ represents: TGAATCTTGAATATCTCATG-, GAATCTTGAATATCTCATG-, AATCTTGAATATCTCATG-, ATCTTGAATATCTCATG-, TCTTGAATATCTCATG-, CTTGAATATCTCATG-, TTGAATATCTCATG-, TGAATATCTCATG-, GAATATCTCATG-, AATATCTCATG-, ATATCTCATG-, TATCTCATG-, ATCTCATG-, TCTCATG-, CTCATG-, TCATG-, CATG-, ATG-, TG-, or G-;
   and
N⁸ is selected from: -AGTATTCTAGAAACTCACCA, -AGTATTCTAGAAACTCACC, -AGTATTCTAGAAACTCAC, -AGTATTCTAGAAACTCA, -AGTATTCTAGAAACTC, -AGTATTCTAGAAACT, -AGTATTCTAGAAAC, -AGTATTCTAGAAA, -AGTATTCTAGAA, -AGTATTCTAGA, -AGTATTCTAG, -AGTATTCTA, -AGTATTCT, -AGTATTC, -AGTATT, -AGTAT, -AGTA, -AGT, -AG, or -A.

Preferred in accordance with the present invention is general formula (S6):
5'-N⁷-**AATGGACC**-N⁸-3'
wherein
N⁷ represents: AATCTTGAATATCTCATG-, ATCTTGAATATCTCATG-, TCTTGAATATCTCATG-, CTTGAATATCTCATG-, TTGAATATCTCATG-, TGAATATCTCATG-, GAATATCTCATG-, AATATCTCATG-, ATATCTCATG-, TATCTCATG-, ATCTCATG-, TCTCATG-, CTCATG-, TCATG-, CATG-, ATG-, TG-, or G-;
   and
N⁸ is selected from: -AGTATTCTAGAAACTCAC, -AGTATTCTAGAAACTCA, -AGTATTCTAGAAACTC, -AGTATTCTAGAAACT, -AGTATTCTAGAAAC, -AGTATTCTAGAAA, -AGTATTCTAGAA, -AGTATTCTAGA, -AGTATTCTAG, -AGTATTCTA, -AGTATTCT, -AGTATTC, -AGTATT, -AGTAT, -AGTA, -AGT, -AG, or -A.

More preferred in accordance with the present invention is general formula (S6):
5'-N⁷-**AATGGACC**-N⁸-3'
wherein
N⁷ represents: TGAATATCTCATG-, GAATATCTCATG-, AATATCTCATG-, ATATCTCATG-, TATCTCATG-, ATCTCATG-, TCTCATG-, CTCATG-, TCATG-, CATG-, ATG-, TG-, or G-;
   and
N⁸ is selected from: -AGTATTCTAGAAA, -AGTATTCTAGAA, -AGTATTCTAGA, -AGTATTCTAG, -AGTATTCTA, -AGTATTCT, -AGTATTC, -AGTATT, -AGTAT, -AGTA, -AGT, -AG, or -A.

Even more preferred in accordance with the present invention is general formula (S6):
5'-N⁷-**AATGGACC**-N⁸-3'
wherein
N⁷ represents: ATCTCATG-, TCTCATG-, CTCATG-, TCATG-, CATG-, ATG-, TG-, or G-;
   and
N⁸ is selected from: -AGTATTCT, -AGTATTC, -AGTATT, -AGTAT, -AGTA, -AGT, -AG, or -A.

Seq. ID No. 101 represented by the following general formula (S7):
5'-N⁹-**ATTAATAA**-N¹⁰-3'
wherein
N⁹ represents: ATTCATATTTATATACAGGC-, TTCATATTTATATACAGGC-, TCATATTTATATACAGGC-, CATATTTATATACAGGC-, ATATTTATATACAGGC-, TATTTATATACAGGC-, ATTTATATACAGGC-,
TTTATATACAGGC-, TTATATACAGGC-, TATATACAGGC-, ATATACAGGC-, TATACAGGC-, ATACAGGC-, TACAGGC-,
ACAGGC-, CAGGC-, AGGC-, GGC-, GC-, or C-;
   and
N¹⁰ is selected from: -AGTGCAAATGTTATTGGCTA, -AGTGCAAATGTTATTGGCT, -AGTGCAAATGTTATTGGC, -AGTGCAAATGTTATTGG, -AGTGCAAATGTTATTG, -AGTGCAAATGTTATT, -AGTGCAAATGTTAT, -AGTGCAAATGTTA, -AGTGCAAATGTT, -AGTGCAAATGT, -AGTGCAAATG, -AGTGCAAAT, -AGTGCAAA, -AGTGCAA, -AGTGCA, -AGTGC, -AGTG, -AGT, -AG, or -A.

Preferred in accordance with the present invention is general formula (S7):
5-N⁹-**ATTAATAA**-N¹⁰-3'
wherein
N⁹ represents: TCATATTTATATACAGGC-, CATATTTATATACAGGC-, ATATTTATATACAGGC-, TATTTATATACAGGC-, ATTTATATACAGGC-, TTTATATACAGGC-, TTATATACAGGC-, TATATACAGGC-, ATATACAGGC-, TATACAGGC-, ATACAGGC-, TACAGGC-, ACAGGC-, CAGGC-, AGGC-, GGC-, GC-, or C-;
   and
N¹⁰ is selected from: -AGTGCAAATGTTATTGGC, -AGTGCAAATGTTATTGG, -AGTGCAAATGTTATTG, -AGTGCAAATGTTATT, -AGTGCAAATGTTAT, -AGTGCAAATGTTA, -AGTGCAAATGTT, -AGTGCAAATGT, -AGTGCAAATG, -AGTGCAAAT, -AGTGCAAA, -AGTGCAA, -AGTGCA, -AGTGC, -AGTG, -AGT, -AG, or -A.

More preferred in accordance with the present invention is general formula (S7):
5-N⁹-**ATTAATAA**-N¹⁰-3'
wherein
N⁹ represents: TTTATATACAGGC-, TTATATACAGGC-, TATATACAGGC-, ATATACAGGC-, TATACAGGC-, ATACAGGC-, TACAGGC-, ACAGGC-, CAGGC-, AGGC-, GGC-, GC-, or C-;
   and
N¹⁰ is selected from: -AGTGCAAATGTTA, -AGTGCAAATGTT, -AGTGCAAATGT, -AGTGCAAATG, -AGTGCAAAT, -AGTGCAAA, -AGTGCAA, -AGTGCA, -AGTGC, -AGTG, -AGT, -AG, or -A.

Even more preferred in accordance with the present invention is general formula (S7):
5'-N⁹-**ATTAATAA**-N¹⁰-3'
wherein
N⁹ represents: ATACAGGC-, TACAGGC-, ACAGGC-, CAGGC-, AGGC-, GGC-, GC-, or C-;
   and
N¹⁰ is selected from: -AGTGCAAA, -AGTGCAA, -AGTGCA, -AGTGC, -AGTG, -AGT, -AG, or -A.

Still more preferred are the following antisense-oligonucleotides (Table 1):

| **SP** | **L** | **Seq ID No.** | **Sequence, 5'-3'** |
|---|---|---|---|
| 89 | 17 | 311 | GCGAGTGACTCACTCAA |
| 90 | 15 | 312 | CGAGTGACTCACTCA |
| 90 | 16 | 313 | GCGAGTGACTCACTCA |
| 90 | 17 | 314 | CGCGAGTGACTCACTCA |
| 91 | 14 | 315 | CGAGTGACTCACTC |
| 91 | 16 | 316 | CGCGAGTGACTCACTC |
| 91 | 17 | 317 | GCGCGAGTGACTCACTC |
| 92 | 14 | 318 | GCGAGTGACTCACT |
| 92 | 16 | 319 | GCGCGAGTGACTCACT |
| 92 | 17 | 320 | CGCGCGAGTGACTCACT |
| 93 | 12 | 321 | CGAGTGACTCAC |
| 93 | 13 | 322 | GCGAGTGACTCAC |
| 93 | 14 | 323 | CGCGAGTGACTCAC |
| 93 | 16 | 324 | CGCGCGAGTGACTCAC |
| 93 | 17 | 325 | GCGCGCGAGTGACTCAC |
| 94 | 13 | 326 | CGCGAGTGACTCA |
| 94 | 14 | 327 | GCGCGAGTGACTCA |
| 94 | 15 | 328 | CGCGCGAGTGACTCA |
| 94 | 16 | 329 | GCGCGCGAGTGACTCA |
| 94 | 17 | 330 | TGCGCGCGAGTGACTCA |
| 95 | 14 | 331 | CGCGCGAGTGACTC |
| 95 | 16 | 332 | TGCGCGCGAGTGACTC |
| 95 | 17 | 333 | GTGCGCGCGAGTGACTC |
| 96 | 13 | 334 | CGCGCGAGTGACT |
| 97 | 14 | 335 | TGCGCGCGAGTGAC |
| 97 | 16 | 336 | CGTGCGCGCGAGTGAC |
| 98 | 13 | 337 | TGCGCGCGAGTGA |
| 107 | 16 | 338 | GTCGTCGCTCCGTGCG |
| 108 | 15 | 339 | GTCGTCGCTCCGTGC |
| 108 | 17 | 340 | GTGTCGTCGCTCCGTGC |
| 109 | 13 | 341 | TCGTCGCTCCGTG |
| 109 | 15 | 342 | TGTCGTCGCTCCGTG |
| 110 | 12 | 343 | TCGTCGCTCCGT |
| 110 | 13 | 344 | GTCGTCGCTCCGT |
| 110 | 14 | 345 | TGTCGTCGCTCCGT |
| 110 | 15 | 346 | GTGTCGTCGCTCCGT |
| 110 | 16 | 347 | GGTGTCGTCGCTCCGT |
| 351 | 16 | 348 | CGTCATAGACCGAGCC |
| 351 | 12 | 349 | ATAGACCGAGCC |
| 354 | 16 | 350 | GCTCGTCATAGACCGA |
| 354 | 13 | 351 | CGTCATAGACCGA |
| 355 | 14 | 352 | CTCGTCATAGACCG |
| 355 | 15 | 353 | GCTCGTCATAGACCG |
| 356 | 14 | 354 | GCTCGTCATAGACC |
| 381 | 17 | 355 | CAGCCCCCGACCCATGG |
| 382 | 16 | 356 | CAGCCCCCGACCCATG |
| 383 | 14 | 357 | AGCCCCCGACCCAT |
| 384 | 14 | 358 | CAGCCCCCGACCCA |
| 422 | 17 | 359 | CGCGTCCACAGGACGAT |
| 425 | 14 | 360 | CGCGTCCACAGGAC |
| 429 | 15 | 361 | CGATACGCGTCCACA |
| 431 | 13 | 362 | CGATACGCGTCCA |
| 431 | 16 | 363 | TGGCGATACGCGTCCA |
| 432 | 12 | 364 | CGATACGCGTCC |
| 432 | 13 | 365 | GCGATACGCGTCC |
| 432 | 17 | 366 | GCTGGCGATACGCGTCC |
| 433 | 15 | 367 | CTGGCGATACGCGTC |
| 433 | 12 | 368 | GCGATACGCGTC |
| 433 | 16 | 369 | GCTGGCGATACGCGTC |
| 433 | 14 | 370 | TGGCGATACGCGTC |
| 434 | 13 | 371 | TGGCGATACGCGT |
| 434 | 14 | 372 | CTGGCGATACGCGT |
| 434 | 12 | 373 | GGCGATACGCGT |
| 435 | 13 | 374 | CTGGCGATACGCG |
| 435 | 12 | 375 | TGGCGATACGCG |
| 437 | 17 | 376 | ATCGTGCTGGCGATACG |
| 449 | 16 | 377 | CGTGCGGTGGGATCGT |
| 449 | 17 | 378 | ACGTGCGGTGGGATCGT |
| 450 | 17 | 379 | AACGTGCGGTGGGATCG |
| 452 | 15 | 380 | AACGTGCGGTGGGAT |
| 452 | 17 | 381 | TGAACGTGCGGTGGGAT |
| 459 | 17 | 382 | CGACTTCTGAACGTGCG |
| 941 | 17 | 383 | TTAACGCGGTAGCAGTA |
| 941 | 16 | 384 | TAACGCGGTAGCAGTA |
| 942 | 17 | 385 | GTTAACGCGGTAGCAGT |
| 943 | 15 | 386 | TTAACGCGGTAGCAG |
| 944 | 13 | 387 | TAACGCGGTAGCA |
| 945 | 12 | 388 | TAACGCGGTAGC |
| 945 | 13 | 389 | TTAACGCGGTAGC |
| 946 | 12 | 390 | TTAACGCGGTAG |
| 946 | 13 | 391 | GTTAACGCGGTAG |
| 946 | 15 | 392 | CGGTTAACGCGGTAG |
| 946 | 16 | 393 | CCGGTTAACGCGGTAG |
| 947 | 14 | 394 | CGGTTAACGCGGTA |
| 947 | 13 | 395 | GGTTAACGCGGTA |
| 947 | 15 | 396 | CCGGTTAACGCGGTA |
| 947 | 16 | 397 | GCCGGTTAACGCGGTA |
| 947 | 17 | 398 | TGCCGGTTAACGCGGTA |
| 948 | 13 | 399 | CGGTTAACGCGGT |
| 949 | 13 | 400 | CCGGTTAACGCGG |
| 949 | 14 | 401 | GCCGGTTAACGCGG |
| 949 | 15 | 402 | TGCCGGTTAACGCGG |
| 950 | 13 | 403 | GCCGGTTAACGCG |
| 950 | 15 | 404 | CTGCCGGTTAACGCG |
| 950 | 16 | 405 | GCTGCCGGTTAACGCG |
| 1387 | 16 | 406 | ATGCCGCGTCAGGTAC |
| 1392 | 13 | 407 | ACATGCCGCGTCA |
| 1393 | 16 | 408 | GATGACATGCCGCGTC |
| 1394 | 12 | 409 | GACATGCCGCGT |
| 1394 | 15 | 410 | GATGACATGCCGCGT |
| 1395 | 13 | 411 | ATGACATGCCGCG |
| 1805 | 17 | 412 | TCCCGCACCTTGGAACC |
| 1851 | 16 | 413 | CGATCTCTCAACACGT |
| 1851 | 17 | 414 | TCGATCTCTCAACACGT |
| 1852 | 15 | 415 | CGATCTCTCAACACG |
| 1852 | 16 | 416 | TCGATCTCTCAACACG |
| 1852 | 17 | 417 | CTCGATCTCTCAACACG |
| 2064 | 16 | 418 | GTAGTGTTTAGGGAGC |
| 2072 | 16 | 419 | GCTATTTGGTAGTGTT |
| 2284 | 15 | 420 | AGCTTATCCTATGAC |
| 2285 | 14 | 421 | AGCTTATCCTATGA |
| 2355 | 17 | 422 | CAGGCATTAATAAAGTG |
| 4120 | 16 | 423 | CTAGGCGCCTCTATGC |
| 4121 | 14 | 424 | TAGGCGCCTCTATG |
| 4121 | 15 | 425 | CTAGGCGCCTCTATG |
| 4122 | 13 | 426 | TAGGCGCCTCTAT |
| 4217 | 16 | 427 | CATGAATGGACCAGTA |

| | | | |
|---|---|---|---|
| SP: start position or start nucleotide on **Seq. ID No. 2** L: length of the sequence | | | |

The antisense-oligonucleotides of the invention such as the antisense-oligonucleotides of the sequences Seq. ID No.s 311 - 427 consist of nucleotides, preferably DNA nucleotides, which are non-LNA units (also named herein non-LNA nucleotides) as well as LNA units (also named herein LNA nucleotides).

The use of LNA units is preferred especially at the 3' terminal and the 5' terminal end. Thus it is preferred if the last 1 - 5 nucleotides at the 3' terminal end and also the last 1 - 5 nucleotides at the 5' terminal end especially of the sequences disclosed herein and particularly of Seq. ID No.s 311 - 427 are LNA units (also named LNA nucleotides) while in between the 1 - 5 LNA units at the 3' and 5' end 2 - 14, preferably 3 - 12, more preferably 4 - 10, more preferably 5 - 9, still more preferably 6 - 8, non-LNA units (also named non-LNA nucleotides) are present. Such kind of antisense-oligonucleotides are called gapmers and are disclosed in more detail below. More preferred are 2 - 5 LNA nucleotides at the 3' end and 2 - 5 LNA nucleotides at the 5' end or 1 - 4 LNA nucleotides at the 3' end and 1 - 4 LNA nucleotides at the 5' end and still more preferred are 2 - 4 LNA nucleotides at the 3' end and 2 - 4 LNA nucleotides at the 5' end of the antisense-oligonucleotides with a number of preferably 4 - 10, more preferably 5 - 9, still more preferably 6 - 8 non-LNA units in between the LNA units at the 3' and the 5' end.

Moreover instead of the use of LNA units or in addition to the use of the LNA units, the use of phosphorothioates or phosphorodithioates and preferably phosphorothioates as internucleotide linkages is preferred.

Thus further preferred are antisense-oligonucleotides wherein more than 50%, preferably more than 60%, more preferably more than 70%, still more preferably more than 80%, and most preferably more than 90% of the internucleotide linkages are phosphorothioates or phosphorodithioates and preferably phosphorothioates groups and wherein the last 1 - 4 nucleotides at the 3' end are LNA units and the last 1 - 4 nucleotides at the 5' end are LNA units and between the LNA units at the ends a sequence of 6 - 12 nucleotides, preferably 7 - 11, preferably 7 - 10, more preferably 8 - 10 are present which are non-LNA units. Moreover it is preferred that these antisense-oligonucleotides in form of gapmers consist in total of 12 to 18 nucleotides.

### Gapmers

The antisense-oligonucleotides of the invention may consist of nucleotide sequences which comprise both DNA nucleotides which are non-LNA units as well as LNA nucleotides, and may be arranged in the form of a gapmer.

Thus, the antisense-oligonucleotides of the present invention are preferably gapmers. A gapmer consists of a middle part of DNA nucleotide units which are not locked thus which are non-LNA units. The DNA nucleotides of this middle part could be linked to each other by the internucleotide linkages (IL) as disclosed herein which may be phosphate groups, phosphorothioate groups or phosphorodithioate groups and may be connected to nucleobase analogues such as 5-propynyl cytosine, 7-methylguanine, 7-methyladenine, 2-thiothymine, 2-thiocytosine, or 5-methylcytosine, but are not bicyclic pentose structures. The middle part of non-LNA units is flanked at the 3' end and the 5' end by sequences consisting of LNA units. Thus gapmers have the general formula:

### LNA sequence 1 - non-LNA sequence - LNA sequence 2 or region A - region B - region C

The middle part of the antisense-oligonucleotide which consists of DNA nucleotide units which are non-LNA units is, when formed in a duplex with the complementary target RNA, capable of recruiting RNase. The 3' and 5' terminal nucleotide units are LNA units which are preferably in alpha-L configuration, particularly preferred being alpha-L-oxy LNAs.

Thus, a gapmer is an antisense-oligonucleotide which comprises a contiguous stretch of DNA nucleotides which is capable of recruiting an RNase, such as RNaseH, such as a region of at least 6 or 7 DNA nucleotides which are non-LNA units, referred to herein as middle part or region B, wherein region B is flanked both 5' and 3' by regions of affinity enhancing nucleotide analogues which are LNA units, such as between 1 - 6 LNA units 5' and 3' to the contiguous stretch of DNA nucleotides which is capable of recruiting RNase - these flanking regions are referred to as regions A and C respectively.

Preferably the gapmer comprises a (poly)nucleotide sequence of formula (5' to 3'), A-B-C, or optionally A-B-C-D or D-A-B-C, wherein; region A (5' region) consists of at least one nucleotide analogue, such as at least one LNA unit, such as between 1-6 LNA units, and region B consists of at least five consecutive DNA nucleotides which are non-LNA units and which are capable of recruiting RNase (when formed in a duplex with a complementary RNA molecule, such as the mRNA target), and region C (3'region) consists of at least one nucleotide analogue, such as at least one LNA unit, such as between 1-6 LNA units, and region D, when present consists of 1, 2 or 3 DNA nucleotide units which are non-LNA units.

In some embodiments, region A consists of 1, 2, 3, 4, 5 or 6 LNA units, such as between 2-5 LNA units, such as 3 or 4 LNA units; and/or region C consists of 1, 2, 3, 4, 5 or 6 LNA units, such as between 2-5 LNA units, such as 3 or 4 LNA units.

In some embodiments B consists of 5, 6, 7, 8, 9, 10, 11 or 12 consecutive DNA nucleotides which are capable of recruiting RNase, or between 6-10, or between 7-9, such as 8 consecutive nucleotides which are capable of recruiting RNase. In some embodiments region B consists of at least one DNA nucleotide unit, such as 1-12 DNA nucleotide units, preferably between 4-12 DNA nucleotide units, more preferably between 6-10 DNA nucleotide units, still more preferred such as between 7-10 DNA nucleotide units, and most preferably 8, 9 or 10 DNA nucleotide units which are non-LNA units.

In some embodiments region A consist of 3 or 4 LNA, region B consists of 7, 8, 9 or 10 DNA nucleotide units, and region C consists of 3 or 4 LNA units. Such designs include (A-B-C): 1-7-2, 2-7-1, 2-7-2, 3-7-1, 3-7-2, 1-7-3, 2-7-3, 3-7-3, 2-7-4, 3-7-4, 4-7-2, 4-7-3, 4-7-4, 1-8-1, 1-8-2, 2-8-1, 2-8-2, 1-8-3, 3-8-1, 3-8-3, 2-8-3, 3-8-2, 4-8-1, 4-8-2, 1-8-4, 2-8-4, 3-8-4, 4-8-3, 4-8-4, 1-9-1, 1-9-2, 2-9-1, 2-9-2, 2-9-3, 3-9-2, 3-9-3, 1-9-3, 3-9-1, 4-9-1, 1-9-4, 4-9-2, 2-9-4, 4-9-3, 3-9-4, 4-9-4, 1-10-1, 1-10-2, 2-10-1, 2-10-2, 1-10-3, 3-10-1, 2-10-2, 2-10-3, 3-10-2, 3-10-3, 2-10-4, 4-10-2, 3-10-4, 4-10-3, 4-10-4, 1-11-1, 1-11-2, 2-11-1, 2-11-2, 1-11-3, 3-11-1, 2-11-2, 2-11-3, 3-11-2, 3-11-3, 2-11-4, 4-11-2, 3-11-4, 4-11-3, 4-11-4, and may further include region D, which may have one or 2 DNA nucleotide units, which are non-LNA units.

Further gapmer designs are disclosed in WO2004/046160A and are hereby incorporated by reference. US provisional application, 60/977409, hereby incorporated by reference, refers to 'shortmer' gapmer antisense-oligonucleotide, which are also suitable for the present invention.

In some embodiments the antisense-oligonucleotide consists of a contiguous nucleotide sequence of a total of 10, 11, 12, 13 or 14 nucleotide units (LNA units and non-LNA units together), wherein the contiguous nucleotide sequence is of formula (5' - 3'), A-B-C, or optionally A-B-C-D or D-A-B-C, wherein A consists of 1, 2 or 3 LNA units, and B consists of 7, 8 or 9 contiguous DNA nucleotide units which are non-LNA units and which are capable of recruiting RNase when formed in a duplex with a complementary RNA molecule (such as a mRNA target), and C consists of 1, 2 or 3 LNA units. When present, D consists of a single DNA nucleotide unit which is a non-LNA unit.

In some embodiments A consists of 1 LNA unit. In some embodiments A consists of 2 LNA units. In some embodiments A consists of 3 LNA units. In some embodiments C consists of 1 LNA unit. In some embodiments C consists of 2 LNA units. In some embodiments C consists of 3 LNA units. In some embodiments B consists of 7 DNA nucleotide units which are non-LNA units. In some embodiments B consists of 8 DNA nucleotide units. In some embodiments B consists of 9 DNA nucleotide units. In some embodiments B consists of 1 - 9 DNA nucleotide units, such as 2, 3, 4, 5, 6, 7 or 8 DNA nucleotide units. The DNA nucleotide units are always non-LNA units. In some embodiments B comprises 1, 2 or 3 LNA units which are preferably in the alpha-L configuration and which are more preferably alpha-L-oxy LNA units. In some embodiments the number of nucleotides present in A-B-C are selected from the group consisting of (LNA units - region B - LNA units and more preferably alpha-L-oxy LNA units (region A) - region B - (region C) alpha-L-oxy LNA units): 1-8-1, 1-8-2, 2-8-1, 2-8-2, 1-8-3, 3-8-1, 3-8-3, 2-8-3, 3-8-2, 4-8-1, 4-8-2, 1-8-4, 2-8-4, 3-8-4, 4-8-3, 4-8-4, 1-9-1, 1-9-2, 2-9-1, 2-9-2, 2-9-3, 3-9-2, 3-9-3, 1-9-3, 3-9-1, 4-9-1, 1-9-4, 4-9-2, 2-9-4, 4-9-3, 3-9-4, 4-9-4, 1-10-1, 1-10-2, 2-10-1, 2-10-2, 1-10-3, 3-10-1, 2-10-2, 2-10-3, 3-10-2, 3-10-3, 2-10-4, 4-10-2, 3-10-4, 4-10-3, 4-10-4, 1-11-1, 1-11-2, 2-11-1, 2-11-2, 1-11-3, 3-11-1, 2-11-2, 2-11-3, 3-11-2, 3-11-3, 2-11-4, 4-11-2, 3-11-4, 4-11-3, 4-11-4. In some embodiments the number of nucleotides in A-B-C are selected from the group consisting of:
Phosphorothioate or phosphorodithioate and especially phosphorothioate internucleotide linkages are also preferred, particularly for the gapmer region B. Phosphorothioate or phosphorodithioate linkages may also be used for the flanking regions (A and C, and for linking A or C to D, and within region D, if present).

Regions A, B and C, may however comprise internucleotide linkages other than phosphorothioate or phosphorodithioate, such as phosphodiester linkages, particularly, for instance when the use of nucleotide analogues protects the internucleotide linkages within regions A and C from endo-nuclease degradation - such as when regions A and C consist of LNA units.

The internucleotide linkages in the antisense-oligonucleotide may be phosphodiester, phosphorothioate, phosphorodithioate or boranophosphate so as to allow RNase H cleavage of targeted RNA. Phosphorothioate or phosphorodithioate is preferred, for improved nuclease resistance and other reasons, such as ease of manufacture. In one aspect of the oligomer of the invention, the LNA units and/or the non-LNA units are linked together by means of phosphorothioate groups.

It is recognized that the inclusion of phosphodiester linkages, such as one or two linkages, into an otherwise phosphorothioate antisense-oligonucleotide, particularly between or adjacent to LNA units (typically in region A and or C) can modify the bioavailability and/or bio-distribution of an antisense-oligonucleotide (see WO2008/053314A which is hereby incorporated by reference).

In some embodiments, such as in the sequences of the antisense-oligonucleotides disclosed herein and where suitable and not specifically indicated, all remaining internucleotide linkage groups are either phosphodiester groups or phosphorothioate groups, or a mixture thereof.

In some embodiments all the internucleotide linkage groups are phosphorothioate groups. When referring to specific gapmer antisense-oligonucleotide sequences, such as those provided herein, it will be understood that, in various embodiments, when the linkages are phosphorothioate linkages, alternative linkages, such as those disclosed herein may be used, for example phosphate (also named phosphodiester) linkages may be used, particularly for linkages between nucleotide analogues, such as LNA units. Likewise, when referring to specific gapmer antisense-oligonucleotide sequences, such as those provided herein, when the C residues are annotated as 5'-methyl modified cytosine, in various embodiments, one or more of the Cs present in the oligomer may be unmodified C residues.

### Gapmer Sequences

The following antisense-oligonucleotides in form of gapmers as listed in Table 2 and Table 3 are especially preferred.

As used herein the abbreviations b, d, s, ss have the following meaning:
- b: LNA unit or LNA nucleotide (any one selected from b¹ - b⁷)
- b¹: β-D-oxy-LNA
- b²: β-D-thio-LNA
- b³: β-D-amino-LNA
- b⁴: α-L-oxy-LNA
- b⁵: β-D-ENA
- b⁶: β-D-(NH)-LNA
- b⁷: β-D-(NCH₃)-LNA
- d: 2-deoxy, that means 2-deoxyribose units (e.g. formula **B3** or **B5** with R = -H)
- s: the internucleotide linkage is a phosphorothioate group (-O-P(O)(S⁻)-O-)
- ss: the internucleotide linkage is a phosphorodithioate group (-O-P(S)(S⁻)-O-)
- C*: methyl-C (5-methylcytosine); [consequently dC* is 5-methyldeoxycytosine]
nucleotides in **bold** are LNA nucleotides
nucleotides not in bold are non-LNA nucleotides

**Table 2**

| **SP** | **L** | **Seq ID No.** | **Sequence, 5'-3'** |
|---|---|---|---|
| 89 | 17 | 102 | **GbsCbsGbsAb**sdGsdTsdGsdAsdCsdTsdCsdAsdCs**TbsCbsAbsAb** |
| 90 | 15 | 103 | **CbsGbsAb**sdGsdTsdGsdAsdCsdTsdCsdAsdCs**TbsCbsAb** |
| 90 | 16 | 104 | **GbsCbsGb**sdAsdGsdTsdGsdAsdCsdTsdCsdAsdCs**TbsCbsAb** |
| 90 | 17 | 105 | **CbsGbsCbsGb**sdAsdGsdTsdGsdAsdCsdTsdCsdAs**CbsTbsCbsAb** |
| 91 | 14 | 106 | **CbsGbsAb**sdGsdTsdGsdAsdCsdTsdCsdAs**CbsTbsCb** |
| 91 | 16 | 107 | **CbsGbsCb**sdGsdAsdGsdTsdGsdAsdCsdTsdCsdAs**CbsTbsCb** |
| 91 | 17 | 108 | **GbsCbsGbsCb**sdGsdAsdGsdTsdGsdAsdCsdTsdCs**AbsCbsTbsCb** |
| 92 | 14 | 109 | **GbsCbsGb**sdAsdGsdTsdGsdAsdCsdTsdCs**AbsCbsTb** |
| 92 | 16 | 110 | **GbsCbsGb**sdCsdGsdAsdGsdTsdGsdAsdCsdTsdCs**AbsCbsTb** |
| 92 | 17 | 111 | **CbsGbsCbsGb**sdCsdGsdAsdGsdTsdGsdAsdCsdTs**CbsAbsCbsTb** |
| 93 | 12 | 112 | **CbsGb**sdAsdGsdTsdGsdAsdCsdTsdCs**AbsCb** |
| 93 | 13 | 113 | **GbsCbsGb**sdAsdGsdTsdGsdAsdCsdTsdCs**AbsCb** |
| 93 | 14 | 114 | **CbsGbsCb**sdGsdAsdGsdTsdGsdAsdCsdTs**CbsAbsCb** |
| 93 | 16 | 115 | **CbsGbsCb**sdGsdCsdGsdAsdGsdTsdGsdAsdCsdTs**CbsAbsCb** |
| 93 | 17 | 116 | **GbsCbsGbsCb**sdGsdCsdGsdAsdGsdTsdGsdAsdCs**TbsCbsAbsCb** |
| 94 | 13 | 117 | **CbsGbsCb**sdGsdAsdGsdTsdGsdAsdCsdTs**CbsAb** |
| 94 | 14 | 118 | **GbsCbsGb**sdCsdGsdAsdGsdTsdGsdAsdCs**TbsCbsAb** |
| 94 | 15 | 119 | **CbsGbsCb**sdGsdCsdGsdAsdGsdTsdGsdAsdCs**TbsCbsAb** |
| 94 | 16 | 120 | **GbsCbsGb**sdCsdGsdCsdGsdAsdGsdTsdGsdAsdCs**TbsCbsAb** |
| 94 | 17 | 121 | **TbsGbsCbsGb**sdCsdGsdCsdGsdAsdGsdTsdGsdAs**CbsTbsCbsAb** |
| 95 | 14 | 122 | **CbsGbsCb**sdGsdCsdGsdAsdGsdTsdGsdAs**CbsTbsCb** |
| 95 | 16 | 123 | **TbsGbsCb**sdGsdCsdGsdCsdGsdAsdGsdTsdGsdAs**CbsTbsCb** |
| 95 | 17 | 124 | **GbsTbsGbsCb**sdGsdCsdGsdCsdGsdAsdGsdTsdGs**AbsCbsTbsCb** |
| 96 | 13 | 125 | **CbsGbsCb**sdGsdCsdGsdAsdGsdTsdGsdAs**CbsTb** |
| 97 | 14 | 126 | **TbsGbsCb**sdGsdCsdGsdCsdGsdAsdGsdTs**GbsAbsCb** |
| 97 | 16 | 127 | **CbsGbsTb**sdGsdCsdGsdCsdGsdCsdGsdAsdGsdTs**GbsAbsCb** |
| 98 | 13 | 128 | **TbsGbsCb**sdGsdCsdGsdCsdGsdAsdGsdTs**GbsAb** |
| 107 | 16 | 129 | **GbsTbsCb**sdGsdTsdCsdGsdCsdTsdCsdCsdGsdTs**GbsCbsGb** |
| 108 | 15 | 130 | **GbsTbsCb**sdGsdTsdCsdGsdCsdTsdCsdCsdGs**TbsGbsCb** |
| 108 | 17 | 131 | **GbsTbsGbsTb**sdCsdGsdTsdCsdGsdCsdTsdCsdCs**GbsTbsGbsCb** |
| 109 | 13 | 132 | **TbsCbsGb**sdTsdCsdGsdCsdTsdCsdCsdGs**TbsGb** |
| 109 | 15 | 133 | **TbsGbsTb**sdCsdGsdTsdCsdGsdCsdTsdCsdCs**GbsTbsGb** |
| 110 | 12 | 134 | **TbsCb**sdGsdTsdCsdGsdCsdTsdCsdCs**GbsTb** |
| 110 | 13 | 135 | **GbsTbsCb**sdGsdTsdCsdGsdCsdTsdCsdCs**GbsTb** |
| 110 | 14 | 136 | **TbsGbsTb**sdCsdGsdTsdCsdGsdCsdTsdCs**CbsGbsTb** |
| 110 | 15 | 137 | **GbsTbsGb**sdTsdCsdGsdTsdCsdGsdCsdTsdCs**CbsGbsTb** |
| 110 | 16 | 138 | **GbsGbsTb**sdGsdTsdCsdGsdTsdCsdGsdCsdTsdCs**CbsGbsTb** |
| 351 | 16 | 139 | **CbsGbsTb**sdCsdAsdTsdAsdGsdAsdCsdCsdGsdAs**GbsCbsCb** |
| 351 | 12 | 140 | **AbsTb**sdAsdGsdAsdCsdCsdGsdAsdGs**CbsCb** |
| 354 | 16 | 141 | **GbsCbsTb**sdCsdGsdTsdCsdAsdTsdAsdGsdAsdCs**CbsGbsAb** |
| 354 | 13 | 142 | **CbsGbsTb**sdCsdAsdTsdAsdGsdAsdCsdCs**GbsAb** |
| 355 | 14 | 143 | **CbsTb**sdCsdGsdTsdCsdAsdTsdAsdGsdAs**CbsCbsGb** |
| 355 | 14 | 144 | **CbsTbsCb**sdGsdTsdCsdAsdTsdAsdGsdAsdCs**CbsGb** |
| 355 | 14 | 145 | **CbsTbsCb**sdGsdTsdCsdAsdTsdAsdGsdAs**CbsCbsGb** |
| 355 | 15 | 146 | **GbsCbsTb**sdCsdGsdTsdCsdAsdTsdAsdGsdAs**CbsCbsGb** |
| 356 | 14 | 147 | **GbsCbsTb**sdCsdGsdTsdCsdAsdTsdAsdGs**AbsCbsCb** |
| 381 | 17 | 148 | **CbsAbsGbsCb**sdCsdCsdCsdCsdGsdAsdCsdCsdCs**AbsTbsGbsGb** |
| 382 | 16 | 149 | **CbsAbsGb**sdCsdCsdCsdCsdCsdGsdAsdCsdCsdCs**AbsTbsGb** |
| 382 | 16 | 150 | **dCsdAsdG**sdCsdCsdCsdCsdCsdGsdAsdCsdCsdCs**dAsdTsdG** |
| 382 | 16 | 151 | **CbsAbsGb**sdCsdCsdCsdCsdCsdGsdAsdCsdCs**CbsAbsTbsGb** |
| 382 | 16 | 152 | **CbsAbsGbsCb**sdCsdCsdCsdCsdGsdAsdCsdCsdCs**AbsTbsGb** |
| 382 | 16 | 153 | **CbsAbsGbsCb**sdCsdCsdCsdCsdGsdAsdCsdCs**CbsAbsTbsGb** |
| 383 | 14 | 154 | **AbsGb**sdCsdCsdCsdCsdCsdGsdAsdCsdCs**CbsAbsTb** |
| 383 | 14 | 155 | **AbsGbsCb**sdCsdCsdCsdCsdGsdAsdCsdCsdCs**AbsTb** |
| 383 | 14 | 156 | **AbsGbsCb**sdCsdCsdCsdCsdGsdAsdCsdCs**CbsAbsTb** |
| 384 | 14 | 157 | **CbsAbsGb**sdCsdCsdCsdCsdCsdGsdAsdCs**CbsCbsAb** |
| 422 | 17 | 158 | **CbsGbsCbsGb**sdTsdCsdCsdAsdCsdAsdGsdGsdAs**CbsGbsAbsTb** |
| 425 | 14 | 159 | **CbsGbsCb**sdGsdTsdCsdCsdAsdCsdAsdGs**GbsAbsCb** |
| 429 | 15 | 160 | **CbsGbsAb**sdTsdAsdCsdGsdCsdGsdTsdCsdCs**AbsCbsAb** |
| 429 | 15 | 161 | **CbsGbsAb**sdTsdAsdCsdGsdCsdGsdTsdCs**CbsAbsCbsAb** |
| 429 | 15 | 162 | **CbsGbsAbsTb**sdAsdCsdGsdCsdGsdTsdCsdCs**AbsCbsAb** |
| 432 | 12 | 163 | **CbsGb**sdAsdTsdAsdCsdGsdCsdGsdTs**CbsCb** |
| 431 | 13 | 164 | **CbsGbsAb**sdTsdAsdCsdGsdCsdGsdTsdCs**CbsAb** |
| 431 | 13 | 165 | **CbsGb**sdAsdTsdAsdCsdGsdCsdGsdTs**CbsCbsAb** |
| 431 | 16 | 166 | **TbsGbsGb**sdCsdGsdAsdTsdAsdCsdGsdCsdGsdTs**CbsCbsAb** |
| 432 | 12 | 167 | **CbsGb**sdAsdTsdAsdCsdGsdCsdGsdTsdCs**Cb** |
| 432 | 12 | 168 | **Cb**sdGsdAsdTsdAsdCsdGsdCsdGsdTs**CbsCb** |
| 432 | 13 | 169 | **GbsCbsGb**sdAsdTsdAsdCsdGsdCsdGsdTs**CbsCb** |
| 432 | 17 | 170 | **GbsCbsTbsGb**sdGsdCsdGsdAsdTsdAsdCsdGsdCs**GbsTbsCbsCb** |
| 433 | 15 | 171 | **CbsTbsGb**sdGsdCsdGsdAsdTsdAsdCsdGsdCs**GbsTbsCb** |
| 433 | 12 | 172 | **GbsCb**sdGsdAsdTsdAsdCsdGsdCsdGs**TbsCb** |
| 433 | 16 | 173 | **GbsCbsTb**sdGsdGsdCsdGsdAsdTsdAsdCsdGsdCs**GbsTbsCb** |
| 433 | 14 | 174 | **TbsGbsGb**sdCsdGsdAsdTsdAsdCsdGsdCs**GbsTbsCb** |
| 434 | 12 | 175 | **GbsGb**sdCsdGsdAsdTsdAsdCsdGsdCs**GbsTb** |
| 434 | 13 | 176 | **TbsGbsGb**sdCsdGsdAsdTsdAsdCsdGsdCs**GbsTb** |
| 434 | 13 | 177 | **TbsGb**sdGsdCsdGsdAsdTsdAsdCsdGs**CbsGbsTb** |
| 434 | 14 | 178 | **CbsTbsGb**sdGsdCsdGsdAsdTsdAsdCsdGs**CbsGbsTb** |
| 435 | 13 | 179 | **CbsTbsGb**sdGsdCsdGsdAsdTsdAsdCsdGs**CbsGb** |
| 435 | 12 | 180 | **TbsGb**sdGsdCsdGsdAsdTsdAsdCsdGs**CbsGb** |
| 437 | 17 | 181 | **AbsTbsCbsGb**sdTsdGsdCsdTsdGsdGsdCsdGsdAs**TbsAbsCbsGb** |
| 449 | 16 | 182 | **CbsGbsTb**sdGsdCsdGsdGsdTsdGsdGsdGsdAsdTs**CbsGbsTb** |
| 449 | 17 | 183 | **AbsCbsGbsTb**sdGsdCsdGsdGsdTsdGsdGsdGsdAs**TbsCbsGbsTb** |
| 450 | 17 | 184 | **AbsAbsCbsGb**sdTsdGsdCsdGsdGsdTsdGsdGsdGs**AbsTbsCbsGb** |
| 452 | 15 | 185 | **AbsAbsCb**sdGsdTsdGsdCsdGsdGsdTsdGsdGs**GbsAbsTb** |
| 452 | 17 | 186 | **TbsGbsAbsAb**sdCsdGsdTsdGsdCsdGsdGsdTsdGs**GbsGbsAbsTb** |
| 459 | 17 | 187 | **CbsGbsAbsCb**sdTsdTsdCsdTsdGsdAsdAsdCsdGs**TbsGbsCbsGb** |
| 941 | 17 | 188 | **TbsTbsAbsAb**sdCsdGsdCsdGsdGsdTsdAsdGsdCs**AbsGbsTbsAb** |
| 941 | 16 | 189 | **TbsAbsAb**sdCsdGsdCsdGsdGsdTsdAsdGsdCsdAs**GbsTbsAb** |
| 942 | 17 | 190 | **GbsTbsTbsAb**sdAsdCsdGsdCsdGsdGsdTsdAsdGs**CbsAbsGbsTb** |
| 943 | 15 | 191 | **TbsTbsAb**sdAsdCsdGsdCsdGsdGsdTsdAsdGs**CbsAbsGb** |
| 944 | 13 | 192 | **TbsAbsAb**sdCsdGsdCsdGsdGsdTsdAsdGs**CbsAb** |
| 945 | 12 | 193 | **TbsAb**sdAsdCsdGsdCsdGsdGsdTsdAs**GbsCb** |
| 945 | 13 | 194 | **TbsTbsAb**sdAsdCsdGsdCsdGsdGsdTsdAs**GbsCb** |
| 946 | 12 | 195 | **TbsTb**sdAsdAsdCsdGsdCsdGsdGsdTs**AbsGb** |
| 946 | 13 | 196 | **GbsTbsTb**sdAsdAsdCsdGsdCsdGsdGsdTs**AbsGb** |
| 946 | 15 | 197 | **CbsGbsGb**sdTsdTsdAsdAsdCsdGsdCsdGsdGs**TbsAbsGb** |
| 946 | 16 | 198 | **CbsCbsGb**sdGsdTsdTsdAsdAsdCsdGsdCsdGsdGs**TbsAbsGb** |
| 947 | 14 | 199 | **CbsGbsGb**sdTsdTsdAsdAsdCsdGsdCsdGs**GbsTbsAb** |
| 947 | 13 | 200 | **GbsGbsTb**sdTsdAsdAsdCsdGsdCsdGsdGs**TbsAb** |
| 947 | 15 | 201 | **CbsCbsGb**sdGsdTsdTsdAsdAsdCsdGsdCsdGs**GbsTbsAb** |
| 947 | 16 | 202 | **GbsCbsCb**sdGsdGsdTsdTsdAsdAsdCsdGsdCsdGs**GbsTbsAb** |
| 947 | 17 | 203 | **TbsGbsCbsCb**sdGsdGsdTsdTsdAsdAsdCsdGsdCs**GbsGbsTbsAb** |
| 948 | 13 | 204 | **CbsGbsGb**sdTsdTsdAsdAsdCsdGsdCsdGs**GbsTb** |
| 949 | 13 | 205 | **CbsCbsGb**sdGsdTsdTsdAsdAsdCsdGsdCs**GbsGb** |
| 949 | 14 | 206 | **GbsCbsCb**sdGsdGsdTsdTsdAsdAsdCsdGs**CbsGbsGb** |
| 949 | 15 | 207 | **TbsGbsCb**sdCsdGsdGsdTsdTsdAsdAsdCsdGs**CbsGbsGb** |
| 950 | 13 | 208 | **GbsCbsCb**sdGsdGsdTsdTsdAsdAsdCsdGs**CbsGb** |
| 950 | 15 | 209 | **CbsTbsGb**sdCsdCsdGsdGsdTsdTsdAsdAsdCs**GbsCbsGb** |
| 950 | 16 | 210 | **GbsCbsTb**sdGsdCsdCsdGsdGsdTsdTsdAsdAsdCs**GbsCbsGb** |
| 1387 | 16 | 211 | **AbsTbsGb**sdCsdCsdGsdCsdGsdTsdCsdAsdGsdGs**TbsAbsCb** |
| 1392 | 13 | 212 | **AbsCbsAb**sdTsdGsdCsdCsdGsdCsdGsdTs**CbsAb** |
| 1393 | 16 | 213 | **GbsAbsTb**sdGsdAsdCsdAsdTsdGsdCsdCsdGsdCs**GbsTbsCb** |
| 1393 | 16 | 214 | **GbsAbsTb**sdGsdAsdCsdAsdTsdGsdCsdCsdGs**CbsGbsTbsCb** |
| 1393 | 16 | 215 | **GbsAbsTbsGb**sdAsdCsdAsdTsdGsdCsdCsdGsdCs**GbsTbsCb** |
| 1393 | 16 | 216 | **GbsAbsTbsGb**sdAsdCsdAsdTsdGsdCsdCsdGs**CbsGbsTbsCb** |
| 1394 | 12 | 217 | **GbsAb**sdCsdAsdTsdGsdCsdCsdGsdCs**GbsTb** |
| 1394 | 15 | 218 | **GbsAbsTb**sdGsdAsdCsdAsdTsdGsdCsdCsdGs**CbsGbsTb** |
| 1395 | 13 | 219 | **AbsTbsGb**sdAsdCsdAsdTsdGsdCsdCsdGs**CbsGb** |
| 1805 | 17 | 220 | **TbsCbsCbsCb**sdGsdCsdAsdCsdCsdTsdTsdGsdGs**AbsAbsCbsCb** |
| 1851 | 16 | 221 | **CbsGbsAb**sdTsdCsdTsdCsdTsdCsdAsdAsdCsdAs**CbsGbsTb** |
| 1851 | 17 | 222 | **TbsCbsGbsAb**sdTsdCsdTsdCsdTsdCsdAsdAsdCs**AbsCbsGbsTb** |
| 1852 | 15 | 223 | **CbsGbsAb**sdTsdCsdTsdCsdTsdCsdAsdAsdCs**AbsCbsGb** |
| 1852 | 16 | 224 | **TbsCbsGb**sdAsdTsdCsdTsdCsdTsdCsdAsdAsdCs**AbsCbsGb** |
| 1852 | 17 | 225 | **CbsTbsCbsGb**sdAsdTsdCsdTsdCsdTsdCsdAsdAs**CbsAbsCbsGb** |
| 2064 | 16 | 226 | **GbsTb**sdAsdGsdTsdGsdTsdTsdTsdAsdGsdGsdGsAbs**GbsCb** |
| 2064 | 16 | 227 | **GbsTbsAbsGb**sdTsdGsdTsdTsdTsdAsdGsdGsdGs**AbsGbsCb** |
| 2064 | 16 | 228 | **GbsTbsAb**sdGsdTsdGsdTsdTsdTsdAsdGsdGsdGs**AbsGbsCb** |
| 2064 | 16 | 229 | **GbsTbsAb**sdGsdTsdGsdTsdTsdTsdAsdGsdGsdGsdAs**GbsCb** |
| 2064 | 16 | 230 | **GbsTbsAb**sdGsdTsdGsdTsdTsdTsdAsdGsdGs**GbsAbsGbsCb** |
| 2064 | 16 | 231 | **GbsTbsAbsGb**sdTsdGsdTsdTsdTsdAsdGsdGs**GbsAbsGbsCb** |
| 2064 | 16 | 232 | **GbsTb**sdAsdGsdTsdGsdTsdTsdTsdAsdGsdGsdGs**AbsGbsCb** |
| 2064 | 16 | 233 | **GbsTbsAbsGbsTb**sdGsdTsdTsdTsdAsdGsdGs**GbsAbsGbsCb** |
| 2064 | 16 | 234 | **GbsTbsAbsGb**sdTsdGsdTsdTsdTsdAsdGsGbs**GbsAbsGbsCb** |
| 2064 | 16 | 235 | **GbsTbsAbsGbsTb**sdGsdTsdTsdTsdAsdGs**GbsGbsAbsGbsCb** |
| 2072 | 16 | 236 | **GbsCb**sdTsdAsdTsdTsdTsdGsdGsdTsdAsdGsdTs**GbsTbsTb** |
| 2072 | 16 | 237 | **GbsCbsTb**sdAsdTsdTsdTsdGsdGsdTsdAsdGsdTsdGs**TbsTb** |
| 2072 | 16 | 238 | **GbsCbsTb**sdAsdTsdTsdTsdGsdGsdTsdAsdGsdTs**GbsTbsTb** |
| 2072 | 16 | 239 | **GbsCbsTb**sAbsdTsdTsdTsdGsdGsdTsdAsdGsdTs**GbsTbsTb** |
| 2072 | 16 | 240 | **GbsCbsTb**sdAsdTsdTsdTsdGsdGsdTsdAsdGs**TbsGbsTbsTb** |
| 2072 | 16 | 241 | **GbsCbsTb**sAbsdTsdTsdTsdGsdGsdTsdAsdGs**TbsGbsTbsTb** |
| 2284 | 15 | 242 | **AbsGbsCb**sdTsdTsdAsdTsdCsdCsdTsdAsdTs**GbsAbsCb** |
| 2284 | 15 | 243 | **AbsGbsCb**sdTsdTsdAsdTsdCsdCsdTsdAs**TbsGbsAbsCb** |
| 2284 | 15 | 244 | **AbsGbsCbsTb**sdTsdAsdTsdCsdCsdTsdAsdTs**GbsAbsCb** |
| 2285 | 14 | 245 | **AbsGb**sdCsdTsdTsdAsdTsdCsdCsdTsdAs**TbsGbsAb** |
| 2285 | 14 | 246 | **AbsGbsCb**sdTsdTsdAsdTsdCsdCsdTsdAsdTs**GbsAb** |
| 2285 | 14 | 247 | **AbsGbsCb**sdTsdTsdAsdTsdCsdCsdTsdAs**TbsGbsAb** |
| 2355 | 17 | 248 | **CbsAbsGb**sdGsdCsdAsdTsdTsdAsdAsdTsdAsdAsdAs**GbsTbsGb** |
| 2355 | 17 | 249 | **CbsAbsGbsGb**sdCsdAsdTsdTsdAsdAsdTsdAsdAsdAs**GbsTbsGb** |
| 2355 | 17 | 250 | **CbsAbsGb**sdGsdCsdAsdTsdTsdAsdAsdTsdAsdAs**AbsGbsTbsGb** |
| 2355 | 17 | 251 | **CbsAbsGbs**GbsdCsdAsdTsdTsdAsdAsdTsdAsdAs**AbsGbsTbsGb** |
| 4217 | 16 | 252 | **CbsAbsTb**sdGsdAsdAsdTsdGsdGsdAsdCsdCsdAs**GbsTbsAb** |
| 4217 | 16 | 253 | **CbsAbsTb**sdGsdAsdAsdTsdGsdGsdAsdCsdCs**AbsGbsTbsAb** |
| 4217 | 16 | 254 | **CbsAbsTbsGb**sdAsdAsdTsdGsdGsdAsdCsdCsdAs**GbsTbsAb** |
| 4217 | 16 | 255 | **CbsAbsTbsGb**sdAsdAsdTsdGsdGsdAsdCsdCs**AbsGbsTbsAb** |
| 4120 | 16 | 256 | **CbsTbsAb**sdGsdGsdCsdGsdCsdCsdTsdCsdTsdAs**TbsGbsCb** |
| 4121 | 14 | 257 | **TbsAbsGb**sdGsdCsdGsdCsdCsdTsdCsdTs**AbsTbsGb** |
| 4121 | 15 | 258 | **CbsTbsAb**sdGsdGsdCsdGsdCsdCsdTsdCsdTs**AbsTbsGb** |
| 4122 | 13 | 259 | **TbsAbsGb**sdGsdCsdGsdCsdCsdTsdCsdTs**AbsTb** |

**Table 3**

| **SP** | **L** | **Seq ID No.** | **Sequence, 5'-3'** |
|---|---|---|---|
| 2064 | 16 | 260 | **GbsTbsAbsGb**sdTsdGsdTsdTsdTsdAsdGsdGsdGs**AbsGbsC*b** |
| 2064 | 16 | 261 | **GbsTbsAb**sdGsdTsdGsdTsdTsdTsdAsdGsdGsdGs**AbsGbsC*b** |
| 2064 | 16 | 262 | **GbsTbsAb**sdGsdTsdGsdTsdTsdTsdAsdGsdGsdGsdAs**GbsC*b** |
| 2064 | 16 | 263 | **GbsTbsAb**sdGsdTsdGsdTsdTsdTsdAsdGsdGs**GbsAbsGbsC*b** |
| 2064 | 16 | 264 | **GbsTbsAbsGb**sdTsdGsdTsdTsdTsdAsdGsdGs**GbsAbsGbsC*b** |
| 2064 | 16 | 265 | **GbsTb**sdAsdGsdTsdGsdTsdTsdTsdAsdGsdGsdGs**AbsGbsC*b** |
| 429 | 15 | 266 | **C*bsGbsAbsTb**sdAsdC*sdGsdC*sdGsdTsdC*sdC*s**AbsC*bsAb** |
| 4217 | 16 | 267 | **C*bsAbsTb**sdGsdAsdAsdTsdGsdGsdAsdC*sdC*s**AbsGbsTbsAb** |
| 2355 | 17 | 268 | **C*bsAbsGb**sdGsdC*sdAsdTsdTsdAsdAsdTsdAsdAs**AbsGbsTbsGb** |
| 2355 | 17 | 269 | **C*bsAbsGb**sdGsdC*sdAsdTsdTsdAsdAsdTsdAsdAsdAs**GbsTbsGb** |
| 432 | 12 | 270 | **C*bsGb**sdAsdTsdAsdC*sdGsdC*sdGsdTs**C*bsC*b** |
| 4217 | 16 | 271 | **C*bsAbsTbsGb**sdAsdAsdTsdGsdGsdAsdC*sdC*s**AbsGbsTbsAb** |
| 2072 | 16 | 272 | **GbsC*bsTbsAb**sdTsdTsdTsdGsdGsdTsdAsdGsdTs**GbsTbsTb** |
| 2072 | 16 | 273 | **GbsC*b**sdTsdAsdTsdTsdTsdGsdGsdTsdAsdGsdTs**GbsTbsTb** |
| 432 | 12 | 274 | **C*bsGb**sdAsdTsdAsdC*sdGsdC*sdGsdTsdC*s**C*b** |
| 2072 | 16 | 275 | **GbsC*bsTb**sdAsdTsdTsdTsdGsdGsdTsdAsdGsdTsdGs**TbsTb** |
| 432 | 12 | 276 | **C*b**sdGsdAsdTsdAsdC*sdGsdC*sdGsdTs**C*bsC*b** |
| 431 | 13 | 277 | **C*bsGbsAb**sdTsdAsdC*sdGsdC*sdGsdTsdC*s**C*bsAb** |
| 429 | 15 | 278 | **C*bsGbsAb**sdTsdAsdC*sdGsdC*sdGsdTsdC*sdC*s**AbsC*bsAb** |
| 4217 | 16 | 279 | **C*bsAbsTbsGb**sdAsdAsdTsdGsdGsdAsdC*sdC*sdAs**GbsTbsAb** |
| 1393 | 16 | 280 | **GbsAbsTb**sdGsdAsdC*sdAsdTsdGsdC*sdC*sdGsdC*s**GbsTbsC*b** |
| 2285 | 14 | 281 | **AbsGbsC*b**sdTsdTsdAsdTsdC*sdC*sdTsdAsdTs**GbsAb** |
| 355 | 14 | 282 | **C*bsTb**sdC*sdGsdTsdC*sdAsdTsdAsdGsdAs**C*bsC*bsGb** |
| 2072 | 16 | 283 | **GbsC*bsTb**sdAsdTsdTsdTsdGsdGsdTsdAsdGsdTs**GbsTbsTb** |
| 1393 | 16 | 284 | **GbsAbsTb**sdGsdAsdC*sdAsdTsdGsdC*sdC*sdGs**C*bsGbsTbsC*b** |
| 2355 | 17 | 285 | **C*bsAbsGbsGb**sdC*sdAsdTsdTsdAsdAsdTsdAsdAs**AbsGbsTbsGb** |
| 429 | 15 | 286 | **C*bsGbsAb**sdTsdAsdC*sdGsdC*sdGsdTsdC*s**C*bsAbsC*bsAb** |
| 2285 | 14 | 287 | **AbsGbsC*b**sdTsdTsdAsdTsdC*sdC*sdTsdAs**TbsGbsAb** |
| 355 | 14 | 288 | **C*bsTbsC*b**sdGsdTsdC*sdAsdTsdAsdGsdAs**C*bsC*bsGb** |
| 1393 | 16 | 289 | **GbsAbsTbsGb**sdAsdC*sdAsdTsdGsdC*sdC*sdGs**C*bsGbsTbsC*b** |
| 1393 | 16 | 290 | **GbsAbsTbsGb**sdAsdC*sdAsdTsdGsdC*sdC*sdGsdC*s**GbsTbsC*b** |
| 4217 | 16 | 291 | **C*bsAbsTb**sdGsdAsdAsdTsdGsdGsdAsdC*sdC*sdAs**GbsTbsAb** |
| 2285 | 14 | 292 | **AbsGb**sdC*sdTsdTsdAsdTsdC*sdC*sdTsdAs**TbsGbsAb** |
| 434 | 13 | 293 | **TbsGbsGb**sdC*sdGsdAsdTsdAsdC*sdGsdC*s**GbsTb** |
| 383 | 14 | 294 | **AbsGbsC*b**sdC*sdC*sdC*sdC*sdGsdAsdC*sdC*sdC*s**AbsTb** |
| 431 | 13 | 295 | **C*bsGb**sdAsdTsdAsdC*sdGsdC*sdGsdTs**C*bsC*bsAb** |
| 2284 | 15 | 296 | **AbsGbsC*b**sdTsdTsdAsdTsdC*sdC*sdTsdAsdTs**GbsAbsC*b** |
| 355 | 14 | 297 | **C*bsTbsC*b**sdGsdTsdC*sdAsdTsdAsdGsdAsdC*s**C*bsGb** |
| 2284 | 15 | 298 | **AbsGbsC*b**sdTsdTsdAsdTsdC*sdC*sdTsdAs**TbsGbsAbsC*b** |
| 383 | 14 | 299 | **AbsGbsC*b**sdC*sdC*sdC*sdC*sdGsdAsdC*sdC*s**C*bsAbsTb** |
| 383 | 14 | 300 | **AbsGb**sdC*sdC*sdC*sdC*sdC*sdGsdAsdC*sdC*s**C*bsAbsTb** |
| 382 | 16 | 301 | **C*bsAbsGb**sdC*sdC*sdC*sdC*sdC*sdGsdAsdC*sdC*sdC*s**AbsTbsGb** |
| 2072 | 16 | 302 | **GbsC*bsTb**sdAsdTsdTsdTsdGsdGsdTsdAsdGs**TbsGbsTbsTb** |
| 2072 | 16 | 303 | **GbsC*bsTb**sAbsdTsdTsdTsdGsdGsdTsdAsdGs**TbsGbsTbsTb** |
| 434 | 13 | 304 | **TbsGb**sdGsdC*sdGsdAsdTsdAsdC*sdGs**C*bsGbsTb** |
| 2284 | 15 | 305 | **AbsGbsC*bsTb**sdTsdAsdTsdC*sdC*sdTsdAsdTs**GbsAbsC*b** |
| 382 | 16 | 306 | **C*bsAbsGb**sdC*sdC*sdC*sdC*sdC*sdGsdAsdC*sdC*s**C*bsAbsTbsGb** |
| 382 | 16 | 307 | **C*bsAbsGbsC*b**sdC*sdC*sdC*sdC*sdGsdAsdC*sdC*sdC*s**AbsTbsGb** |
| 382 | 16 | 308 | **dC*bsdAbsdGb**sdC*sdC*sdC*sdC*sdC*sdGsdAsdC*sdC* sdC*s**dAbsdTbsdGb** |
| 2355 | 17 | 309 | **C*bsAbsGb**sGbsdC*sdAsdTsdTsdAsdAsdTsdAsdAsdAs**GbsTbsGb** |
| 382 | 16 | 310 | **C*bsAbsGbsC*b**sdC*sdC*sdC*sdC*sdGsdAsdC*sdC*sC***bsAbsTbsGb** |

### Pharmaceutical Compositions

The antisense-oligonucleotides of the present invention are preferably administered in form of their pharmaceutically active salts optionally using substantially nontoxic pharmaceutically acceptable carriers, excipients, adjuvants, solvents or diluents. The medications of the present invention are prepared in a conventional solid or liquid carrier or diluents and a conventional pharmaceutically-made adjuvant at suitable dosage level in a known way. The preferred preparations and formulations are in administratable form which is suitable for infusion or injection (intrathecal, intracerebroventricular, intracranial, intravenous, intraperitoneal, intramuscular, subcutaneous), local administration into the brain, inhalation, local administration into a solid tumor or oral application. However also other applications form are possible such as absorption through epithelial or mucocutaneous linings (oral mucosa, rectal and vaginal epithelial linings, nasopharyngial mucosa, intestinal mucosa), rectally, transdermally, topically, intradermally, intragastrically, intracutaneously, intravaginally, intravasally, intranasally, intrabuccally, percutaneously, sublingually, or any other means available within the pharmaceutical arts.
The administratable formulations, for example, include injectable liquid formulations, retard formulations, powders especially for inhalation, pills, tablets, film tablets, coated tablets, dispersible granules, dragees, gels, syrups, slurries, suspensions, emulsions, capsules and deposits. Other administratable galenical formulations are also possible like a continuous injection through an implantable pump or a catheter into the brain.

As used herein the term "pharmaceutically acceptable" refers to any carrier which does not interfere with the effectiveness of the biological activity of the antisense-oligonucleotides as active ingredient in the formulation and that is not toxic to the host to which it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and the active compound can be administered to the subject at an effective dose.
An "effective dose" refers to an amount of the antisense-oligonucleotide as active ingredient that is sufficient to affect the course and the severity of the disease, leading to the reduction or remission of such pathology. An "effective dose" useful for treating and/or preventing these diseases or disorders may be determined using methods known to one skilled in the art. Furthermore, the antisense-oligonucleotides of the present invention may be mixed and administered together with liposomes, complex forming agents, receptor targeted molecules, solvents, preservatives and/or diluents.

Preferred are pharmaceutical preparations in form of **infusion** solutions or solid matrices for continuous release of the active ingredient, especially for continuous infusion for **intrathecal** administration, intracerebroventricular administration or intracranial administration of at least one antisense-oligonucleotide of the present invention. Also preferred are pharmaceutical preparations in form of solutions or solid matrices suitable for local administration into the brain. For fibrotic diseases of the lung, inhalation formulations are especially preferred.

A ready-to-use sterile solution comprises for example at least one antisense-oligonucleotide at a concentration ranging from 1 to 10 mg/ml, preferably from 5 to 10mg/ml and an isotonic agent selected, for example, amongst sugars such as sucrose, lactose, mannitol or sorbitol. A suitable buffering agent, to control the solution pH to 4 to 6 (preferably 5), may be also included. Another optional ingredient of the formulation can be a non-ionic surfactant, such as Tween 20 or Tween 80.

A sterile lyophilized **powder** to be reconstituted for use comprises at least one antisense-oligonucleotide, and optionally a bulking agent (e.g. mannitol, trehalose, sorbitol, glycine) and/or a cryoprotectent (e.g. trehalose, mannitol). The solvent for reconstitution can be water for injectable compounds, with or without a buffering salt to control the pH to 4 to 6.

Aerosol preparations suitable for **inhalation** may include solutions and solids in powder form, which may be in combination with a pharmaceutically acceptable carrier such as inert compressed gas, e.g. nitrogen.

A particularly preferred pharmaceutical composition is a lyophilised (freeze-dried) preparation (lyophilisate) suitable for administration by inhalation or for intravenous administration. To prepare the preferred lyophilised preparation at least one antisense-oligonucleotide of the invention is solubilised in a 4 to 5% (w/v) mannitol solution and the solution is then lyophilised. The mannitol solution can also be prepared in a suitable buffer solution as described above.

Further examples of suitable cryo- / lyoprotectants (otherwise referred to as bulking agents or stabilizers) include thiol-free albumin, immunoglobulins, polyalkyleneoxides (e.g. PEG, polypropylene glycols), trehalose, glucose, sucrose, sorbitol, dextran, maltose, raffinose, stachyose and other saccharides (cf. for instance WO 97/29782), while mannitol is used preferably. These can be used in conventional amounts in conventional lyophilization techniques. Methods of lyophilisation are well known in the art of preparing pharmaceutical formulations.

For administration by inhalation the particle diameter of the lyophilised preparation is preferably between 2 to 5 µm, more preferably between 3 to 4 µm. The lyophilised preparation is particularly suitable for administration using an inhalator, for example the OPTINEB^{®} or VENTA-NEB^{®} inhalator (NEBU-TEC, Elsenfeld, Germany). The lyophilised product can be rehydrated in sterile distilled water or any other suitable liquid for inhalation administration. Alternatively for intravenous administration the lyophilised product can be rehydrated in sterile distilled water or any other suitable liquid for intravenous administration.

After rehydration for administration in sterile distilled water or another suitable liquid the lyophilised preparation should have the approximate physiological osmolality of the target tissue for the rehydrated peptide preparation i.e. blood for intravenous administration or lung tissue for inhalation administration. Thus it is preferred that the rehydrated formulation is substantially isotonic.

The preferred dosage concentration for either intravenous, oral, or inhalation administration is between 100 to 2000 µmol/ml, and more preferably is between 200 to 800 µmol/ml.

For oral administration in the form of tablets or capsules, the at least one antisense-oligonucleotide may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethyl-cellulose, polyethylene glycol and waxes. Among the lubricants that may be mentioned for use in these dosage forms, boric acid, sodium benzoate, sodium acetate, sodium chloride, and the like. Disintegrants include starch, methylcellulose, guar gum and the like.

Additionally, the compositions of the present invention may be formulated in sustained release form to provide the rate controlled release of the at least one antisense-oligonucleotide to optimize the therapeutic effects. Suitable dosage forms for sustained release include implantable biodegradable matrices for sustained release containing the at least one antisense-oligonucleotide, layered tablets containing layers of varying disintegration rates or controlled release polymeric matrices impregnated with the at least one antisense-oligonucleotide.

Liquid form preparations include solutions, suspensions and emulsions. As an example may be mentioned water or water-propylene glycol solutions for parenteral injections or addition of sweeteners and opacifiers for oral solutions, suspensions and emulsions.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5% to about 95% by weight of the total composition, preferably from about 25% to about 75 by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 1 to about 40% by weight of the composition, preferably 2 to about 30% by weight of the composition, more preferably from about 3 to 20% by weight of the composition, and most preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropyl-methylcellulose; polyvinylpyrrolidone; and inorganics such as magnesium aluminum silicate. The amount of binder in the composition can range from about 1 to 30% by weight of the composition, preferably from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate, stearic acid; high melting point waxes; and water soluble lubricants such as sodium chloride, sodium benzoate, sodium acetate, sodium oleate, polyethylene glycols and d'l-leucine. Lubricants are usually added at the very last step before compression, since they must be present on the surfaces of the granules and in between them and the parts of the tablet press. The amount of lubricant in the composition can range from about 0.05 to about 15% by weight of the composition, preferably 0.2 to about 5% by weight of the composition, more preferably from about 0.3 to about 3%, and most preferably from about 0.3 to about 1.5% by weight of the composition.

Glidents are materials that prevent caking and improve the flow characteristics of granulations, so that flow is smooth and uniform. Suitable glidents include silicon dioxide and talc. The amount of glident in the composition can range from about 0.01 to 10% by weight of the composition, preferably 0.1 % to about 7% by weight of the total composition, more preferably from about 0.2 to 5% by weight, and most preferably from about 0.5 to about 2% by weight.

In the pharmaceutical compositions disclosed herein the antisense-oligonucleotides are incorporated preferably in the form of their salts and optionally together with other components which increase stability of the antisense-oligonucleotides, increase recruitment of RNase H, increase target finding properties, enhance cellular uptake and the like. In order to achieve these goals, the antisense-oligonucleotides may be chemically modified instead of or in addition to the use of the further components useful for achieving these purposes. Thus the antisense-oligonucleotides of the invention may be chemically linked to moieties or components which enhance the activity, cellular distribution or cellular uptake etc. of the antisense-oligonucleotides. Such moieties include lipid moieties such as a cholesterol moiety, cholic acid, a thioether, hexyl-S-tritylthiol, a thiocholesterol, an aliphatic chain, e.g., dodecandiol or undecyl residues, a phospholipid such as dihexadecyl-rac-glycerol or triethylammonium-1,2-di-O-hexadecyl-rac-glycero-3H-phosphonate, a polyamine or a polyethylene glycol chain, or adamantine acetic acid, a palmityl moiety, or an octadecylamine or hexylamino-carbonyl-oxycholesterol moiety. The present invention also includes antisense-oligonucleotides which are chimeric compounds.

"Chimeric" antisense-oligonucleotides in the context of this invention, are antisense-oligonucleotides, which contain two or more chemically distinct regions, one is the oligonucleotide sequence as disclosed herein which is connected to a moiety or component for increasing cellular uptake, increasing resistance to nuclease degradation, increasing binding affinity for the target nucleic acid, increasing recruitment of RNase H and so on. For instance, the additional region or moiety or component of the antisense-oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target which is the mRNA coding for the TGF-R_{II}, thereby greatly enhancing the efficiency of antisense-oligonucleotide inhibition of gene expression. Consequently, comparable results can often be obtained with shorter oligonucleotides when chimeric oligonucleotides are used.

### Indications

The present invention relates to the use of the antisense-oligonucleotides disclosed herein for prophylaxis and treatment of neurodegenerative diseases, neurotrauma, neurovascular and neuroinflammatory including postinfectious and inflammatory disorders of the central nervous system (CNS).

The antisense-oligonucleotides of the present invention are especially useful for promoting regeneration and functional reconnection of damaged neural pathways and/or for the treatment and compensation of age induced decreases in neuronal stem cell renewal.

Thus, another aspect of the present invention relates to the use of an antisense-oligonucleotide as disclosed herein for promoting regeneration by reactivating neurogenesis, allowing neuronal differentiation and migration, and inducing integration of new neurons into anatomic and functional neuronal circuits.

A further aspect of the present invention relates to the use of an antisense-oligonucleotide as disclosed herein for promoting regeneration and clinical repair in patients with damage to the nervous system or damage to other organ systems induced by fibrosis or loss of stem cell turnover.

Moreover the antisense-oligonucleotides are useful for compensation and treatment of decreases in neuronal stem cell renewal induced by age, inflammation or a gene defect.

The antisense-oligonucleotides of the present invention inhibit the TGF-R_{II} expression and are consequently used for the treatment of diseases associated with up-regulated or enhanced TGF-R and/or TGF-R_{II} levels.

Thus another aspect of the present invention relates to the use of the antisense-oligonucleotides in the prophylaxis and treatment of neurodegenerative diseases, neuroinflammatory disorders, traumatic or posttraumatic disorders, vascular or more precisely neurovascular disorders, hypoxic disorders, postinfectious central nervous system disorders, fibrotic diseases, hyperproliferative diseases, cancer, tumors, presbyakusis and presbyopie.

The term "neurodegenerative disease" or "neurological disease" or "neuroinflammatory disorder" refers any disease, disorder, or condition affecting the central or peripheral nervous system, including ADHD, AIDS-neurological complications, absence of the Septum Pellucidum, acquired epileptiform aphasia, acute disseminated encephalomyelitis, adrenoleukodystrophy, agenesis of the Corpus Callosum, agnosia, Aicardi Syndrome, Alexander Disease, Alpers' Disease, alternating hemiplegia, Alzheimer's Disease, amyotrophic lateral sclerosis (ALS), anencephaly, aneurysm, Angelman Syndrome, angiomatosis, anoxia, aphasia, apraxia, arachnoid cysts, arachnoiditis, Arnold-Chiari Malformation, arteriovenous malformation, aspartame, Asperger Syndrome, ataxia telangiectasia, ataxia, attention deficit-hyperactivity disorder, autism, autonomic dysfunction, back pain, Barth Syndrome, Batten Disease, Behcet's Disease, Bell's Palsy, benign essential blepharospasm, benign focal amyotrophy, benign intracranial hypertension, Bernhardt-Roth Syndrome, Binswanger's Disease, blepharospasm, Bloch-Sulzberger Syndrome, brachial plexus birth injuries, brachial plexus injuries, Bradbury-Eggleston Syndrome, brain aneurysm, brain injury, brain and spinal tumors, Brown-Sequard Syndrome, bulbospinal muscular atrophy, Canavan Disease, Carpal Tunnel Syndrome, causalgia, cavernomas, cavernous angioma, cavernous malformation, central cervical cord syndrome, central cord syndrome, central pain syndrome, cephalic disorders, cerebellar degeneration, cerebellar hypoplasia, cerebral aneurysm, cerebral arteriosclerosis, cerebral atrophy, cerebral beriberi, cerebral gigantism, cerebral hypoxia, cerebral palsy, cerebro-oculo-facio-skeletal syndrome, Charcot-Marie-Tooth Disorder, Chiari Malformation, chorea, choreoacanthocytosis, chronic inflammatory demyelinating polyneuropathy (CIDP), chronic orthostatic intolerance, chronic pain, Cockayne Syndrome Type II, Coffin Lowry Syndrome, coma, including persistent vegetative state, complex regional pain syndrome, congenital facial diplegia, congenital myasthenia, congenital myopathy, congenital vascular cavernous malformations, corticobasal degeneration, cranial arteritis, craniosynostosis, Creutzfeldt-Jakob Disease, cumulative trauma disorders, Cushing's Syndrome, cytomegalic inclusion body disease (CIBD), cytomegalovirus infection, dancing eyes-dancing feet syndrome, Dandy-Walker Syndrome, Dawson Disease, De Morsier's Syndrome, Dejerine-Klumpke Palsy, dementia-multi-infarct, dementia-subcortical, dementia with Lewy Bodies, dermatomyositis, developmental dyspraxia, Devic's Syndrome, diabetic neuropathy, diffuse sclerosis, Dravet's Syndrome, dysautonomia, dysgraphia, dyslexia, dysphagia, dyspraxia, dystonias, early infantile epileptic encephalopathy, Empty Sella Syndrome, encephalitis lethargica, encephalitis and meningitis, encephaloceles, encephalopathy, encephalotrigeminal angiomatosis, epilepsy, Erb's Palsy, Erb-Duchenne and Dejerine-Klumpke Palsies, Fabry's Disease, Fahr's Syndrome, fainting, familial dysautonomia, familial hemangioma, familial idiopathic basal ganglia calcification, familial spastic paralysis, febrile seizures (e.g., GEFS and GEFS plus), Fisher Syndrome, Floppy Infant Syndrome, Friedreich's Ataxia, Gaucher's Disease, Gerstmann's Syndrome, Gerstmann-Straussler-Scheinker Disease, giant cell arteritis, giant cell inclusion disease, globoid cell leukodystrophy, glossopharyngeal neuralgia, Guillain-Barre Syndrome, HTLV-1 associated myelopathy, Hallervorden-Spatz Disease, head injury, headache, hemicrania continua, hemifacial spasm, hemiplegia alterans, hereditary neuropathies, hereditary spastic **paraplegia,** heredopathia atactica polyneuritiformis, Herpes Zoster Oticus, Herpes Zoster, Hirayama Syndrome, holoprosencephaly, Huntington's Disease, hydranencephaly, hydrocephalus-normal pressure, **hydrocephalus,** hydromyelia, hypercortisolism, hypersomnia, hypertonia, hypotonia, hypoxia, immune-mediated encephalomyelitis, inclusion body myositis, incontinentia pigmenti, infantile hypotonia, infantile phytanic acid storage disease, infantile refsum disease, infantile spasms, inflammatory myopathy, intestinal lipodystrophy, intracranial cysts, intracranial hypertension, Isaac's Syndrome, Joubert Syndrome, Kearns-Sayre Syndrome, Kennedy's Disease, Kinsbourne syndrome, Kleine-Levin syndrome, Klippel Feil Syndrome, Klippel-Trenaunay Syndrome (KTS), Klüver-Bucy Syndrome, Korsakoffs Amnesic Syndrome, Krabbe Disease, Kugelberg-Welander Disease, kuru, Lambert-Eaton Myasthenic Syndrome, Landau-Kleffner Syndrome, lateral femoral cutaneous nerve entrapment, lateral medullary syndrome, learning disabilities, Leigh's Disease, Lennox-Gastaut Syndrome, Lesch-Nyhan Syndrome, leukodystrophy, Levine-Critchley Syndrome, Lewy Body Dementia, lissencephaly, locked-in syndrome, Lou Gehrig's Disease, lupus-neurological sequelae, Lyme Disease-Neurological Complications, Machado-Joseph Disease, macrencephaly, megalencephaly, Melkersson-Rosenthal Syndrome, meningitis, Menkes Disease, meralgia paresthetica, metachromatic leukodystrophy, microcephaly, migraine, Miller Fisher Syndrome, mini-strokes, mitochondrial myopathies, Mobius Syndrome, monomelic amyotrophy, motor neuron diseases, Moyamoya Disease, mucolipidoses, mucopolysaccharidoses, multi-infarct dementia, multifocal motor neuropathy, multiple sclerosis (MS), multiple systems atrophy (MSA-C and MSA-P), multiple system atrophy with orthostatic hypotension, muscular dystrophy, myasthenia-congenital, myasthenia gravis, myelinoclastic diffuse sclerosis, myoclonic encephalopathy of infants, myoclonus, myopathy-congenital, myopathy-thyrotoxic, myopathy, myotonia congenita, myotonia, narcolepsy, neuroacanthocytosis, neurodegeneration with brain iron accumulation, neurofibromatosis, neuroleptic malignant syndrome, neurological complications of AIDS, neurological manifestations of Pompe Disease, neuromyelitis optica, neuromyotonia, neuronal ceroid lipofuscinosis, neuronal migration disorders, neuropathy-hereditary, neurosarcoidosis, neurotoxicity, nevus cavernosus, Niemann-Pick Disease, O'Sullivan-McLeod Syndrome, occipital neuralgia, occult spinal dysraphism sequence, Ohtahara Syndrome, olivopontocerebellar atrophy, opsoclonus myoclonus, orthostatic hypotension, Overuse Syndrome, pain-chronic, paraneoplastic syndromes, paresthesia, Parkinson's Disease, parmyotonia congenita, paroxysmal choreoathetosis, paroxysmal hemicrania, Parry-Romberg, Pelizaeus-Merzbacher Disease, Pena Shokeir II Syndrome, perineural cysts, periodic paralyses, peripheral neuropathy, periventricular leukomalacia, persistent vegetative state, pervasive developmental disorders, phytanic acid storage disease, Pick's Disease, Piriformis Syndrome, pituitary tumors, polymyositis, Pompe Disease, porencephaly, Post-Polio Syndrome, postherpetic neuralgia, postinfectious encephalomyelitis, postural hypotension, postural orthostatic tachycardia syndrome, postural tachycardia syndrome, primary lateral sclerosis, prion diseases, progressive hemifacial atrophy, progressive locomotor ataxia, progressive multifocal leukoencephalopathy, progressive sclerosing poliodystrophy, progressive supranuclear palsy, pseudotumor cerebri, pyridoxine dependent and pyridoxine responsive siezure disorders, Ramsay Hunt Syndrome Type I, Ramsay Hunt Syndrome Type II, Rasmussen's Encephalitis and other autoimmune epilepsies, reflex sympathetic dystrophy syndrome, refsum disease-infantile, refsum disease, repetitive motion disorders, repetitive stress injuries, restless legs syndrome, retrovirus-associated myelopathy, Rett Syndrome, Reye's Syndrome, Riley-Day Syndrome, SUNCT headache, sacral nerve root cysts, Saint Vitus Dance, Salivary Gland Disease, Sandhoff Disease, Schilder's Disease, schizencephaly, seizure disorders, septo-optic dysplasia, severe myoclonic epilepsy of infancy (SMEI), shaken baby syndrome, shingles, Shy-Drager Syndrome, Sjogren's Syndrome, sleep apnea, sleeping sickness, Soto's Syndrome, spasticity, spina bifida, spinal cord infarction, spinal cord injury, spinal cord tumors, spinal muscular atrophy, spinocerebellar atrophy, Steele-Richardson-Olszewski Syndrome, Stiff-Person Syndrome, striatonigral degeneration, stroke, Sturge-Weber Syndrome, subacute sclerosing panencephalitis, subcortical arteriosclerotic encephalopathy, Swallowing Disorders, Sydenham Chorea, syncope, syphilitic spinal sclerosis, syringohydromyelia, syringomyelia, systemic lupus erythematosus, Tabes Dorsalis, Tardive Dyskinesia, Tarlov Cysts, Tay-Sachs Disease, temporal arteritis, tethered spinal cord syndrome, Thomsen Disease, thoracic outlet syndrome, thyrotoxic myopathy, Tic Douloureux, Todd's Paralysis, Tourette Syndrome, transient ischemic attack, transmissible spongiform encephalopathies, transverse myelitis, traumatic brain injury, tremor, trigeminal neuralgia, tropical spastic paraparesis, tuberous sclerosis, vascular erectile tumor, vasculitis including temporal arteritis, Von Economo's Disease, Von Hippel-Lindau disease (VHL), Von Recklinghausen's Disease, Wallenberg's Syndrome, Werdnig-Hoffinan Disease, Wernicke-Korsakoff Syndrome, West Syndrome, Whipple's Disease, Williams Syndrome, Wilson's Disease, X-Linked Spinal and Bulbar Muscular Atrophy, and Zellweger Syndrome.

Preferred examples of neurodegenerative diseases and neuroinflammatory disorders are selected from the group comprising or consisting of:
Alzheimer's disease, Parkinson's disease, Creutzfeldt Jakob disease (CJD), new variant of Creutzfeldt Jakobs disease (nvCJD), Hallervorden Spatz disease, Huntington's disease, multisystem atrophy, dementia, frontotemporal dementia, motor neuron disorders of multiple spontaneous or genetic background, amyotrophic lateral sclerosis (ALS), spinal muscular atrophy, spinocerebellar atrophies (SCAs), schizophrenia, affective disorders, major depression, meningoencephalitis, bacterial meningoencephalitis, viral meningoencephalitis, CNS autoimmune disorders, multiple sclerosis (MS), acute ischemic / hypoxic lesions, stroke, CNS and spinal cord trauma, head and spinal trauma, brain traumatic injuries, arteriosclerosis, atherosclerosis, microangiopathic dementia, Binswanger' disease (Leukoaraiosis), retinal degeneration, cochlear degeneration, macular degeneration, cochlear deafness, AIDS-related dementia, retinitis pigmentosa, fragile X-associated tremor/ataxia syndrome (FXTAS), progressive supranuclear palsy (PSP), striatonigral degeneration (SND), olivopontocerebellear degeneration (OPCD), Shy Drager syndrome (SDS), age dependant memory deficits, neurodevelopmental disorders associated with dementia, Down's Syndrome, synucleinopathies, superoxide dismutase mutations, trinucleotide repeat disorders as Huntington's Disease, trauma, hypoxia, vascular diseases, vascular inflammations, CNS-ageing. Also age dependant decrease of stem cell renewal may be addressed.

The antisense-oligonucleotides are also useful for prophylaxis and treatment of **fibrotic diseases.** Fibrosis or fibrotic disease is the formation of excess fibrous connective tissue in an organ or tissue in a reparative or reactive process. This can be a reactive, benign, or pathological state. In response to injury this is called scarring and if fibrosis arises from a single cell line this is called a fibroma. Physiologically this acts to deposit connective tissue, which can obliterate the architecture and function of the underlying organ or tissue. Fibrosis can be used to describe the pathological state of excess deposition of fibrous tissue, as well as the process of connective tissue deposition in healing. Fibrosis is a process involving stimulated cells to form connective tissue, including collagen and glycosaminoglycans. Subsequently macrophages and damaged tissue between the interstitium release TGFβ. TGFβ stimulates the proliferation and activation of fibroblasts which deposit connective tissue. Reducing the TGFβ levels prevents and decreases the formation of connective tissue and thus prevents and treats fibrosis.

Examples for fibrotic diseases are

| | |
|---|---|
| Lungs: | • pulmonary fibrosis |
| | • idiopathic pulmonary fibrosis (idiopathic means cause is unknown) |
| | • cystic fibrosis |
| | |
| Liver: | • hepatic cirrhosis of multiple origin |
| | |
| Heart: | • endomyocardial fibrosis |
| | • old myocardial infarction |
| | • atrial fibrosis |
| | |
| Other: | • mediastinal fibrosis (soft tissue of the mediastinum) |
| | • myelofibrosis (bone marrow) |
| | • retroperitoneal fibrosis (soft tissue of the retroperitoneum) |
| | • progressive massive fibrosis (lungs); a complication of coal workers' pneumoconiosis |
| | • nephrogenic systemic fibrosis (skin) |
| | • Crohn's Disease (intestine) |
| | • keloid (skin) |
| | • scleroderma/systemic sclerosis (skin, lungs) |
| | • arthrofibrosis (knee, shoulder, other joints) |
| | • Peyronie's disease (penis) |
| | • Dupuytren's contracture (hands, fingers) |
| | • some forms of adhesive capsulitis (shoulder) |
| | • residuums after Lupus erythematodes |

Thus another aspect of the present invention relates to the use of an antisense-oligonucleotide for prophylaxis and/or treatment of or to the use of an antisense-oligonucleotide for the preparation of a pharmaceutical composition for prophylaxis and/or treatment of pulmonary fibrosis, cystic fibrosis, hepatic cirrhosis, endomyocardial fibrosis, old myocardial infarction, atrial fibrosis, mediastinal fibrosis, myelofibrosis, retroperitoneal fibrosis, progressive massive fibrosis, nephrogenic systemic fibrosis, Crohn's Disease, keloid, systemic sclerosis, arthrofibrosis, Peyronie's disease, Dupuytren's contracture, and residuums after Lupus erythematodes.

Still another aspect of the present invention relates to the use of an antisense-oligonucleotide for prophylaxis and/or treatment of hyperproliferative diseases, cancer, tumors and their metastases or to the use of an antisense-oligonucleotide for the preparation of a pharmaceutical composition for prophylaxis and/or treatment of hyperproliferative diseases, cancer, tumors and their metastases.

Examples for hyperproliferative diseases, cancer, tumors are selected from the group comprising or consisting of: adenocarcinoma, melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas, e.g. astrocytomas, oligodendrogliomas, medulloblastomas, PNET's, mixed gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma and tongue cancer.

For the treatment of hyperproliferative diseases, cancer, tumors and their metastases the antisense-oligonucleotides may be administered at regular intervals (dose intervals, DI) of between 3 days and two weeks, such as 4, 5, 6, 7, 8, 9, 10, 11, 12, or 13 days, such as about 1 week, such as 6, 7 or 8 days. Suitably at least two doses are provides with a DI period between the two dosages, such as 3, 4, 5, 6, 7, 8, 9 or 10 dosages, each with a dose interval (DI) between each dose of the antisense-oligonucleotide. The DI period between each dosage may the same, such as between 3 days and two weeks, such as 4, 5, 6, 7, 8, 9, 10, 1 1 , 12, 13 days, such as about 1 week, such as 6, 7 or 8 days.

Preferably each dose of the antisense-oligonucleotide may be between about 0.25mg/kg - about 10mg/kg, such as about 0.5mg/kg, about 1 mg/kg, about 2mg/kg, about 3mg/kg, about 4mg/kg, about 5mg/kg, about 6mg/kg, about 7mg/kg, about 8mg/kg, about 9mg/kg. In some embodiments, each does of the antisense-oligonucleotide may be between about 2 mg/kg - about 8mg/kg, or about 4 to about 6 mg/kg or about 4mg/kg to about 5mg/kg. In some embodiments, each does of the antisense-oligonucleotide is at least 2mg/kg, such as 2, 3, 4, 5, 6, 7 or 8 mg/kg, such as 6 mg/kg. In some embodiments the dosage regime for the antisense-oligonucleotide may be repeated after an initial dosage regime, for example after a rest period where no antisense-oligonucleotide is administered. Such as rest period may be more than 2 weeks in duration, such as about 3 weeks or about 4 weeks, or about 5 weeks or about 6 weeks. In some embodiments the dosage regimen for the antisense-oligonucleotide is one weekly dosage, repeated three, four or five times. This dosage regimen may then be repeated after a rest period of, for example, about 3 - 5 weeks, such as about 4 weeks. In some embodiments, the antisense-oligonucleotide is administered during a first dosage regimen at regular dosage intervals (DI) of between 4 and 13 days for between 2 - 10 administrations. Administration of the antisense-oligonucleotide is typically performed by parenteral administration, such as subcutaneous, intramuscular, intravenous or intraperitoneal administration.

### Description of Figures

**Fig. 1** shows the inhibitory effect of the antisense-oligonucleotides (ASO). The DNA is transcribed to the Pre-mRNA to which in the nucleus of the cell, the antisense-oligonucleotides (ASO) can bind or hybridize to the complementary sequence within an exon (as represented by the first ASO from the right side and the first ASO from the left side) or within an intron (as represented by the second ASO from the right side) or at allocation consisting of an area of an exon and an area of an adjacent intron (as represented by the second ASO from the left side). By post-transcriptional modification, i.e. the splicing, the mRNA is formed to which the ASO can bind or hybridize in the cytoplasma of the cell in order to inhibit translation of the mRNA into the protein sequence. Thus, the ASO knock down the target gene and the protein expression selectively.
**Fig. 2** shows a nucleoside unit (without internucleotide linkage) or nucleotide unit (with internucleotide linkage) which are non-LNA units and which may be contained in the antisense-oligonucleotides of the present invention especially in the region B in case the antisense-oligonucleotide of the present invention is a gapmer.

### Examples

### Example 1

### Gapmer antisense-oligonucleotide synthesis and purification

The antisense-oligonucleotides in form of gapmers were assembled on an ABI 3900 or on an ABI 394 synthesizer (Applied Biosystems) according to the phosphoramidite oligomerization chemistry. On the ABI3900, the solid support was polystyrene loaded with UnySupport (purchased from Glen Research, Sterling, Virginia, USA) to give a synthesis scale of 0.2 µmol. On the ABI 394 the solid support was 500 A controlled pore glass (CPG) loaded with Unylinker™ purchased from Chemgenes (Wilmington, MA, USA) to give a 3 µmol synthesis scale.

Ancillary synthesis reagents such as "Deblock", "Oxidizer", "CapA" and "CapB" as well as DNA phosphoramidites were obtained from SAFC Proligo (Hamburg, Germany). Specifically, 5'-O-(4,4'-dimethoxytrityl)-3'-O-(2-cyanoethyl-N,N-diisopropyl) phosphoramidite monomers of deoxy thymidine (dT), 4-N-benzoyl-2'-deoxy-cytidine (dC^{Bz}), 6-N-benzoyl-2'-deoxy-adenosine (dA^{Bz}) and 2-N-isobutyryl-2'-deoxy-guanosine (dG^{iBu}) were used. In addition, LNA phosphoramidites carrying the same protecting groups as the standard DNA phosphoramidites were purchased from Exiqon (Vedbaek, Denmark) and were used to build the antisense sequences. All phosphoramidites were dissolved in dry acetonitrile to give 70 mM-oligonucleotide. Coupling time for all phosphoramidites was 4 min employing 5-ethylthio-1 H-tetrazole (ETT) as activator (0.5 M in acetonitrile). Phosphorothioate linkages were generated using a 50 mM solution of 3-((dimethylamino-methylidene)amino)-3H-1,2,4-dithiazole-3-thione (DDTT, obtained from Chemgenes (Wilmington, MA, USA)) in anhydrous acetonitrile/pyridine (1:1 v/v). All antisense-oligonucleotides were synthesized "DMT off', which means that the final 5'-O-(4,4'-dimethoxytrityl) group was removed on the synthesizer using the "Deblock" reagent.

Upon completion of the solid phase synthesis antisense-oligonucleotides were treated with a 20% diethylamine solution in acetonitrile (Biosolve BV, Valkenswaard, The Netherlands) for 20 minutes to remove the cyanoethyl protecting groups on the phosphate backbone. Subsequently, the antisense-oligonucleotides were cleaved from the solid support and deprotected using 1 to 5 mL concentrated aqueous ammonia (obtained from Sigma Aldrich) for 16 hours at 55°C. The solid support was separated from the antisense-oligonucleotides by filtration or centrifugation.

Next, the crude antisense-oligonucleotides were purified by anion-exchange high-performance liquid chromatography (HPLC) on an AKTA Explorer System (GE Healthcare, Freiburg, Germany) and a column packed with Source Q15 (GE Helthcare). Buffer A was 10 mM sodium perchlorate, 20 mM Tris, 1 mM EDTA, pH 7.4 and contained 20% acetonitrile and buffer B was the same as buffer A with the exception of 500 mM sodium perchlorate. A gradient of 15%B to 55%B within 32 column volumes (CV) was employed. UV traces at 280 nm were recorded. Appropriate fractions were pooled and precipitated with 3M NaOAc, pH=5.2 and 70% ethanol. Finally, the pellet was washed with 70% ethanol.

Identity of the antisense-oligonucleotides was confirmed by electrospray ionization mass spectrometry (ESI-MS) and purity was assessed by analytical anion-exchange HPLC using a Dionex DNA Pac 200 (4x 250 mm) column on a Dionex UltiMate 3000 HPLC system employing a flow rate of 1 mL/min.

### Determination of therapeutic activity

The activity of the antisense-oligonucleotides directed to TGF-R_{II} was tested in A549, Panc-1 and 4T1 cells.

TGF-R_{II} mRNA was quantified by branched DNA in total mRNA isolated from cells incubated with TGF-R_{II} specific oligonucleotides.

All cells were obtained from American Type Culture Collection (Rockville, Md., cat. No. CCL-185 (A549), CRL-2539 (4T1) and CRL-1469 (Panc-1)). A549 (human) and 4T1 (mouse) cells were cultured in RPMI1640 medium (Biochrom #FG1215), supplemented with 10% fetal calf serum (FCS) (Biochrom AG, Berlin, Germany, cat. No. S0115) and penicillin 100 U/ml, streptomycin 100 mg/ml (Biochrom AG, Berlin, Germany, cat. No. A2213) at 37°C in an atmosphere with 5% CO₂ in a humidified incubator (Heraeus HERAcell, Kendro Laboratory Products, Langenselbold, Germany). Panc-1 cells (human) were cultured in Dulbeccos modified Eagle's medium (DMEM) (Biochrom #F0435) supplemented with 10% fetal calf serum (FCS) (Biochrom AG, Berlin, Germany, cat. No. S0115), penicillin 100 U/ml, streptomycin 100 mg/ml (Biochrom AG, Berlin, Germany, cat. No. A2213) and 1 mM Sodium pyruvate (Biochrom AG, Berlin, Germany, cat. No. L0473).

Transfection of antisense-oligonucleotides was performed directly after seeding 10,000 A549 cells / well on a 96-well plate, and was carried out with Lipofectamine 2000 (Invitrogen GmbH, Karlsruhe, Germany, cat.No. 11668-019) as described by the manufacturer. In two independent single dose experiments performed in quadruplicates, oligonucleotides were transfected at a concentration of 20 nM or 5 nM respectively. After transfection cells were incubated for 24 h at 37°C and 5 % CO₂ in a humidified incubator (Heraeus GmbH, Hanau, Germany). Most effective antisense-oligonucleotides against TGF-R_{II} from the transfection screens were further characterized by direct incubation without transfection reagent ("gymnotic uptake"). 8000 A549 cells / well were incubated with oligonucleotides at 7.5 µM concentration over 72 h. Results were verified with Panc-1 cells (10000 cells /well, 5 µM). Antisense-oligonucleotides cross-reactive to mouse TGF-R_{II} were also tested on mouse 4T1 cells for gymnotic uptake efficacy (5000 cells / well, 7.5 µM).

In a second screening round, new chemical modifications of the antisense-oligonucleotides were synthesized and characterized by gymnotic delivery in all 3 cell lines, as described above. Most effective antisense-oligonucleotides against TGF-R_{II} from the single dose screens were further characterized by dose response curves. For dose response curves, gymnotic uptake experiments in Panc-1 and 4T1 were performed as for the single dose screen above, but with concentrations starting with 10 µM (Panc-1) or 20µM (4T1) and decreasing in 3-fold dilutions down to 0.12 µM (Panc-1) or 0.25 µM (4T1). For measurement of TGFBR2-mRNA cells were harvested and lysed at 53°C following procedures recommended by the manufacturer of the Quantigene Explore Kit (Panomics, Fremont, Calif., USA, cat. No. QG0004) for bDNA. For quantitation of GAPDH-mRNA the Quantigene Explore Kit was used, whereas quantitation of TGF-R_{II}-mRNA was conducted with QuantiGene 2.0 (custom manufacturing for Axolabs GmbH, Kulmbach, Germany). After incubation and lysis, 10 µl of the lysates were incubated with probe-sets specific to human or mouse TGF-R_{II} and human or mouse GAPDH. Both reaction types were processed according to the manufacturer's protocol for the respective QuantiGene kit. Chemoluminescence was measured in a Victor2-Light (Perkin Elmer, Wiesbaden, Germany) as RLUs (relative light units) and values obtained with the TGF-R_{II} probe-sets were normalized to the respective GAPDH values for each well and then normalized to the corresponding mRNA readout from mock-treated cells.

Sequence Listings

Seq. ID No. 1: Homo sapiens transforming growth factor, beta receptor II (70/80kDa) (TGFBR2), transcript variant 2, (antisense; DNA code)

TTTAGCTACT AGGAATGGGA ACAGGAGGCA GGATGCTCAC CTGAGTATTT TGCTTTATTC 60
AATCTAATAA ACATTTTATT TATGTAAAAG ACAAACAATG CATAGAATAA AAATAAGTGC 120
TTGAGACTTT TGATATAAAA AGAGTATATA GCATTCACAT TCCTATTTTA ATACATGAGT 180
ACAGCTGAAG TGTTCCATAA AAGAATAAAA CTTTCCCTTT ATGTATAGTA GTGAAAAAAG 240
TCAGTATTTT TAGGAACTAC AGAATGTTAT TCCTTGGTCT TTTTTCTTGA ATAAGAAAAA 300
AAAACATAAA CAAAACAAGC CACAGTATCC TCTGACACTA CATTCCAGTT TATGCTGATA 360
ACCCAGAAGT GAGAATACTC TTGAATCTTG AATATCTCAT GAATGGACCA GTATTCTAGA 420
AACTCACCAC TAGAGGTCAA TGGGCAACAG CTATTGGGAT GGTATCAGCA TGCCCTACGG 480
TGCAAGTGGA ATTTCTAGGC GCCTCTATGC TACTGCAGCC ACACTGTCTT TAACTCTCAG 540
CCCACCCACA CTGAGGAGGG TGCCTAGAGG TTCTATTTCC AAACCTTTGC ATGTATCTTA 600
AAAATCTCAA TAAAATGAGA CCTTCCACCA TCCAAACAGA GCTGATATTC TCACTACCAG 660
TCCCTCTCTA ATATTCCTAT TTGGCTGAAA ATAAGTAGCT TCAAAAAGTT TTAAAAAAGA 720
GATTACTTGC AGCATTAACA CTTCTTTGTT GATTAACAAG TTTCCTATGG AGTTTTAAAG 780
CTCATACTTT GTTCTTGTCC TTGTGGACAC AAATTTTCTA ACTGCAAATG GGACCTTTGT 840
GTCCCACATT CAAATCCTCT CTAGTAATTT CTGCAAAGGT TGAGAAGGCT GGCATGATGG 900
AGAGAACGGT AACCATGAGG AAAGCTTCTT GGAGTAAAGC ACTCCTCTCT CCAATGCAGA 960
GGGTAAAACT ATTAACATAT AAGCAAAAGA AACTTGGGCT AACTGAGACC CTTAAAGGAG 1020
TTCCCCTTTA GTCCAATAAA AGGCCAACTT CAAATCTTAA CACCAGATAA GGTAGTCAAA 1080
ATCATATTAT ATACCCAGAG AATGACTGCT TGAATGGACA TTTCTTACAA GGGACCTTGG 1140
TTAGGTGCAG ATTTAATTCC TAGACTGGGG TCCAGGTAGG CAGTGGAAAG AGCTAATGTT 1200
TACAGTGAGA AGTGAGGCAG CTTTGTAAGT GTCTCCACAC CTTCACATTT TGTGAACGTG 1260
GACTGGAGAT AACTGAAAAC CATCTGCTAT CCTTACCTGG GGATCCAGAT TTTCCTGCAA 1320
AATCTCCAAA TATTTATAAA GTGGCTTCAC TTTTTGAAAC GCTGTGCTGA CCAAACAAAA 1380
CATATGTTTA GAGTGCCTGA GGTCATAGTC CTGACAATGA TAGTATTGTG TAGTTGAAAT 1440
CCTCTTCATC AGGCCAAACT GTGCTTGAGC AATCAGGAGC CCAGAAAGAT GGAACCCATT 1500
GGTGTTTGTA TAGAAAACTA GAAAATCAAG TCAAGTGTAA TGAAAAAGTA AACACGATAA 1560
AGCCTAGAGT GAGAATTTGC TCCTTTTTAG AAAAGGATGA AGGCTGGGAG CAGAGAATAG 1620
TAACATAAGT GCAGGGGAAA GATGAAAAAA AGAACAATTT TTCATTAGTA GATGGTGGGG 1680
CAATCGCATG GATGGGGACA TCTGTTCTGA TTTTTCTGCA ACCCATGAAG GTAAAAAGTG 1740
GGGTTCAAAA CATTCAAGGT ATTAAAGATG GGGTAGAGTT TCTAAACTAG GTTGAGGGAG 1800
AGTTTCTAAA CTAGCCCCCC AGATTTGGGG CTTGGAGCTT AAATGAAAAG TCCAGGAGAA 1860
ATAAGGGCAC ACAGGAACCC CGGGAACACT GGTCCTCAAA CAGTGCCACT GTACTTAGTT 1920
CCATGGCCAG AAGAGAAGTG CTAGGCAGGG AATGATTATT TTGCAAAAGC AAGTGCAATG 1980
TGGTCATAGC TGGCTGTGAG ACATGGAGCC TCTTTCCTCA TGCAAAGTTC ACTGTTTTAC 2040

AGTCAGAGAA CCACTGCATG TGTGATTGTC AAATGCTAAT GCTGTCATGG GTCCCTTCCT 2100
TCTCTGCTTG GTTCTGGAGT TCTCCAATAA AACCAATTTC CTGGGAATAT TTGATGTTTT 2160
TCCTTGTCTC TTTTCAAGGT ATGGCTATAT ATATAGAGCT ATAGACATAT ATAGATATAT 2220
ATATATATAT ATAAAACATA GCTATTCATA TTTATATACA GGCATTAATA AAGTGCAAAT 2280
GTTATTGGCT ATTGTAAAAA TCAATCTCAT TTCCTGAGGA AGTGCTAACA CAGCTTATCC 2340
TATGACAATG TCAAAGGCAT AGAATGCTCT ATGTCACCCA CTCCCTGCTG CTGTTGTTTC 2400
TGCTTATCCC CACAGCTTAC AGGGAGGGGA GTGACCCCCT TGGTTTTCCA GGAAGCATCA 2460
GTTCAGGGGC AGCTTCCTGC TGCCTCTGTT CTTTGGTGAG AGGGGCAGCC TCTTTGGACA 2520
TGGCCCAGCC TGCCCCAGAA GAGCTATTTG GTAGTGTTTA GGGAGCCGTC TTCAGGAATC 2580
TTCTCCTCCG AGCAGCTCCT CCCCGAGAGC CTGTCCAGAT GCTCCAGCTC ACTGAAGCGT 2640
TCTGCCACAC ACTGGGCTGT GAGACGGGCC TCTGGGTCGT GGTCCCAGCA CTCAGTCAAC 2700
GTCTCACACA CCATCTGGAT GCCCTGGTGG TTGAGCCAGA AGCTGGGAAT TTCTGGTCGC 2760
CCTCGATCTC TCAACACGTT GTCCTTCATG CTTTCGACAC AGGGGTGCTC CCGCACCTTG 2820
GAACCAAATG GAGGCTCATA ATCTTTTACT TCTCCCACTG CATTACAGCG AGATGTCATT 2880
TCCCAGAGCA CCAGAGCCAT GGAGTAGACA TCGGTCTGCT TGAAGGACTC AACATTCTCC 2940
AAATTCATCC TGGATTCTAG GACTTCTGGA GCCATGTATC TTGCAGTTCC CACCTGCCCA 3000
CTGTTAGCCA GGTCATCCAC AGACAGAGTA GGGTCCAGAC GCAGGGAAAG CCCAAAGTCA 3060
CACAGGCAGC AGGTTAGGTC GTTCTTCACG AGGATATTGG AGCTCTTGAG GTCCCTGTGC 3120
ACGATGGGCA TCTTGGGCCT CCCACATGGA GTGTGATCAC TGTGGAGGTG AGCAATCCCC 3180
CGGGCGAGGG AGCTGCCCAG CTTGCGCAGG TCCTCCCAGC TGATGACATG CCGCGTCAGG 3240
TACTCCTGTA GGTTGCCCTT GGCGTGGAAG GCGGTGATCA GCCAGTATTG TTTCCCCAAC 3300
TCCGTCTTCC GCTCCTCAGC CGTCAGGAAC TGGAGTATGT TCTCATGCTT CAGATTGATG 3360
TCTGAGAAGA TGTCCTTCTC TGTCTTCCAA GAGGCATACT CCTCATAGGG AAAGATCTTG 3420
ACTGCCACTG TCTCAAACTG CTCTGAAGTG TTCTGCTTCA GCTTGGCCTT ATAGACCTCA 3480
GCAAAGCGAC CTTTCCCCAC CAGGGTGTCC AGCTCAATGG GCAGCAGCTC TGTGTTGTGG 3540
TTGATGTTGT TGGCACACGT GGAGCTGATG TCAGAGCGGT CATCTTCCAG GATGATGGCA 3600
CAGTGCTCGC TGAACTCCAT GAGCTTCCGC GTCTTGCCGG TTTCCCAGGT TGAACTCAGC 3660
TTCTGCTGCC GGTTAACGCG GTAGCAGTAG AAGATGATGA TGACAGATAT GGCAACTCCC 3720
AGTGGTGGCA GGAGGCTGAT GCCTGTCACT TGAAATATGA CTAGCAACAA GTCAGGATTG 3780
CTGGTGTTAT ATTCTTCTGA GAAGATGATG TTGTCATTGC ACTCATCAGA GCTACAGGAA 3840
CACATGAAGA AAGTCTCACC AGGCTTTTTT TTTTCCTTCA TAATGCACTT TGGAGAAGCA 3900
GCATCTTCCA GAATAAAGTC ATGGTAGGGG AGCTTGGGGT CATGGCAAAC TGTCTCTAGT 3960
GTTATGTTCT CGTCATTCTT TCTCCATACA GCCACACAGA CTTCCTGTGG CTTCTCACAG 4020
ATGGAGGTGA TGCTGCAGTT GCTCATGCAG GATTTCTGGT TGTCACAGGT GGAAAATCTC 4080
ACATCACAAA ATTTACACAG TTGTGGAAAC TTGACTGCAC CGTTGTTGTC AGTGACTATC 4140
ATGTCGTTAT TAACCGACTT CTGAACGTGC GGTGGGATCG TGCTGGCGAT ACGCGTCCAC 4200
AGGACGATGT GCAGCGGCCA CAGGCCCCTG AGCAGCCCCC GACCCATGGC AGACCCCGCT 4260
GCTCGTCATA GACCGAGCCC CCAGCGCAGC GGACGGCGCC TTCCCGGACC CCTGGCTGCG 4320
CCTCCGCGCC GCGCCCTCTC CGGACCCCGC GCCGGGCCGG CAGCGCAGAT GTGCGGGCCA 4380
GATGTGGCGC CCGCTCGCCA GCCAGGAGGG GGCCTGGAGG CCGGCGAGGC GCGGGGAGGC 4440
CCCCGGCGGC CGAGGGAAGC TGCACAGGAG TCCGGCTCCT GTCCCGAGCG GGTGCACGCG 4500
CGGGGGTGTC GTCGCTCCGT GCGCGCGAGT GACTCACTCA ACTTCAACTC AGCGCTGCGG 4560

GGGAAACAGG AAACTCCTCG CCAACAGCTG GGCAGGACCT CTCTCCGCCC GAGAGCCTTC 4620
TCCCTCTCC 4629

Seq. ID No. 2: Homo sapiens transforming growth factor, beta receptor II (70/80kDa) (TGFBR2), transcript variant 2, mRNA (sense; written in DNA code)

GGAGAGGGAG AAGGCTCTCG GGCGGAGAGA GGTCCTGCCC AGCTGTTGGC GAGGAGTTTC 60
CTGTTTCCCC CGCAGCGCTG AGTTGAAGTT GAGTGAGTCA CTCGCGCGCA CGGAGCGACG 120
ACACCCCCGC GCGTGCACCC GCTCGGGACA GGAGCCGGAC TCCTGTGCAG CTTCCCTCGG 180
CCGCCGGGGG CCTCCCCGCG CCTCGCCGGC CTCCAGGCCC CCTCCTGGCT GGCGAGCGGG 240
CGCCACATCT GGCCCGCACA TCTGCGCTGC CGGCCCGGCG CGGGGTCCGG AGAGGGCGCG 300
GCGCGGAGGC GCAGCCAGGG GTCCGGGAAG GCGCCGTCCG CTGCGCTGGG GGCTCGGTCT 360
ATGACGAGCA GCGGGGTCTG CCATGGGTCG GGGGCTGCTC AGGGGCCTGT GGCCGCTGCA 420
CATCGTCCTG TGGACGCGTA TCGCCAGCAC GATCCCACCG CACGTTCAGA AGTCGGTTAA 480
TAACGACATG ATAGTCACTG ACAACAACGG TGCAGTCAAG TTTCCACAAC TGTGTAAATT 540
TTGTGATGTG AGATTTTCCA CCTGTGACAA CCAGAAATCC TGCATGAGCA ACTGCAGCAT 600
CACCTCCATC TGTGAGAAGC CACAGGAAGT CTGTGTGGCT GTATGGAGAA AGAATGACGA 660
GAACATAACA CTAGAGACAG TTTGCCATGA CCCCAAGCTC CCCTACCATG ACTTTATTCT 720
GGAAGATGCT GCTTCTCCAA AGTGCATTAT GAAGGAAAAA AAAAAGCCTG GTGAGACTTT 780
CTTCATGTGT TCCTGTAGCT CTGATGAGTG CAATGACAAC ATCATCTTCT CAGAAGAATA 840
TAACACCAGC AATCCTGACT TGTTGCTAGT CATATTTCAA GTGACAGGCA TCAGCCTCCT 900
GCCACCACTG GGAGTTGCCA TATCTGTCAT CATCATCTTC TACTGCTACC GCGTTAACCG 960
GCAGCAGAAG CTGAGTTCAA CCTGGGAAAC CGGCAAGACG CGGAAGCTCA TGGAGTTCAG 1020
CGAGCACTGT GCCATCATCC TGGAAGATGA CCGCTCTGAC ATCAGCTCCA CGTGTGCCAA 1080
CAACATCAAC CACAACACAG AGCTGCTGCC CATTGAGCTG GACACCCTGG TGGGGAAAGG 1140
TCGCTTTGCT GAGGTCTATA AGGCCAAGCT GAAGCAGAAC ACTTCAGAGC AGTTTGAGAC 1200
AGTGGCAGTC AAGATCTTTC CCTATGAGGA GTATGCCTCT TGGAAGACAG AGAAGGACAT 1260
CTTCTCAGAC ATCAATCTGA AGCATGAGAA CATACTCCAG TTCCTGACGG CTGAGGAGCG 1320
GAAGACGGAG TTGGGGAAAC AATACTGGCT GATCACCGCC TTCCACGCCA AGGGCAACCT 1380
ACAGGAGTAC CTGACGCGGC ATGTCATCAG CTGGGAGGAC CTGCGCAAGC TGGGCAGCTC 1440
CCTCGCCCGG GGGATTGCTC ACCTCCACAG TGATCACACT CCATGTGGGA GGCCCAAGAT 1500
GCCCATCGTG CACAGGGACC TCAAGAGCTC CAATATCCTC GTGAAGAACG ACCTAACCTG 1560
CTGCCTGTGT GACTTTGGGC TTTCCCTGCG TCTGGACCCT ACTCTGTCTG TGGATGACCT 1620
GGCTAACAGT GGGCAGGTGG GAACTGCAAG ATACATGGCT CCAGAAGTCC TAGAATCCAG 1680
GATGAATTTG GAGAATGTTG AGTCCTTCAA GCAGACCGAT GTCTACTCCA TGGCTCTGGT 1740
GCTCTGGGAA ATGACATCTC GCTGTAATGC AGTGGGAGAA GTAAAAGATT ATGAGCCTCC 1800
ATTTGGTTCC AAGGTGCGGG AGCACCCCTG TGTCGAAAGC ATGAAGGACA ACGTGTTGAG 1860
AGATCGAGGG CGACCAGAAA TTCCCAGCTT CTGGCTCAAC CACCAGGGCA TCCAGATGGT 1920
GTGTGAGACG TTGACTGAGT GCTGGGACCA CGACCCAGAG GCCCGTCTCA CAGCCCAGTG 1980
TGTGGCAGAA CGCTTCAGTG AGCTGGAGCA TCTGGACAGG CTCTCGGGGA GGAGCTGCTC 2040
GGAGGAGAAG ATTCCTGAAG ACGGCTCCCT AAACACTACC AAATAGCTCT TCTGGGGCAG 2100
GCTGGGCCAT GTCCAAAGAG GCTGCCCCTC TCACCAAAGA ACAGAGGCAG CAGGAAGCTG 2160

CCCCTGAACT GATGCTTCCT GGAAAACCAA GGGGGTCACT CCCCTCCCTG TAAGCTGTGG 2220
GGATAAGCAG AAACAACAGC AGCAGGGAGT GGGTGACATA GAGCATTCTA TGCCTTTGAC 2280
ATTGTCATAG GATAAGCTGT GTTAGCACTT CCTCAGGAAA TGAGATTGAT TTTTACAATA 2340
GCCAATAACA TTTGCACTTT ATTAATGCCT GTATATAAAT ATGAATAGCT ATGTTTTATA 2400
TATATATATA TATATCTATA TATGTCTATA GCTCTATATA TATAGCCATA CCTTGAAAAG 2460
AGACAAGGAA AAACATCAAA TATTCCCAGG AAATTGGTTT TATTGGAGAA CTCCAGAACC 2520
AAGCAGAGAA GGAAGGGACC CATGACAGCA TTAGCATTTG ACAATCACAC ATGCAGTGGT 2580
TCTCTGACTG TAAAACAGTG AACTTTGCAT GAGGAAAGAG GCTCCATGTC TCACAGCCAG 2640
CTATGACCAC ATTGCACTTG CTTTTGCAAA ATAATCATTC CCTGCCTAGC ACTTCTCTTC 2700
TGGCCATGGA ACTAAGTACA GTGGCACTGT TTGAGGACCA GTGTTCCCGG GGTTCCTGTG 2760
TGCCCTTATT TCTCCTGGAC TTTTCATTTA AGCTCCAAGC CCCAAATCTG GGGGGCTAGT 2820
TTAGAAACTC TCCCTCAACC TAGTTTAGAA ACTCTACCCC ATCTTTAATA CCTTGAATGT 2880
TTTGAACCCC ACTTTTTACC TTCATGGGTT GCAGAAAAAT CAGAACAGAT GTCCCCATCC 2940
ATGCGATTGC CCCACCATCT ACTAATGAAA AATTGTTCTT TTTTTCATCT TTCCCCTGCA 3000
CTTATGTTAC TATTCTCTGC TCCCAGCCTT CATCCTTTTC TAAAAAGGAG CAAATTCTCA 3060
CTCTAGGCTT TATCGTGTTT ACTTTTTCAT TACACTTGAC TTGATTTTCT AGTTTTCTAT 3120
ACAAACACCA ATGGGTTCCA TCTTTCTGGG CTCCTGATTG CTCAAGCACA GTTTGGCCTG 3180
ATGAAGAGGA TTTCAACTAC ACAATACTAT CATTGTCAGG ACTATGACCT CAGGCACTCT 3240
AAACATATGT TTTGTTTGGT CAGCACAGCG TTTCAAAAAG TGAAGCCACT TTATAAATAT 3300
TTGGAGATTT TGCAGGAAAA TCTGGATCCC CAGGTAAGGA TAGCAGATGG TTTTCAGTTA 3360
TCTCCAGTCC ACGTTCACAA AATGTGAAGG TGTGGAGACA CTTACAAAGC TGCCTCACTT 3420
CTCACTGTAA ACATTAGCTC TTTCCACTGC CTACCTGGAC CCCAGTCTAG GAATTAAATC 3480
TGCACCTAAC CAAGGTCCCT TGTAAGAAAT GTCCATTCAA GCAGTCATTC TCTGGGTATA 3540
TAATATGATT TTGACTACCT TATCTGGTGT TAAGATTTGA AGTTGGCCTT TTATTGGACT 3600
AAAGGGGAAC TCCTTTAAGG GTCTCAGTTA GCCCAAGTTT CTTTTGCTTA TATGTTAATA 3660
GTTTTACCCT CTGCATTGGA GAGAGGAGTG CTTTACTCCA AGAAGCTTTC CTCATGGTTA 3720
CCGTTCTCTC CATCATGCCA GCCTTCTCAA CCTTTGCAGA AATTACTAGA GAGGATTTGA 3780
ATGTGGGACA CAAAGGTCCC ATTTGCAGTT AGAAAATTTG TGTCCACAAG GACAAGAACA 3840
AAGTATGAGC TTTAAAACTC CATAGGAAAC TTGTTAATCA ACAAAGAAGT GTTAATGCTG 3900
CAAGTAATCT CTTTTTTAAA ACTTTTTGAA GCTACTTATT TTCAGCCAAA TAGGAATATT 3960
AGAGAGGGAC TGGTAGTGAG AATATCAGCT CTGTTTGGAT GGTGGAAGGT CTCATTTTAT 4020
TGAGATTTTT AAGATACATG CAAAGGTTTG GAAATAGAAC CTCTAGGCAC CCTCCTCAGT 4080
GTGGGTGGGC TGAGAGTTAA AGACAGTGTG GCTGCAGTAG CATAGAGGCG CCTAGAAATT 4140
CCACTTGCAC CGTAGGGCAT GCTGATACCA TCCCAATAGC TGTTGCCCAT TGACCTCTAG 4200
TGGTGAGTTT CTAGAATACT GGTCCATTCA TGAGATATTC AAGATTCAAG AGTATTCTCA 4260
CTTCTGGGTT ATCAGCATAA ACTGGAATGT AGTGTCAGAG GATACTGTGG CTTGTTTTGT 4320
TTATGTTTTT TTTTCTTATT CAAGAAAAAA GACCAAGGAA TAACATTCTG TAGTTCCTAA 4380
AAATACTGAC TTTTTTCACT ACTATACATA AAGGGAAAGT TTTATTCTTT TATGGAACAC 4440
TTCAGCTGTA CTCATGTATT AAAATAGGAA TGTGAATGCT ATATACTCTT TTTATATCAA 4500
AAGTCTCAAG CACTTATTTT TATTCTATGC ATTGTTTGTC TTTTACATAA ATAAAATGTT 4560
TATTAGATTG AATAAAGCAA AATACTCAGG TGAGCATCCT GCCTCCTGTT CCCATTCCTA 4620
GTAGCTAAA 4629

Seq. ID No. 3: Homo sapiens transforming growth factor, beta receptor II (70/80kDa) (TGFBR2), transcript variant 2, mRNA (sense; written in RNA code)

GGAGAGGGAG AAGGCUCUCG GGCGGAGAGA GGUCCUGCCC AGCUGUUGGC GAGGAGUUUC 60
CUGUUUCCCC CGCAGCGCUG AGUUGAAGUU GAGUGAGUCA CUCGCGCGCA CGGAGCGACG 120
ACACCCCCGC GCGUGCACCC GCUCGGGACA GGAGCCGGAC UCCUGUGCAG CUUCCCUCGG 180
CCGCCGGGGG CCUCCCCGCG CCUCGCCGGC CUCCAGGCCC CCUCCUGGCU GGCGAGCGGG 240
CGCCACAUCU GGCCCGCACA UCUGCGCUGC CGGCCCGGCG CGGGGUCCGG AGAGGGCGCG 300
GCGCGGAGGC GCAGCCAGGG GUCCGGGAAG GCGCCGUCCG CUGCGCUGGG GGCUCGGUCU 360
AUGACGAGCA GCGGGGUCUG CCAUGGGUCG GGGGCUGCUC AGGGGCCUGU GGCCGCUGCA 420
CAUCGUCCUG UGGACGCGUA UCGCCAGCAC GAUCCCACCG CACGUUCAGA AGUCGGUUAA 480
UAACGACAUG AUAGUCACUG ACAACAACGG UGCAGUCAAG UUUCCACAAC UGUGUAAAUU 540
UUGUGAUGUG AGAUUUUCCA CCUGUGACAA CCAGAAAUCC UGCAUGAGCA ACUGCAGCAU 600
CACCUCCAUC UGUGAGAAGC CACAGGAAGU CUGUGUGGCU GUAUGGAGAA AGAAUGACGA 660
GAACAUAACA CUAGAGACAG UUUGCCAUGA CCCCAAGCUC CCCUACCAUG ACUUUAUUCU 720
GGAAGAUGCU GCUUCUCCAA AGUGCAUUAU GAAGGAAAAA AAAAAGCCUG GUGAGACUUU 780
CUUCAUGUGU UCCUGUAGCU CUGAUGAGUG CAAUGACAAC AUCAUCUUCU CAGAAGAAUA 840
UAACACCAGC AAUCCUGACU UGUUGCUAGU CAUAUUUCAA GUGACAGGCA UCAGCCUCCU 900
GCCACCACUG GGAGUUGCCA UAUCUGUCAU CAUCAUCUUC UACUGCUACC GCGUUAACCG 960
GCAGCAGAAG CUGAGUUCAA CCUGGGAAAC CGGCAAGACG CGGAAGCUCA UGGAGUUCAG 1020
CGAGCACUGU GCCAUCAUCC UGGAAGAUGA CCGCUCUGAC AUCAGCUCCA CGUGUGCCAA 1080
CAACAUCAAC CACAACACAG AGCUGCUGCC CAUUGAGCUG GACACCCUGG UGGGGAAAGG 1140
UCGCUUUGCU GAGGUCUAUA AGGCCAAGCU GAAGCAGAAC ACUUCAGAGC AGUUUGAGAC 1200
AGUGGCAGUC AAGAUCUUUC CCUAUGAGGA GUAUGCCUCU UGGAAGACAG AGAAGGACAU 1260
CUUCUCAGAC AUCAAUCUGA AGCAUGAGAA CAUACUCCAG UUCCUGACGG CUGAGGAGCG 1320
GAAGACGGAG UUGGGGAAAC AAUACUGGCU GAUCACCGCC UUCCACGCCA AGGGCAACCU 1380
ACAGGAGUAC CUGACGCGGC AUGUCAUCAG CUGGGAGGAC CUGCGCAAGC UGGGCAGCUC 1440
CCUCGCCCGG GGGAUUGCUC ACCUCCACAG UGAUCACACU CCAUGUGGGA GGCCCAAGAU 1500
GCCCAUCGUG CACAGGGACC UCAAGAGCUC CAAUAUCCUC GUGAAGAACG ACCUAACCUG 1560
CUGCCUGUGU GACUUUGGGC UUUCCCUGCG UCUGGACCCU ACUCUGUCUG UGGAUGACCU 1620
GGCUAACAGU GGGCAGGUGG GAACUGCAAG AUACAUGGCU CCAGAAGUCC UAGAAUCCAG 1680
GAUGAAUUUG GAGAAUGUUG AGUCCUUCAA GCAGACCGAU GUCUACUCCA UGGCUCUGGU 1740
GCUCUGGGAA AUGACAUCUC GCUGUAAUGC AGUGGGAGAA GUAAAAGAUU AUGAGCCUCC 1800
AUUUGGUUCC AAGGUGCGGG AGCACCCCUG UGUCGAAAGC AUGAAGGACA ACGUGUUGAG 1860
AGAUCGAGGG CGACCAGAAA UUCCCAGCUU CUGGCUCAAC CACCAGGGCA UCCAGAUGGU 1920
GUGUGAGACG UUGACUGAGU GCUGGGACCA CGACCCAGAG GCCCGUCUCA CAGCCCAGUG 1980
UGUGGCAGAA CGCUUCAGUG AGCUGGAGCA UCUGGACAGG CUCUCGGGGA GGAGCUGCUC 2040
GGAGGAGAAG AUUCCUGAAG ACGGCUCCCU AAACACUACC AAAUAGCUCU UCUGGGGCAG 2100
GCUGGGCCAU GUCCAAAGAG GCUGCCCCUC UCACCAAAGA ACAGAGGCAG CAGGAAGCUG 2160
CCCCUGAACU GAUGCUUCCU GGAAAACCAA GGGGGUCACU CCCCUCCCUG UAAGCUGUGG 2220
GGAUAAGCAG AAACAACAGC AGCAGGGAGU GGGUGACAUA GAGCAUUCUA UGCCUUUGAC 2280

AUUGUCAUAG GAUAAGCUGU GUUAGCACUU CCUCAGGAAA UGAGAUUGAU UUUUACAAUA 2340
GCCAAUAACA UUUGCACUUU AUUAAUGCCU GUAUAUAAAU AUGAAUAGCU AUGUUUUAUA 2400
UAUAUAUAUA UAUAUCUAUA UAUGUCUAUA GCUCUAUAUA UAUAGCCAUA CCUUGAAAAG 2460
AGACAAGGAA AAACAUCAAA UAUUCCCAGG AAAUUGGUUU UAUUGGAGAA CUCCAGAACC 2520
AAGCAGAGAA GGAAGGGACC CAUGACAGCA UUAGCAUUUG ACAAUCACAC AUGCAGUGGU 2580
UCUCUGACUG UAAAACAGUG AACUUUGCAU GAGGAAAGAG GCUCCAUGUC UCACAGCCAG 2640
CUAUGACCAC AUUGCACUUG CUUUUGCAAA AUAAUCAUUC CCUGCCUAGC ACUUCUCUUC 2700
UGGCCAUGGA ACUAAGUACA GUGGCACUGU UUGAGGACCA GUGUUCCCGG GGUUCCUGUG 2760
UGCCCUUAUU UCUCCUGGAC UUUUCAUUUA AGCUCCAAGC CCCAAAUCUG GGGGGCUAGU 2820
UUAGAAACUC UCCCUCAACC UAGUUUAGAA ACUCUACCCC AUCUUUAAUA CCUUGAAUGU 2880
UUUGAACCCC ACUUUUUACC UUCAUGGGUU GCAGAAAAAU CAGAACAGAU GUCCCCAUCC 2940
AUGCGAUUGC CCCACCAUCU ACUAAUGAAA AAUUGUUCUU UUUUUCAUCU UUCCCCUGCA 3000
CUUAUGUUAC UAUUCUCUGC UCCCAGCCUU CAUCCUUUUC UAAAAAGGAG CAAAUUCUCA 3060
CUCUAGGCUU UAUCGUGUUU ACUUUUUCAU UACACUUGAC UUGAUUUUCU AGUUUUCUAU 3120
ACAAACACCA AUGGGUUCCA UCUUUCUGGG CUCCUGAUUG CUCAAGCACA GUUUGGCCUG 3180
AUGAAGAGGA UUUCAACUAC ACAAUACUAU CAUUGUCAGG ACUAUGACCU CAGGCACUCU 3240
AAACAUAUGU UUUGUUUGGU CAGCACAGCG UUUCAAAAAG UGAAGCCACU UUAUAAAUAU 3300
UUGGAGAUUU UGCAGGAAAA UCUGGAUCCC CAGGUAAGGA UAGCAGAUGG UUUUCAGUUA 3360
UCUCCAGUCC ACGUUCACAA AAUGUGAAGG UGUGGAGACA CUUACAAAGC UGCCUCACUU 3420
CUCACUGUAA ACAUUAGCUC UUUCCACUGC CUACCUGGAC CCCAGUCUAG GAAUUAAAUC 3480
UGCACCUAAC CAAGGUCCCU UGUAAGAAAU GUCCAUUCAA GCAGUCAUUC UCUGGGUAUA 3540
UAAUAUGAUU UUGACUACCU UAUCUGGUGU UAAGAUUUGA AGUUGGCCUU UUAUUGGACU 3600
AAAGGGGAAC UCCUUUAAGG GUCUCAGUUA GCCCAAGUUU CUUUUGCUUA UAUGUUAAUA 3660
GUUUUACCCU CUGCAUUGGA GAGAGGAGUG CUUUACUCCA AGAAGCUUUC CUCAUGGUUA 3720
CCGUUCUCUC CAUCAUGCCA GCCUUCUCAA CCUUUGCAGA AAUUACUAGA GAGGAUUUGA 3780
AUGUGGGACA CAAAGGUCCC AUUUGCAGUU AGAAAAUUUG UGUCCACAAG GACAAGAACA 3840
AAGUAUGAGC UUUAAAACUC CAUAGGAAAC UUGUUAAUCA ACAAAGAAGU GUUAAUGCUG 3900
CAAGUAAUCU CUUUUUUAAA ACUUUUUGAA GCUACUUAUU UUCAGCCAAA UAGGAAUAUU 3960
AGAGAGGGAC UGGUAGUGAG AAUAUCAGCU CUGUUUGGAU GGUGGAAGGU CUCAUUUUAU 4020
UGAGAUUUUU AAGAUACAUG CAAAGGUUUG GAAAUAGAAC CUCUAGGCAC CCUCCUCAGU 4080
GUGGGUGGGC UGAGAGUUAA AGACAGUGUG GCUGCAGUAG CAUAGAGGCG CCUAGAAAUU 4140
CCACUUGCAC CGUAGGGCAU GCUGAUACCA UCCCAAUAGC UGUUGCCCAU UGACCUCUAG 4200
UGGUGAGUUU CUAGAAUACU GGUCCAUUCA UGAGAUAUUC AAGAUUCAAG AGUAUUCUCA 4260
CUUCUGGGUU AUCAGCAUAA ACUGGAAUGU AGUGUCAGAG GAUACUGUGG CUUGUUUUGU 4320
UUAUGUUUUU UUUUCUUAUU CAAGAAAAAA GACCAAGGAA UAACAUUCUG UAGUUCCUAA 4380
AAAUACUGAC UUUUUUCACU ACUAUACAUA AAGGGAAAGU UUUAUUCUUU UAUGGAACAC 4440
UUCAGCUGUA CUCAUGUAUU AAAAUAGGAA UGUGAAUGCU AUAUACUCUU UUUAUAUCAA 4500
AAGUCUCAAG CACUUAUUUU UAUUCUAUGC AUUGUUUGUC UUUUACAUAA AUAAAAUGUU 4560
UAUUAGAUUG AAUAAAGCAA AAUACUCAGG UGAGCAUCCU GCCUCCUGUU CCCAUUCCUA 4620
GUAGCUAAA 4629

SEQUENCE LISTING

<110>  Axolabs GmbH

<120>  Antisense-Oligonucleotides as Inhibitors of TGF-R signaling

<130>  REG-P03653EP(II)

<160>  428

<170>  PatentIn version 3.5

<210>  1
<211>  4629
<212>  DNA
<213>  Artificial Sequence

<220>
<223>  Homo sapiens transforming growth factor, beta receptor II
       (70/80kDa) (TGFBR2), transcript variant 2, antisense DNA

<400>  1
tttagctact aggaatggga acaggaggca ggatgctcac ctgagtattt tgctttattc      60

aatctaataa acattttatt tatgtaaaag acaaacaatg catagaataa aaataagtgc     120

ttgagacttt tgatataaaa agagtatata gcattcacat tcctatttta atacatgagt     180

acagctgaag tgttccataa aagaataaaa ctttcccttt atgtatagta gtgaaaaaag     240

tcagtatttt taggaactac agaatgttat tccttggtct tttttcttga ataagaaaaa     300

aaaacataaa caaaacaagc cacagtatcc tctgacacta cattccagtt tatgctgata     360

acccagaagt gagaatactc ttgaatcttg aatatctcat gaatggacca gtattctaga     420

aactcaccac tagaggtcaa tgggcaacag ctattgggat ggtatcagca tgccctacgg     480

tgcaagtgga atttctaggc gcctctatgc tactgcagcc acactgtctt taactctcag     540

cccacccaca ctgaggaggg tgcctagagg ttctatttcc aaacctttgc atgtatctta     600

aaaatctcaa taaaatgaga ccttccacca tccaaacaga gctgatattc tcactaccag     660

tccctctcta atattcctat ttggctgaaa ataagtagct tcaaaaagtt ttaaaaaaga     720

gattacttgc agcattaaca cttctttgtt gattaacaag tttcctatgg agttttaaag     780

ctcatacttt gttcttgtcc ttgtggacac aaattttcta actgcaaatg ggacctttgt     840

gtcccacatt caaatcctct ctagtaattt ctgcaaaggt tgagaaggct ggcatgatgg     900

agagaacggt aaccatgagg aaagcttctt ggagtaaagc actcctctct ccaatgcaga     960

gggtaaaact attaacatat aagcaaaaga aacttgggct aactgagacc cttaaaggag    1020

ttccccttta gtccaataaa aggccaactt caaatcttaa caccagataa ggtagtcaaa    1080

atcatattat atacccagag aatgactgct tgaatggaca tttcttacaa gggaccttgg    1140

ttaggtgcag atttaattcc tagactgggg tccaggtagg cagtggaaag agctaatgtt    1200

tacagtgaga agtgaggcag ctttgtaagt gtctccacac cttcacattt tgtgaacgtg    1260

gactggagat aactgaaaac catctgctat ccttacctgg ggatccagat tttcctgcaa 1320

aatctccaaa tatttataaa gtggcttcac tttttgaaac gctgtgctga ccaaacaaaa 1380

catatgttta gagtgcctga ggtcatagtc ctgacaatga tagtattgtg tagttgaaat 1440

cctcttcatc aggccaaact gtgcttgagc aatcaggagc ccagaaagat ggaacccatt 1500

ggtgtttgta tagaaaacta gaaaatcaag tcaagtgtaa tgaaaaagta aacacgataa 1560

agcctagagt gagaatttgc tcctttttag aaaaggatga aggctgggag cagagaatag 1620

taacataagt gcaggggaaa gatgaaaaaa agaacaattt ttcattagta gatggtgggg 1680

caatcgcatg gatggggaca tctgttctga tttttctgca acccatgaag gtaaaaagtg 1740

gggttcaaaa cattcaaggt attaaagatg gggtagagtt tctaaactag gttgagggag 1800

agtttctaaa ctagcccccc agatttgggg cttggagctt aaatgaaaag tccaggagaa 1860

ataagggcac acaggaaccc cgggaacact ggtcctcaaa cagtgccact gtacttagtt 1920

ccatggccag aagagaagtg ctaggcaggg aatgattatt ttgcaaaagc aagtgcaatg 1980

tggtcatagc tggctgtgag acatggagcc tctttcctca tgcaaagttc actgttttac 2040

agtcagagaa ccactgcatg tgtgattgtc aaatgctaat gctgtcatgg gtcccttcct 2100

tctctgcttg gttctggagt tctccaataa aaccaatttc ctgggaatat ttgatgtttt 2160

tccttgtctc ttttcaaggt atggctatat atatagagct atagacatat atagatatat 2220

atatatatat ataaaacata gctattcata tttatataca ggcattaata aagtgcaaat 2280

gttattggct attgtaaaaa tcaatctcat ttcctgagga agtgctaaca cagcttatcc 2340

tatgacaatg tcaaaggcat agaatgctct atgtcaccca ctccctgctg ctgttgtttc 2400

tgcttatccc cacagcttac agggagggga gtgaccccct tggttttcca ggaagcatca 2460

gttcaggggc agcttcctgc tgcctctgtt ctttggtgag aggggcagcc tctttggaca 2520

tggcccagcc tgccccagaa gagctatttg gtagtgttta gggagccgtc ttcaggaatc 2580

ttctcctccg agcagctcct ccccgagagc ctgtccagat gctccagctc actgaagcgt 2640

tctgccacac actgggctgt gagacgggcc tctgggtcgt ggtcccagca ctcagtcaac 2700

gtctcacaca ccatctggat gccctggtgg ttgagccaga agctgggaat ttctggtcgc 2760

cctcgatctc tcaacacgtt gtccttcatg ctttcgacac aggggtgctc ccgcaccttg 2820

gaaccaaatg gaggctcata atcttttact tctcccactg cattacagcg agatgtcatt 2880

tcccagagca ccagagccat ggagtagaca tcggtctgct tgaaggactc aacattctcc 2940

aaattcatcc tggattctag gacttctgga gccatgtatc ttgcagttcc cacctgccca 3000

ctgttagcca ggtcatccac agacagagta gggtccagac gcagggaaag cccaaagtca 3060

cacaggcagc aggttaggtc gttcttcacg aggatattgg agctcttgag gtccctgtgc 3120

acgatgggca tcttgggcct cccacatgga gtgtgatcac tgtggaggtg agcaatcccc 3180

cgggcgaggg agctgcccag cttgcgcagg tcctcccagc tgatgacatg ccgcgtcagg 3240

tactcctgta ggttgccctt ggcgtggaag gcggtgatca gccagtattg tttccccaac 3300

tccgtcttcc gctcctcagc cgtcaggaac tggagtatgt tctcatgctt cagattgatg 3360

tctgagaaga tgtccttctc tgtcttccaa gaggcatact cctcataggg aaagatcttg 3420

actgccactg tctcaaactg ctctgaagtg ttctgcttca gcttggcctt atagacctca 3480

gcaaagcgac ctttccccac cagggtgtcc agctcaatgg gcagcagctc tgtgttgtgg 3540

ttgatgttgt tggcacacgt ggagctgatg tcagagcggt catcttccag gatgatggca 3600

cagtgctcgc tgaactccat gagcttccgc gtcttgccgg tttcccaggt tgaactcagc 3660

ttctgctgcc ggttaacgcg gtagcagtag aagatgatga tgacagatat ggcaactccc 3720

agtggtggca ggaggctgat gcctgtcact tgaaatatga ctagcaacaa gtcaggattg 3780

ctggtgttat attcttctga gaagatgatg ttgtcattgc actcatcaga gctacaggaa 3840

cacatgaaga aagtctcacc aggctttttt ttttccttca taatgcactt tggagaagca 3900

gcatcttcca gaataaagtc atggtagggg agcttggggt catggcaaac tgtctctagt 3960

gttatgttct cgtcattctt tctccataca gccacacaga cttcctgtgg cttctcacag 4020

atggaggtga tgctgcagtt gctcatgcag gatttctggt tgtcacaggt ggaaaatctc 4080

acatcacaaa atttacacag ttgtggaaac ttgactgcac cgttgttgtc agtgactatc 4140

atgtcgttat taaccgactt ctgaacgtgc ggtgggatcg tgctggcgat acgcgtccac 4200

aggacgatgt gcagcggcca caggcccctg agcagccccc gacccatggc agaccccgct 4260

gctcgtcata gaccgagccc ccagcgcagc ggacggcgcc ttcccggacc cctggctgcg 4320

cctccgcgcc gcgccctctc cggaccccgc gccgggccgg cagcgcagat gtgcgggcca 4380

gatgtggcgc ccgctcgcca gccaggaggg ggcctggagg ccggcgaggc gcggggaggc 4440

ccccggcggc cgagggaagc tgcacaggag tccggctcct gtcccgagcg ggtgcacgcg 4500

cgggggtgtc gtcgctccgt gcgcgcgagt gactcactca acttcaactc agcgctgcgg 4560

gggaaacagg aaactcctcg ccaacagctg ggcaggacct ctctccgccc gagagccttc 4620

tccctctcc 4629

<210> 2
<211> 4629
<212> DNA
<213> Artificial Sequence

<220>
<223> Homo sapiens transforming growth factor, beta receptor II
 (70/80kDa) (TGFBR2), transcript variant 2, sense DNA

<400> 2

ggagagggag aaggctctcg ggcggagaga ggtcctgccc agctgttggc gaggagtttc 60

ctgtttcccc cgcagcgctg agttgaagtt gagtgagtca ctcgcgcgca cggagcgacg 120

acacccccgc gcgtgcaccc gctcgggaca ggagccggac tcctgtgcag cttccctcgg 180

ccgccggggg cctccccgcg cctcgccggc ctccaggccc cctcctggct ggcgagcggg 240

cgccacatct ggcccgcaca tctgcgctgc cggcccggcg cggggtccgg agagggcgcg 300

gcgcggaggc gcagccaggg gtccgggaag gcgccgtccg ctgcgctggg ggctcggtct 360

atgacgagca gcggggtctg ccatgggtcg ggggctgctc aggggcctgt ggccgctgca 420

catcgtcctg tggacgcgta tcgccagcac gatcccaccg cacgttcaga agtcggttaa 480

taacgacatg atagtcactg acaacaacgg tgcagtcaag tttccacaac tgtgtaaatt 540

ttgtgatgtg agattttcca cctgtgacaa ccagaaatcc tgcatgagca actgcagcat 600

cacctccatc tgtgagaagc cacaggaagt ctgtgtggct gtatggagaa agaatgacga 660

gaacataaca ctagagacag tttgccatga ccccaagctc ccctaccatg actttattct 720

ggaagatgct gcttctccaa agtgcattat gaaggaaaaa aaaaagcctg gtgagacttt 780

cttcatgtgt tcctgtagct ctgatgagtg caatgacaac atcatcttct cagaagaata 840

taacaccagc aatcctgact tgttgctagt catatttcaa gtgacaggca tcagcctcct 900

gccaccactg ggagttgcca tatctgtcat catcatcttc tactgctacc gcgttaaccg 960

gcagcagaag ctgagttcaa cctgggaaac cggcaagacg cggaagctca tggagttcag 1020

cgagcactgt gccatcatcc tggaagatga ccgctctgac atcagctcca cgtgtgccaa 1080

caacatcaac cacaacacag agctgctgcc cattgagctg gacaccctgg tggggaaagg 1140

tcgctttgct gaggtctata aggccaagct gaagcagaac acttcagagc agtttgagac 1200

agtggcagtc aagatctttc cctatgagga gtatgcctct tggaagacag agaaggacat 1260

cttctcagac atcaatctga agcatgagaa catactccag ttcctgacgg ctgaggagcg 1320

gaagacggag ttggggaaac aatactggct gatcaccgcc ttccacgcca agggcaacct 1380

acaggagtac ctgacgcggc atgtcatcag ctgggaggac ctgcgcaagc tgggcagctc 1440

cctcgcccgg gggattgctc acctccacag tgatcacact ccatgtggga ggcccaagat 1500

gcccatcgtg cacagggacc tcaagagctc caatatcctc gtgaagaacg acctaacctg 1560

ctgcctgtgt gactttgggc tttccctgcg tctggaccct actctgtctg tggatgacct 1620

ggctaacagt gggcaggtgg gaactgcaag atacatggct ccagaagtcc tagaatccag 1680

gatgaatttg gagaatgttg agtccttcaa gcagaccgat gtctactcca tggctctggt 1740

gctctgggaa atgacatctc gctgtaatgc agtgggagaa gtaaaagatt atgagcctcc 1800

atttggttcc aaggtgcggg agcacccctg tgtcgaaagc atgaaggaca acgtgttgag 1860

agatcgaggg cgaccagaaa ttcccagctt ctggctcaac caccagggca tccagatggt 1920

gtgtgagacg ttgactgagt gctgggacca cgacccagag gcccgtctca cagcccagtg 1980

tgtggcagaa cgcttcagtg agctggagca tctggacagg ctctcgggga ggagctgctc 2040

ggaggagaag attcctgaag acggctccct aaacactacc aaatagctct tctggggcag 2100

gctgggccat gtccaaagag gctgcccctc tcaccaaaga acagaggcag caggaagctg 2160

cccctgaact gatgcttcct ggaaaaccaa gggggtcact cccctccctg taagctgtgg 2220

ggataagcag aaacaacagc agcagggagt gggtgacata gagcattcta tgcctttgac 2280

attgtcatag gataagctgt gttagcactt cctcaggaaa tgagattgat ttttacaata 2340

gccaataaca tttgcacttt attaatgcct gtatataaat atgaatagct atgttttata 2400

tatatatata tatatctata tatgtctata gctctatata tatagccata ccttgaaaag 2460

agacaaggaa aaacatcaaa tattcccagg aaattggttt tattggagaa ctccagaacc 2520

aagcagagaa ggaagggacc catgacagca ttagcatttg acaatcacac atgcagtggt 2580

tctctgactg taaaacagtg aactttgcat gaggaaagag gctccatgtc tcacagccag 2640

ctatgaccac attgcacttg cttttgcaaa ataatcattc cctgcctagc acttctcttc 2700

tggccatgga actaagtaca gtggcactgt ttgaggacca gtgttcccgg ggttcctgtg 2760

tgcccttatt tctcctggac ttttcattta agctccaagc cccaaatctg gggggctagt 2820

ttagaaactc tccctcaacc tagtttagaa actctacccc atctttaata ccttgaatgt 2880

tttgaacccc actttttacc ttcatgggtt gcagaaaaat cagaacagat gtccccatcc 2940

atgcgattgc cccaccatct actaatgaaa aattgttctt tttttcatct ttcccctgca 3000

cttatgttac tattctctgc tcccagcctt catccttttc taaaaaggag caaattctca 3060

ctctaggctt tatcgtgttt actttttcat tacacttgac ttgattttct agttttctat 3120

acaaacacca atgggttcca tctttctggg ctcctgattg ctcaagcaca gtttggcctg 3180

atgaagagga tttcaactac acaatactat cattgtcagg actatgacct caggcactct 3240

aaacatatgt tttgtttggt cagcacagcg tttcaaaaag tgaagccact ttataaatat 3300

ttggagattt tgcaggaaaa tctggatccc caggtaagga tagcagatgg ttttcagtta 3360

tctccagtcc acgttcacaa aatgtgaagg tgtggagaca cttacaaagc tgcctcactt 3420

ctcactgtaa acattagctc tttccactgc ctacctggac cccagtctag gaattaaatc 3480

tgcacctaac caaggtccct tgtaagaaat gtccattcaa gcagtcattc tctgggtata 3540

taatatgatt ttgactacct tatctggtgt taagatttga agttggcctt ttattggact 3600

aaaggggaac tcctttaagg gtctcagtta gcccaagttt cttttgctta tatgttaata 3660

gttttaccct ctgcattgga gagaggagtg ctttactcca agaagctttc ctcatggtta 3720

ccgttctctc catcatgcca gccttctcaa cctttgcaga aattactaga gaggatttga 3780

atgtgggaca caaaggtccc atttgcagtt agaaaatttg tgtccacaag gacaagaaca 3840

aagtatgagc tttaaaactc cataggaaac ttgttaatca acaaagaagt gttaatgctg 3900

caagtaatct cttttttaaa actttttgaa gctacttatt ttcagccaaa taggaatatt 3960

agagagggac tggtagtgag aatatcagct ctgtttggat ggtggaaggt ctcattttat 4020

tgagattttt aagatacatg caaaggtttg gaaatagaac ctctaggcac cctcctcagt 4080

gtgggtgggc tgagagttaa agacagtgtg gctgcagtag catagaggcg cctagaaatt 4140

ccacttgcac cgtagggcat gctgatacca tcccaatagc tgttgcccat tgacctctag 4200

tggtgagttt ctagaatact ggtccattca tgagatattc aagattcaag agtattctca 4260

cttctgggtt atcagcataa actggaatgt agtgtcagag gatactgtgg cttgttttgt 4320

ttatgttttt ttttcttatt caagaaaaaa gaccaaggaa taacattctg tagttcctaa 4380

aaatactgac ttttttcact actatacata aagggaaagt tttattcttt tatggaacac 4440

ttcagctgta ctcatgtatt aaaataggaa tgtgaatgct atatactctt tttatatcaa 4500

aagtctcaag cacttatttt tattctatgc attgtttgtc ttttacataa ataaaatgtt 4560

tattagattg aataaagcaa aatactcagg tgagcatcct gcctcctgtt cccattccta 4620

gtagctaaa 4629

<210> 3
<211> 4629
<212> RNA
<213> Artificial Sequence

<220>
<223> Homo sapiens transforming growth factor, beta receptor II
      (70/80kDa) (TGFBR2), transcript variant 2, sense RNA

<400> 3
ggagagggag aaggcucucg ggcggagaga gguccugccc agcuguuggc gaggaguuuc 60

cuguuucccc cgcagcgcug aguugaaguu gagugaguca cucgcgcgca cggagcgacg 120

acacccccgc gcgugcaccc gcucgggaca ggagccggac uccugugcag cuucccucgg 180

ccgccggggg ccuccccgcg ccucgccggc cuccaggccc ccuccuggcu ggcgagcggg 240

cgccacaucu ggcccgcaca ucugcgcugc cggcccggcg cgggguccgg agagggcgcg 300

gcgcggaggc gcagccaggg guccgggaag gcgccguccg cugcgcuggg ggcucggucu 360

augacgagca gcggggucug ccaugggucg ggggcugcuc aggggccugu ggccgcugca 420

caucguccug uggacgcgua ucgccagcac gaucccaccg cacguucaga agucgguuaa 480

uaacgacaug auagucacug acaacaacgg ugcagucaag uuuccacaac uguguaaauu 540

uugugaugug agauuuucca ccugugacaa ccagaaaucc ugcaugagca acugcagcau 600

caccuccauc ugugagaagc cacaggaagu cuguguggcu guauggagaa agaaugacga 660

gaacauaaca cuagagacag uuugccauga ccccaagcuc cccuaccaug acuuuauucu 720

ggaagaugcu gcuucuccaa agugcauuau gaaggaaaaa aaaaagccug gugagacuuu 780

cuucaugugu uccuguagcu cugaugagug caaugacaac aucaucuucu cagaagaaua 840

uaacaccagc aauccugacu uguugcuagu cauauuucaa gugacaggca ucagccuccu 900

gccaccacug ggaguugcca uaucugucau caucaucuuc uacugcuacc gcguuaaccg 960

gcagcagaag cugaguucaa ccugggaaac cggcaagacg cggaagcuca uggaguucag 1020

cgagcacugu gccaucaucc uggaagauga ccgcucugac aucagcucca cgugugccaa 1080

caacaucaac cacaacacag agcugcugcc cauugagcug gacacccugg uggggaaagg 1140

ucgcuuugcu gaggucuaua aggccaagcu gaagcagaac acuucagagc aguuugagac 1200

aguggcaguc aagaucuuuc ccuaugagga guaugccucu uggaagacag agaaggacau 1260

cuucucagac aucaaucuga agcaugagaa cauacuccag uuccugacgg cugaggagcg 1320

gaagacggag uuggggaaac aauacuggcu gaucaccgcc uuccacgcca agggcaaccu 1380

acaggaguac cugacgcggc augucaucag cugggaggac cugcgcaagc ugggcagcuc 1440

ccucgcccgg gggauugcuc accuccacag ugaucacacu ccauguggga ggcccaagau 1500

gcccaucgug cacagggacc ucaagagcuc caauauccuc gugaagaacg accuaaccug 1560

cugccugugu gacuuugggc uuucccugcg ucuggacccu acucugucug uggaugaccu 1620

ggcuaacagu gggcaggugg gaacugcaag auacauggcu ccagaagucc uagaauccag 1680

gaugaauuug gagaauguug aguccuucaa gcagaccgau gucuacucca uggcucuggu 1740

gcucugggaa augacaucuc gcuguaaugc agugggagaa guaaaagauu augagccucc 1800

auuugguucc aaggugcggg agcaccccug ugucgaaagc augaaggaca acguguugag 1860

agaucgaggg cgaccagaaa uucccagcuu cuggcucaac caccagggca uccagauggu 1920

gugugagacg uugacugagu gcugggacca cgacccagag gcccgucuca cagcccagug 1980

uguggcagaa cgcuucagug agcuggagca ucuggacagg cucucgggga ggagcugcuc 2040

ggaggagaag auuccugaag acggcucccu aaacacuacc aaauagcucu ucuggggcag 2100

gcugggccau guccaaagag gcugccccuc ucaccaaaga acagaggcag caggaagcug 2160

ccccugaacu gaugcuuccu ggaaaaccaa gggggucacu ccccucccug uaagcugugg 2220

ggauaagcag aaacaacagc agcagggagu gggugacaua gagcauucua ugccuuugac 2280

auugucauag gauaagcugu guuagcacuu ccucaggaaa ugagauugau uuuuacaaua 2340

gccaauaaca uuugcacuuu auuaaugccu guauauaaau augaauagcu auguuuuaua 2400

uauauauaua uauaucuaua uaugucuaua gcucuauaua uauagccaua ccuugaaaag 2460

agacaaggaa aaacaucaaa uauucccagg aaauugguuu uauuggagaa cuccagaacc 2520

aagcagagaa ggaagggacc caugacagca uuagcauuug acaaucacac augcaguggu 2580

ucucugacug uaaaacagug aacuuugcau gaggaaagag gcuccauguc ucacagccag 2640

cuaugaccac auugcacuug cuuuugcaaa auaaucauuc ccugccuagc acuucucuuc 2700

uggccaugga acuaaguaca guggcacugu uugaggacca guguucccgg gguuccugug 2760

ugcccuuauu ucuccuggac uuuucauuua agcuccaagc cccaaaucug gggggcuagu 2820

uuagaaacuc ucccucaacc uaguuuagaa acucuacccc aucuuuaaua ccuugaaugu 2880

uuugaacccc acuuuuuacc uucaugguuu gcagaaaaau cagaacagau guccccaucc 2940

augcgauugc cccaccaucu acuaaugaaa aauuguucuu uuuuucaucu uuccccugca 3000

cuuauguuac uauucucugc ucccagccuu cauccuuuuc uaaaaaggag caaauucuca 3060

cucuaggcuu uaucguguuu acuuuuucau uacacuugac uugauuuucu aguuuucuau 3120

acaaacacca auggguucca ucuuucuggg cuccugauug cucaagcaca guuuggccug 3180

augaagagga uuucaacuac acaauacuau cauugucagg acuaugaccu caggcacucu 3240

aaacauaugu uuuguuuggu cagcacagcg uuucaaaaag ugaagccacu uuauaaauau 3300

uuggagauuu ugcaggaaaa ucuggauccc cagguaagga uagcagaugg uuuucaguua 3360

ucuccagucc acguucacaa aaugugaagg uguggagaca cuuacaaagc ugccucacuu 3420

cucacuguaa acauuagcuc uuuccacugc cuaccuggac cccagucuag gaauuaaauc 3480

ugcaccuaac caaggucccu uguaagaaau guccauucaa gcagucauuc ucugguauaa 3540

uaauaugauu uugacuaccu uaucuggugu uaagauuuga aguuggccuu uuauuggacu 3600

aaaggggaac uccuuuaagg gucucaguua gcccaaguuu cuuuugcuua uauguuaaua 3660

guuuuacccu cugcauugga gagaggagug cuuuacucca agaagcuuuc cucaugguua 3720

ccguucucuc caucaugcca gccuucucaa ccuuugcaga aauuacuaga gaggauuuga 3780

augugggaca caaagguccc auuugcaguu agaaaauuug uguccacaag gacaagaaca 3840

aaguaugagc uuuaaaacuc cauaggaaac uuguuaauca acaaagaagu guuaaugcug 3900

caaguaaucu cuuuuuuaaa acuuuuugaa gcuacuuauu uucagccaaa uaggaauauu 3960

agagagggac ugguagugag aauaucagcu cuguuuggau gguggaaggu cucauuuuau 4020

ugagauuuuu aagauacaug caaagguuug gaaauagaac cucuaggcac ccuccucagu 4080

gugggugggc ugagaguuaa agacagugug gcugcaguag cauagaggcg ccuagaaauu 4140

ccacuugcac cguagggcau gcugauacca ucccaauagc uguugcccau ugaccucuag 4200

uggugaguuu cuagaauacu gguccauuca ugagauauuc aagauucaag aguauucuca 4260

cuucuggguu aucagcauaa acuggaaugu agugucagag gauacugugg cuuguuuugu 4320

uuauguuuuu uuuucuuauu caagaaaaaa gaccaaggaa uaacauucug uaguuccuaa 4380

aaauacugac uuuuuucacu acuauacaua aagggaaagu uuuauucuuu uauggaacac 4440

uucagcugua cucauguauu aaaauaggaa ugugaaugcu auauacucuu uuuauaucaa 4500

aagucucaag cacuuauuuu uauucuaugc auuguuuguc uuuuacauaa auaaaauguu 4560

uauuagauug aauaaagcaa aauacucagg ugagcauccu gccuccuguu cccauuccua 4620

guagcuaaa 4629

<210> 4
<211> 61
<212> DNA
<213> Artificial Sequence

<220>
<223> Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400> 4
gaatactctt gaatcttgaa tatctcatga atggaccagt attctagaaa ctcaccacta 60

g 61

<210> 5
<211> 63
<212> DNA
<213> Artificial Sequence

<220>
<223> Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400> 5
gcatgcccta cggtgcaagt ggaatttcta ggcgcctcta tgctactgca gccacactgt 60

ctt 63

<210> 6
<211> 61
<212> DNA
<213> Artificial Sequence

<220>
<223> Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400> 6
aacatagcta ttcatattta tatacaggca ttaataaagt gcaaatgtta ttggctattg 60

t 61

<210> 7
<211> 62
<212> DNA

<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  7
tctcatttcc tgaggaagtg ctaacacagc ttatcctatg acaatgtcaa aggcatagaa      60

tg                                                                     62


<210>  8
<211>  69
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  8
acatggccca gcctgcccca gaagagctat ttggtagtgt ttagggagcc gtcttcagga      60

atcttctcc                                                              69


<210>  9
<211>  108
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  9
agaagctggg aatttctggt cgccctcgat ctctcaacac gttgtccttc atgctttcga      60

cacaggggtg ctcccgcacc ttggaaccaa atggaggctc ataatctt                  108


<210>  10
<211>  69
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  10
gcccagcttg cgcaggtcct cccagctgat gacatgccgc gtcaggtact cctgtaggtt      60

gcccttggc                                                              69

<210>  11
<211>  70
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  11
gtttcccagg ttgaactcag cttctgctgc cggttaacgc ggtagcagta gaagatgatg      60

atgacagata                                                              70


<210>  12
<211>  169
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  12
agtgactatc atgtcgttat taaccgactt ctgaacgtgc ggtgggatcg tgctggcgat      60

acgcgtccac aggacgatgt gcagcggcca caggcccctg agcagccccc gacccatggc     120

agaccccgct gctcgtcata gaccgagccc ccagcgcagc ggacggcgc                 169


<210>  13
<211>  98
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  13
agtgactatc atgtcgttat taaccgactt ctgaacgtgc ggtgggatcg tgctggcgat      60

acgcgtccac aggacgatgt gcagcggcca caggcccc                              98


<210>  14
<211>  94
<212>  DNA
<213>  Artificial Sequence

<220>
<223>  Antisense oligonucleotide against Homo sapiens transforming
       growth factor, beta receptor II (70/80kDa) (TGFBR2), transcript

variant 2, wherein at least 2 nucleotides are LNA units (i.e.
      locked nucleotides)

<400>  14
gatgtgcagc ggccacaggc ccctgagcag cccccgaccc atggcagacc ccgctgctcg      60

tcatagaccg agcccccagc gcagcggacg gcgc                                  94


<210>  15
<211>  82
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  15
tgtcccgagc gggtgcacgc gcgggggtgt cgtcgctccg tgcgcgcgag tgactcactc      60

aacttcaact cagcgctgcg gg                                               82


<210>  16
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  16
aacatagcta ttcat                                                       15


<210>  17
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  17
acatagctat tcata                                                       15


<210>  18
<211>  15
<212>  DNA
<213>  Artificial Sequence

<220>
<223>  Antisense oligonucleotide against Homo sapiens transforming

growth factor, beta receptor II (70/80kDa) (TGFBR2), transcript
      variant 2, wherein at least 2 nucleotides are LNA units (i.e.
      locked nucleotides)

<400>  18
catagctatt catat                                                        15


<210>  19
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  19
atagctattc atatt                                                        15


<210>  20
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  20
tagctattca tattt                                                        15


<210>  21
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  21
agctattcat attta                                                        15


<210>  22
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  22
gctattcata tttat                                                        15


<210>  23
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  23
ctattcatat ttata                                                        15


<210>  24
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  24
tattcatatt tatat                                                        15


<210>  25
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  25
attcatattt atata                                                        15


<210>  26
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  26
ttcatattta tatac                                                        15

<210>  27
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  27
tcatatttat ataca                                                        15


<210>  28
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  28
catatttata tacag                                                        15


<210>  29
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  29
atatttatat acagg                                                        15


<210>  30
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  30
tatttatata caggc                                                        15


<210>  31

<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  31
atttatatac aggca                                                        15


<210>  32
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  32
tttatataca ggcat                                                        15


<210>  33
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  33
ttatatacag gcatt                                                        15


<210>  34
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  34
tatatacagg catta                                                        15


<210>  35
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  35
atatacaggc attaa                                                        15


<210>  36
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  36
tatacaggca ttaat                                                        15


<210>  37
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  37
atacaggcat taata                                                        15


<210>  38
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  38
tacaggcatt aataa                                                        15


<210>  39
<211>  15
<212>  DNA
<213>  Artificial Sequence

<220>
<223>  Antisense oligonucleotide against Homo sapiens transforming

growth factor, beta receptor II (70/80kDa) (TGFBR2), transcript
      variant 2, wherein at least 2 nucleotides are LNA units (i.e.
      locked nucleotides)

<400>  39
acaggcatta ataaa                                                        15


<210>  40
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  40
caggcattaa taaag                                                        15


<210>  41
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  41
aggcattaat aaagt                                                        15


<210>  42
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  42
ggcattaata aagtg                                                        15


<210>  43
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  43
gcattaataa agtgc                                                        15


<210>  44
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  44
cattaataaa gtgca                                                        15


<210>  45
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  45
attaataaag tgcaa                                                        15


<210>  46
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  46
ttaataaagt gcaaa                                                        15


<210>  47
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  47
taataaagtg caaat                                                        15

<210>  48
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  48
aataaagtgc aaatg                                                        15


<210>  49
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  49
ataaagtgca aatgt                                                        15


<210>  50
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  50
taaagtgcaa atgtt                                                        15


<210>  51
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  51
aaagtgcaaa tgtta                                                        15


<210>  52

<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  52
aagtgcaaat gttat                                                        15


<210>  53
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  53
agtgcaaatg ttatt                                                        15


<210>  54
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  54
gtgcaaatgt tattg                                                        15


<210>  55
<211>  15
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  55
tgcaaatgtt attgg                                                        15


<210>  56
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  56
gcaaatgtta ttggc                                                        15


<210>  57
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  57
caaatgttat tggct                                                        15


<210>  58
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  58
aaatgttatt ggcta                                                        15


<210>  59
<211>  15
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  59
aatgttattg gctat                                                        15


<210>  60
<211>  15
<212>  DNA
<213>  Artificial Sequence

<220>
<223>  Antisense oligonucleotide against Homo sapiens transforming

growth factor, beta receptor II (70/80kDa) (TGFBR2), transcript
variant 2, wherein at least 2 nucleotides are LNA units (i.e.
locked nucleotides)

<400> 60
atgttattgg ctatt 15

<210> 61
<211> 15
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 61
tgttattggc tattg 15

<210> 62
<211> 15
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 62
gttattggct attgt 15

<210> 63
<211> 51
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 63
ctcttgaatc ttgaatatct catgaatgga ccagtattct agaaactcac c 51

<210> 64
<211> 53
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400>  64
ccctacggtg caagtggaat ttctaggcgc ctctatgcta ctgcagccac act          53

<210>  65
<211>  51
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  65
agctattcat atttatatac aggcattaat aaagtgcaaa tgttattggc t            51

<210>  66
<211>  52
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  66
tttcctgagg aagtgctaac acagcttatc ctatgacaat gtcaaaggca ta           52

<210>  67
<211>  59
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  67
gcccagcctg ccccagaaga gctatttggt agtgtttagg gagccgtctt caggaatct    59

<210>  68
<211>  98
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  68
ctgggaattt ctggtcgccc tcgatctctc aacacgttgt ccttcatgct ttcgacacag   60

gggtgctccc gcaccttgga accaaatgga ggctcata 98

<210> 69
<211> 59
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 69
gcttgcgcag gtcctcccag ctgatgacat gccgcgtcag gtactcctgt aggttgccc 59

<210> 70
<211> 60
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 70
ccaggttgaa ctcagcttct gctgccggtt aacgcggtag cagtagaaga tgatgatgac 60

<210> 71
<211> 88
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 71
ctatcatgtc gttattaacc gacttctgaa cgtgcggtgg gatcgtgctg gcgatacgcg 60

tccacaggac gatgtgcagc ggccacag 88

<210> 72
<211> 84
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 72

gcagcggcca caggcccctg agcagccccc gacccatggc agaccccgct gctcgtcata      60

gaccgagccc ccagcgcagc ggac                                            84


<210>  73
<211>  72
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  73
cgagcgggtg cacgcgcggg ggtgtcgtcg ctccgtgcgc gcgagtgact cactcaactt      60

caactcagcg ct                                                         72


<210>  74
<211>  159
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  74
ctatcatgtc gttattaacc gacttctgaa cgtgcggtgg gatcgtgctg gcgatacgcg      60

tccacaggac gatgtgcagc ggccacaggc ccctgagcag cccccgaccc atggcagacc     120

ccgctgctcg tcatagaccg agcccccagc gcagcggac                           159


<210>  75
<211>  41
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  75
gaatcttgaa tatctcatga atggaccagt attctagaaa c                         41


<210>  76
<211>  43
<212>  DNA
<213>  Artificial Sequence

<220>
<223>  Antisense oligonucleotide against Homo sapiens transforming

growth factor, beta receptor II (70/80kDa) (TGFBR2), transcript
variant 2, wherein at least 2 nucleotides are LNA units (i.e.
locked nucleotides)

<400> 76
cggtgcaagt ggaatttcta ggcgcctcta tgctactgca gcc 43

<210> 77
<211> 41
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 77
ttcatattta tatacaggca ttaataaagt gcaaatgtta t 41

<210> 78
<211> 42
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 78
tgaggaagtg ctaacacagc ttatcctatg acaatgtcaa ag 42

<210> 79
<211> 49
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 79
gcctgcccca gaagagctat ttggtagtgt ttagggagcc gtcttcagg 49

<210> 80
<211> 88
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400>  80
aatttctggt cgccctcgat ctctcaacac gttgtccttc atgctttcga cacaggggtg      60

ctcccgcacc ttggaaccaa atggaggc                                          88


<210>  81
<211>  49
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  81
cgcaggtcct cccagctgat gacatgccgc gtcaggtact cctgtaggt                   49


<210>  82
<211>  50
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  82
ttgaactcag cttctgctgc cggttaacgc ggtagcagta gaagatgatg                  50


<210>  83
<211>  78
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  83
atgtcgttat taaccgactt ctgaacgtgc ggtgggatcg tgctggcgat acgcgtccac      60

aggacgatgt gcagcggc                                                     78


<210>  84
<211>  74
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.

locked nucleotides)

<400> 84
ggccacaggc ccctgagcag cccccgaccc atggcagacc ccgctgctcg tcatagaccg 60

agcccccagc gcag 74


<210> 85
<211> 62
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 85
gggtgcacgc gcgggggtgt cgtcgctccg tgcgcgcgag tgactcactc aacttcaact 60

ca 62


<210> 86
<211> 149
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 86
atgtcgttat taaccgactt ctgaacgtgc ggtgggatcg tgctggcgat acgcgtccac 60

aggacgatgt gcagcggcca caggcccctg agcagccccc gacccatggc agaccccgct 120

gctcgtcata gaccgagccc ccagcgcag 149


<210> 87
<211> 31
<212> DNA
<213> Artificial Sequence

<220>
<223> Antisense oligonucleotide against Homo sapiens transforming
growth factor, beta receptor II (70/80kDa) (TGFBR2), transcript
variant 2, wherein at least 2 nucleotides are LNA units (i.e.
locked nucleotides)

<400> 87
ttgaatatct catgaatgga ccagtattct a 31


<210> 88
<211> 33
<212> DNA
<213> Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  88
caagtggaat ttctaggcgc ctctatgcta ctg                                    33


<210>  89
<211>  31
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  89
atttatatac aggcattaat aaagtgcaaa t                                      31


<210>  90
<211>  32
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  90
aagtgctaac acagcttatc ctatgacaat gt                                     32


<210>  91
<211>  39
<212>  DNA
<213>  Artificial Sequence

<220>
<223>  Antisense oligonucleotide against Homo sapiens transforming
       growth factor, beta receptor II (70/80kDa) (TGFBR2), transcript
       variant 2, wherein at least 2 nucleotides are LNA units (i.e.
       locked nucleotides)

<400>  91
ccccagaaga gctatttggt agtgtttagg gagccgtct                              39


<210>  92
<211>  78
<212>  DNA
<213>  Artificial Sequence

<220>
<223>  Antisense oligonucleotide against Homo sapiens transforming

growth factor, beta receptor II (70/80kDa) (TGFBR2), transcript
      variant 2, wherein at least 2 nucleotides are LNA units (i.e.
      locked nucleotides)

<400>  92
ctggtcgccc tcgatctctc aacacgttgt ccttcatgct ttcgacacag gggtgctccc      60

gcaccttgga accaaatg                                                    78


<210>  93
<211>  39
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  93
gtcctcccag ctgatgacat gccgcgtcag gtactcctg                             39


<210>  94
<211>  40
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  94
ctcagcttct gctgccggtt aacgcggtag cagtagaaga                            40


<210>  95
<211>  68
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  95
gttattaacc gacttctgaa cgtgcggtgg gatcgtgctg gcgatacgcg tccacaggac      60

gatgtgca                                                               68


<210>  96
<211>  64
<212>  DNA
<213>  Artificial Sequence

<220>

<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  96
caggcccctg agcagccccc gacccatggc agaccccgct gctcgtcata gaccgagccc      60

ccag                                                                    64


<210>  97
<211>  52
<212>  DNA
<213>  Artificial Sequence

<220>
<223>  Antisense oligonucleotide against Homo sapiens transforming
      growth factor, beta receptor II (70/80kDa) (TGFBR2), transcript
      variant 2, wherein at least 2 nucleotides are LNA units (i.e.
      locked nucleotides)

<400>  97
cacgcgcggg ggtgtcgtcg ctccgtgcgc gcgagtgact cactcaactt ca              52


<210>  98
<211>  10
<212>  DNA
<213>  Artificial Sequence

<220>
<223>  General formula


<220>
<221>  misc_feature
<222>  (1)..(1)
<223>  w may represent 5'GGTGGGATCGTGCTGGCGAT'3 or sequences derived
      from 5'GGTGGGATCGTGCTGGCGAT'3, wherein one or more nucleotides
      are eliminated from the 5'-end and wherein w is at least T

<220>
<221>  misc_feature
<222>  (10)..(10)
<223>  r may represent 5'ACAGGACGATGTGCAGCGGC'3 or sequences derived
      from 5'ACAGGACGATGTGCAGCGGC'3, wherein one or more nucleotides
      are eliminated from the 3'-end and wherein w is at least A

<220>
<221>  misc_feature
<222>  (10)..(10)
<223>  r may represent 5'ACAGGACGATGTGCAGCGGC'3 or sequences derived
      from 5'ACAGGACGATGTGCAGCGGC'3, wherein one or more nucleotides
      are eliminated from the 3'-end and wherein r is at least A

<400>  98
wacgcgtccr                                                              10


<210>  99
<211>  10
<212>  DNA

<213>  Artificial Sequence

<220>
<223>  General formula



<220>
<221>  misc_feature
<222>  (1)..(1)
<223>  w may represent 5'CCCAGCCTGCCCCAGAAGAGCTATTTG'3 or sequences
      derived from 5'CCCAGCCTGCCCCAGAAGAGCTATTTG'3, wherein one or more
      nucleotides are eliminated from the 5'-end and wherein w is at
      least G

<220>
<221>  misc_feature
<222>  (10)..(10)
<223>  r may represent 5'TAGGGAGCCGTCTTCAGGAATCTTCTC'3 or sequences
      derived from 5'TAGGGAGCCGTCTTCAGGAATCTTCTC'3, wherein one or more
      nucleotides are eliminated from the 3'-end and wherein w is at
      least G

<220>
<221>  misc_feature
<222>  (10)..(10)
<223>  r may represent 5'TAGGGAGCCGTCTTCAGGAATCTTCTC'3 or sequences
      derived from 5'TAGGGAGCCGTCTTCAGGAATCTTCTC'3, wherein one or more
      nucleotides are eliminated from the 3'-end and wherein r is at
      least G

<400>  99
wgtagtgttr                                                              10


<210>  100
<211>  10
<212>  DNA
<213>  Artificial Sequence

<220>
<223>  General formula



<220>
<221>  misc_feature
<222>  (1)..(1)
<223>  w may represent 5'TGAATCTTGAATATCTCATG'3 or sequences derived
      from 5'TGAATCTTGAATATCTCATG'3, wherein one or more nucleotides
      are eliminated from the 5'-end and wherein w is at least G

<220>
<221>  misc_feature
<222>  (10)..(10)
<223>  r may represent 5'AGTATTCTAGAAACTCACCA'3 or sequences derived
      from 5'AGTATTCTAGAAACTCACCA'3, wherein one or more nucleotides
      are eliminated from the 3'-end and wherein w is at least A

<220>
<221>  misc_feature
<222>  (10)..(10)
<223>  r may represent 5'AGTATTCTAGAAACTCACCA'3 or sequences derived
      from 5'AGTATTCTAGAAACTCACCA'3, wherein one or more nucleotides
      are eliminated from the 3'-end and wherein r is at least A

<400>  100
waatggaccr                                                              10


<210>  101
<211>  10
<212>  DNA
<213>  Artificial Sequence

<220>
<223>  General formula


<220>
<221>  misc_feature
<222>  (1)..(1)
<223>  w may represent 5'ATTCATATTTATATACAGGC'3 or sequences derived
       from 5'ATTCATATTTATATACAGGC'3, wherein one or more nucleotides
       are eliminated from the 5'-end and wherein w is at least C

<220>
<221>  misc_feature
<222>  (10)..(10)
<223>  r may represent 5'AGTGCAAATGTTATTGGCTA'3 or sequences derived
       from 5'AGTGCAAATGTTATTGGCTA'3, wherein one or more nucleotides
       are eliminated from the 3'-end and wherein w is at least A

<220>
<221>  misc_feature
<222>  (10)..(10)
<223>  r may represent 5'AGTGCAAATGTTATTGGCTA'3 or sequences derived
       from 5'AGTGCAAATGTTATTGGCTA'3, wherein one or more nucleotides
       are eliminated from the 3'-end and wherein r is at least A

<400>  101
wattaataar                                                              10


<210>  102
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  102
gcgagtgact cactcaa                                                      17


<210>  103
<211>  15
<212>  DNA
<213>  Artificial Sequence

<220>
<223>  Antisense oligonucleotide against hTGFbRII wherein the first 3
       nucleotides and the last 3 nucleotides are LNA units and wherein

the remaining non LNA units in between have 2-deoxyribose as
sugar and all internucleotide linkages are phosphorothioate

<400> 103
cgagtgactc actca 15

<210> 104
<211> 16
<212> DNA
<213> Artificial Sequence

<220>
<223> Antisense oligonucleotide against hTGFbRII wherein the first 3
nucleotides and the last 3 nucleotides are LNA units and wherein
the remaining non LNA units in between have 2-deoxyribose as
sugar and all internucleotide linkages are phosphorothioate

<400> 104
gcgagtgact cactca 16

<210> 105
<211> 17
<212> DNA
<213> Artificial Sequence

<220>
<223> Antisense oligonucleotide against hTGFbRII wherein the first 4
nucleotides and the last 4 nucleotides are LNA units and wherein
the remaining non LNA units in between have 2-deoxyribose as
sugar and all internucleotide linkages are phosphorothioate

<400> 105
cgcgagtgac tcactca 17

<210> 106
<211> 14
<212> DNA
<213> Artificial Sequence

<220>
<223> Antisense oligonucleotide against hTGFbRII wherein the first 3
nucleotides and the last 3 nucleotides are LNA units and wherein
the remaining non LNA units in between have 2-deoxyribose as
sugar and all internucleotide linkages are phosphorothioate

<400> 106
cgagtgactc actc 14

<210> 107
<211> 16
<212> DNA
<213> Artificial Sequence

<220>
<223> Antisense oligonucleotide against hTGFbRII wherein the first 3

nucleotides and the last 3 nucleotides are LNA units and wherein
the remaining non LNA units in between have 2-deoxyribose as
sugar and all internucleotide linkages are phosphorothioate

<400> 107
cgcgagtgac tcactc 16

<210> 108
<211> 17
<212> DNA
<213> Artificial Sequence

<220>
<223> Antisense oligonucleotide against hTGFbRII wherein the first 4
nucleotides and the last 4 nucleotides are LNA units and wherein
the remaining non LNA units in between have 2-deoxyribose as
sugar and all internucleotide linkages are phosphorothioate

<400> 108
gcgcgagtga ctcactc 17

<210> 109
<211> 14
<212> DNA
<213> Artificial Sequence

<220>
<223> Antisense oligonucleotide against hTGFbRII wherein the first 3
nucleotides and the last 3 nucleotides are LNA units and wherein
the remaining non LNA units in between have 2-deoxyribose as
sugar and all internucleotide linkages are phosphorothioate

<400> 109
gcgagtgact cact 14

<210> 110
<211> 16
<212> DNA
<213> Artificial Sequence

<220>
<223> Antisense oligonucleotide against hTGFbRII wherein the first 3
nucleotides and the last 3 nucleotides are LNA units and wherein
the remaining non LNA units in between have 2-deoxyribose as
sugar and all internucleotide linkages are phosphorothioate

<400> 110
gcgcgagtga ctcact 16

<210> 111
<211> 17
<212> DNA
<213> Artificial Sequence

<220>

<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate

<400>  111
cgcgcgagtg actcact                                                    17

<210>  112
<211>  12
<212>  DNA
<213>  Artificial Sequence

<220>
<223>  Antisense oligonucleotide against hTGFbRII wherein the first 2
      nucleotides and the last 2 nucleotides are LNA units and wherein
      the remaining non LNA units in between have 2-deoxyribose as
      sugar and all internucleotide linkages are phosphorothioate

<400>  112
cgagtgactc ac                                                         12

<210>  113
<211>  13
<212>  DNA
<213>  Artificial Sequence

<220>
<223>  Antisense oligonucleotide against hTGFbRII wherein the first 3
      nucleotides and the last 2 nucleotides are LNA units and wherein
      the remaining non LNA units in between have 2-deoxyribose as
      sugar and all internucleotide linkages are phosphorothioate

<400>  113
gcgagtgact cac                                                        13

<210>  114
<211>  14
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate

<400>  114
cgcgagtgac tcac                                                       14

<210>  115
<211>  16
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate

<400>  115
cgcgcgagtg actcac                                                    16

<210>  116
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate

<400>  116
gcgcgcgagt gactcac                                                   17

<210>  117
<211>  13
<212>  DNA
<213>  Artificial Sequence

<220>
<223>  Antisense oligonucleotide against hTGFbRII wherein the first 3
      nucleotides and the last 2 nucleotides are LNA units and wherein
      the remaining non LNA units in between have 2-deoxyribose as
      sugar and all internucleotide linkages are phosphorothioate

<400>  117
cgcgagtgac tca                                                       13

<210>  118
<211>  14
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate

<400>  118
gcgcgagtga ctca                                                      14

<210>  119
<211>  15
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate


<400>  119
cgcgcgagtg actca                                                        15


<210>  120
<211>  16
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate


<400>  120
gcgcgcgagt gactca                                                       16


<210>  121
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate


<400>  121
tgcgcgcgag tgactca                                                      17


<210>  122
<211>  14
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate


<400>  122
cgcgcgagtg actc                                                         14


<210>  123
<211>  16
<212>  DNA

<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  123
tgcgcgcgag tgactc                                                       16


<210>  124
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  124
gtgcgcgcga gtgactc                                                      17


<210>  125
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  125
cgcgcgagtg act                                                          13


<210>  126
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  126
tgcgcgcgag tgac                                                         14


<210>  127
<211>  16

<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  127
cgtgcgcgcg agtgac                                                       16


<210>  128
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  128
tgcgcgcgag tga                                                          13


<210>  129
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  129
gtcgtcgctc cgtgcg                                                       16


<210>  130
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  130
gtcgtcgctc cgtgc                                                        15


<210>  131

<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  131
gtgtcgtcgc tccgtgc                                                    17


<210>  132
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  132
tcgtcgctcc gtg                                                        13


<210>  133
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  133
tgtcgtcgct ccgtg                                                      15


<210>  134
<211>  12
<212>  DNA
<213>  Artificial Sequence

<220>
<223>  Antisense oligonucleotide against hTGFbRII wherein the first 2
       nucleotides and the last 2 nucleotides are LNA units and wherein
       the remaining non LNA units in between have 2-deoxyribose as
       sugar and all internucleotide linkages are phosphorothioate


<400>  134
tcgtcgctcc gt                                                         12

<210>  135
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  135
gtcgtcgctc cgt                                                          13


<210>  136
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  136
tgtcgtcgct ccgt                                                         14


<210>  137
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  137
gtgtcgtcgc tccgt                                                        15


<210>  138
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  138
ggtgtcgtcg ctccgt                                                       16

<210>  139
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  139
cgtcatagac cgagcc                                                       16


<210>  140
<211>  12
<212>  DNA
<213>  Artificial Sequence

<220>
<223>  Antisense oligonucleotide against hTGFbRII wherein the first 2
       nucleotides and the last 2 nucleotides are LNA units and wherein
       the remaining non LNA units in between have 2-deoxyribose as
       sugar and all internucleotide linkages are phosphorothioate


<400>  140
atagaccgag cc                                                           12


<210>  141
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  141
gctcgtcata gaccga                                                       16


<210>  142
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  142
cgtcatagac cga                                                          13

<210>  143
<211>  14
<212>  DNA
<213>  Artificial Sequence

<220>
<223>  Antisense oligonucleotide against hTGFbRII wherein the first 2
       nucleotides and the last 3 nucleotides are LNA units and wherein
       the remaining non LNA units in between have 2-deoxyribose as
       sugar and all internucleotide linkages are phosphorothioate


<400>  143
ctcgtcatag accg                                                         14


<210>  144
<211>  14
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  144
ctcgtcatag accg                                                         14


<210>  145
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  145
ctcgtcatag accg                                                         14


<210>  146
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  146

gctcgtcata gaccg                                                        15


<210>  147
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  147
gctcgtcata gacc                                                         14


<210>  148
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  148
cagcccccga cccatgg                                                      17


<210>  149
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  149
cagcccccga cccatg                                                       16


<210>  150
<211>  16
<212>  DNA
<213>  Artificial Sequence

<220>
<223>  Antisense oligonucleotide against hTGFbRII wherein the non LNA
       units have 2-deoxyribose as sugar and all internucleotide
       linkages are phosphorothioate groups

<400>  150
cagcccccga cccatg                                                       16

<210>  151
<211>  16
<212>  DNA
<213>  Artificial Sequence

<220>
<223>  Antisense oligonucleotide against hTGFbRII wherein the first 3
       nucleotides and the last 4 nucleotides are LNA units and wherein
       the remaining non LNA units in between have 2-deoxyribose as
       sugar and all internucleotide linkages are phosphorothioate


<400>  151
cagcccccga cccatg                                                       16


<210>  152
<211>  16
<212>  DNA
<213>  Artificial Sequence

<220>
<223>  Antisense oligonucleotide against hTGFbRII wherein the first 4
       nucleotides and the last 3 nucleotides are LNA units and wherein
       the remaining non LNA units in between have 2-deoxyribose as
       sugar and all internucleotide linkages are phosphorothioate


<400>  152
cagcccccga cccatg                                                       16


<210>  153
<211>  16
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  153
cagcccccga cccatg                                                       16


<210>  154
<211>  14
<212>  DNA
<213>  Artificial Sequence

<220>
<223>  Antisense oligonucleotide against hTGFbRII wherein the first 2
       nucleotides and the last 3 nucleotides are LNA units and wherein
       the remaining non LNA units in between have 2-deoxyribose as
       sugar and all internucleotide linkages are phosphorothioate


<400>  154

agcccccgac ccat                                                          14

<210>  155
<211>  14
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate

<400>  155
agcccccgac ccat                                                          14

<210>  156
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate

<400>  156
agcccccgac ccat                                                          14

<210>  157
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate

<400>  157
cagcccccga ccca                                                          14

<210>  158
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate

<400>  158
cgcgtccaca ggacgat                                                    17


<210>  159
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  159
cgcgtccaca ggac                                                       14


<210>  160
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  160
cgatacgcgt ccaca                                                      15


<210>  161
<211>  15
<212>  DNA
<213>  Artificial Sequence

<220>
<223>  Antisense oligonucleotide against hTGFbRII wherein the first 3
       nucleotides and the last 4 nucleotides are LNA units and wherein
       the remaining non LNA units in between have 2-deoxyribose as
       sugar and all internucleotide linkages are phosphorothioate


<400>  161
cgatacgcgt ccaca                                                      15


<210>  162
<211>  15
<212>  DNA
<213>  Artificial Sequence

<220>
<223>  Antisense oligonucleotide against hTGFbRII wherein the first 4
       nucleotides and the last 3 nucleotides are LNA units and wherein
       the remaining non LNA units in between have 2-deoxyribose as
       sugar and all internucleotide linkages are phosphorothioate

<400>  162
cgatacgcgt ccaca                                                        15


<210>  163
<211>  12
<212>  DNA
<213>  Artificial Sequence

<220>
<223>  Antisense oligonucleotide against hTGFbRII wherein the first 2
       nucleotides and the last 2 nucleotides are LNA units and wherein
       the remaining non LNA units in between have 2-deoxyribose as
       sugar and all internucleotide linkages are phosphorothioate


<400>  163
cgatacgcgt cc                                                           12


<210>  164
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  164
cgatacgcgt cca                                                          13


<210>  165
<211>  13
<212>  DNA
<213>  Artificial Sequence

<220>
<223>  Antisense oligonucleotide against hTGFbRII wherein the first 2
       nucleotides and the last 3 nucleotides are LNA units and wherein
       the remaining non LNA units in between have 2-deoxyribose as
       sugar and all internucleotide linkages are phosphorothioate


<400>  165
cgatacgcgt cca                                                          13


<210>  166
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate

<400>  166
tggcgatacg cgtcca                                                        16


<210>  167
<211>  12
<212>  DNA
<213>  Artificial Sequence

<220>
<223>  Antisense oligonucleotide against hTGFbRII wherein the first 2
       nucleotides and the last 1 nucleotide are LNA units and wherein
       the remaining non LNA units in between have 2-deoxyribose as
       sugar and all internucleotide linkages are phosphorothioate


<400>  167
cgatacgcgt cc                                                            12


<210>  168
<211>  12
<212>  DNA
<213>  Artificial Sequence

<220>
<223>  Antisense oligonucleotide against hTGFbRII wherein the first 1
       nucleotide and the last 2 nucleotides are LNA units and wherein
       the remaining non LNA units in between have 2-deoxyribose as
       sugar and all internucleotide linkages are phosphorothioate


<400>  168
cgatacgcgt cc                                                            12


<210>  169
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  169
gcgatacgcg tcc                                                           13


<210>  170
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as

sugar and all internucleotide linkages are phosphorothioate

<400> 170
gctggcgata cgcgtcc 17

<210> 171
<211> 15
<212> DNA
<213> Artificial Sequence

<220>
<223> Antisense oligonucleotide against hTGFbRII wherein the first 3
nucleotides and the last 3 nucleotides are LNA units and wherein
the remaining non LNA units in between have 2-deoxyribose as
sugar and all internucleotide linkages are phosphorothioate

<400> 171
ctggcgatac gcgtc 15

<210> 172
<211> 12
<212> DNA
<213> Artificial Sequence

<220>
<223> Antisense oligonucleotide against hTGFbRII wherein the first 2
nucleotides and the last 2 nucleotides are LNA units and wherein
the remaining non LNA units in between have 2-deoxyribose as
sugar and all internucleotide linkages are phosphorothioate

<400> 172
gcgatacgcg tc 12

<210> 173
<211> 16
<212> DNA
<213> Artificial Sequence

<220>
<223> Antisense oligonucleotide against hTGFbRII wherein the first 3
nucleotides and the last 3 nucleotides are LNA units and wherein
the remaining non LNA units in between have 2-deoxyribose as
sugar and all internucleotide linkages are phosphorothioate

<400> 173
gctggcgata cgcgtc 16

<210> 174
<211> 14
<212> DNA
<213> Artificial Sequence

<220>
<223> Antisense oligonucleotide against hTGFbRII wherein the first 3
nucleotides and the last 3 nucleotides are LNA units and wherein

the remaining non LNA units in between have 2-deoxyribose as
sugar and all internucleotide linkages are phosphorothioate

<400> 174
tggcgatacg cgtc 14

<210> 175
<211> 12
<212> DNA
<213> Artificial Sequence

<220>
<223> Antisense oligonucleotide against hTGFbRII wherein the first 2
nucleotides and the last 2 nucleotides are LNA units and wherein
the remaining non LNA units in between have 2-deoxyribose as
sugar and all internucleotide linkages are phosphorothioate

<400> 175
ggcgatacgc gt 12

<210> 176
<211> 13
<212> DNA
<213> Artificial Sequence

<220>
<223> Antisense oligonucleotide against hTGFbRII wherein the first 3
nucleotides and the last 2 nucleotides are LNA units and wherein
the remaining non LNA units in between have 2-deoxyribose as
sugar and all internucleotide linkages are phosphorothioate

<400> 176
tggcgatacg cgt 13

<210> 177
<211> 13
<212> DNA
<213> Artificial Sequence

<220>
<223> Antisense oligonucleotide against hTGFbRII wherein the first 2
nucleotides and the last 3 nucleotides are LNA units and wherein
the remaining non LNA units in between have 2-deoxyribose as
sugar and all internucleotide linkages are phosphorothioate

<400> 177
tggcgatacg cgt 13

<210> 178
<211> 14
<212> DNA
<213> Artificial Sequence

<220>
<223> Antisense oligonucleotide against hTGFbRII wherein the first 3

nucleotides and the last 3 nucleotides are LNA units and wherein
      the remaining non LNA units in between have 2-deoxyribose as
      sugar and all internucleotide linkages are phosphorothioate

<400>  178
ctggcgatac gcgt                                                         14

<210>  179
<211>  13
<212>  DNA
<213>  Artificial Sequence

<220>
<223>  Antisense oligonucleotide against hTGFbRII wherein the first 3
      nucleotides and the last 2 nucleotides are LNA units and wherein
      the remaining non LNA units in between have 2-deoxyribose as
      sugar and all internucleotide linkages are phosphorothioate

<400>  179
ctggcgatac gcg                                                          13

<210>  180
<211>  12
<212>  DNA
<213>  Artificial Sequence

<220>
<223>  Antisense oligonucleotide against hTGFbRII wherein the first 2
      nucleotides and the last 2 nucleotides are LNA units and wherein
      the remaining non LNA units in between have 2-deoxyribose as
      sugar and all internucleotide linkages are phosphorothioate

<400>  180
tggcgatacg cg                                                           12

<210>  181
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate

<400>  181
atcgtgctgg cgatacg                                                      17

<210>  182
<211>  16
<212>  DNA
<213>  Artificial Sequence

<220>

<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate

<400>  182
cgtgcggtgg gatcgt                                                    16

<210>  183
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate

<400>  183
acgtgcggtg ggatcgt                                                   17

<210>  184
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate

<400>  184
aacgtgcggt gggatcg                                                   17

<210>  185
<211>  15
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate

<400>  185
aacgtgcggt gggat                                                     15

<210>  186
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate


<400>  186
tgaacgtgcg gtgggat                                                    17


<210>  187
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate


<400>  187
cgacttctga acgtgcg                                                    17


<210>  188
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate


<400>  188
ttaacgcggt agcagta                                                    17


<210>  189
<211>  16
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate


<400>  189
taacgcggta gcagta                                                     16


<210>  190
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate


<400>  190
gttaacgcgg tagcagt                                                      17


<210>  191
<211>  15
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate


<400>  191
ttaacgcggt agcag                                                        15


<210>  192
<211>  13
<212>  DNA
<213>  Artificial Sequence

<220>
<223>  Antisense oligonucleotide against hTGFbRII wherein the first 3
      nucleotides and the last 2 nucleotides are LNA units and wherein
      the remaining non LNA units in between have 2-deoxyribose as
      sugar and all internucleotide linkages are phosphorothioate


<400>  192
taacgcggta gca                                                          13


<210>  193
<211>  12
<212>  DNA
<213>  Artificial Sequence

<220>
<223>  Antisense oligonucleotide against hTGFbRII wherein the first 2
      nucleotides and the last 2 nucleotides are LNA units and wherein
      the remaining non LNA units in between have 2-deoxyribose as
      sugar and all internucleotide linkages are phosphorothioate


<400>  193
taacgcggta gc                                                           12


<210>  194
<211>  13
<212>  DNA

<213>  Artificial Sequence

<220>
<223>  Antisense oligonucleotide against hTGFbRII wherein the first 3
      nucleotides and the last 2 nucleotides are LNA units and wherein
      the remaining non LNA units in between have 2-deoxyribose as
      sugar and all internucleotide linkages are phosphorothioate


<400>  194
ttaacgcggt agc                                                          13


<210>  195
<211>  12
<212>  DNA
<213>  Artificial Sequence

<220>
<223>  Antisense oligonucleotide against hTGFbRII wherein the first 2
      nucleotides and the last 2 nucleotides are LNA units and wherein
      the remaining non LNA units in between have 2-deoxyribose as
      sugar and all internucleotide linkages are phosphorothioate


<400>  195
ttaacgcggt ag                                                           12


<210>  196
<211>  13
<212>  DNA
<213>  Artificial Sequence

<220>
<223>  Antisense oligonucleotide against hTGFbRII wherein the first 3
      nucleotides and the last 2 nucleotides are LNA units and wherein
      the remaining non LNA units in between have 2-deoxyribose as
      sugar and all internucleotide linkages are phosphorothioate


<400>  196
gttaacgcgg tag                                                          13


<210>  197
<211>  15
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate


<400>  197
cggttaacgc ggtag                                                        15


<210>  198
<211>  16

<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  198
ccggttaacg cggtag                                                       16


<210>  199
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  199
cggttaacgc ggta                                                         14


<210>  200
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  200
ggttaacgcg gta                                                          13


<210>  201
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  201
ccggttaacg cggta                                                        15


<210>  202

<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  202
gccggttaac gcggta                                                       16


<210>  203
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  203
tgccggttaa cgcggta                                                      17


<210>  204
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  204
cggttaacgc ggt                                                          13


<210>  205
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  205
ccggttaacg cgg                                                          13

<210>  206
<211>  14
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  206
gccggttaac gcgg                                                         14


<210>  207
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  207
tgccggttaa cgcgg                                                        15


<210>  208
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  208
gccggttaac gcg                                                          13


<210>  209
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  209
ctgccggtta acgcg                                                        15

<210>  210
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  210
gctgccggtt aacgcg                                                       16


<210>  211
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  211
atgccgcgtc aggtac                                                       16


<210>  212
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  212
acatgccgcg tca                                                          13


<210>  213
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  213
gatgacatgc cgcgtc                                                       16

<210>  214
<211>  16
<212>  DNA
<213>  Artificial Sequence

<220>
<223>  Antisense oligonucleotide against hTGFbRII wherein the first 3
       nucleotides and the last 4 nucleotides are LNA units and wherein
       the remaining non LNA units in between have 2-deoxyribose as
       sugar and all internucleotide linkages are phosphorothioate


<400>  214
gatgacatgc cgcgtc                                                       16


<210>  215
<211>  16
<212>  DNA
<213>  Artificial Sequence

<220>
<223>  Antisense oligonucleotide against hTGFbRII wherein the first 4
       nucleotides and the last 3 nucleotides are LNA units and wherein
       the remaining non LNA units in between have 2-deoxyribose as
       sugar and all internucleotide linkages are phosphorothioate


<400>  215
gatgacatgc cgcgtc                                                       16


<210>  216
<211>  16
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  216
gatgacatgc cgcgtc                                                       16


<210>  217
<211>  12
<212>  DNA
<213>  Artificial Sequence

<220>
<223>  Antisense oligonucleotide against hTGFbRII wherein the first 2
       nucleotides and the last 2 nucleotides are LNA units and wherein
       the remaining non LNA units in between have 2-deoxyribose as
       sugar and all internucleotide linkages are phosphorothioate


<400>  217

gacatgccgc gt                                                          12

<210>  218
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate

<400>  218
gatgacatgc cgcgt                                                       15

<210>  219
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate

<400>  219
atgacatgcc gcg                                                         13

<210>  220
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate

<400>  220
tcccgcacct tggaacc                                                     17

<210>  221
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate

<400>  221
cgatctctca acacgt                                                        16


<210>  222
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  222
tcgatctctc aacacgt                                                       17


<210>  223
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  223
cgatctctca acacg                                                         15


<210>  224
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  224
tcgatctctc aacacg                                                        16


<210>  225
<211>  17
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate

<400>  225
ctcgatctct caacacg                                                    17


<210>  226
<211>  16
<212>  DNA
<213>  Artificial Sequence

<220>
<223>  Antisense oligonucleotide against hTGFbRII wherein the first 2
       nucleotides and the last 2 nucleotides are LNA units and wherein
       the remaining non LNA units in between have 2-deoxyribose as
       sugar and all internucleotide linkages are phosphorothioate


<400>  226
gtagtgttta gggagc                                                     16


<210>  227
<211>  16
<212>  DNA
<213>  Artificial Sequence

<220>
<223>  Antisense oligonucleotide against hTGFbRII wherein the first 4
       nucleotides and the last 3 nucleotides are LNA units and wherein
       the remaining non LNA units in between have 2-deoxyribose as
       sugar and all internucleotide linkages are phosphorothioate


<400>  227
gtagtgttta gggagc                                                     16


<210>  228
<211>  16
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  228
gtagtgttta gggagc                                                     16


<210>  229
<211>  16
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate

<400>  229
gtagtgttta gggagc                                                        16


<210>  230
<211>  16
<212>  DNA
<213>  Artificial Sequence

<220>
<223>  Antisense oligonucleotide against hTGFbRII wherein the first 3
       nucleotides and the last 4 nucleotides are LNA units and wherein
       the remaining non LNA units in between have 2-deoxyribose as
       sugar and all internucleotide linkages are phosphorothioate


<400>  230
gtagtgttta gggagc                                                        16


<210>  231
<211>  16
<212>  DNA
<213>  Artificial Sequence

<220>
<223>  Antisense oligonucleotide against hTGFbRII wherein the first 4
       nucleotides and the last 4 nucleotides are LNA units and wherein
       the remaining non LNA units in between have 2-deoxyribose as
       sugar and all internucleotide linkages are phosphorothioate


<400>  231
gtagtgttta gggagc                                                        16


<210>  232
<211>  16
<212>  DNA
<213>  Artificial Sequence

<220>
<223>  Antisense oligonucleotide against hTGFbRII wherein the first 2
       nucleotides and the last 3 nucleotides are LNA units and wherein
       the remaining non LNA units in between have 2-deoxyribose as
       sugar and all internucleotide linkages are phosphorothioate


<400>  232
gtagtgttta gggagc                                                        16


<210>  233
<211>  16
<212>  DNA
<213>  Artificial Sequence

<220>
<223>  Antisense oligonucleotide against hTGFbRII wherein the first 5
       nucleotides and the last 4 nucleotides are LNA units and wherein
       the remaining non LNA units in between have 2-deoxyribose as

sugar and all internucleotide linkages are phosphorothioate

<400> 233
gtagtgttta gggagc 16

<210> 234
<211> 16
<212> DNA
<213> Artificial Sequence

<220>
<223> Antisense oligonucleotide against hTGFbRII wherein the first 4
nucleotides and the last 5 nucleotides are LNA units and wherein
the remaining non LNA units in between have 2-deoxyribose as
sugar and all internucleotide linkages are phosphorothioate

<400> 234
gtagtgttta gggagc 16

<210> 235
<211> 16
<212> DNA
<213> Artificial Sequence

<220>
<223> Antisense oligonucleotide against hTGFbRII wherein the first 5
nucleotides and the last 5 nucleotides are LNA units and wherein
the remaining non LNA units in between have 2-deoxyribose as
sugar and all internucleotide linkages are phosphorothioate

<400> 235
gtagtgttta gggagc 16

<210> 236
<211> 16
<212> DNA
<213> Artificial Sequence

<220>
<223> Antisense oligonucleotide against hTGFbRII wherein the first 2
nucleotides and the last 3 nucleotides are LNA units and wherein
the remaining non LNA units in between have 2-deoxyribose as
sugar and all internucleotide linkages are phosphorothioate

<400> 236
gctatttggt agtgtt 16

<210> 237
<211> 16
<212> DNA
<213> Artificial Sequence

<220>
<223> Antisense oligonucleotide against hTGFbRII wherein the first 3
nucleotides and the last 2 nucleotides are LNA units and wherein

the remaining non LNA units in between have 2-deoxyribose as
sugar and all internucleotide linkages are phosphorothioate

<400> 237
gctatttggt agtgtt 16

<210> 238
<211> 16
<212> DNA
<213> Artificial Sequence

<220>
<223> Antisense oligonucleotide against hTGFbRII wherein the first 3
nucleotides and the last 3 nucleotides are LNA units and wherein
the remaining non LNA units in between have 2-deoxyribose as
sugar and all internucleotide linkages are phosphorothioate

<400> 238
gctatttggt agtgtt 16

<210> 239
<211> 16
<212> DNA
<213> Artificial Sequence

<220>
<223> Antisense oligonucleotide against hTGFbRII wherein the first 4
nucleotides and the last 3 nucleotides are LNA units and wherein
the remaining non LNA units in between have 2-deoxyribose as
sugar and all internucleotide linkages are phosphorothioate

<400> 239
gctatttggt agtgtt 16

<210> 240
<211> 16
<212> DNA
<213> Artificial Sequence

<220>
<223> Antisense oligonucleotide against hTGFbRII wherein the first 3
nucleotides and the last 4 nucleotides are LNA units and wherein
the remaining non LNA units in between have 2-deoxyribose as
sugar and all internucleotide linkages are phosphorothioate

<400> 240
gctatttggt agtgtt 16

<210> 241
<211> 16
<212> DNA
<213> Artificial Sequence

<220>
<223> Antisense oligonucleotide against hTGFbRII wherein the first 4

nucleotides and the last 4 nucleotides are LNA units and wherein
the remaining non LNA units in between have 2-deoxyribose as
sugar and all internucleotide linkages are phosphorothioate

<400> 241
gctatttggt agtgtt 16

<210> 242
<211> 15
<212> DNA
<213> Artificial Sequence

<220>
<223> Antisense oligonucleotide against hTGFbRII wherein the first 3
nucleotides and the last 3 nucleotides are LNA units and wherein
the remaining non LNA units in between have 2-deoxyribose as
sugar and all internucleotide linkages are phosphorothioate

<400> 242
agcttatcct atgac 15

<210> 243
<211> 15
<212> DNA
<213> Artificial Sequence

<220>
<223> Antisense oligonucleotide against hTGFbRII wherein the first 3
nucleotides and the last 4 nucleotides are LNA units and wherein
the remaining non LNA units in between have 2-deoxyribose as
sugar and all internucleotide linkages are phosphorothioate

<400> 243
agcttatcct atgac 15

<210> 244
<211> 15
<212> DNA
<213> Artificial Sequence

<220>
<223> Antisense oligonucleotide against hTGFbRII wherein the first 4
nucleotides and the last 3 nucleotides are LNA units and wherein
the remaining non LNA units in between have 2-deoxyribose as
sugar and all internucleotide linkages are phosphorothioate

<400> 244
agcttatcct atgac 15

<210> 245
<211> 14
<212> DNA
<213> Artificial Sequence

<220>

<223>  Antisense oligonucleotide against hTGFbRII wherein the first 2
      nucleotides and the last 3 nucleotides are LNA units and wherein
      the remaining non LNA units in between have 2-deoxyribose as
      sugar and all internucleotide linkages are phosphorothioate

<400>  245
agcttatcct atga                                                    14

<210>  246
<211>  14
<212>  DNA
<213>  Artificial Sequence

<220>
<223>  Antisense oligonucleotide against hTGFbRII wherein the first 3
      nucleotides and the last 2 nucleotides are LNA units and wherein
      the remaining non LNA units in between have 2-deoxyribose as
      sugar and all internucleotide linkages are phosphorothioate

<400>  246
agcttatcct atga                                                    14

<210>  247
<211>  14
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate

<400>  247
agcttatcct atga                                                    14

<210>  248
<211>  17
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate

<400>  248
caggcattaa taaagtg                                                 17

<210>  249
<211>  17
<212>  DNA
<213>  Artificial Sequence

<220>
<223>  Antisense oligonucleotide against hTGFbRII wherein the first 4
      nucleotides and the last 3 nucleotides are LNA units and wherein
      the remaining non LNA units in between have 2-deoxyribose as
      sugar and all internucleotide linkages are phosphorothioate


<400>  249
caggcattaa taaagtg                                                    17


<210>  250
<211>  17
<212>  DNA
<213>  Artificial Sequence

<220>
<223>  Antisense oligonucleotide against hTGFbRII wherein the first 3
      nucleotides and the last 4 nucleotides are LNA units and wherein
      the remaining non LNA units in between have 2-deoxyribose as
      sugar and all internucleotide linkages are phosphorothioate


<400>  250
caggcattaa taaagtg                                                    17


<210>  251
<211>  17
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate


<400>  251
caggcattaa taaagtg                                                    17


<210>  252
<211>  16
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate


<400>  252
catgaatgga ccagta                                                     16


<210>  253
<211>  16
<212>  DNA
<213>  Artificial Sequence

<220>
<223>  Antisense oligonucleotide against hTGFbRII wherein the first 3
      nucleotides and the last 4 nucleotides are LNA units and wherein
      the remaining non LNA units in between have 2-deoxyribose as
      sugar and all internucleotide linkages are phosphorothioate


<400>  253
catgaatgga ccagta                                                       16


<210>  254
<211>  16
<212>  DNA
<213>  Artificial Sequence

<220>
<223>  Antisense oligonucleotide against hTGFbRII wherein the first 4
      nucleotides and the last 3 nucleotides are LNA units and wherein
      the remaining non LNA units in between have 2-deoxyribose as
      sugar and all internucleotide linkages are phosphorothioate


<400>  254
catgaatgga ccagta                                                       16


<210>  255
<211>  16
<212>  DNA
<213>  Artificial Sequence

<220>
<223>  Antisense oligonucleotide against hTGFbRII wherein the first 4
      nucleotides and the last 4 nucleotides are LNA units and wherein
      the remaining non LNA units in between have 2-deoxyribose as
      sugar and all internucleotide linkages are phosphorothioate


<400>  255
catgaatgga ccagta                                                       16


<210>  256
<211>  16
<212>  DNA
<213>  Artificial Sequence

<220>
<223>  Antisense oligonucleotide against hTGFbRII wherein the first 3
      nucleotides and the last 3 nucleotides are LNA units and wherein
      the remaining non LNA units in between have 2-deoxyribose as
      sugar and all internucleotide linkages are phosphorothioate


<400>  256
ctaggcgcct ctatgc                                                       16


<210>  257
<211>  14
<212>  DNA

<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  257
taggcgcctc tatg                                                         14


<210>  258
<211>  15
<212>  DNA
<213>  Artificial Sequence

<220>
<223>  Antisense oligonucleotide against hTGFbRII wherein the first 3
       nucleotides and the last 3 nucleotides are LNA units and wherein
       the remaining non LNA units in between have 2-deoxyribose as
       sugar and all internucleotide linkages are phosphorothioate


<400>  258
ctaggcgcct ctatg                                                        15


<210>  259
<211>  13
<212>  DNA
<213>  Artificial Sequence

<220>
<223>  Antisense oligonucleotide against hTGFbRII wherein the first 3
       nucleotides and the last 2 nucleotides are LNA units and wherein
       the remaining non LNA units in between have 2-deoxyribose as
       sugar and all internucleotide linkages are phosphorothioate


<400>  259
taggcgcctc tat                                                          13


<210>  260
<211>  16
<212>  DNA
<213>  Artificial Sequence

<220>
<223>  Antisense oligonucleotide against hTGFbRII wherein the first 4
       nts & the last 3 nts are LNA units; wherein the remaining non LNA
       units in between have 2-deoxyribose as sugar & all nt-linkages
       are phosphorothioate groups; all cytosine residues are methyl-C

<400>  260
gtagtgttta gggagc                                                       16


<210>  261
<211>  16
<212>  DNA

<213>  Artificial Sequence

<220>
<223>  Antisense oligonucleotide against hTGFbRII wherein the first 3
       nts & the last 3 nts are LNA units; wherein the remaining non LNA
       units in between have 2-deoxyribose as sugar & all nt-linkages
       are phosphorothioate groups; all cytosine residues are methyl-C

<400>  261
gtagtgttta gggagc                                                    16


<210>  262
<211>  16
<212>  DNA
<213>  Artificial Sequence

<220>
<223>  Antisense oligonucleotide against hTGFbRII wherein the first 3
       nts & the last 2 nts are LNA units; wherein the remaining non LNA
       units in between have 2-deoxyribose as sugar & all nt-linkages
       are phosphorothioate groups; all cytosine residues are methyl-C

<400>  262
gtagtgttta gggagc                                                    16


<210>  263
<211>  16
<212>  DNA
<213>  Artificial Sequence

<220>
<223>  Antisense oligonucleotide against hTGFbRII wherein the first 3
       nts & the last 4 nts are LNA units; wherein the remaining non LNA
       units in between have 2-deoxyribose as sugar & all nt-linkages
       are phosphorothioate groups; all cytosine residues are methyl-C

<400>  263
gtagtgttta gggagc                                                    16


<210>  264
<211>  16
<212>  DNA
<213>  Artificial Sequence

<220>
<223>  Antisense oligonucleotide against hTGFbRII wherein the first 4
       nts & the last 4 nts are LNA units; wherein the remaining non LNA
       units in between have 2-deoxyribose as sugar & all nt-linkages
       are phosphorothioate groups; all cytosine residues are methyl-C

<400>  264
gtagtgttta gggagc                                                    16


<210>  265
<211>  16
<212>  DNA
<213>  Artificial Sequence

<220>

<223>  Antisense oligonucleotide against hTGFbRII wherein the first 2
      nts & the last 3 nts are LNA units; wherein the remaining non LNA
      units in between have 2-deoxyribose as sugar & all nt-linkages
      are phosphorothioate groups; all cytosine residues are methyl-C

<400>  265
gtagtgttta gggagc                                                    16


<210>  266
<211>  15
<212>  DNA
<213>  Artificial Sequence

<220>
<223>  Antisense oligonucleotide against hTGFbRII wherein the first 4
      nts & the last 3 nts are LNA units; wherein the remaining non LNA
      units in between have 2-deoxyribose as sugar & all nt-linkages
      are phosphorothioate groups; all cytosine residues are methyl-C

<400>  266
cgatacgcgt ccaca                                                     15


<210>  267
<211>  16
<212>  DNA
<213>  Artificial Sequence

<220>
<223>  Antisense oligonucleotide against hTGFbRII wherein the first 3
      nts & the last 4 nts are LNA units; wherein the remaining non LNA
      units in between have 2-deoxyribose as sugar & all nt-linkages
      are phosphorothioate groups; all cytosine residues are methyl-C

<400>  267
catgaatgga ccagta                                                    16


<210>  268
<211>  17
<212>  DNA
<213>  Artificial Sequence

<220>
<223>  Antisense oligonucleotide against hTGFbRII wherein the first 3
      nts & the last 4 nts are LNA units; wherein the remaining non LNA
      units in between have 2-deoxyribose as sugar & all nt-linkages
      are phosphorothioate groups; all cytosine residues are methyl-C

<400>  268
caggcattaa taaagtg                                                   17


<210>  269
<211>  17
<212>  DNA
<213>  Artificial Sequence

<220>
<223>  Antisense oligonucleotide against hTGFbRII wherein the first 3
      nts & the last 3 nts are LNA units; wherein the remaining non LNA
      units in between have 2-deoxyribose as sugar & all nt-linkages

are phosphorothioate groups; all cytosine residues are methyl-C

<400> 269
caggcattaa taaagtg 17

<210> 270
<211> 12
<212> DNA
<213> Artificial Sequence

<220>
<223> Antisense oligonucleotide against hTGFbRII wherein the first 2
nts & the last 2 nts are LNA units; wherein the remaining non LNA
units in between have 2-deoxyribose as sugar & all nt-linkages
are phosphorothioate groups; all cytosine residues are methyl-C

<400> 270
cgatacgcgt cc 12

<210> 271
<211> 16
<212> DNA
<213> Artificial Sequence

<220>
<223> Antisense oligonucleotide against hTGFbRII wherein the first 4
nts & the last 4 nts are LNA units; wherein the remaining non LNA
units in between have 2-deoxyribose as sugar & all nt-linkages
are phosphorothioate groups; all cytosine residues are methyl-C

<400> 271
catgaatgga ccagta 16

<210> 272
<211> 16
<212> DNA
<213> Artificial Sequence

<220>
<223> Antisense oligonucleotide against hTGFbRII wherein the first 4
nts & the last 3 nts are LNA units; wherein the remaining non LNA
units in between have 2-deoxyribose as sugar & all nt-linkages
are phosphorothioate groups; all cytosine residues are methyl-C

<400> 272
gctatttggt agtgtt 16

<210> 273
<211> 16
<212> DNA
<213> Artificial Sequence

<220>
<223> Antisense oligonucleotide against hTGFbRII wherein the first 2
nts & the last 3 nts are LNA units; wherein the remaining non LNA
units in between have 2-deoxyribose as sugar & all nt-linkages
are phosphorothioate groups; all cytosine residues are methyl-C

<400> 273

gctatttggt agtgtt                                                    16

<210>  274
<211>  12
<212>  DNA
<213>  Artificial Sequence

<220>
<223>  Antisense oligonucleotide against hTGFbRII wherein the first 2
       nts & the last 1 nt are LNA units; wherein the remaining non LNA
       units in between have 2-deoxyribose as sugar & all nt-linkages
       are phosphorothioate groups; all cytosine residues are methyl-C

<400>  274
cgatacgcgt cc                                                        12

<210>  275
<211>  16
<212>  DNA
<213>  Artificial Sequence

<220>
<223>  Antisense oligonucleotide against hTGFbRII wherein the first 3
       nts & the last 2 nts are LNA units; wherein the remaining non LNA
       units in between have 2-deoxyribose as sugar & all nt-linkages
       are phosphorothioate groups; all cytosine residues are methyl-C

<400>  275
gctatttggt agtgtt                                                    16

<210>  276
<211>  12
<212>  DNA
<213>  Artificial Sequence

<220>
<223>  Antisense oligonucleotide against hTGFbRII wherein the first 1 nt
       & the last 2 nts are LNA units; wherein the remaining non LNA
       units in between have 2-deoxyribose as sugar & all nt-linkages
       are phosphorothioate groups; all cytosine residues are methyl-C

<400>  276
cgatacgcgt cc                                                        12

<210>  277
<211>  13
<212>  DNA
<213>  Artificial Sequence

<220>
<223>  Antisense oligonucleotide against hTGFbRII wherein the first 3
       nts & the last 2 nts are LNA units; wherein the remaining non LNA
       units in between have 2-deoxyribose as sugar & all nt-linkages
       are phosphorothioate groups; all cytosine residues are methyl-C

<400>  277
cgatacgcgt cca                                                       13

<210>  278
<211>  15
<212>  DNA
<213>  Artificial Sequence

<220>
<223>  Antisense oligonucleotide against hTGFbRII wherein the first 3
       nts & the last 3 nts are LNA units; wherein the remaining non LNA
       units in between have 2-deoxyribose as sugar & all nt-linkages
       are phosphorothioate groups; all cytosine residues are methyl-C

<400>  278
cgatacgcgt ccaca                                                        15


<210>  279
<211>  16
<212>  DNA
<213>  Artificial Sequence

<220>
<223>  Antisense oligonucleotide against hTGFbRII wherein the first 4
       nts & the last 3 nts are LNA units; wherein the remaining non LNA
       units in between have 2-deoxyribose as sugar & all nt-linkages
       are phosphorothioate groups; all cytosine residues are methyl-C

<400>  279
catgaatgga ccagta                                                       16


<210>  280
<211>  16
<212>  DNA
<213>  Artificial Sequence

<220>
<223>  Antisense oligonucleotide against hTGFbRII wherein the first 3
       nts & the last 3 nts are LNA units; wherein the remaining non LNA
       units in between have 2-deoxyribose as sugar & all nt-linkages
       are phosphorothioate groups; all cytosine residues are methyl-C

<400>  280
gatgacatgc cgcgtc                                                       16


<210>  281
<211>  14
<212>  DNA
<213>  Artificial Sequence

<220>
<223>  Antisense oligonucleotide against hTGFbRII wherein the first 3
       nts & the last 2 nts are LNA units; wherein the remaining non LNA
       units in between have 2-deoxyribose as sugar & all nt-linkages
       are phosphorothioate groups; all cytosine residues are methyl-C

<400>  281
agcttatcct atga                                                         14


<210>  282
<211>  14
<212>  DNA

<213>  Artificial Sequence

<220>
<223>  Antisense oligonucleotide against hTGFbRII wherein the first 2
       nts & the last 3 nts are LNA units; wherein the remaining non LNA
       units in between have 2-deoxyribose as sugar & all nt-linkages
       are phosphorothioate groups; all cytosine residues are methyl-C

<400>  282
ctcgtcatag accg                                                          14


<210>  283
<211>  16
<212>  DNA
<213>  Artificial Sequence

<220>
<223>  Antisense oligonucleotide against hTGFbRII wherein the first 3
       nts & the last 3 nts are LNA units; wherein the remaining non LNA
       units in between have 2-deoxyribose as sugar & all nt-linkages
       are phosphorothioate groups; all cytosine residues are methyl-C

<400>  283
gctatttggt agtgtt                                                        16


<210>  284
<211>  16
<212>  DNA
<213>  Artificial Sequence

<220>
<223>  Antisense oligonucleotide against hTGFbRII wherein the first 3
       nts & the last 4 nts are LNA units; wherein the remaining non LNA
       units in between have 2-deoxyribose as sugar & all nt-linkages
       are phosphorothioate groups; all cytosine residues are methyl-C

<400>  284
gatgacatgc cgcgtc                                                        16


<210>  285
<211>  17
<212>  DNA
<213>  Artificial Sequence

<220>
<223>  Antisense oligonucleotide against hTGFbRII wherein the first 4
       nts & the last 4 nts are LNA units; wherein the remaining non LNA
       units in between have 2-deoxyribose as sugar & all nt-linkages
       are phosphorothioate groups; all cytosine residues are methyl-C

<400>  285
caggcattaa taaagtg                                                       17


<210>  286
<211>  15
<212>  DNA
<213>  Artificial Sequence

<220>

<223>  Antisense oligonucleotide against hTGFbRII wherein the first 3
      nts & the last 4 nts are LNA units; wherein the remaining non LNA
      units in between have 2-deoxyribose as sugar & all nt-linkages
      are phosphorothioate groups; all cytosine residues are methyl-C

<400>  286
cgatacgcgt ccaca                                                        15


<210>  287
<211>  14
<212>  DNA
<213>  Artificial Sequence

<220>
<223>  Antisense oligonucleotide against hTGFbRII wherein the first 3
      nts & the last 3 nts are LNA units; wherein the remaining non LNA
      units in between have 2-deoxyribose as sugar & all nt-linkages
      are phosphorothioate groups; all cytosine residues are methyl-C

<400>  287
agcttatcct atga                                                         14


<210>  288
<211>  14
<212>  DNA
<213>  Artificial Sequence

<220>
<223>  Antisense oligonucleotide against hTGFbRII wherein the first 3
      nts & the last 3 nts are LNA units; wherein the remaining non LNA
      units in between have 2-deoxyribose as sugar & all nt-linkages
      are phosphorothioate groups; all cytosine residues are methyl-C

<400>  288
ctcgtcatag accg                                                         14


<210>  289
<211>  16
<212>  DNA
<213>  Artificial Sequence

<220>
<223>  Antisense oligonucleotide against hTGFbRII wherein the first 4
      nts & the last 4 nts are LNA units; wherein the remaining non LNA
      units in between have 2-deoxyribose as sugar & all nt-linkages
      are phosphorothioate groups; all cytosine residues are methyl-C

<400>  289
gatgacatgc cgcgtc                                                       16


<210>  290
<211>  16
<212>  DNA
<213>  Artificial Sequence

<220>
<223>  Antisense oligonucleotide against hTGFbRII wherein the first 4
      nts & the last 3 nts are LNA units; wherein the remaining non LNA
      units in between have 2-deoxyribose as sugar & all nt-linkages

are phosphorothioate groups; all cytosine residues are methyl-C

<400>  290
gatgacatgc cgcgtc                                                    16


<210>  291
<211>  16
<212>  DNA
<213>  Artificial Sequence

<220>
<223>  Antisense oligonucleotide against hTGFbRII wherein the first 3
       nts & the last 3 nts are LNA units; wherein the remaining non LNA
       units in between have 2-deoxyribose as sugar & all nt-linkages
       are phosphorothioate groups; all cytosine residues are methyl-C

<400>  291
catgaatgga ccagta                                                    16


<210>  292
<211>  14
<212>  DNA
<213>  Artificial Sequence

<220>
<223>  Antisense oligonucleotide against hTGFbRII wherein the first 2
       nts & the last 3 nts are LNA units; wherein the remaining non LNA
       units in between have 2-deoxyribose as sugar & all nt-linkages
       are phosphorothioate groups; all cytosine residues are methyl-C

<400>  292
agcttatcct atga                                                      14


<210>  293
<211>  13
<212>  DNA
<213>  Artificial Sequence

<220>
<223>  Antisense oligonucleotide against hTGFbRII wherein the first 3
       nts & the last 2 nts are LNA units; wherein the remaining non LNA
       units in between have 2-deoxyribose as sugar & all nt-linkages
       are phosphorothioate groups; all cytosine residues are methyl-C

<400>  293
tggcgatacg cgt                                                       13


<210>  294
<211>  14
<212>  DNA
<213>  Artificial Sequence

<220>
<223>  Antisense oligonucleotide against hTGFbRII wherein the first 3
       nts & the last 2 nts are LNA units; wherein the remaining non LNA
       units in between have 2-deoxyribose as sugar & all nt-linkages
       are phosphorothioate groups; all cytosine residues are methyl-C

<400>  294

agcccccgac ccat                                                    14

<210>  295
<211>  13
<212>  DNA
<213>  Artificial Sequence

<220>
<223>  Antisense oligonucleotide against hTGFbRII wherein the first 2
       nts & the last 3 nts are LNA units; wherein the remaining non LNA
       units in between have 2-deoxyribose as sugar & all nt-linkages
       are phosphorothioate groups; all cytosine residues are methyl-C

<400>  295
cgatacgcgt cca                                                     13

<210>  296
<211>  15
<212>  DNA
<213>  Artificial Sequence

<220>
<223>  Antisense oligonucleotide against hTGFbRII wherein the first 3
       nts & the last 3 nts are LNA units; wherein the remaining non LNA
       units in between have 2-deoxyribose as sugar & all nt-linkages
       are phosphorothioate groups; all cytosine residues are methyl-C

<400>  296
agcttatcct atgac                                                   15

<210>  297
<211>  14
<212>  DNA
<213>  Artificial Sequence

<220>
<223>  Antisense oligonucleotide against hTGFbRII wherein the first 3
       nts & the last 2 nts are LNA units; wherein the remaining non LNA
       units in between have 2-deoxyribose as sugar & all nt-linkages
       are phosphorothioate groups; all cytosine residues are methyl-C

<400>  297
ctcgtcatag accg                                                    14

<210>  298
<211>  15
<212>  DNA
<213>  Artificial Sequence

<220>
<223>  Antisense oligonucleotide against hTGFbRII wherein the first 3
       nts & the last 4 nts are LNA units; wherein the remaining non LNA
       units in between have 2-deoxyribose as sugar & all nt-linkages
       are phosphorothioate groups; all cytosine residues are methyl-C

<400>  298
agcttatcct atgac                                                   15

<210>  299
<211>  14
<212>  DNA
<213>  Artificial Sequence

<220>
<223>  Antisense oligonucleotide against hTGFbRII wherein the first 3
       nts & the last 3 nts are LNA units; wherein the remaining non LNA
       units in between have 2-deoxyribose as sugar & all nt-linkages
       are phosphorothioate groups; all cytosine residues are methyl-C

<400>  299
agcccccgac ccat                                                    14


<210>  300
<211>  14
<212>  DNA
<213>  Artificial Sequence

<220>
<223>  Antisense oligonucleotide against hTGFbRII wherein the first 2
       nts & the last 3 nts are LNA units; wherein the remaining non LNA
       units in between have 2-deoxyribose as sugar & all nt-linkages
       are phosphorothioate groups; all cytosine residues are methyl-C

<400>  300
agcccccgac ccat                                                    14


<210>  301
<211>  16
<212>  DNA
<213>  Artificial Sequence

<220>
<223>  Antisense oligonucleotide against hTGFbRII wherein the first 3
       nts & the last 3 nts are LNA units; wherein the remaining non LNA
       units in between have 2-deoxyribose as sugar & all nt-linkages
       are phosphorothioate groups; all cytosine residues are methyl-C

<400>  301
cagcccccga cccatg                                                  16


<210>  302
<211>  16
<212>  DNA
<213>  Artificial Sequence

<220>
<223>  Antisense oligonucleotide against hTGFbRII wherein the first 3
       nts & the last 4 nts are LNA units; wherein the remaining non LNA
       units in between have 2-deoxyribose as sugar & all nt-linkages
       are phosphorothioate groups; all cytosine residues are methyl-C

<400>  302
gctatttggt agtgtt                                                  16


<210>  303
<211>  16
<212>  DNA

<213>  Artificial Sequence

<220>
<223>  Antisense oligonucleotide against hTGFbRII wherein the first 4
       nts & the last 4 nts are LNA units; wherein the remaining non LNA
       units in between have 2-deoxyribose as sugar & all nt-linkages
       are phosphorothioate groups; all cytosine residues are methyl-C

<400>  303
gctatttggt agtgtt                                                    16


<210>  304
<211>  13
<212>  DNA
<213>  Artificial Sequence

<220>
<223>  Antisense oligonucleotide against hTGFbRII wherein the first 2
       nts & the last 3 nts are LNA units; wherein the remaining non LNA
       units in between have 2-deoxyribose as sugar & all nt-linkages
       are phosphorothioate groups; all cytosine residues are methyl-C

<400>  304
tggcgatacg cgt                                                       13


<210>  305
<211>  15
<212>  DNA
<213>  Artificial Sequence

<220>
<223>  Antisense oligonucleotide against hTGFbRII wherein the first 4
       nts & the last 3 nts are LNA units; wherein the remaining non LNA
       units in between have 2-deoxyribose as sugar & all nt-linkages
       are phosphorothioate groups; all cytosine residues are methyl-C

<400>  305
agcttatcct atgac                                                     15


<210>  306
<211>  16
<212>  DNA
<213>  Artificial Sequence

<220>
<223>  Antisense oligonucleotide against hTGFbRII wherein the first 3
       nts & the last 4 nts are LNA units; wherein the remaining non LNA
       units in between have 2-deoxyribose as sugar & all nt-linkages
       are phosphorothioate groups; all cytosine residues are methyl-C

<400>  306
cagcccccga cccatg                                                    16


<210>  307
<211>  16
<212>  DNA
<213>  Artificial Sequence

<220>

<223>  Antisense oligonucleotide against hTGFbRII wherein the first 4
      nts & the last 3 nts are LNA units; wherein the remaining non LNA
      units in between have 2-deoxyribose as sugar & all nt-linkages
      are phosphorothioate groups; all cytosine residues are methyl-C

<400>  307
cagcccccga cccatg                                                    16


<210>  308
<211>  16
<212>  DNA
<213>  Artificial Sequence

<220>
<223>  Antisense oligonucleotide against hTGFbRII wherein the non LNA
      units have 2-deoxyribose as sugar & all nt-linkages are
      phosphorothioate groups; all cytosine residues are methyl-C

<400>  308
cagcccccga cccatg                                                    16


<210>  309
<211>  17
<212>  DNA
<213>  Artificial Sequence

<220>
<223>  Antisense oligonucleotide against hTGFbRII wherein the first 4
      nts & the last 3 nts are LNA units; wherein the remaining non LNA
      units in between have 2-deoxyribose as sugar & all nt-linkages
      are phosphorothioate groups; all cytosine residues are methyl-C

<400>  309
caggcattaa taaagtg                                                   17


<210>  310
<211>  16
<212>  DNA
<213>  Artificial Sequence

<220>
<223>  Antisense oligonucleotide against hTGFbRII wherein the first 4
      nts & the last 4 nts are LNA units; wherein the remaining non LNA
      units in between have 2-deoxyribose as sugar & all nt-linkages
      are phosphorothioate groups; all cytosine residues are methyl-C

<400>  310
cagcccccga cccatg                                                    16


<210>  311
<211>  17
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  311
gcgagtgact cactcaa                                                    17


<210>  312
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  312
cgagtgactc actca                                                      15


<210>  313
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  313
gcgagtgact cactca                                                     16


<210>  314
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  314
cgcgagtgac tcactca                                                    17


<210>  315
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  315
cgagtgactc actc                                                       14

<210>  316
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  316
cgcgagtgac tcactc                                                       16


<210>  317
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  317
gcgcgagtga ctcactc                                                      17


<210>  318
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  318
gcgagtgact cact                                                         14


<210>  319
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  319
gcgcgagtga ctcact                                                       16


<210>  320

<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  320
cgcgcgagtg actcact                                                    17


<210>  321
<211>  12
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  321
cgagtgactc ac                                                         12


<210>  322
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  322
gcgagtgact cac                                                        13


<210>  323
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  323
cgcgagtgac tcac                                                       14


<210>  324
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  324
cgcgcgagtg actcac                                                    16


<210>  325
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  325
gcgcgcgagt gactcac                                                   17


<210>  326
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  326
cgcgagtgac tca                                                       13


<210>  327
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  327
gcgcgagtga ctca                                                      14


<210>  328
<211>  15
<212>  DNA
<213>  Artificial Sequence

<220>
<223>  Antisense oligonucleotide against hTGFbRII which consists of

nucleotides, preferably DNA nucleotides, which are non-LNA units
      (also named herein non-LNA nucleotides) as well as LNA units
      (also named herein LNA nucleotides)

<400>  328
cgcgcgagtg actca                                                        15


<210>  329
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  329
gcgcgcgagt gactca                                                       16


<210>  330
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  330
tgcgcgcgag tgactca                                                      17


<210>  331
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  331
cgcgcgagtg actc                                                         14


<210>  332
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  332
tgcgcgcgag tgactc                                                        16


<210>  333
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  333
gtgcgcgcga gtgactc                                                       17


<210>  334
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  334
cgcgcgagtg act                                                           13


<210>  335
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  335
tgcgcgcgag tgac                                                          14


<210>  336
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  336
cgtgcgcgcg agtgac                                                        16

<210>  337
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  337
tgcgcgcgag tga                                                          13


<210>  338
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  338
gtcgtcgctc cgtgcg                                                       16


<210>  339
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  339
gtcgtcgctc cgtgc                                                        15


<210>  340
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  340
gtgtcgtcgc tccgtgc                                                      17


<210>  341

<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  341
tcgtcgctcc gtg                                                          13


<210>  342
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  342
tgtcgtcgct ccgtg                                                        15


<210>  343
<211>  12
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  343
tcgtcgctcc gt                                                           12


<210>  344
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  344
gtcgtcgctc cgt                                                          13


<210>  345
<211>  14
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  345
tgtcgtcgct ccgt                                                    14


<210>  346
<211>  15
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  346
gtgtcgtcgc tccgt                                                   15


<210>  347
<211>  16
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  347
ggtgtcgtcg ctccgt                                                  16


<210>  348
<211>  16
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  348
cgtcatagac cgagcc                                                  16


<210>  349
<211>  12
<212>  DNA
<213>  Artificial Sequence

<220>
<223>  Antisense oligonucleotide against hTGFbRII which consists of

nucleotides, preferably DNA nucleotides, which are non-LNA units
      (also named herein non-LNA nucleotides) as well as LNA units
      (also named herein LNA nucleotides)

<400>  349
atagaccgag cc                                                          12


<210>  350
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  350
gctcgtcata gaccga                                                      16


<210>  351
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  351
cgtcatagac cga                                                         13


<210>  352
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  352
ctcgtcatag accg                                                        14


<210>  353
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  353
gctcgtcata gaccg                                                        15


<210>  354
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  354
gctcgtcata gacc                                                         14


<210>  355
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  355
cagcccccga cccatgg                                                      17


<210>  356
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  356
cagcccccga cccatg                                                       16


<210>  357
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  357
agcccccgac ccat                                                         14

<210>  358
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  358
cagcccccga ccca                                                          14


<210>  359
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  359
cgcgtccaca ggacgat                                                       17


<210>  360
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  360
cgcgtccaca ggac                                                          14


<210>  361
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  361
cgatacgcgt ccaca                                                         15


<210>  362

<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  362
cgatacgcgt cca                                                          13


<210>  363
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  363
tggcgatacg cgtcca                                                       16


<210>  364
<211>  12
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  364
cgatacgcgt cc                                                           12


<210>  365
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  365
gcgatacgcg tcc                                                          13


<210>  366
<211>  17
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  366
gctggcgata cgcgtcc                                                    17


<210>  367
<211>  15
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  367
ctggcgatac gcgtc                                                      15


<210>  368
<211>  12
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  368
gcgatacgcg tc                                                         12


<210>  369
<211>  16
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  369
gctggcgata cgcgtc                                                     16


<210>  370
<211>  14
<212>  DNA
<213>  Artificial Sequence

<220>
<223>  Antisense oligonucleotide against hTGFbRII which consists of

nucleotides, preferably DNA nucleotides, which are non-LNA units
      (also named herein non-LNA nucleotides) as well as LNA units
      (also named herein LNA nucleotides)

<400>  370
tggcgatacg cgtc                                                          14


<210>  371
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  371
tggcgatacg cgt                                                           13


<210>  372
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  372
ctggcgatac gcgt                                                          14


<210>  373
<211>  12
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  373
ggcgatacgc gt                                                            12


<210>  374
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  374
ctggcgatac gcg                                                          13


<210>  375
<211>  12
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  375
tggcgatacg cg                                                           12


<210>  376
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  376
atcgtgctgg cgatacg                                                      17


<210>  377
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  377
cgtgcggtgg gatcgt                                                       16


<210>  378
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  378
acgtgcggtg ggatcgt                                                      17

<210>  379
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  379
aacgtgcggt gggatcg                                                    17


<210>  380
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  380
aacgtgcggt gggat                                                      15


<210>  381
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  381
tgaacgtgcg gtgggat                                                    17


<210>  382
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  382
cgacttctga acgtgcg                                                    17


<210>  383

<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  383
ttaacgcggt agcagta                                                    17


<210>  384
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  384
taacgcggta gcagta                                                     16


<210>  385
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  385
gttaacgcgg tagcagt                                                    17


<210>  386
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  386
ttaacgcggt agcag                                                      15


<210>  387
<211>  13
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  387
taacgcggta gca                                                          13


<210>  388
<211>  12
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  388
taacgcggta gc                                                           12


<210>  389
<211>  13
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  389
ttaacgcggt agc                                                          13


<210>  390
<211>  12
<212>  DNA
<213>  Artificial Sequence

<220>
<223>  Antisense oligonucleotide against hTGFbRII which consists of
      nucleotides, preferably DNA nucleotides, which are non-LNA units
      (also named herein non-LNA nucleotides) as well as LNA units
      (also named herein LNA nucleotides)

<400>  390
ttaacgcggt ag                                                           12


<210>  391
<211>  13
<212>  DNA
<213>  Artificial Sequence

<220>
<223>  Antisense oligonucleotide against hTGFbRII which consists of

nucleotides, preferably DNA nucleotides, which are non-LNA units
      (also named herein non-LNA nucleotides) as well as LNA units
      (also named herein LNA nucleotides)

<400>  391
gttaacgcgg tag                                                          13


<210>  392
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  392
cggttaacgc ggtag                                                        15


<210>  393
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  393
ccggttaacg cggtag                                                       16


<210>  394
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  394
cggttaacgc ggta                                                         14


<210>  395
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  395
ggttaacgcg gta                                                          13


<210>  396
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  396
ccggttaacg cggta                                                        15


<210>  397
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  397
gccggttaac gcggta                                                       16


<210>  398
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  398
tgccggttaa cgcggta                                                      17


<210>  399
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  399
cggttaacgc ggt                                                          13

<210>  400
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  400
ccggttaacg cgg                                                          13


<210>  401
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  401
gccggttaac gcgg                                                         14


<210>  402
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  402
tgccggttaa cgcgg                                                        15


<210>  403
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  403
gccggttaac gcg                                                          13


<210>  404

<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  404
ctgccggtta acgcg                                                        15


<210>  405
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  405
gctgccggtt aacgcg                                                       16


<210>  406
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  406
atgccgcgtc aggtac                                                       16


<210>  407
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  407
acatgccgcg tca                                                          13


<210>  408
<211>  16
<212>  DNA
<213>  Artificial Sequence

<220>
<223>  Antisense oligonucleotide against hTGFbRII which consists of
      nucleotides, preferably DNA nucleotides, which are non-LNA units
       (also named herein non-LNA nucleotides) as well as LNA units
       (also named herein LNA nucleotides)

<400>  408
gatgacatgc cgcgtc                                                       16


<210>  409
<211>  12
<212>  DNA
<213>  Artificial Sequence

<220>
<223>  Antisense oligonucleotide against hTGFbRII which consists of
      nucleotides, preferably DNA nucleotides, which are non-LNA units
       (also named herein non-LNA nucleotides) as well as LNA units
       (also named herein LNA nucleotides)

<400>  409
gacatgccgc gt                                                           12


<210>  410
<211>  15
<212>  DNA
<213>  Artificial Sequence

<220>
<223>  Antisense oligonucleotide against hTGFbRII which consists of
      nucleotides, preferably DNA nucleotides, which are non-LNA units
       (also named herein non-LNA nucleotides) as well as LNA units
       (also named herein LNA nucleotides)

<400>  410
gatgacatgc cgcgt                                                        15


<210>  411
<211>  13
<212>  DNA
<213>  Artificial Sequence

<220>
<223>  Antisense oligonucleotide against hTGFbRII which consists of
      nucleotides, preferably DNA nucleotides, which are non-LNA units
       (also named herein non-LNA nucleotides) as well as LNA units
       (also named herein LNA nucleotides)

<400>  411
atgacatgcc gcg                                                          13


<210>  412
<211>  17
<212>  DNA
<213>  Artificial Sequence

<220>
<223>  Antisense oligonucleotide against hTGFbRII which consists of

nucleotides, preferably DNA nucleotides, which are non-LNA units
      (also named herein non-LNA nucleotides) as well as LNA units
      (also named herein LNA nucleotides)

<400>  412
tcccgcacct tggaacc                                                    17


<210>  413
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  413
cgatctctca acacgt                                                     16


<210>  414
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  414
tcgatctctc aacacgt                                                    17


<210>  415
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  415
cgatctctca acacg                                                      15


<210>  416
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  416
tcgatctctc aacacg                                                        16


<210>  417
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  417
ctcgatctct caacacg                                                       17


<210>  418
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  418
gtagtgttta gggagc                                                        16


<210>  419
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  419
gctatttggt agtgtt                                                        16


<210>  420
<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  420
agcttatcct atgac                                                         15

<210>  421
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  421
agcttatcct atga                                                         14


<210>  422
<211>  17
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  422
caggcattaa taaagtg                                                      17


<210>  423
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  423
ctaggcgcct ctatgc                                                       16


<210>  424
<211>  14
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  424
taggcgcctc tatg                                                         14


<210>  425

<211>  15
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  425
ctaggcgcct ctatg                                                        15


<210>  426
<211>  13
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  426
taggcgcctc tat                                                          13


<210>  427
<211>  16
<212>  DNA
<213>  Artificial Sequence

<220>
<223>  Antisense oligonucleotide against hTGFbRII which consists of
       nucleotides, preferably DNA nucleotides, which are non-LNA units
       (also named herein non-LNA nucleotides) as well as LNA units
       (also named herein LNA nucleotides)

<400>  427
catgaatgga ccagta                                                       16


<210>  428
<211>  10
<212>  DNA
<213>  Artificial Sequence

<220>
<223>  General formula


<220>
<221>  misc_feature
<222>  (1)..(1)
<223>  w may represent 5'CATGGCAGACCCCGCTGCTC'3 or sequences derived
       from 5'CATGGCAGACCCCGCTGCTC'3, wherein one or more nucleotides
       are eliminated from the 5'-end and wherein w is at least C

<220>
<221>  misc_feature
<222>  (10)..(10)

<223>  r may represent 5′CCGAGCCCCCAGCGCAGCGG′3 or sequences derived
      from 5′CCGAGCCCCCAGCGCAGCGG′3, wherein one or more nucleotides
      are eliminated from the 3′-end and wherein r is at least C

<400>  428
wgtcatagar                                                              10

## Claims

1. Antisense-oligonucleotide having a length of at least 8 nucleotides, wherein at least two of the nucleotides are LNAs and the sequence of the antisense-oligonucleotide of the at least 8 nucleotides is selected from the group of sequences of at least 8 nucleotides contained in a sequence selected from the following group:
GAATACTCTTGAATCTTGAATATCTCATGAATGGACCAGTATTCTAGAAACTCACCACTAG
(Seq. ID No. 4),
GCATGCCCTACGGTGCAAGTGGAATTTCTAGGCGCCTCTATGCTACTGCAGCCACACTGTCTT
(Seq. ID No. 5),
AACATAGCTATTCATATTTATATACAGGCATTAATAAAGTGCAAATGTTATTGGCTATTGT
(Seq. ID No. 6),
TCTCATTTCCTGAGGAAGTGCTAACACAGCTTATCCTATGACAATGTCAAAGGCATAGAATG
(Seq. ID No. 7),
ACATGGCCCAGCCTGCCCCAGAAGAGCTATTTGGTAGTGTTTAGGGAGCCGTCTTCAGGAATCTTCTCC
(Seq. ID No. 8), 
AGAAGCTGGGAATTTCTGGTCGCCCTCGATCTCTCAACACGTTGTCCTTCATGCTTTCGACACAG
GGGTGCTCCCGCACCTTGGAACCAAATGGAGGCTCATAATCTT (Seq. ID No. 9),
GCCCAGCTTGCGCAGGTCCTCCCAGCTGATGACATGCCGCGTCAGGTACTCCTGTAGGTTGCCCTTGGC
(Seq. ID No. 10),
GTTTCCCAGGTTGAACTCAGCTTCTGCTGCCGGTTAACGCGGTAGCAGTAGAAGATGATGATGACAGATA
(Seq. ID No. 11), 
AGTGACTATCATGTCGTTATTAACCGACTTCTGAACGTGCGGTGGGATCGTGCTGGCGATACGCG
TCCACAGGACGATGTGCAGCGGCCACAGGCCCCTGAGCAGCCCCCGACCCATGGCAGACCCCGCT
GCTCGTCATAGACCGAGCCCCCAGCGCAGCGGACGGCGC (Seq. ID No. 12),
 
TGTCCCGAGCGGGTGCACGCGCGGGGGTGTCGTCGCTCCGTGCGCGCGAGTGACTCACTCAACTT
CAACTCAGCGCTGCGGG   (Seq. ID No. 15),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

2. Antisense-oligonucleotide according to claim 1 having a length of 10 to 28 nucleotides, wherein at least two of the nucleotides are LNAs and the sequence of the antisense-oligonucleotide of the 10 to 28 nucleotides is selected from the group of sequences of 10 to 28 nucleotides contained in a sequence selected from the following group:
GAATACTCTTGAATCTTGAATATCTCATGAATGGACCAGTATTCTAGAAACTCACCACTAG
(Seq. ID No. 4),
GCATGCCCTACGGTGCAAGTGGAATTTCTAGGCGCCTCTATGCTACTGCAGCCACACTGTCTT
(Seq. ID No. 5),
AACATAGCTATTCATATTTATATACAGGCATTAATAAAGTGCAAATGTTATTGGCTATTGT
(Seq. ID No. 6),
TCTCATTTCCTGAGGAAGTGCTAACACAGCTTATCCTATGACAATGTCAAAGGCATAGAATG
(Seq. ID No. 7),
ACATGGCCCAGCCTGCCCCAGAAGAGCTATTTGGTAGTGTTTAGGGAGCCGTCTTCAGGAATCTTCTCC
(Seq. ID No. 8), 
AGAAGCTGGGAATTTCTGGTCGCCCTCGATCTCTCAACACGTTGTCCTTCATGCTTTCGACACAG
GGGTGCTCCCGCACCTTGGAACCAAATGGAGGCTCATAATCTT (Seq. ID No. 9),
GCCCAGCTTGCGCAGGTCCTCCCAGCTGATGACATGCCGCGTCAGGTACTCCTGTAGGTTGCCCTTGGC
(Seq. ID No. 10),
GTTTCCCAGGTTGAACTCAGCTTCTGCTGCCGGTTAACGCGGTAGCAGTAGAAGATGATGATGACAGATA
(Seq. ID No. 11), 
AGTGACTATCATGTCGTTATTAACCGACTTCTGAACGTGCGGTGGGATCGTGCTGGCGATACGCG
TCCACAGGACGATGTGCAGCGGCCACAGGCCCCTGAGCAGCCCCCGACCCATGGCAGACCCCGCT
GCTCGTCATAGACCGAGCCCCCAGCGCAGCGGACGGCGC (Seq. ID No. 12),
 
TGTCCCGAGCGGGTGCACGCGCGGGGGTGTCGTCGCTCCGTGCGCGCGAGTGACTCACTCAACTT
CAACTCAGCGCTGCGGG   (Seq. ID No. 15),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

3. Antisense-oligonucleotide according to claim 1 or 2 having a length of 10 to 26 nucleotides, wherein at least two of the nucleotides are LNAs and the sequence of the antisense-oligonucleotide of the 10 to 26 nucleotides is selected from the group of sequences of 10 to 26 nucleotides contained in a sequence selected from the following group:
CTCTTGAATCTTGAATATCTCATGAATGGACCAGTATTCTAGAAACTCACC
(Seq. ID No. 63),
CCCTACGGTGCAAGTGGAATTTCTAGGCGCCTCTATGCTACTGCAGCCACACT
(Seq. ID No. 64),
AGCTATTCATATTTATATACAGGCATTAATAAAGTGCAAATGTTATTGGCT
(Seq. ID No. 65),
TTTCCTGAGGAAGTGCTAACACAGCTTATCCTATGACAATGTCAAAGGCATA
(Seq. ID No. 66),
GCCCAGCCTGCCCCAGAAGAGCTATTTGGTAGTGTTTAGGGAGCCGTCTTCAGGAATCT
(Seq. ID No. 67), 
CTGGGAATTTCTGGTCGCCCTCGATCTCTCAACACGTTGTCCTTCATGCTTTCGACACAGGGGTG
CTCCCGCACCTTGGAACCAAATGGAGGCTCATA   (Seq. ID No. 68),
GCTTGCGCAGGTCCTCCCAGCTGATGACATGCCGCGTCAGGTACTCCTGTAGGTTGCCC
(Seq. ID No. 69),
CCAGGTTGAACTCAGCTTCTGCTGCCGGTTAACGCGGTAGCAGTAGAAGATGATGATGAC
(Seq. ID No. 70), 
CTATCATGTCGTTATTAACCGACTTCTGAACGTGCGGTGGGATCGTGCTGGCGATACGCGTCCAC
AGGACGATGTGCAGCGGCCACAG   (Seq. ID No. 71),
 
GCAGCGGCCACAGGCCCCTGAGCAGCCCCCGACCCATGGCAGACCCCGCTGCTCGTCATAGACCG
AGCCCCCAGCGCAGCGGAC   (Seq. ID No. 72),
 
CGAGCGGGTGCACGCGCGGGGGTGTCGTCGCTCCGTGCGCGCGAGTGACTCACTCAACTTCA
ACTCAGCGCT   (Seq. ID No. 73),
wherein the antisense-oligonucleotide is capable of hybridizing with the open reading frame of the gene encoding TGF-R_{II} or with a region of the mRNA encoding TGF-R_{II} and salts and optical isomers of said antisense-oligonucleotide.

4. Antisense-oligonucleotide according to claim 1, 2 or 3, wherein the last 1 to 4 nucleotides at the 5' terminal end are LNA nucleotides and the last 1 to 4 nucleotides at the 3' terminal end are LNA nucleotides and between the LNA nucleotides at the 5' terminal end and the LNA nucleotides at the 3' terminal end at least 4 consecutive nucleotides are present which are not LNA nucleotides.

5. Antisense-oligonucleotide according to any one of the claims 1 - 4, wherein the LNA nucleotides are linked to each other through a phosphorothioate group or a phosphorodithioate group or wherein all nucleotides are linked to each other through a phosphorothioate group or a phosphorodithioate group.

6. Antisense-oligonucleotide according to any one of the claims 1 - 5, wherein the LNA nucleotides are selected from the following group: 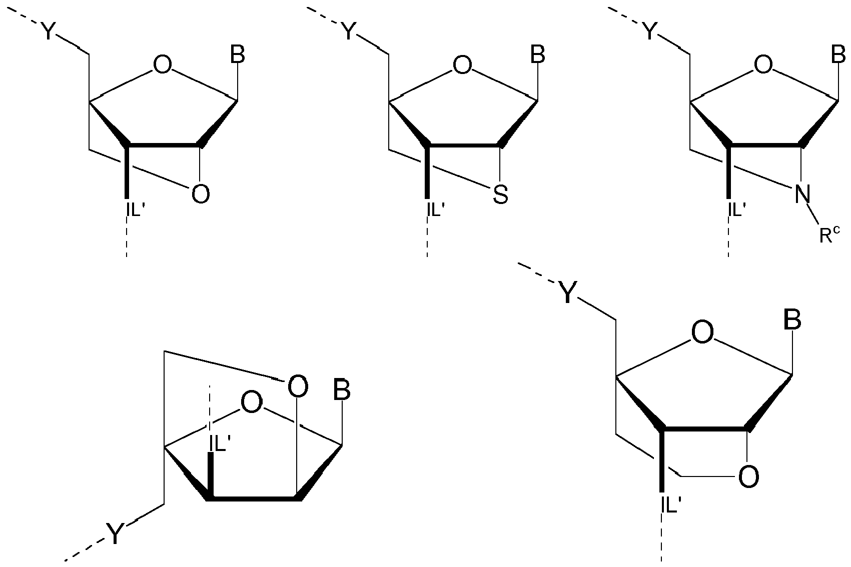 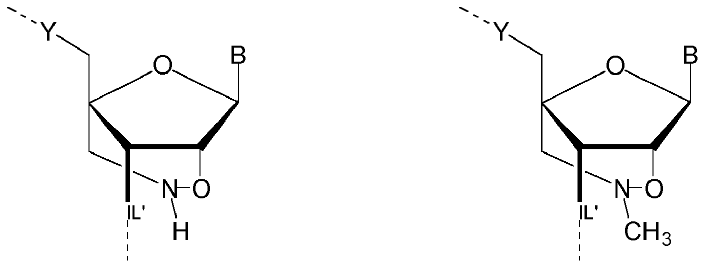 wherein
IL' represents -X"-P(=X')(X⁻)-;
X' represents =O or =S;
X⁻ represents -O⁻, -OH, -OR^{H}, -NHR^{H}, -N(R^{H})₂, -OCH₂CH₂OR^{H}, -OCH₂CH₂SR^{H}, -BH₃⁻, -R^{H}, -SH, -SR^{H}, or -S⁻;
X" represents -O-, -NH-, -NR^{H}- -CH₂-, or -S-;
Y is -O-, -NH-, -NR^{H}- -CH₂- or -S-;
R^{C} and R^{H} are independently of each other selected from hydrogen and C₁₋₄alkyl.
B represents a nucleobase selected from the following group:
adenine, thymine, guanine, cytosine, uracile, 5-methylcytosine, 5-methyldeoxycytosine, 5-hydroxymethyl cytosine, xanthine, hypoxanthine, 2-aminoadenine, 6-methyladenine, 6-methylguanine, 6-ethyladenine, 6-ethylguanine, 2-propyladenine, 2-propylguanine, 6-carboxyuracile, N⁶-methyl-adenine, 5-halouracil, 5-halocytosine, 5-propynyl uracil, 5-propynyl cytosine, 6-azo uracil, 6-azo cytosine, 6-azo thymine, 5-uracil, 4-thiouracil, 8-halo-, 8-amino-, 8-thiol-, 8-thioalkyl-, 8-hydroxyl-adenines and -guanines, 5-halo-, 5-trifluoromethyl-uracils and -cytosines, 7-methylguanine, 7-methyladenine, 8-azaguanine, 8-azaadenine, 7-deazaguanine, 7-deazaadenine, 3-deazaguanine, 3-deazaadenine, 2-thiouracil, 2-thiothymine, and 2-thiocytosine.

7. Antisense-oligonucleotide according to any one of the claims 1 - 6, 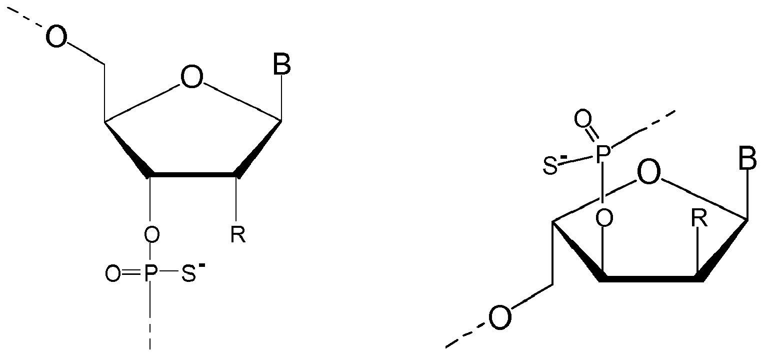 wherein
B represents a nucleobase as defined in claim 6;
R represents -H, -F, -OH, -NH₂, -N(CH₃)₂, -OCH₃, -OCH₂CH₂OCH₃, -OCH₂CH₂CH₂OH, -OCH₂CH₂CH₂NH₂.

8. Antisense-oligonucleotide according to any one of the claims 1 - 7, wherein the antisense-oligonucleotides bind with 100% complementarity specific to the gene encoding TGF-R_{II} or to the mRNA encoding TGF-R_{II} and do not bind to any other region in the complete human transcriptome.

9. Antisense-oligonucleotide selected from the following group:
| **Seq ID No.** | **Sequence, 5'-3'** |
|---|---|
| 311 | GCGAGTGACTCACTCAA |
| 312 | CGAGTGACTCACTCA |
| 313 | GCGAGTGACTCACTCA |
| 314 | CGCGAGTGACTCACTCA |
| 315 | CGAGTGACTCACTC |
| 316 | CGCGAGTGACTCACTC |
| 317 | GCGCGAGTGACTCACTC |
| 318 | GCGAGTGACTCACT |
| 319 | GCGCGAGTGACTCACT |
| 320 | CGCGCGAGTGACTCACT |
| 321 | CGAGTGACTCAC |
| 322 | GCGAGTGACTCAC |
| 323 | CGCGAGTGACTCAC |
| 324 | CGCGCGAGTGACTCAC |
| 325 | GCGCGCGAGTGACTCAC |
| 326 | CGCGAGTGACTCA |
| 327 | GCGCGAGTGACTCA |
| 328 | CGCGCGAGTGACTCA |
| 329 | GCGCGCGAGTGACTCA |
| 330 | TGCGCGCGAGTGACTCA |
| 331 | CGCGCGAGTGACTC |
| 332 | TGCGCGCGAGTGACTC |
| 333 | GTGCGCGCGAGTGACTC |
| 334 | CGCGCGAGTGACT |
| 335 | TGCGCGCGAGTGAC |
| 336 | CGTGCGCGCGAGTGAC |
| 337 | TGCGCGCGAGTGA |
| 338 | GTCGTCGCTCCGTGCG |
| 339 | GTCGTCGCTCCGTGC |
| 340 | GTGTCGTCGCTCCGTGC |
| 341 | TCGTCGCTCCGTG |
| 342 | TGTCGTCGCTCCGTG |
| 343 | TCGTCGCTCCGT |
| 344 | GTCGTCGCTCCGT |
| 345 | TGTCGTCGCTCCGT |
| 346 | GTGTCGTCGCTCCGT |
| 347 | GGTGTCGTCGCTCCGT |
| 348 | CGTCATAGACCGAGCC |
| 349 | ATAGACCGAGCC |
| 350 | GCTCGTCATAGACCGA |
| 351 | CGTCATAGACCGA |
| 352 | CTCGTCATAGACCG |
| 353 | GCTCGTCATAGACCG |
| 354 | GCTCGTCATAGACC |
| 355 | CAGCCCCCGACCCATGG |
| 356 | CAGCCCCCGACCCATG |
| 357 | AGCCCCCGACCCAT |
| 358 | CAGCCCCCGACCCA |
| 359 | CGCGTCCACAGGACGAT |
| 360 | CGCGTCCACAGGAC |
| 361 | CGATACGCGTCCACA |
| 362 | CGATACGCGTCCA |
| 363 | TGGCGATACGCGTCCA |
| 364 | CGATACGCGTCC |
| 365 | GCGATACGCGTCC |
| 366 | GCTGGCGATACGCGTCC |
| 367 | CTGGCGATACGCGTC |
| 368 | GCGATACGCGTC |
| 369 | GCTGGCGATACGCGTC |
| 370 | TGGCGATACGCGTC |
| 371 | TGGCGATACGCGT |
| 372 | CTGGCGATACGCGT |
| 373 | GGCGATACGCGT |
| 374 | CTGGCGATACGCG |
| 375 | TGGCGATACGCG |
| 376 | ATCGTGCTGGCGATACG |
| 377 | CGTGCGGTGGGATCGT |
| 378 | ACGTGCGGTGGGATCGT |
| 379 | AACGTGCGGTGGGATCG |
| 380 | AACGTGCGGTGGGAT |
| 381 | TGAACGTGCGGTGGGAT |
| 382 | CGACTTCTGAACGTGCG |
| 383 | TTAACGCGGTAGCAGTA |
| 384 | TAACGCGGTAGCAGTA |
| 385 | GTTAACGCGGTAGCAGT |
| 386 | TTAACGCGGTAGCAG |
| 387 | TAACGCGGTAGCA |
| 388 | TAACGCGGTAGC |
| 389 | TTAACGCGGTAGC |
| 390 | TTAACGCGGTAG |
| 391 | GTTAACGCGGTAG |
| 392 | CGGTTAACGCGGTAG |
| 393 | CCGGTTAACGCGGTAG |
| 394 | CGGTTAACGCGGTA |
| 395 | GGTTAACGCGGTA |
| 396 | CCGGTTAACGCGGTA |
| 397 | GCCGGTTAACGCGGTA |
| 398 | TGCCGGTTAACGCGGTA |
| 399 | CGGTTAACGCGGT |
| 400 | CCGGTTAACGCGG |
| 401 | GCCGGTTAACGCGG |
| 402 | TGCCGGTTAACGCGG |
| 403 | GCCGGTTAACGCG |
| 404 | CTGCCGGTTAACGCG |
| 405 | GCTGCCGGTTAACGCG |
| 406 | ATGCCGCGTCAGGTAC |
| 407 | ACATGCCGCGTCA |
| 408 | GATGACATGCCGCGTC |
| 409 | GACATGCCGCGT |
| 410 | GATGACATGCCGCGT |
| 411 | ATGACATGCCGCG |
| 412 | TCCCGCACCTTGGAACC |
| 413 | CGATCTCTCAACACGT |
| 414 | TCGATCTCTCAACACGT |
| 415 | CGATCTCTCAACACG |
| 416 | TCGATCTCTCAACACG |
| 417 | CTCGATCTCTCAACACG |
| 418 | GTAGTGTTTAGGGAGC |
| 419 | GCTATTTGGTAGTGTT |
| 420 | AGCTTATCCTATGAC |
| 421 | AGCTTATCCTATGA |
| 422 | CAGGCATTAATAAAGTG |
| 423 | CTAGGCGCCTCTATGC |
| 424 | TAGGCGCCTCTATG |
| 425 | CTAGGCGCCTCTATG |
| 426 | TAGGCGCCTCTAT |
| 427 | CATGAATGGACCAGTA |
wherein
the last 1 - 5 nucleotides at the 3' terminal end and also the last 1 - 5 nucleotides at the 5' terminal end are LNA nucleotides while in between the 1 - 5 LNA nucleotides at the 3' end and at the 5' end 4 - 10 non-LNA nucleotides are present.

10. Antisense-oligonucleotide according to any one of the claims 1 - 9 for promoting regeneration and functional reconnection of damaged neural pathways and/or for treatment and compensation of age induced decreases in neuronal stem cell renewal.

11. Antisense-oligonucleotide according to any one of the claims 1 - 9 for use in the prophylaxis and treatment of neurodegenerative diseases, neuroinflammatory disorders, traumatic or posttraumatic disorders, neurovascular disorders, hypoxic disorders, postinfectious central nervous system disorders, fibrotic diseases, hyperproliferative diseases, cancer, tumors, presbyakusis and presbyopie.

12. Use according to claim 11, wherein the neurodegenerative diseases and neuroinflammatory disorders are selected from the group consisting of:
Alzheimer's disease, Parkinson's disease, Creutzfeldt Jakob disease, new variant of Creutzfeldt Jakobs disease, Hallervorden Spatz disease, Huntington's disease, multisystem atrophy, dementia, frontotemporal dementia, motor neuron disorders, amyotrophic lateral sclerosis, spinal muscular atrophy, spinocerebellar atrophies, schizophrenia, affective disorders, major depression, meningoencephalitis, bacterial meningoencephalitis, viral meningoencephalitis, CNS autoimmune disorders, multiple sclerosis, acute ischemic / hypoxic lesions, stroke, CNS and spinal cord trauma, head and spinal trauma, brain traumatic injuries, arteriosclerosis, atherosclerosis, microangiopathic dementia, Binswanger' disease, retinal degeneration, cochlear degeneration, macular degeneration, cochlear deafness, AIDS-related dementia, retinitis pigmentosa, fragile X-associated tremor/ataxia syndrome, progressive supranuclear palsy, striatonigral degeneration, olivopontocerebellear degeneration, Shy Drager syndrome, age dependant memory deficits, neurodevelopmental disorders associated with dementia, Down's Syndrome, synucleinopathies, superoxide dismutase mutations, trinucleotide repeat disorders, trauma, hypoxia, vascular diseases, vascular inflammations, and CNS-ageing and wherein the fibrotic diseases are selected from the group consisting of: pulmonary fibrosis, cystic fibrosis, hepatic cirrhosis, endomyocardial fibrosis, old myocardial infarction, atrial fibrosis, mediastinal fibrosis, myelofibrosis, retroperitoneal fibrosis, progressive massive fibrosis, nephrogenic systemic fibrosis, Crohn's Disease, keloid, systemic sclerosis, arthrofibrosis, Peyronie's disease, Dupuytren's contracture, and residuums after Lupus erythematodes.

13. Pharmaceutical composition containing at least one antisense-oligonucleotide according to any one of claims 1 - 9 together with at least one pharmaceutically acceptable carrier, excipient, adjuvant, solvent or diluent.

14. Pharmaceutical preparation according to claim 13, wherein the pharmaceutical preparation is an infusion solution or a solid matrix for continuous release of the active ingredient.

15. Pharmaceutical preparation according to claim 13 or 14, wherein the pharmaceutical preparation is suitable for local administration into the brain.
